# EUROPEAN PATENT SPECIFICATION

(11) **EP 3 458 468 B1**
(45) Date of publication and mention of the grant of the patent: **09.12.2020**
(21) Application number: 17725744.1
(22) Date of filing: 18.05.2017
(51) Int. Cl.: C07J 9/00, C07J 41/00, C07J 71/00, C07J 13/00, C07J 21/00, C07J 17/00, C07J 31/00, C07J 43/00, C07J 51/00

(54) **STEROID 6.7.BETA.-EPOXIDES AS CHEMICAL INTERMEDIATES**
STEROIDE 6.7.BETA.-EPOXIDE ALS CHEMISCHE ZWISCHENPRODUKTE
BÊTA-ÉPOXYDES DE STÉROÏDES 6.7 COMME INTERMÉDIAIRES CHIMIQUES

(30) Priority: 18.05.2016 GB 201608779
(43) Date of publication of application: 27.03.2019
(73) Proprietor: NZP UK Limited, Bristol, BS2 0ZX (GB)
(72) Inventor: WEYMOUTH-WILSON, Alexander Charles, Reading Berkshire RG6 6BZ (GB); KOMSTA, Zofia, Reading Berkshire RG6 6BZ (GB); WALLIS, Laura, Reading Berkshire RG6 6BZ (GB); DAVIES, Ieuan, Reading Berkshire RG6 6BZ (GB); WANG, Jingjing, Reading Berkshire RG6 6BZ (GB)
(74) Representative: O'Kane, Jessica Ann
(86) International application number: PCT/GB2017/051393
(87) International publication number: WO 2017/199039

(56) References cited:
- EP-A1- 1 985 621
- WO-A1-2016/079517
- WO-A2-2006/122977
- TOCHTROP G P ET AL: "Synthesis of [3,4-(13)c(2)]-enriched bile salts as NMR probes of protein-ligand interactions", THE JOURNAL OF ORGANIC CHEMISTRY, AMERICAN CHEMICAL SOCIETY ETC.|, vol. 67, no. 19, 20 September 2002 (2002-09-20), pages 6764-6771, XP002260968, ISSN: 0022-3263, DOI: 10.1021/JO0259109
- UEKAWA T ET AL: "Short-step Synthesis of Chenodiol from Stigmasterol", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 68, no. 6, 1 January 2004 (2004-01-01), pages 1332-1337, XP003016017, ISSN: 0916-8451, DOI: 10.1271/BBB.68.1332
- M. Stocks: "On Medicinal Chemistry" In: "On Medicinal Chemistry", 1 January 2007 (2007-01-01), Sci-ink Ltd, XP55674042, ISBN: 978-0-9550072-3-1 pages 214-219,

## Description

### Field of the invention

The present invention relates to methods of preparing compounds which are intermediates in the synthesis of bile acid derivatives with pharmacological activity. In particular, the invention relates to methods of preparing intermediates in the synthesis of obeticholic acid and its analogues. The invention further relates to novel intermediates *per se.*

### Background of the invention

Bile acids are steroid acids which are found in the bile of mammals and include compounds such as cholic acid, chenodeoxycholic acid, lithocholic acid and deoxycholic acid, all of which are found in humans. Many bile acids are natural ligands of the farnesoid X receptor (FXR) which is expressed in the liver and intestine of mammals, including humans.

Bile acids are derivatives of steroids and are numbered in the same way. The following shows the general numbering system for steroids and the numbering of the carbon atoms in chenodeoxycholic acid.

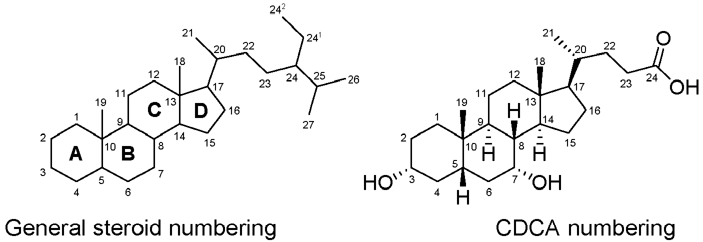

Agonists of FXR have been found to be of use in the treatment of cholestatic liver disorders including primary biliary cholangitis and non-alcoholic steatohepatitis (see review by Jonker et al., in Journal of Steroid Biochemistry & Molecular Biology, 2012, 130, 147-158).

Ursodeoxycholic acid (UDCA), a bile acid originally isolated from the gall bladder of bears, is currently used in the treatment of cholestatic liver disorders, although it appears to be inactive at the FXR.

As well as their action at the FXR, bile acids and their derivatives are also modulators of the G protein-coupled receptor TGR5. This is a member of the rhodopsin-like superfamily of G-protein coupled receptors and has an important role in the bile acid signalling network, which complements the role of the FXR.

Because of the importance of FXR and TGR5 agonists in the treatment of cholestatic liver disorders, efforts have been made to develop new compounds which have agonist activity at these receptors. One particularly active compound is obeticholic acid, which is a potent agonist of both FXR and TGR5. Obeticholic acid is described in WO02/072598 and EP1568706, both of which describe a process for the preparation of obeticholic acid from 7-keto lithocholic acid, which is derived from cholic acid. Further processes for the production of obeticholic acid and its derivatives are described in WO2006/122977, US2009/0062256 and WO2013/192097 and all of these processes also start from 7-keto lithocholic acid.

It is clear from the number of patent publications directed to processes for the production of obeticholic acid that it is by no means simple to synthesise this compound and indeed the process which is currently used starts from cholic acid, has 12 steps and a low overall yield.

In addition to the inefficiency and high cost of this process, there are also problems with the cost and availability of the starting materials. Cholic acid, the current starting material for the production of obeticholic acid, is a natural bile acid which is usually obtained from the slaughter of cows and other animals. This means that the availability of cholic acid and other bile acids is limited by the number of cattle available for slaughter. Since the incidence of cholestatic liver disease is increasing worldwide, the demand for synthetic bile acids such as obeticholic acid is also likely to increase and it is doubtful whether the supply of naturally derived bile acids will continue to be sufficient to meet demand.

Furthermore, the use of a starting material derived from animals means that there is the possibility of the contamination of the material with infectious agents such as viruses or prions, which can not only be hazardous to workers but could potentially contaminate the end products if steps are not taken to prevent this.

Although some patients with cholestatic liver disease can be treated with ursodeoxycholic acid, this is also a natural bile acid and faces the same problems of limited availability and high cost.

In an attempt to solve the problems associated with the use of bile acids as starting materials, the present inventors have devised a process for the synthesis of synthetic bile acid derivatives, such as obeticholic acid (OCA, referred to herein as compound (XA)), which uses plant sterols as starting materials.

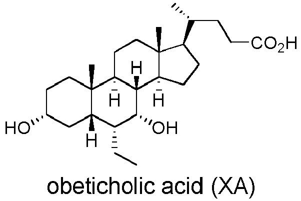

The inventors have developed a process for the production of synthetic bile acids which proceeds *via* novel intermediates and which provides the final product in significantly higher yield than current processes. The process is flexible and can use a variety of different starting materials including animal, fungal and plant sterols.

Suitable animal sterols which can be used as starting materials include deoxycholic acid, cholic acid, while fungal sterols include ergosterol.

Plant sterols are widely available at significantly lower cost than bile acids and, indeed, are often waste products of other processes. Suitable plant sterol and plant sterol derivatives which can be used as starting materials include bis-norcholenol (also known as 20-hydroxymethylpregn-4-en-3-one), androstenedione, androstadienedione, dehydroepiandrosterone, stigmasterol, brassicasterol, campesterol and β-sitosterol.

Our patent applications Nos. PCT/GB2015/053516 (WO2016/079517), PCT/GB2015/053517 (WO2016/079518), PCT/GB2015/053518 (WO2016/079519) and PCT/GB2015/053519 (WO2016/079520) relate to intermediates in the process of synthesizing obeticholic acid (and analogues) as well as to processes for preparing the intermediates and processes for converting them to the desired products.

The present application relates to further compounds which are intermediates in the synthesis of obeticholic acid and analogues thereof.

### Summary of the invention

In one aspect of the invention is provided a compound of general formula (Ia): 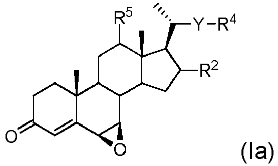 wherein:
R² is H, halo, OH or a protected OH group;
Y is a bond, or a C₁₋₂₀ alkylene, C₂₋₂₀ alkenylene or C₂₋₂₀ alkynylene linker group any of which is optionally substituted with one or more R³;
   wherein each R³ is independently H, halo, OH, a protected OH group or NR⁸R⁹; wherein each of R⁸ and R⁹ is independently H, C₁₋₆ alkyl, C(O)Ph, benzyl, phthalimide, *tert*-butyloxycarbonyl or carboxybenzyl;
R⁴ is C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azide, NO₂, NR¹⁰C(O)NR¹⁰SO₂R¹¹, NR¹⁰C(O)NR¹⁰SO₂N R¹⁰R¹¹, NR¹⁰SO₂R¹¹, C(O)NR¹⁰SO₂R¹¹, CH(XR¹⁰)(XR¹¹), CH(R¹⁰)(XR¹¹), phthalimide or a carboxylic acid mimetic group such as tetrazole;
   wherein each X is independently O, S or NR⁸;
   wherein each R¹⁰ and R¹¹ is independently:
      a. hydrogen;
         or
      b. C₁₋₂₀ alkyl, C₃₋₇ cycloalkyl, C₂₋₂₀ alkenyl or C₂₋₂₀ alkynyl, any of which is optionally substituted with one or more substituents selected from:
         C₁₋₄ alkyl, C₁₋₄ haloalkyl, halo, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ and a 6-to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
         or
      c. a 6- to 14- membered aryl, 5- to 14-membered heteroaryl group or 3- to 10-membered heterocyclic ring, any of which is optionally substituted with one or more substituents selected from:
         C₁₋₆ alkyl, C₃₋₇ cycloalkyl, C₁₋₆ haloalkyl, halo, NO₂, CN, OR¹⁹, C(O)C₁₋₄ alkyl, SR¹⁹, C(O)OR¹⁹, C(O)N(R¹⁹)₂, SO₂R¹⁹, SO₃R¹⁹, N(R¹⁹)₂, phenyl, 5- to 14-membered heteroaryl, 3- to 10-membered heterocyclic ring, methylenedioxy and ethylenedioxy;
         or
      d. a polyethylene glycol residue;
         or
      e. when R⁴ is C(O)NR¹⁰R¹¹, CH(XR¹⁰)(XR¹¹), NR¹⁰R¹¹, BR¹⁰R¹¹, CH[C(O)OR¹⁰]₂ or CH(BR¹⁰R¹¹)₂ an R¹⁰ and an R¹¹ group, together with the atom or atoms to which they are attached, may combine to form a 3- to 10-membered heterocyclic ring;
         wherein each R¹⁹ is independently:
         H, C₁₋₆ alkyl, C₁₋₆ haloalkyl, or a 6- to 14- membered aryl or 5- to 14-membered heteroaryl group either of which is optionally substituted with one or more substituents selected from halo, C₁₋₆ alkyl and C₁₋₆ haloalkyl;
      and wherein each R¹³ is independently:
      a. C₁₋₂₀ alkyl, C₂₋₂₀ alkenyl or C₂₋₂₀ alkynyl, any of which is optionally substituted with one or more substituents selected from:
         halo, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SO₂R¹⁹, SO₃R¹⁹ and N(R¹⁹)₂;
         or
      b. a 6- to 14- membered aryl or 5- to 14-membered heteroaryl group either of which is optionally substituted with one or more substituents selected from: C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
         wherein each R¹⁹ is independently:
         H, C₁₋₆ alkyl or C₁₋₆ haloalkyl; or
Y and R⁴ together form a =CH₂ group; and
R⁵ is H, OH or a protected OH group;
or a salt or isotopic variant thereof.

In another aspect of the invention is provided a compound of general formula (I): 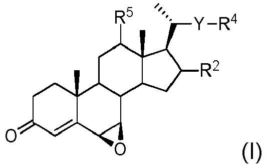 wherein:
R² is H, halo, OH or a protected OH group;
Y is a bond, or a C₁₋₂₀ alkylene, C₂₋₂₀ alkenylene or C₂₋₂₀ alkynylene linker group any of which is optionally substituted with one or more R³;
   wherein each R³ is independently halo, OR⁸ or NR⁸R⁹;
   wherein each of R⁸ and R⁹ is independently H or C₁₋₄ alkyl;
R⁴ is C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, CH(OR¹⁰)(OR¹¹), CH(R¹⁰)(OR¹¹), CH(SR¹⁰)(SR¹¹), NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azide or a carboxylic acid mimetic group such as tetrazole;
   wherein each R¹⁰ and R¹¹ is independently:
   a. hydrogen;
      or
   b. C₁₋₂₀ alkyl, C₂₋₂₀ alkenyl or C₂₋₂₀ alkynyl, any of which is optionally substituted with one or more substituents selected from:
      halo, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
      or
   c. a 6- to 14- membered aryl or 5- to 14-membered heteroaryl group either of which is optionally substituted with one or more substituents selected from: C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
      or
   d. a polyethylene glycol residue;
      or
   e. when R⁴ is C(O)NR¹⁰R¹¹, CH(OR¹⁰)(OR¹¹), CH(SR¹⁰)(SR¹¹), NR¹⁰R¹¹, BR¹⁰R¹¹, CH[C(O)OR¹⁰]₂ or CH(BR¹⁰R¹¹)₂ an R¹⁰
      and an R¹¹ group, together with the atom or atoms to which they are attached, may combine to form a 3- to 10-membered heterocyclic ring;
      wherein each R¹⁹ is independently:
         H, C₁₋₆ alkyl, C₁₋₆ haloalkyl, or a 6- to 14- membered aryl or 5- to 14-membered heteroaryl group either of which is optionally substituted with one or more substituents selected from halo, C₁₋₆ alkyl and C₁₋₆ haloalkyl;
   and wherein each R¹³ is independently:
   a. C₁₋₂₀ alkyl, C₂₋₂₀ alkenyl or C₂₋₂₀ alkynyl, any of which is optionally substituted with one or more substituents selected from:
      halo, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SO₂R¹⁹, SO₃R¹⁹ and N(R¹⁹)₂;
      or
   b. a 6- to 14- membered aryl or 5- to 14-membered heteroaryl group either of which is optionally substituted with one or more substituents selected from:
      C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
      wherein each R¹⁹ is independently:
      H, C₁₋₆ alkyl or C₁₋₆ haloalkyl; or
Y and R⁴ together form a =CH₂ group; and
R⁵ is H, OH or a protected OH group;
or a salt or isotopic variant thereof.

Compounds of general formula (Ia) and (I) are of use as intermediates in the synthesis of obeticholic acid and analogues thereof.

In another aspect of the invention is provided a process for preparing a compound of general formula (Ia) comprising:
reacting a compound of general formula (IIa) with a halogenating agent followed by reaction with base to give a compound of general formula (Ia): 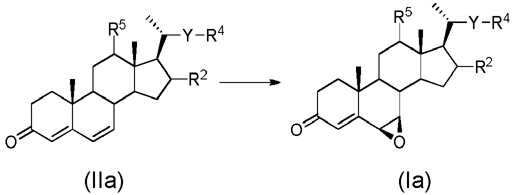 wherein Y, R², R⁴ and R⁵ are as above defined for a compound of general formula (Ia).

In another aspect of the invention is provided a process for preparing a compound of general formula (I) comprising:
reacting a compound of general formula (II) with trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), or 1,3-diiodo-5,5-dimethylhydantoin (DIDMH) followed by reaction with base to give a compound of general formula (I): 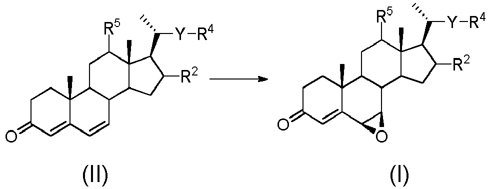 wherein Y, R², R⁴ and R⁵ are as above defined for a compound of general formula (I).

In another aspect of the invention is provided a process for preparing a compound of general formula (Ia) comprising the steps of:
(A) reacting a compound of general formula (IIa) with a halogenating agent to give a compound of general formula (IIIxa) and/or a compound of general formula (IIIya) 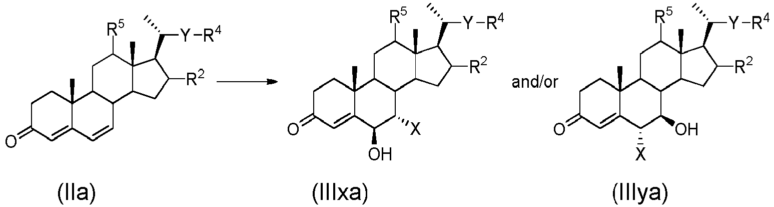
   wherein X is Cl, Br or I; and
   wherein Y, R², R⁴ and R⁵ are as above defined for a compound of general formula (Ia) and
(B) reacting a compound of general formula (IIIxa) and/or a compound of general formula (IIIya) with a base to form a compound of general formula (la): 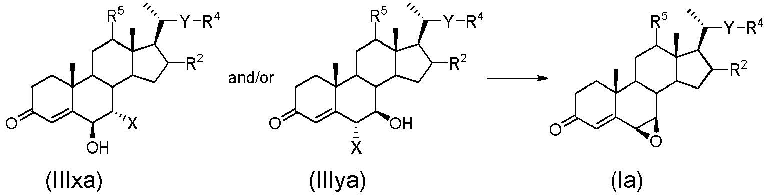
   wherein X is Cl, Br or I; and
   wherein Y, R², R⁴ and R⁵ are as above defined for a compound of general formula (Ia).

In another aspect of the invention is provided a process for preparing a compound of general formula (I) comprising the steps of:
(A) reacting a compound of general formula (II) with trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), or 1,3-diiodo-5,5-dimethylhydantoin (DIDMH) to give a compound of general formula (IIIx) and/or a compound of general formula (Illy) 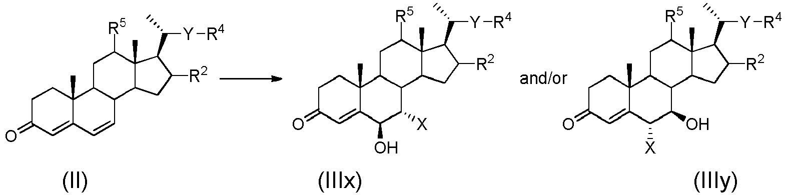
   wherein X is Cl, Br or I; and
   wherein Y, R², R⁴ and R⁵ are as above defined for a compound of general formula (I) and
(B) reacting a compound of general formula (IIIx) and/or a compound of general formula (Illy) with a base to form a compound of general formula (I): 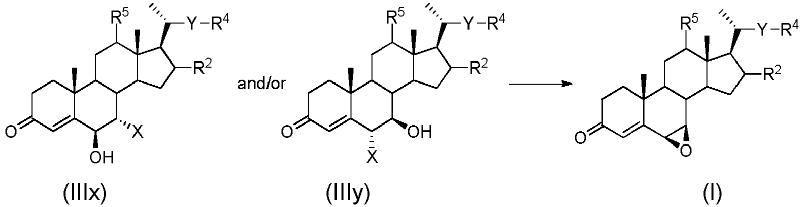
   wherein X is Cl, Br or I; and
   wherein Y, R², R⁴ and R⁵ are as above defined for a compound of general formula (I).

As such, the compounds of general formula (IIIxa) and general formula (IIIya), and compounds of general formula (IIIx) and general formula (Illy) *per se*, are useful as intermediates in the synthesis of a compound of general formula (Ia) or a compound of general formula (I), respectively.

Thus, there is provided a compound of general formula (IIIxa): 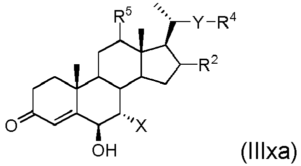 or a salt an isotopic variant thereof,
wherein X is Cl, Br or I; and
wherein Y, R², R⁴ and R⁵ are as above defined for a compound of general formula (Ia).

There is also provided a compound of general formula (IIIx): 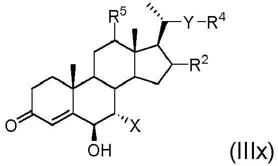 or a salt an isotopic variant thereof,
wherein X is Cl, Br or I; and
wherein Y, R², R⁴ and R⁵ are as above defined for a compound of general formula (I).

There is further provided a compound of general formula (IIIya): 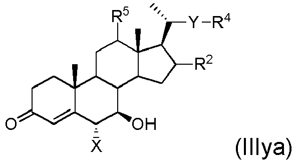 or a salt or an isotopic variant thereof,
wherein X is Cl, Br or I; and
wherein Y, R², R⁴ and R⁵ are as above defined for a compound of general formula (Ia).

There is yet further provided a compound of general formula (IIIy): 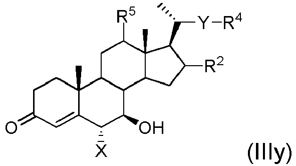 or a salt or an isotopic variant thereof,
wherein X is Cl, Br or I; and
wherein Y, R², R⁴ and R⁵ are as above defined for a compound of general formula (I).

Also provided is a mixture of a compound of formula (IIIxa) and a compound of general formula (IIIya). In one embodiment is provided a mixture of a compound of formula (IIIx) and a compound of general formula (Illy).

Further provided is a mixture comprising a compound of formula (IIIxa) and a compound of general formula (IIIya). In one embodiment is provided a mixture comprising a compound of formula (IIIx) and a compound of general formula (Illy).

In another aspect of the invention is provided a process for preparing a compound of general formula (Ia) comprising the steps of:
(A) reacting a compound of general formula (IIa) with a halogenating agent to give a compound of general formula (IIIxa) and/or a compound of general formula (IIIya) and/or a compound of general formula (IIIxz) and/or a compound of general formula (IIIyz): 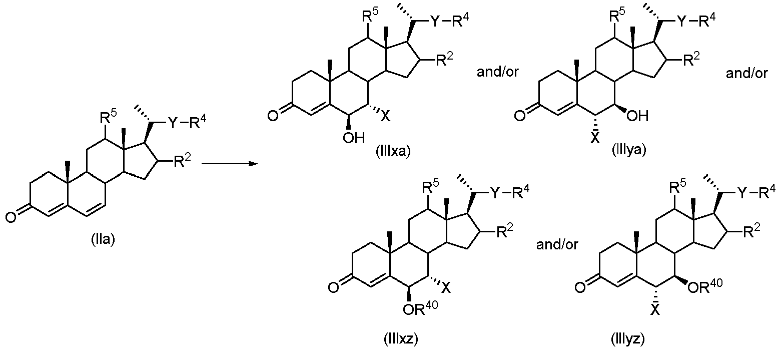
   wherein X is Cl, Br or I;
   R⁴⁰ is C(O)H, C(O)C₁₋₄ alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and
   wherein Y, R², R⁴ and R⁵ are as above defined for a compound of general formula (Ia) and
(B) reacting a compound of general formula (IIIxa) and/or a compound of general formula (IIIxa) and/or a compound of general formula (IIIxz) and/or a compound of general formula (IIIyz) with a base to form a compound of general formula (la): 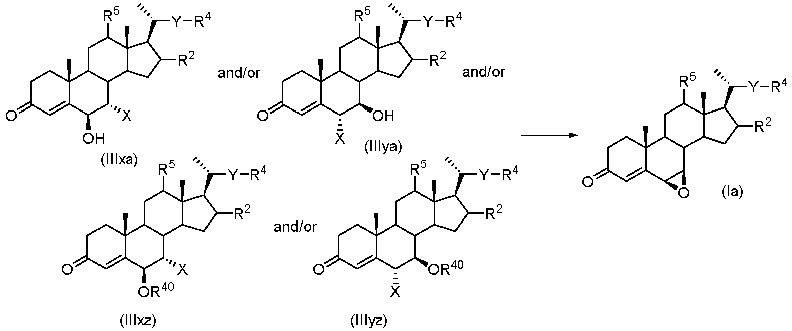
   wherein X is Cl, Br or I;
   R⁴⁰ is C(O)H, C(O)C₁₋₄ alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and
   wherein Y, R², R⁴ and R⁵ are as above defined for a compound of general formula (Ia).

In this embodiment, suitably step (A) is carried out in the presence of HOC(O)R^{x}, HOR^{y}, or HSO₃R^{z}; wherein R^{x} is H, C₁₋₄ alkyl (e.g. methyl or ethyl), phenyl or benzyl; R^{y} is phenyl, benzyl or C₂₋₄ alkene (e.g. allyl); and R^{z} is CF₃, wherein phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl. HOR^{x}, HOR^{y} or HSO₃R^{z} may be present as an additive in the reaction (suitably in the absence of water and in the presence of an aprotic solvent) or as the reaction solvent itself.

In this aspect of the invention, mixtures of compounds of general formula (IIIxa) and/or, compounds of general formula (IIIya) and/or compounds of general formula (IIIxz) and/or compounds of general formula (IIIyz) are formed in step A and then reacted in step B.

In one embodiment, the process further includes the step of removal of group R⁴⁰ before treatment with base. In one embodiment, the process further includes the step of removal of group R⁴⁰ before step (B) is carried out.

There is provided a compound of general formula (IIIxz): 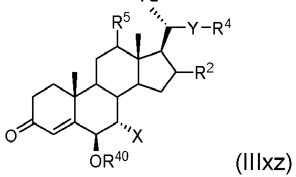 or a salt an isotopic variant thereof,
wherein X is Cl, Br or I;
R⁴⁰ is C(O)H, C(O)C₁₋₄ alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and
wherein Y, R², R⁴ and R⁵ are as above defined for a compound of general formula (Ia).

There is also provided a compound of general formula (IIIyz): 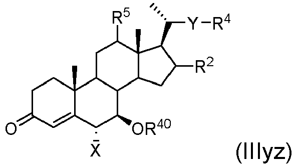 or a salt or an isotopic variant thereof,
wherein X is Cl, Br or I;
R⁴⁰ is C(O)H, C(O)C₁₋₄ alkyl, C(O)phenyl, C(O)benzyl, phenyl, benzyl, C₂₋₄ alkenyl, or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and
wherein Y, R², R⁴ and R⁵ are as above defined for a compound of general formula (Ia).

Further provided is a mixture comprising a compound of formula (IIIxz) and a compound of general formula (IIIyz).

Compounds of general formulae (Ia) and (I) are of use in the synthesis of obeticholic acid and analogues thereof.

Compounds of general formula (Ia) are of use in the preparation of compounds of general formula (Xa): 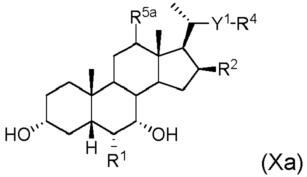 or a salt or an isotopic variant thereof,
wherein,
R¹ is C₁₋₄ alkyl, C₂₋₄ alkenyl or C₂₋₄ alkynyl optionally substituted with one or more substituents selected from halo, OR⁶ and NR⁶R⁷;
   wherein each of R⁶ and R⁷ is independently H or C₁₋₄ alkyl;
R² is H, halo or OH; and
Y¹ is a bond, or a C₁₋₂₀ alkylene linker group which is optionally substituted with one or more R³; or
Y¹ and R⁴ together form a =CH₂ group; and
R^{5a} is H or OH;
wherein R³ and R⁴ are as described above for a compound of general formula (Ia).

Compounds of general formula (I) are of use in the preparation of compounds of general formula (X): 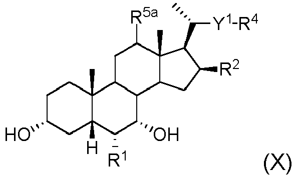 or a salt or an isotopic variant thereof,
wherein,
R¹ is C₁₋₄ alkyl optionally substituted with one or more substituents selected from halo, OR⁶ and NR⁶R⁷;
   wherein each of R⁶ and R⁷ is independently H or C₁₋₄ alkyl;
R² is H, halo or OH; and
Y¹ is a bond, or a C₁₋₂₀ alkylene linker group which is optionally substituted with one or more R³; or
Y¹ and R⁴ together form a =CH₂ group; and
R^{5a} is H or OH;
wherein R³ and R⁴ are as described above for a compound of general formula (I).

In one aspect of the invention is provided a process for preparing a compound of general formula (Xa) from a compound of formula (Ia): 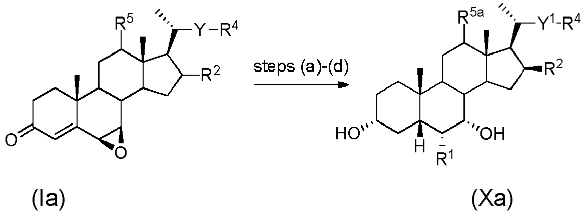 the process comprising the steps of:
(a) selective alkylation of a compound of general formula (Ia) with an organometallic regent to give a compound of general formula (IVa): 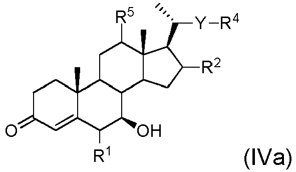
   wherein R¹ is as defined for a compound of formula (Xa);
   and Y, R², R⁴ and R⁵ are as defined for a compound of general formula (Ia);
(b) reducing the compound of general formula (IVa) using a suitable reducing agent to give a compound of general formula (Va): 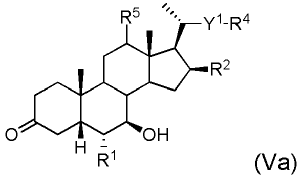
   wherein R¹ and Y¹ are as defined for a compound of formula (Xa);
   and R², R⁴ and R⁵ are as defined for a compound of general formula (Ia);
(c) oxidising the compound of general formula (Va) using a suitable oxidising agent to give a compound of general formula (VIa): 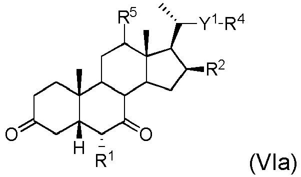
   wherein R¹ and Y¹ are as defined for a compound of formula (Xa);
   and R², R⁴ and R⁵ are as defined for a compound of general formula (Ia);
(d) reduction of the compound of general formula (VIa) using a suitable reducing agent and, where R² and/or R⁵ is a protected OH, removal of the protecting group(s), to give a compound of general formula (Xa) as defined above, wherein removal of the protecting group can take place before or after the reduction.

In one aspect of the invention is provided a process for preparing a compound of general formula (X) from a compound of formula (I): 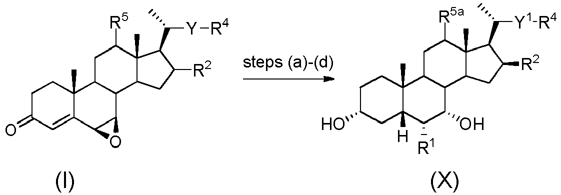 the process comprising the steps of:
(a) selective alkylation of a compound of general formula (I) with an organometallic regent to give a compound of general formula (IV): 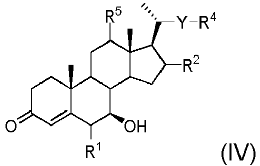
   wherein R¹ is as defined for a compound of formula (X);
   and Y, R², R⁴ and R⁵ are as defined for a compound of general formula (I);
(b) reducing the compound of general formula (IV) using a suitable reducing agent to give a compound of general formula (V): 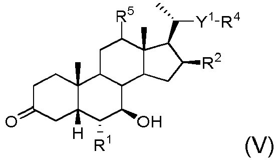
   wherein R¹ and Y¹ are as defined for a compound of formula (X);
   and R², R⁴ and R⁵ are as defined for a compound of general formula (I);
(c) oxidising the compound of general formula (V) using a suitable oxidising agent to give a compound of general formula (VI): 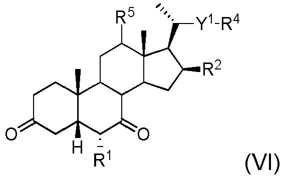
   wherein R¹ and Y¹ are as defined for a compound of formula (X);
   and R², R⁴ and R⁵ are as defined for a compound of general formula (I);
(d) reduction of the compound of general formula (VI) using a suitable reducing agent and, where R² and/or R⁵ is a protected OH, removal of the protecting group(s), to give a compound of general formula (X) as defined above, wherein removal of the protecting group can take place before or after the reduction.

Provided herein is a compound of general formula (IVa) and a compound of general formula (Va) *per se,* and a compound of general formula (IV) and a compound of general formula (V) *per se* such compounds being useful as intermediates in the synthesis of a compound of general formula (Xa) and a compound of general formula (X), respectively.

Thus, provided herein is a compound of general formula (IVa): 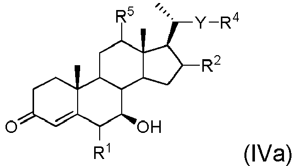 or a salt an isotopic variant thereof,
wherein R¹ is as defined for a compound of general formula (Xa); and
wherein Y, R², R⁴ and R⁵ are as defined for a compound of general formula (Ia).

In one aspect there is provided a compound of general formula (IV): 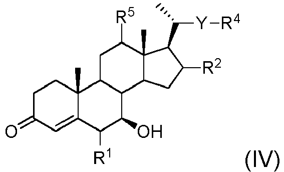 or a salt an isotopic variant thereof,
wherein R¹ is as defined for a compound of general formula (X); and
wherein Y, R², R⁴ and R⁵ are as defined for a compound of general formula (I).

In another aspect there is provided a compound of general formula (Va): 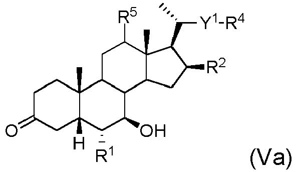 or a salt an isotopic variant thereof,
wherein R¹ and Y¹ are as defined for a compound of formula (Xa);
and R², R⁴ and R⁵ are as defined for compounds of general formula (Ia).

In another aspect there is provided a compound of general formula (V): 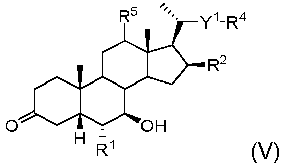 or a salt an isotopic variant thereof,
wherein R¹ and Y¹ are as defined for a compound of formula (X);
and R², R⁴ and R⁵ are as defined for compounds of general formula (I).

### Figures

**Figure 1****:** shows respresentative conversions of a compound of general formula (IIa) or general formula (II) in which the side chain is -CH₂OH to other compounds of general formula (IIa) or general formula (II), respectively, with different side chains.
**Figure 2****:** shows the ¹H NMR spectrum of (6β, 7α, 22E)-6-acetoxy-7-chloro-3-oxo-4,22-choladien-24-oic acid ethyl ester (see Example 2).
**Figure 3****:** shows the ¹³C NMR spectrum of (6β, 7α, 22E)-6-acetoxy-7-chloro-3-oxo-4,22-choladien-24-oic acid ethyl ester (see Example 2).
**Figure 4****:** shows the characteristic C4 protons in the ¹H NMR of a 2:1 mixture of (6β,7β) and (6α,7α) isomers of (22*E*)-6,7-epoxy-3-oxo-4,22-choladien-24-oic acid ethyl ester (See Example 4).
**Figure 5****:** shows an ¹H NMR comparison of (5β, 6α)-3,7-dioxo-6-ethyl-cholan-24-oic acid prepared from (6α, 7α, 22*E*)-6,7-epoxy-3-oxo-4,22-choladien-24-oic acid ethyl ester (alpha) and (6β, 7β, 22*E*)-6,7-epoxy-3-oxo-4,22-choladien-24-oic acid ethyl ester (beta).
**Figure 6****:** shows a ¹³C NMR comparison of (5β, 6α)-3,7-dioxo-6-ethyl-cholan-24-oic acid prepared from (6α, 7α, 22*E*)-6,7-epoxy-3-oxo-4,22-choladien-24-oic acid ethyl ester (alpha) and (6β, 7β, 22*E*)-6,7-epoxy-3-oxo-4,22-choladien-24-oic acid ethyl ester (beta).

### Detailed description of the invention

In the present specification, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

In the present application the term "C₁₋₂₀" alkyl refers to a straight or branched fully saturated hydrocarbon group having from 1 to 20 carbon atoms. The term encompasses methyl, ethyl, *n*-propyl, isopropyl, *n*-butyl, isobutyl, *s*-butyl and *t*-butyl. Other alkyl groups, for example C₁₋₁₂ alkyl, C₁₋₁₀ alkyl, C₁₋₈ alkyl, C₁₋₆ alkyl, C₁₋₅ alkyl, C₁₋₄ alkyl, C₁₋₃ alkyl, or C₁₋₂ alkyl are as defined above but contain different numbers of carbon atoms.

The terms "heterocyclic" and "heterocyclyl" refer to a non-aromatic cyclic group having 3 to 10 ring atoms and at least one heteroatom selected from N, O, S and B and optionally substituted with one or more =O moieties. Examples of heterocyclic groups include pyrrolidine, piperidine, morpholine, piperazine, tetrahydrofuran, dioxolane (e.g. 1,3-dioxolane), dioxane (e.g. 1,3-dioxane) and cyclic thioethers. The term also includes bicyclic and bridged groups such as 9-borabicyclo(3.3.1)nonane (9-BBN).

The term "halogen" refers to fluorine, chlorine, bromine or iodine and the term "halo" to fluoro, chloro, bromo or iodo groups.

The term "C₁₋₆ haloalkyl" refers to a straight or branched alkyl group as defined above having from 1 to 6 carbon atoms and substituted with one or more halo atoms, up to perhalo substitution. Examples include trifluoromethyl, chloroethyl and 1,1-difluoroethyl. Other haloalkyl groups, for example C₁₋₅ haloalkyl, C₁₋₄ haloalkyl, C₁₋₃ haloalkyl or C₁₋₂ haloalkyl are as defined above but contain different numbers of carbon atoms.

The term "C₂₋₂₀ alkenyl" refers to a straight or branched hydrocarbon group having from 2 to 20 carbon atoms and at least one carbon-carbon double bond. Examples include ethenyl (vinyl), prop-1-enyl, prop-2-enyl (allyl), hex-2-enyl etc. Other alkenyl groups, for example C₂₋₁₂ alkenyl, C₂₋₁₀ alkenyl, C₂₋₈ alkenyl, C₂₋₆ alkenyl, C₂₋₅ alkenyl, C₂₋₄ alkenyl or C₂₋₃ alkenyl are as defined above but contain different numbers of carbon atoms.

The term "C₂₋₂₀ alkynyl" refers to a straight or branched hydrocarbon group having from 2 to 20 carbon atoms and at least one carbon-carbon triple bond. Examples include ethynyl, prop-1-ynyl, hex-2-ynyl etc. Other alkynyl groups, for example C₂₋₁₂ alkynyl, C₂₋₁₀ alkynyl, C₂₋₈ alkynyl, C₂₋₆ alkynyl, C₂₋₅ alkynyl, C₂₋₄ alkynyl or C₂₋₃ alkynyl are as defined above but contain different numbers of carbon atoms.

The term "alkylene" refers to a straight or branched fully saturated hydrocarbon chain. Suitably alkylene is C₁₋₂₀ alkylene, C₁₋₁₂ alkylene, C₁₋₁₀ alkylene, C₁₋₈ alkylene, C₁₋₆ alkylene, C₁₋₅ alkylene, C₁₋₄ alkylene, C₁₋₃ alkylene, or C₁₋₂ alkylene. Examples of alkylene groups include -CH₂-, -CH₂CH₂-, -CH(CH₃)-CH₂-, -CH₂CH(CH₃)-, -CH₂CH₂CH₂-, -CH₂CH(CH₂CH₃)- and -CH₂CH(CH₂CH₃)CH₂-.

The term "alkenylene" refers to a straight or branched hydrocarbon chain containing at least one carbon-carbon double bond. Suitably alkenylene is C₂₋₂₀ alkenylene, C₂₋₁₂ alkenylene, C₂₋₁₀ alkenylene, C₂₋₈ alkenylene, C₂₋₆ alkenylene, C₂₋₅ alkenylene, C₂₋₄ alkenylene, or C₂₋₃ alkenylene. Examples of alkenylene groups include -CH=CH-, -CH=C(CH₃)-, -CH₂CH=CH-, -CH=CHCH₂-, -CH₂CH₂CH=CH-, -CH₂CH=C(CH₃)- and -CH₂CH=C(CH₂CH₃)-.

The term "C₂₋₂₀ alkynyl" refers to a straight or branched hydrocarbon group having from 2 to 20 carbon atoms and at least one carbon-carbon triple bond. Examples include ethynyl, prop-1-ynyl, hex-2-ynyl etc. Other alkynyl groups, for example C₂₋₁₂ alkynyl, C₂₋₁₀ alkynyl, C₂₋₈ alkynyl, C₂₋₆ alkynyl, C₂₋₅ alkynyl, C₂₋₄ alkynyl or C₂₋₃ alkynyl are as defined above but contain different numbers of carbon atoms.

The term "alkyl" refers to a straight or branched fully saturated hydrocarbon chain. Suitably alkylene is C₁₋₂₀ alkyl, C₁₋₁₂ alkyl, C₁₋₁₀ alkyl, C₁₋₈ alkyl, C₁₋₆ alkyl, C₁₋₅ alkyl, C₁₋₄ alkyl, C₁₋₃ alkyl, or C₁₋₂ alkyl. Examples of alkyl groups include -CH₃, -CH₂CH₃, -CH(CH₃)-CH₃, - CH₂CH₂CH₃, -C(CH₃)₃ and -CH₂CH₂CH₂CH₃.

The term "alkenyl" refers to a straight or branched hydrocarbon chain containing at least one carbon-carbon double bond. Suitably alkenyl is C₂₋₂₀ alkenyl, C₂₋₁₂ alkenyl, C₂₋₁₀ alkenyl, C₂₋₈ alkenyl, C₂₋₆ alkenyl, C₂₋₅ alkenyl, C₂₋₄ alkenyl, or C₂₋₃ alkenyl. Examples of alkenyl groups include -CH=CH₂, -CH=CH(CH₃), -CH₂CH=CH₂, -CH=CHCH₃, - CH₂CH₂CH=CH₂, -CH₂CH=CH(CH₃)- and -CH₂CH=CH(CH₂CH₃).

The term "alkynylene" refers to a straight or branched hydrocarbon chain containing at least one carbon-carbon triple bond. Suitably alkynylene is C₂₋₂₀ alkynylene, C₂₋₁₂ alkynylene, C₂₋₁₀ alkynylene, C₂₋₈ alkynylene, C₂₋₆ alkynylene, C₂₋₅ alkynylene, C₂₋₄ alkynylene, or C₂₋₃ alkynylene. Examples of alkynylene groups include -C≡C-, -CH₂C≡C-, -C≡C-CH₂-, -CH₂CH₂C≡C-, -CH₂C≡CCH₂- and -CH₂C≡C-CH₂CH₂-.

The terms "aryl" and "aromatic" refer to a cyclic group with aromatic character having from 6 to 14 ring carbon atoms (unless otherwise specified, for example 6 to 10 ring carbon atoms) and containing up to three rings. Where an aryl group contains more than one ring, not all rings must be aromatic in character. Examples include phenyl, naphthyl and anthracenyl as well as partially saturated systems such as tetrahydronaphthyl, indanyl and indenyl. A further example of an aryl group is 1,2,3,4-tetrahydronaphthalene.

The terms "heteroaryl" and "heteroaromatic" refer to a cyclic group with aromatic character having from 5 to 14 ring atoms (unless otherwise specified, for example 5 to 10 ring atoms), at least one of which is a heteroatom selected from N, O and S, and containing up to three rings. Where a heteroaryl group contains more than one ring, not all rings must be aromatic in character. Examples of heteroaryl groups include pyridine, pyrimidine, indole, benzofuran, benzimidazole and indolene. Further examples of heteroaryl groups include quinoline and isoquinoline.

The term "isotopic variant" refers to isotopically-labelled compounds which are identical to those recited in formula (Ia) or formula (I) but for the fact that one or more atoms are replaced by an atom having an atomic mass or mass number different from the atomic mass or mass number most commonly found in nature, or in which the proportion of an atom having an atomic mass or mass number found less commonly in nature has been increased (the latter concept being referred to as "isotopic enrichment"). Examples of isotopes that can be incorporated into compounds of the invention include isotopes of hydrogen, carbon, nitrogen, oxygen, fluorine, iodine and chlorine such as ²H (deuterium), ³H, ¹¹C, ¹³C, ¹⁴C, ¹⁸F, ¹²³I or ¹²⁵I (e.g. ³H, ¹¹C, ¹⁴C, ¹⁸F, ¹²³I or ¹²⁵I), which may be naturally occurring or non-naturally occurring isotopes.

Polyethylene glycol (PEG) is a polyether compound, which in linear form has general formula H-[O-CH₂-CH₂]ₙ-OH. A polyethylene glycol residue is a PEG in which the terminal H is replaced by a bond linking it to the remainder of the molecule.

Branched versions, including hyperbranched and dendritic versions are also contemplated and are generally known in the art. Typically, a branched polymer has a central branch core moiety and a plurality of linear polymer chains linked to the central branch core. PEG is commonly used in branched forms that can be prepared by addition of ethylene oxide to various polyols, such as glycerol, glycerol oligomers, pentaerythritol and sorbitol. The central branch moiety can also be derived from several amino acids, such as lysine. The branched poly (ethylene glycol) can be represented in general form as R(-PEG-OH)ₘ in which R is derived from a core moiety, such as glycerol, glycerol oligomers, or pentaerythritol, and m represents the number of arms. Multi-armed PEG molecules, such as those described in US5,932,462; US5,643,575; US5,229,490; US4,289,872; US2003/0143596; WO96/21469; and WO93/21259 may also be used.

The PEG polymers may have an average molecular weight of, for example, 600-2,000,000 Da, 60,000-2,000,000 Da, 40,000-2,000,000 Da, 400,000-1,600,000 Da, 800-1,200,000 Da, 600-40,000 Da, 600-20,000 Da, 4,000-16,000 Da, or 8,000-12,000 Da.

The term "protected OH" relates to an OH group protected with any suitable protecting group. For example, the protected OH may be a group R⁴ as defined above.

Suitable protecting groups include esters such that, for example when R² and/or R⁵ and/or R³ is a protected OH group, R² and/or R⁵ and/or R³ may independently be a group OC(O)R¹⁴, where R¹⁴ is a group R¹⁰ as defined above. Silyl ethers are also suitable, and in this case, R² and/or R⁵ and/or R³ may independently be a group OSi(R¹⁶)₃, where each R¹⁶ is independently a group R¹³ as defined above.

Other suitable protecting groups for OH are well known to those of skill in the art (see Wuts, PGM and Greene, TW (2006) "Greene's Protective Groups in Organic Synthesis", 4th Edition, John Wiley & Sons, Inc., Hoboken, NJ, USA).

Salts of the compounds of general formula (XVIIIa) and (XVIII) are suitably pharmaceutically or veterinarily acceptable salts. Salts which are not pharmaceutically or veterinarily acceptable may still be valuable as intermediates.

References to a protecting group which is stable in basic conditions mean that the protecting group cannot be removed by treatment with a base.

Appropriate salts of the compounds described herein include basic addition salts such as sodium, potassium, calcium, aluminium, zinc, magnesium and other metal salts as well as choline, diethanolamine, ethanolamine, ethyl diamine, meglumine and other well-known basic addition salts as summarised in Paulekuhn et al., J. Med. Chem. 2007, 50, 6665-6672 and/or known to those skilled in the art.

The term "carboxylic acid mimetic group" relates to known carboxylic acid isosteres including tetrazole, substituted tetrazole, -SO₂-NHR¹⁰, C(O)NH-SO₂R¹⁰ and NHC(O)NH-SO₂R¹⁰;
wherein R¹⁰ is as above defined for a compound of general formulae (Ia) or (I) and is suitably H, C₁₋₆ alkyl, C₃₋₇ cycloalkyl or 6- to 14- membered aryl (e.g. phenyl).

Tetrazole groups include tetrazole-5-yl and tetrazole-1-yl. Substituted tetrazole includes tetrazole substituted with C₁₋₄ alkyl, halo, OH, O(C₁₋₄ alkyl) or SO₂R¹⁰ (e.g. SO₂(C₁₋₄ alkyl), SO₂-phenyl or SO₂-tolyl).

Such carboxylic acid mimetic groups are well known in the art and are discussed, for example in "On Medicinal Chemistry"; M Stocks, L Alcaraz, E Griffen; Pub: Sci-ink Ltd (April 2007).

Particularly suitable carboxylic acid mimetic groups include tetrazole, C(O)NH-SO₂R¹⁰ and NHC(O)NH-SO₂R¹⁰, with tetrazole being particularly suitable.

In one aspect of the invention is provided a compound of general formula (Ia): 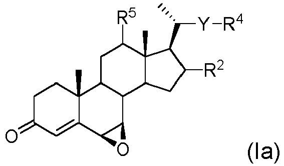 wherein Y, R², R⁴ and R⁵ are as above defined.

Suitably, the compound of general formula (Ia) is: 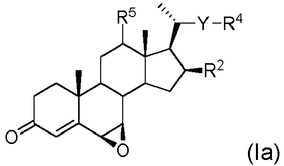 wherein Y, R², R⁴ and R⁵ are as above defined.

In one embodiment, the compound of formula (Ia) is:

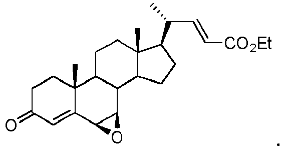

In one embodiment, the compound of formula (Ia) is:

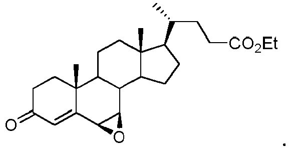

In one embodiment, the compound of formula (Ia) is not:

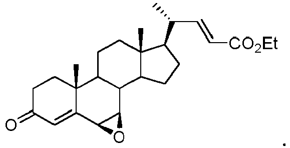

In one embodiment, the compound of formula (Ia) is not:

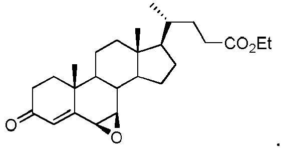

In one aspect of the invention is provided a compound of general formula (I): 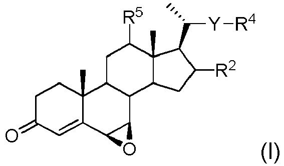 wherein Y, R², R⁴ and R⁵ are as above defined.

Suitably, the compound of general formula (I) is: 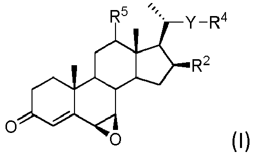 wherein Y, R², R⁴ and R⁵ are as above defined.

In one embodiment, the compound of formula (I) is not:

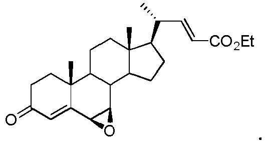

In one embodiment, the compound of formula (I) is not:

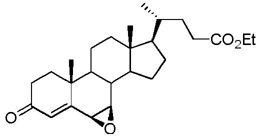

In another aspect there is provided a compound of general formula (IIIxa): 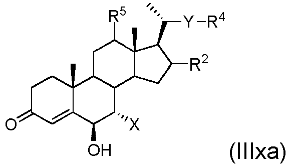 or a salt an isotopic variant thereof,
wherein X is Cl, Br or I; and
wherein Y, R², R⁴ and R⁵ are as above defined for a compound of general formula (Ia).

Suitably, the compound of general formula (IIIxa) is: 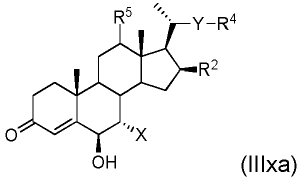 or a salt an isotopic variant thereof,
wherein X is Cl, Br or I; and
wherein Y, R², R⁴ and R⁵ are as above defined for a compound of general formula (Ia).

In another aspect there is provided a compound of general formula (IIIx): 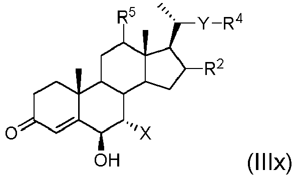 or a salt an isotopic variant thereof,
wherein X is Cl, Br or I; and
wherein Y, R², R⁴ and R⁵ are as above defined for a compound of general formula (I).

Suitably, the compound of general formula (IIIIx) is: 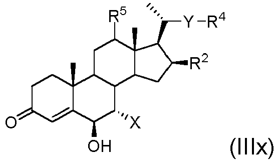 or a salt an isotopic variant thereof,
wherein X is Cl, Br or I; and
wherein Y, R², R⁴ and R⁵ are as above defined for a compound of general formula (I).

In another aspect there is provided a compound of general formula (IIIya): 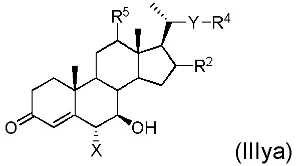 or a salt or an isotopic variant thereof,
wherein X is Cl, Br or I; and
wherein Y, R², R⁴ and R⁵ are as above defined for a compound of general formula (Ia).

Suitably, the compound of general formula (IIIya) is: 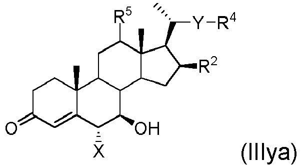 or a salt or an isotopic variant thereof,
wherein X is Cl, Br or I; and
wherein Y, R², R⁴ and R⁵ are as above defined for a compound of general formula (Ia).

In another aspect there is provided a compound of general formula (Illy): 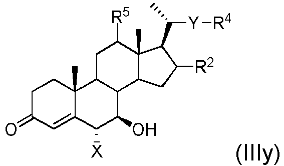 or a salt or an isotopic variant thereof,
wherein X is Cl, Br or I; and
wherein Y, R², R⁴ and R⁵ are as above defined for a compound of general formula (I).

Suitably, the compound of general formula (Illy) is: 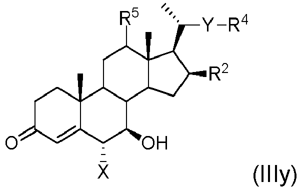 or a salt or an isotopic variant thereof,
wherein X is Cl, Br or I; and
wherein Y, R², R⁴ and R⁵ are as above defined for a compound of general formula (I).

In a further aspect there is provided a compound of general formula (IVa): 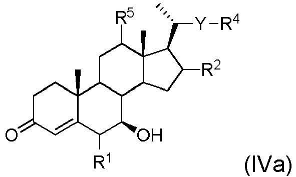 or a salt an isotopic variant thereof,
wherein R¹ is as defined for a compound of formula (Xa); and
Y, R², R⁴ and R⁵ are as defined for compounds of general formula (Ia).

Suitably the compound of general formula (IVa) is: 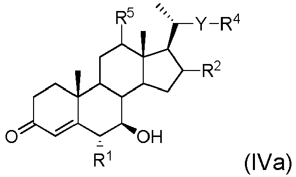 or a salt an isotopic variant thereof,
wherein R¹ is as defined for a compound of formula (Xa); and
Y, R², R⁴ and R⁵ are as defined for compounds of general formula (Ia).

Suitably the compound of general formula (IVa) is: 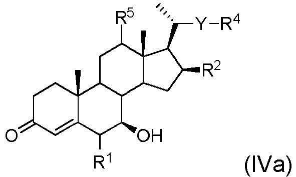 or a salt an isotopic variant thereof,
wherein R¹ is as defined for a compound of formula (Xa); and
Y, R², R⁴ and R⁵ are as defined for compounds of general formula (Ia).

Suitably the compound of general formula (IVa) is: 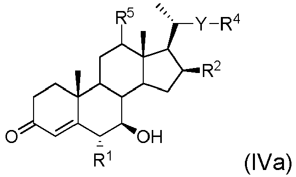 or a salt an isotopic variant thereof,
wherein R¹ is as defined for a compound of formula (Xa); and
Y, R², R⁴ and R⁵ are as defined for compounds of general formula (Ia).

In a further aspect there is provided a compound of general formula (IV): 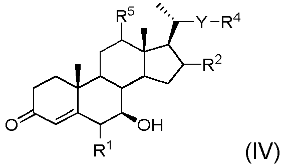 or a salt an isotopic variant thereof,
wherein R¹ is as defined for a compound of formula (X); and
Y, R², R⁴ and R⁵ are as defined for compounds of general formula (I).

Suitably the compound of general formula (IV) is: 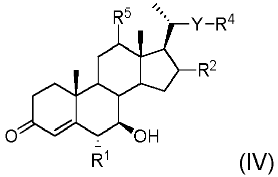 or a salt an isotopic variant thereof,
wherein R¹ is as defined for a compound of formula (X); and
Y, R², R⁴ and R⁵ are as defined for compounds of general formula (I).

Suitably the compound of general formula (IV) is: 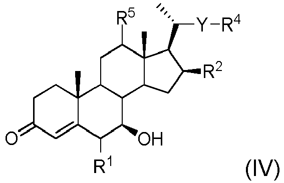 or a salt an isotopic variant thereof,
wherein R¹ is as defined for a compound of formula (X); and
Y, R², R⁴ and R⁵ are as defined for compounds of general formula (I).

Suitably the compound of general formula (IV) is: 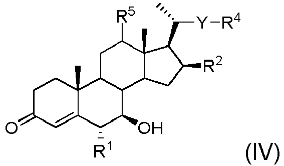 or a salt an isotopic variant thereof,
wherein R¹ is as defined for a compound of formula (Xa); and
Y, R², R⁴ and R⁵ are as defined for compounds of general formula (I).

In a further aspect there is provided a compound of general formula (Va): 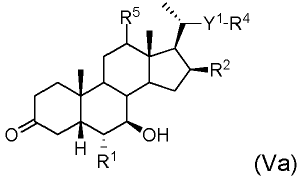 or a salt an isotopic variant thereof,
wherein Y¹ and R¹ are as above defined for compounds of general formula (Xa);
and R², R⁴ and R⁵ are as defined for compounds of general formula (Ia).

In a further aspect there is provided a compound of general formula (V): 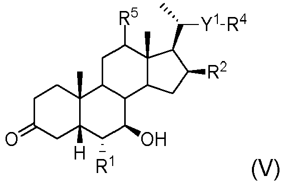 or a salt an isotopic variant thereof,
wherein Y¹ and R¹ are as above defined for compounds of general formula (X);
and R², R⁴ and R⁵ are as defined for compounds of general formula (I).

In one aspect there is provided a compound of general formula (IIIxz): 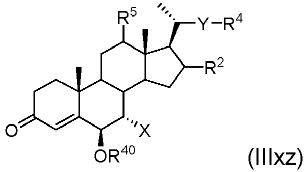 or a salt an isotopic variant thereof,
wherein X is Cl, Br or I;
wherein R⁴⁰ is C(O)H, C(O)C₁₋₄alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and wherein Y, R², R⁴ and R⁵ are as above defined for a compound of general formula (Ia).

Suitably, the compound of general formula (IIIIxz) is: 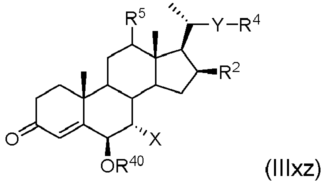 or a salt an isotopic variant thereof,
wherein X is Cl, Br or I;
wherein R⁴⁰ is C(O)H, C(O)C₁₋₄alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and wherein Y, R², R⁴ and R⁵ are as above defined for a compound of general formula (Ia).

In another aspect there is provided a compound of general formula (IIIyz): 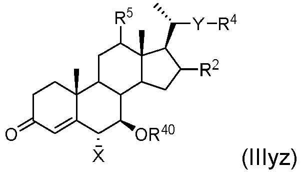 or a salt or an isotopic variant thereof,
wherein X is Cl, Br or I;
wherein R⁴⁰ is C(O)H, C(O)C₁₋₄alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and wherein Y, R², R⁴ and R⁵ are as above defined for a compound of general formula (Ia).

Suitably, the compound of general formula (IIIyz) is: 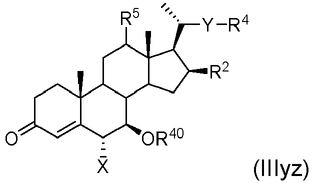 or a salt or an isotopic variant thereof,
wherein X is Cl, Br or I;
wherein R⁴⁰ is C(O)H, C(O)C₁₋₄alkyl, C(O)phenyl, C(O)benzyl, phenyl, benzyl, C₂₋₄ alkenyl or SO₂CF₃; wherein C(O)phenyl, C(O)benzyl, phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl; and wherein Y, R², R⁴ and R⁵ are as above defined for a compound of general formula (Ia).

In one embodiment described herein is provided a mixture of a compound of general formula (IIIxz) and a compound of general formula (IIIyz). In one embodiment is provided a mixture comprising a compound of general formula (IIIxz) and a compound of general formula (IIIyz).

The following embodiments described herein relate to compounds of general formulae (Ia), (I), (IIa), (II), (IIIxa), (IIIx), (IIIya), (IIIy), (IIIxz), (IIIyz), (IVa), (IV), (Va), (V), (VIa), (VI), (Xa) and (X) where applicable, and to methods and intermediates for their preparation as described herein, unless otherwise stated.

Embodiments relating to individual R groups, Y groups and X groups are envisaged as being fully combinable with one or more other R groups to form further embodiments of the invention.

In one embodiment, R² is H. In one embodiment, R² is halo. In one embodiment, R² is OH. In one embodiment, R² is a protected OH group. In one embodiment, R² is a protected OH group which is not stable in a basic environment such that treatment with a base converts the protected OH group to OH. Examples of such groups are well known in the art and include a group OC(O)R¹⁴, wherein R¹⁴ is a group R¹⁰ as defined above for general formula (I), and is suitably C₁₋₆ alkyl or benzyl, or C₁₋₆ alkyl or phenyl. In another embodiment, R² is a protected OH group which is stable in a basic environment. Examples of such groups include OSi(R¹⁶)₃, where each R¹⁶ is independently a group R¹³ as defined above for general formula (I) and general formula (Ia), and is suitably C₁₋₆ alkyl or phenyl. In one embodiment, Si(R¹⁶)₃ is selected from the group consisting of trimethylsilyl (TMS), triethylsilyl (TES), triphenylsilyl (TPS), tri-isopropylsilyl (TIPS), thexyldimethylsilyl (TDS), *tert*-butyldiphenylsilyl (TBDPS), *tert*-butyldimethylsilyl (TBDMS or TBS), di-*tert-*butylmethylsilyl (DTBMS), diethylisopropylsilyl (DEIPS) and dimethylisopropylsilyl (DMIPS), in particular TMS, TES, TIPS, TBDMS and TBDPS.

In one embodiment, R² is in the "up" position i.e. is in the *beta*-configuration.

In one embodiment, Y is a bond.

In one embodiment, for compounds of general formulae (Ia), (I), (IIa), (II), (IIIxa), (IIIx), (IIIya), (IIIy), (IIIxz), (IIIyz), (IVa) and (IV) Y is a C₁₋₂₀, C₁₋₁₂, C₁₋₁₀, C₁₋₈, C₁₋₆, C₁₋₅, C₁₋₄, C₁₋₃ or C₁₋₂alkylene or a C₂₋₁₂, C₂₋₁₀, C₂₋₈, C₂₋₆, C₂₋₅, C₂₋₄, C₂₋₃ or C₂ alkenylene linker group either of which is optionally substituted with one or more groups R³ as defined above.

In one embodiment, for compounds of general formulae (Va), (V), (VIa), (VI), (Xa) and (X) Y is a C₁₋₂₀, C₁₋₁₂, C₁₋₁₀, C₁₋₈, C₁₋₆, C₁₋₅, C₁₋₄, C₁₋₃ or C₁₋₂ alkylene linker group which is optionally substituted with one or more groups R³ as defined above.

In one embodiment, for compounds of general formulae (Ia), (I), (IIa), (II), (IIIxa), (IIIx), (IIIya), (IIIy), (IIIxz), (IIIyz), (IVa) and (IV) Y is bond, or a C₁₋₃ alkylene or C₂₋₃ alkenylene linker group either of which is optionally substituted with one or more groups R³ as defined above. Suitably Y is a C₁₋₃ alkylene or C₂₋₃ alkenylene linker group either of which is optionally substituted with one or more groups R³ as defined above.

In one embodiment, for compounds of general formulae (Va), (V), (VIa), (VI), (Xa) and (X) Y is bond, or a C₁₋₃ alkylene linker group which is optionally substituted with one or more groups R³ as defined above. Suitably Y is a C₁₋₃ alkylene linker group which is optionally substituted with one or more groups R³ as defined above.

In one embodiment, for compounds of general formulae (Ia), (I), (IIa), (II), (IIIxa), (IIIx), (IIIya), (IIIy), (IIIxz), (IIIyz), (IVa) and (IV) Y is a bond, -CH₂-, -CH₂CH₂-, -CH=CH- or-CH=C(CH₃)-; suitably -CH₂-, -CH₂CH₂-, -CH=CH- or -CH=C(CH₃)-, in particular -CH₂CH₂- or -CH=CH-.

In one embodiment, for compounds of general formulae (Va), (V), (VIa), (VI), (Xa) and (X) Y is a bond, -CH₂- or -CH₂CH₂-; suitably -CH₂- or -CH₂CH₂-, in particular -CH₂CH₂.

In one embodiment, for compounds of general formulae (Ia), (I), (IIa), (II), (IIIxa), (IIIx), (IIIya), (IIIy), (IIIxz), (IIIyz), (IVa) and (IV) Y is a bond, an unsubstituted C₁₋₃ alkylene group, a C₁₋₃ alkylene group substituted with OH, or a C₁₋₃ alkenylene group. For example, Y may be a bond, -CH₂-, -CH₂CH₂-, -CH(OH)-CH₂-, -CH=CH- or -CH=C(CH₃)-, in particular a bond, -CH₂-, -CH-₂-CH₂-, -CH=CH- or -CH=C(CH₃)-, especially -CH₂-, -CH₂-CH₂-,-CH=CH- or -CH=C(CH₃)-.

In one embodiment, for compounds of general formulae (Va), (V), (VIa), (VI), (Xa) and (X) Y is a bond, an unsubstituted C₁₋₃ alkylene group or a C₁₋₃ alkylene group substituted with OH. For example, Y may be a bond, -CH₂-, -CH₂CH₂- or -CH(OH)-CH₂-.

In one embodiment, Y is an C₁₋₁₅ alkylene linker, more suitably C₁₋₁₂, C₁₋₁₀ or C₁₋₈ alkylene linker and is substituted with an OH group. In this case, the OH group may be separated from the R⁴ moiety by a single CH₂ group such that the linker Y is a group Y⁴-CH(OH)-CH₂, where Y⁴ is as defined for Y, but is shorter by two carbon atoms. For example, Y may be -CH(OH)-CH₂-.

This Y linker is particularly suitable when R⁴ is CN or R⁴ is CH(XR¹⁰)(XR¹¹) e.g. CH(OR¹⁰)(OR¹¹) wherein R¹⁰ and R¹¹ are as defined above, but particularly wherein the XR¹⁰ and XR¹¹ e.g. OR¹⁰ and OR¹¹ groups together with the carbon atom to which they are attached form a cyclic group, e.g. a cyclic acetal group such as a 1,3-dioxane or 1,3-dioxolane ring.

In one embodiment, R³ is H. In one embodiment, R³ is halo. In one embodiment, R³ is OH. In one embodiment, R³ is NR⁸R⁹, wherein each of R⁸ and R⁹ are suitably independently selected from H, methyl, ethyl, benzyl and *tert*-butyoxycarbonyl. In one embodiment, R³ is a protected OH group. In one embodiment, R³ is a protected OH group which is not stable in a basic environment such that treatment with a base converts the protected OH group to OH. Examples of such groups are well known in the art and include a group OC(O)R¹⁴, wherein R¹⁴ is a group R¹⁰ as defined above for general formula (Ia) or (I), and is suitably C₁₋₆ alkyl or benzyl, or C₁₋₆ alkyl or phenyl. In another embodiment, R³ is a protected OH group which is stable in a basic environment. Examples of such groups include OSi(R¹⁶)₃, where each R¹⁶ is independently a group R¹³ as defined above for general formula (Ia) or (I), and is suitably C₁₋₆ alkyl or phenyl. In one embodiment, Si(R¹⁶)₃ is selected from the group consisting of trimethylsilyl (TMS), triethylsilyl (TES), triphenylsilyl (TPS), tri-isopropylsilyl (TIPS), thexyldimethylsilyl (TDS), *tert*-butyldiphenylsilyl (TBDPS), *tert-*butyldimethylsilyl (TBDMS or TBS), di-*tert*-butylmethylsilyl (DTBMS), diethylisopropylsilyl (DEIPS) and dimethylisopropylsilyl (DMIPS), in particular TMS, TES, TIPS, TBDMS and TBDPS.

In one embodiment R³ is H, halo, OH, OC(O)R¹⁴, OSi(R¹⁶)₃, or NR⁸R⁹;
wherein R¹⁴ is C₁₋₆ alkyl or phenyl;
each R¹⁶ is independently C₁₋₆ alkyl or phenyl; and
each R⁸ and R⁹ is independently H, methyl, ethyl or *tert*-butoxycarbonyl.

In one embodiment, each R³ is independently halo, OR⁸ or NR⁸R⁹; wherein each of R⁸ and R⁹ is independently H or C₁₋₄ alkyl.

In one embodiment, each R³ is independently halo, OR⁸ or NR⁸R⁹; wherein each of R⁸ and R⁹ is independently selected from H, methyl or ethyl, especially H or methyl.

In one embodiment, Y and R⁴ together form a =CH₂ group.

In one embodiment, when present in the R₄ moiety, each R¹⁰ and R¹¹ is independently:
a. hydrogen; or
b. C₁₋₁₀ alkyl, C₂₋₁₀ alkenyl or C₂₋₁₀ alkynyl, any of which is optionally substituted with one or more substituents as described above; or
c. a 6- to 10- membered aryl or 5- to 10-membered heteroaryl group either of which is optionally substituted with one or more substituents as described above; or
d. a polyethylene glycol residue; or
e. when R⁴ is CH(XR¹⁰)(XR¹¹), NR¹⁰R¹¹, BR¹⁰R¹¹, CH[C(O)OR¹⁰]₂ or CH(BR¹⁰R¹¹)₂, an R¹⁰ and an R¹¹ group, together with the atom or atoms to which they are attached, may combine to form a 3- to 10-membered heterocylic ring, more suitably a 5-to 6- membered heterocyclic ring.

In one embodiment, each R¹⁰ and R¹¹ is independently:
a. hydrogen; or
b. C₁₋₁₀ alkyl, C₂₋₁₀ alkenyl or C₂₋₁₀ alkynyl, any of which is optionally substituted with one or more substituents as described above; or
c. a 6- to 10- membered aryl or 5- to 10-membered heteroaryl group either of which is optionally substituted with one or more substituents as described above; or
d. a polyethylene glycol residue; or
e. when R⁴ is CH(OR¹⁰)(OR¹¹), CH(SR¹⁰)(SR¹¹), NR¹⁰R¹¹, BR¹⁰R¹¹, CH[C(O)OR¹⁰]₂ or CH(BR¹⁰R¹¹)₂, an R¹⁰ and an R¹¹ group, together with the atom or atoms to which they are attached, may combine to form a 3- to 10-membered heterocylic ring.

Suitably, each R¹⁰ and R¹¹ is independently
a. hydrogen; or
b. C₁₋₁₀ alkyl, C₂₋₁₀ alkenyl or C₂₋₁₀ alkynyl optionally substituted with one or more substituents as described above; or
c. a 6- to 10-membered aryl group or a 5- to 6-membered heteroaryl group optionally substituted with one or more substituents as described above; or
e. when R⁴ is C(O)NR¹⁰R¹¹ or NR¹⁰R¹¹, an R¹⁰ and an R¹¹ group, together with the nitrogen to which they are attached, combine to form a pyrrolidine or piperidine ring or when R⁴ is CH(XR¹⁰)(XR¹¹), for example CH(OR¹⁰)(OR¹¹), the XR¹⁰ and XR¹¹ group, together with the carbon atom to which they are attached, combine to form a ring; suitably X is O and the ring is a 1,3-dioxane or 1,3-dioxolane ring; or when R⁴ is BR¹⁰R¹¹, the R¹⁰ and R¹¹ groups, together with the boron atom to which they are attached combine to form a bridged boron-containing ring such as 9-BBN.

Suitably, each R¹⁰ and R¹¹ is independently:
a. hydrogen; or
b. C₁₋₁₀ alkyl, C₂₋₁₀ alkenyl or C₂₋₁₀ alkynyl optionally substituted with one or more substituents as described above; or
c. a 6- to 10-membered aryl group optionally substituted with one or more substituents as described above; or
e. when R⁴ is C(O)NR¹⁰R¹¹ or NR¹⁰R¹¹, an R¹⁰ and an R¹¹ group, together with the nitrogen to which they are attached, combine to form a pyrrolidine or piperidine ring or when R⁴ is CH(OR¹⁰)(OR¹¹), the OR¹⁰ and OR¹¹ group, together with the carbon atom to which they are attached, combine to form a 1,3-dioxane or 1,3-dioxolane ring; or when R⁴ is BR¹⁰R¹¹, the R¹⁰ and R¹¹ groups, together with the boron atom to which they are attached combine to form a bridged boron-containing ring such as 9-BBN.

In one embodiment, when R⁴ is NR¹⁰R¹¹, R¹⁰ is H or C₁₋₄ alkyl and R¹¹ is a 5-10 membered heteroaryl group such as tetrazole.

Other examples of R⁴ groups include azide and tetrazole.

When R⁴ is OSi(R¹³)₃, suitably each R¹³ is independently selected from:
a. C₁₋₁₀ alkyl, C₂₋₁₀ alkenyl or C₂₋₁₀ alkynyl optionally substituted with one or more substituents as described above; or
b. a 6- to 10- membered aryl or 5- to 10-membered heteroaryl group optionally substituted with one or more substituents as described above.

More suitably, each R¹³ is independently selected from:
a. C₁₋₁₀ alkyl, C₂₋₁₀ alkenyl or C₂₋₁₀ alkynyl optionally substituted with one or more substituents as described above; or
b. a 6- to 10- membered aryl group optionally substituted with one or more substituents as described above.

Still more suitably, each R¹³ is independently selected from C₁₋₁₀ alkyl or phenyl, either of which is optionally substituted as described above.

In one embodiment, each R¹³ is independently selected from C₁₋₆ alkyl (in particular methyl, ethyl, isopropyl, *tert*-butyl, hexyl) and phenyl.

In one embodiment, Si(R¹³)₃ is selected from the group consisting of trimethylsilyl (TMS), triethylsilyl (TES), triphenylsilyl (TPS), tri-isopropylsilyl (TIPS), thexyldimethylsilyl (TDS), *tert*-butyldiphenylsilyl (TBDPS), *tert*-butyldimethylsilyl (TBDMS or TBS), di-*tert-*butylmethylsilyl (DTBMS), diethylisopropylsilyl (DEIPS) and dimethylisopropylsilyl (DMIPS), in particular TMS, TES, TIPS, TBDMS and TBDPS.

Suitable substituents for alkyl, alkenyl and alkynyl R¹⁰ and R¹¹ groups include halo, NO₂, CN, OR¹⁹, SR¹⁹, C(O)OR¹⁹, SO₂R¹⁹, SO₃R¹⁹, OSO₃R¹⁹, N(R¹⁹)₂, and a 6- to 10- membered aryl or 5- to 14-membered heteroaryl group, either of which is optionally substituted with C₁₋₆ alkyl, C₁₋₆ haloalkyl, halo, NO₂, CN, OR¹⁹, SO₂R¹⁹, SO₃R¹⁹ or N(R¹⁹)₂; where R¹⁹ is as defined above.

Similarly, suitable substituents for alkyl, alkenyl and alkynyl R¹³ groups include halo, NO₂, CN, OR¹⁹, SR¹⁹, C(O)OR¹⁹, SO₂R¹⁹, SO₃R¹⁹, OSO₃R¹⁹, N(R¹⁹)₂, and a 6- to 10- membered aryl or 5- to 14-membered heteroaryl group, either of which is optionally substituted with C₁₋₆ alkyl, C₁₋₆ haloalkyl, halo, NO₂, CN, OR¹⁹, SO₂R¹⁹, SO₃R¹⁹ or N(R¹⁹)₂; where R¹⁹ is as defined above.

More suitable substituents for these R¹⁰ and R¹¹ groups include halo, OR¹⁹, C(O)OR¹⁹, N(R¹⁹)₂, SO₃R¹⁹, OSO₃R¹⁹ or a 6- to 10-membered aryl group optionally substituted as described above, and more suitable substituents for these R¹³ groups include halo, OR¹⁹, C(O)OR¹⁹, N(R¹⁹)₂, SO₃R¹⁹, OSO₃R¹⁹ or a 6- to 10-membered aryl group optionally substituted as described above.

More suitable substituents for these R¹⁰, R¹¹ and R¹³ groups include halo, C₁₋₄ alkyl, C₁₋₄ haloalkyl, -O-C₁₋₄ alkyl, -O-C₁₋₄ haloalkyl, C(O)OH, SO₂OH, -NH(C₁₋₄ alkyl) or -N(C₁₋₄ alkyl)₂; for example fluoro, chloro, methyl, ethyl, trifluoromethyl, methoxy, ethoxy, trifluoromethoxy, C(O)OH, SO₂OH, amino, methyl amino and dimethylamino.

More suitable substituents for these R¹⁰, R¹¹ and R¹³ groups include halo, C₁₋₄ alkyl, C₁₋₄ haloalkyl, -O-C₁₋₄ alkyl, -O-C₁₋₄ haloalkyl, C(O)OH, SO₂OH, -NH₂, -NH(C₁₋₄ alkyl) or -N(C₁₋₄ alkyl)₂; for example fluoro, chloro, methyl, ethyl, trifluoromethyl, methoxy, ethoxy, trifluoromethoxy, C(O)OH, SO₂OH, amino, methyl amino and dimethylamino.

Suitable substituents for aryl and heteroaryl R¹⁰ and R¹¹ groups include C₁₋₆ alkyl, C₁₋₆ haloalkyl, halo, NO₂, CN, OR¹⁹, SR¹⁹ or N(R¹⁹)₂.

Similarly, suitable subsitutents for aryl and heteroaryl R¹³ groups include C₁₋₆ alkyl, C₁₋₆ haloalkyl, halo, NO₂, CN, OR¹⁹, SR¹⁹ or N(R¹⁹)₂.

More suitable substituents for aryl and heteoraryl R¹⁰ and R¹¹ groups include C₁₋₄ alkyl, C₁₋₄ haloalkyl, halo, OR¹⁹ or N(R¹⁹)₂; and similarly, more suitable substituents for aryl and heteroaryl R¹³ groups include C₁₋₄ alkyl, C₁₋₄ haloalkyl, halo, OR¹⁹ or N(R¹⁹)₂.

Particularly suitable substituents for aryl and heteroaryl R¹⁰, R¹¹ and R¹³ groups include halo, C₁₋₄ alkyl, C₁₋₄ haloalkyl, -O-C₁₋₄ alkyl, -O-C₁₋₄ haloalkyl, -NH(C₁₋₄ alkyl) or -N(C₁₋₄ alkyl)₂.

Specific examples of substituents for aryl and heteroaryl R¹⁰, R¹¹ and R¹³ groups include fluoro, chloro, methyl, ethyl, trifluoromethyl, methoxy, ethoxy, trifluoromethoxy, amino, methyl amino and dimethylamino.

As set out above, with respect to groups R¹⁰ and R¹¹, each R¹⁹ is independently selected H, C₁₋₆ alkyl, C₁₋₆ haloalkyl, or a 6- to 14- membered aryl or 5- to 14-membered heteroaryl group either of which is optionally substituted with one or more substituents selected from halo, C₁₋₆ alkyl and C₁₋₆ haloalkyl.

Suitably, R¹⁹ is H, C₁₋₆ alkyl, C₁₋₆ haloalkyl, or a 6- to 10- membered aryl or 5 to 10-membered heteroaryl group optionally substituted with one or more substituents selected from halo, C₁₋₄ alkyl and C₁₋₄ haloalkyl.

More suitably, R¹⁹ is hydrogen, C₁₋₆ alkyl, C₁₋₆ haloalkyl or phenyl optionally substituted with one or more halo, C₁₋₄ alkyl or C₁₋₄ haloalkyl substituents.

Specific examples of R¹⁹ include H, methyl, ethyl, trifluoromethyl or phenyl optionally substituted with one or more substituents selected from fluoro, chloro, methyl, ethyl and trifluoromethyl.

As set out above, with respect to group R¹³, each R¹⁹ is independently H, C₁₋₆ alkyl or C₁₋₆ haloalkyl. In one embodiment, R¹⁹ is H or C₁₋₆ alkyl such as C₁₋₄ alkyl, for example, methyl or ethyl. Specific examples of R¹⁹ include H, methyl, ethyl or trifluoromethyl.

In some particularly suitable compounds of general formula (Ia)), R⁴ is C(O)OR¹⁰, OR¹⁰, SO₃R¹⁰, OSO₃R¹⁰ halo, CN, azide, OSi(R¹³)₃, NR¹⁰C(O)NR¹⁰SO₂R¹¹, NR¹⁰C(O)NR¹⁰SO₂N R¹⁰R¹¹, NR¹⁰SO₂R¹¹, CH(XR¹⁰)(XR¹¹), CH[C(O)OR¹⁰]₂, BR¹⁰R¹¹ or phthalimide.

In some particularly suitable compounds of general formula (Ia), (IIa), (IIIxa), (IIIya), (IIIxz), (IIIyz), (IVa), (Va), (VIa) and (Xa) R⁴ is C(O)OR¹⁰, OR¹⁰, SO₃R¹⁰, OSO₃R¹⁰ halo, CN, CH(XR¹⁰)(XR¹¹), CH[C(O)OR¹⁰]₂ or BR¹⁰R¹¹;and each R¹⁰ and R¹¹ is independently H, C₁₋₆ alkyl or benzyl; or,
when R⁴ is CH(XR¹⁰)(XR¹¹) or BR¹⁰R¹¹, R¹⁰ and R¹¹ together with the atom or atoms to which they are attached, may combine to form a 3- to 10-membered heterocyclic ring; or R⁴ is C(O)NR¹⁰R¹¹ wherein each R¹⁰ and R¹¹ is independently substituted with C(O)OR¹⁹, OR¹⁹, SO₃R¹⁹, or OSO₃R¹⁹ and R¹⁹ is H.

In some particularly suitable compounds of general formula (I), (II), (IIIx), (IIIy), (IV), (V), (VI) and (X) R⁴ is C(O)OR¹⁰, OR¹⁰, SO₃R¹⁰, OSO₃R¹⁰ halo, CN, CH(OR¹⁰)(OR¹¹), CH[C(O)OR¹⁰]₂ or BR¹⁰R¹¹;and each R¹⁰ and R¹¹ is independently H, C₁₋₆ alkyl or benzyl; or,
when R⁴ is CH(OR¹⁰)(OR¹¹) or BR¹⁰R¹¹, R¹⁰ and R¹¹ together with the atom or atoms to which they are attached, may combine to form a 3- to 10-membered heterocyclic ring; or R⁴ is C(O)NR¹⁰R¹¹ wherein each R¹⁰ and R¹¹ is independently substituted with C(O)OR¹⁹, OR¹⁹, SO₃R¹⁹, or OSO₃R¹⁹ and R¹⁹ is H.

When R⁴ is CH(XR¹⁰)(XR¹¹) and R¹⁰ and R¹¹ together with the atom or atoms to which they are attached combine to form a 3- to 10-membered heterocyclic ring, suitably R⁴ is a 3-5 membered heterocyclic ring, in particular a 5-membered heterocyclic ring e.g. R⁴ is selected from: 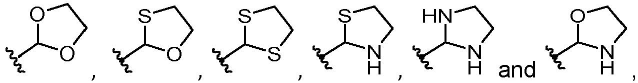 and in particular is

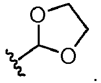

Alternatively, the compound may be in the form of a salt such that:
R⁴ is C(O)O⁻, O⁻, SO₃⁻, or OSO₃⁻; or
R⁴ is C(O)NR¹⁰R¹¹ wherein R¹⁰ and R¹¹ are independently substituted with C(O)O⁻, O⁻, SO₃⁻, or OSO₃⁻;
and a counter ion is present as described above for basic addition salts.

In one embodiment, R⁴ is C(O)OR¹⁰, OR¹⁰, C(O)NR¹⁰R¹¹, SO₃R¹⁰, or OSO₃R¹⁰.

In one embodiment, R⁴ is OSi(R¹³)₃.

In one embodiment, R⁴ is halo, CN, CH(XR¹⁰)(XR¹¹), NR¹⁰R¹¹, BR¹⁰R¹¹, -CH=CH₂, -C≡CH, CH[C(O)OR¹⁰]₂ or CH(BR¹⁰R¹¹)₂ or Y and R⁴ together form a =CH₂ group.

In one embodiment, R⁴ is halo, CN, CH(OR¹⁰)(OR¹¹), NR¹⁰R¹¹,

BR¹⁰R¹¹, -CH=CH₂, -C≡CH, CH[C(O)OR¹⁰]₂ or CH(BR¹⁰R¹¹)₂ or Y and R⁴ together form a =CH₂ group.

In one embodiment, R⁴ is halo, CN, NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azide, NO₂, NR¹⁰C(O)NR¹⁰SO₂R¹¹, C(O)NR¹⁰SO₂R¹¹, CH(XR¹⁰)(XR¹¹), CH(R¹⁰)(XR¹¹) wherein each X is independently O, S or NR⁸.

When R⁴ is CH(XR¹⁰)(XR¹¹), X is suitably O or S, e.g. O. In such compounds, when R¹⁰ and R¹¹ combine to form a ring, it is suitably a 5- or 6-membered ring. More suitably, both X moieties are O and R¹⁰ and R¹¹ form a 1,3-dioxane or 1,3-dioxolane ring.

When R⁴ is CH(R¹⁰)(XR¹¹), X is suitably O or S, e.g. O.

In one embodiment, R⁴ is a carboxylic acid mimetic group.

In one embodiment, R⁴ is a carboxylic acid mimetic group selected from tetrazole, substituted tetrazole, -SO₂-NHR¹⁰, C(O)NH-SO₂R¹⁰ and NHC(O)NH-SO₂R¹⁰;
wherein R¹⁰ is as above defined for a compound of general formulae (Ia) or (I) and is suitably H, C₁₋₆ alkyl, C₃₋₇ cycloalkyl or 6- to 14- membered aryl (e.g. phenyl). Suitably, substituted tetrazole is tetrazole substituted with C₁₋₄ alkyl, halo, OH, O(C₁₋₄ alkyl) or SO₂R¹⁰ (e.g. SO₂(C₁₋₄ alkyl), SO₂-phenyl or SO₂-tolyl).

When R⁴ is a carboxylic acid mimetic group, it is suitably a tetrazolyl group, for example tetrazol-1-yl or tetrazol-5-yl.

In one embodiment, R⁴ is halo, CN, CH(XR¹⁰)(XR¹¹), CH=CH₂, -C≡CH, CH[C(O)OR¹⁰]₂, BR¹⁰R¹¹ or Y and R⁴ together form a =CH₂ group.

In one embodiment, R⁴ is halo, CN, CH(OR¹⁰)(OR¹¹), CH=CH₂, -C≡CH, CH[C(O)OR¹⁰]₂, BR¹⁰R¹¹ or Y and R⁴ together form a =CH₂ group.

Suitably, R⁴ is C(O)OR¹⁰, C(O)NR¹⁰R¹¹, SO₃R¹⁰, or OSO₃R¹⁰.

More suitably, R⁴ is C(O)OR¹⁰, SO₃R¹⁰, or OSO₃R¹⁰ and R¹⁰ is H; or
R⁴ is C(O)NR¹⁰R¹¹ substituted with C(O)OR¹⁹, SO₃R¹⁹, or OSO₃R¹⁹ and R¹⁹ is H.

In other particularly suitable compounds R⁴ is halo, CN, CH(OR¹⁰)(OR¹¹), NR¹⁰R¹¹, CH[C(O)OR¹⁰]₂ or azide;
where R¹⁰ and R¹¹ are as described above but are suitably each independently H or C₁₋₁₀ alkyl, C₂₋₁₀ alkenyl or C₂₋₁₀ alkynyl optionally substituted as described above or, when R⁴ is NR¹⁰R¹¹, R¹¹ may also suitably be a heteroaryl group such as tetrazole; or when R⁴ is CH(OR¹⁰)(OR¹¹), the OR¹⁰ and OR¹¹ groups together with the carbon atom to which they are attached may form a cyclic acetal group, particularly a 1,3-dioxane or 1,3-dioxolane group.

In still other particularly suitable compounds R⁴ is NR¹⁰C(O)NR¹⁰SO₂R¹¹ or C(O)NR¹⁰SO₂R¹¹, where R¹⁰ and R¹¹ are as described above but are suitably each independently H or C₁₋₁₀ alkyl, C₂₋₁₀ alkenyl or C₂₋₁₀ alkynyl optionally substituted as described above.

In one embodiment, R⁴ is C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, NR¹⁰R¹¹, C(O)CH₂N₂, CH[C(O)OR¹⁰]₂, azide, NO₂, NR¹⁰C(O)NR¹⁰SO₂R¹¹, C(O)NR¹⁰SO₂R¹¹, CH(XR¹⁰)(XR¹¹), CH(R¹⁰)(XR¹¹) or a carboxylic acid mimetic group such as tetrazole.

In another embodiment, R⁴ is C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, CH(OR¹⁰)(OR¹¹), CH(R¹⁰)(OR¹¹), CH(SR¹⁰)(SR¹¹), NR¹⁰R¹¹, C(O)CH₂N₂, CH[C(O)OR¹⁰]₂, azide or a carboxylic acid mimetic group such as tetrazole.

In one embodiment, R⁴ is C(O)OR¹⁰, CONR¹⁰R¹¹, OSO₂R¹⁰, OSO₃R¹⁰, CN, azide, OR¹⁰, OSi(R¹³)₃, CH[C(O)OR¹⁰]₂, CH(OR¹⁰)(OR¹¹), NR¹⁰CONR¹⁰SO₂R¹¹ and NR¹⁰SO₂R¹¹ and tetrazole.

In one embodiment, R⁴ is C(O)OR¹⁰, OC(O)R¹⁰, OR¹⁰, OSi(R¹³)₃, OSO₂R¹⁰, halo, CN, NR¹⁰R¹¹, CH[(C(O)OR¹⁰)]₂, azide, C(O)NR¹⁰SO₂R¹¹ CH(XR¹⁰)(XR¹¹); phthalimide, tetrazole or substituted tetrazole.

Other examples of R⁴ groups include azide and tetrazole.

In one embodiment, R⁵ is H. In one embodiment, R⁵ is OH. In one embodiment, R⁵ is a protected OH group. In one embodiment, R⁵ is a protected OH group which is not stable in a basic environment such that treatment with a base converts the protected OH group to OH. Examples of such groups are well known in the art and include a group OC(O)R¹⁴ as defined above in which R¹⁴ is a group R¹⁰ as defined above for general formula (Ia) or formula (I). Particularly suitable R¹⁴ groups are as defined for R¹⁰ above and include C₁₋₆ alkyl such as methyl, or benzyl; or C₁₋₆ alkyl such as methyl, or phenyl. In another embodiment, R⁵ is a protected OH group which is stable in a basic environment. Examples of such groups are well known in the art and include OSi(R¹⁶)₃, where each R¹⁶ is independently a group R¹³ as defined above for general formulae (Ia) and (I), and is suitably C₁₋₆ alkyl or phenyl. In one embodiment, Si(R¹⁶)₃ is selected from the group consisting of trimethylsilyl (TMS), triethylsilyl (TES), triphenylsilyl (TPS), tri-isopropylsilyl (TIPS), thexyldimethylsilyl (TDS), *tert*-butyldiphenylsilyl (TBDPS), *tert*-butyldimethylsilyl (TBDMS or TBS), di-*tert*-butylmethylsilyl (DTBMS), diethylisopropylsilyl (DEIPS) and dimethylisopropylsilyl (DMIPS), in particular TMS, TES, TIPS, TBDMS and TBDPS.

In one aspect of the invention is provided a compound of general formula (Ia) or (I) selected from:
(6β, 7β, 22E)-6,7-epoxy-3-oxo-4,22-choladien-24-oic acid ethyl ester (Example 1);
(6β, 7β, 20S)-6,7-epoxy-3-oxo-4,22-choladien-24-oic acid ethyl ester (Example 5);
(6β, 7β, 20S)-6,7-epoxy-20-hydroxymethyl-pregna-4-en-3-one (Example 41);
(6β, 7β, 20S)-6,7-epoxy-20-bromomethyl-pregna-4-en-3-one (Example 42);
(20S)-methanesulfonyloxymethyl-6,7-β-epoxy-4-pregnen-3-one (Example 43);
(20R)-cyanomethyl-6,7-β-epoxy-4-pregnen-3-one (Example 44);
(20S)-20-acetoxymethyl-6,7-β-epoxy-pregna-4-en-3-one (Example 45);
(6β, 7β, 20S)-6,7-epoxy-20-*tert*-butyldiphenylsiloxymethyl-pregna-4-en-3-one (Example 46);
(6β, 7β, 20S)-6,7-epoxy-20-azidomethyl-pregna-4-en-3-one (Example 47);
(6β, 7β, 20S)-6,7-epoxy-20-(*N*-phthalimidomethyl)-pregna-4,6-dien-3-one (Example 48);
(6β, 7β, 20S)-6,7-epoxy-20-formyl-pregna-4-en-3-one (Example 49);
(6β, 7β, 20S)-6,7-epoxy-20-(ethylenedioxymethyl)-pregna-4-en-3-one (Example 50); and
(6β, 7β)-6,7-epoxy-3-oxo-4-cholen-23-carboxy-24-oic acid dimethyl ester (Example 51);
or a salt or isotopic variant thereof.

### Preparation of compounds of general formula (Ia) and (I)

The epoxidation of dienone (IIA) using *meta*-chloroperoxybenzoic acid (mCPBA) or magnesium monoperoxyphthalate (MMPP) is described by Uekawa et al. in Biosci. Biotechnol. Biochem. 2004, 68, 1332-1337 and is said to yield the alpha-epoxide, as shown in Scheme 1.

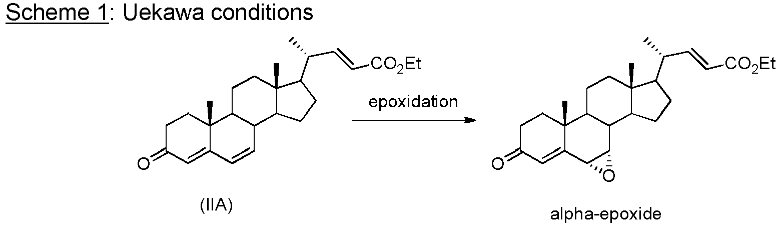

Starting from this alpha-epoxide, the present inventors have developed processes and intermediates for synthesizing obeticholic acid and analogues thereof as described in patent applications Nos. PCT/GB2015/053516 (WO2016/079517), PCT/GB2015/053517 (WO2016/079518), PCT/GB2015/053518 (WO2016/079519) and PCT/GB2015/053519 (WO2016/079520) .

The present inventors have now devised an alternative route to obeticholic acid and its analogues utilising the corresponding beta-epoxide.

The inventors investigated further conditions for the epoxidation of compound (IIA) with a view to forming the beta-epoxide, but found that peroxides (such as mCPBA or MMPP - varying the conditions described in Uekawa *et al*.), dimethyldioxirane (DMDO) and other substituted dioxiranes as well as reactions promoted by various catalysts such as MTO or Mn(II) salts, all produced the alpha-epoxide as a major diastereoisomer. The beta epoxide was observed, but typically in only less than 15% yield.

It is known that epoxides may be formed via reaction of an alkene to form a halohydrin, which then undergoes an intramolecular ring closing reaction upon treatment with a base to form an epoxide. Such a reaction is described by Draper, R. W. in J. Chem. Soc. Perkin Trans. I, 1983, 2781-2786 wherein a 4,6-diene-3-one steroid molecule was reacted with chromyl chloride to form (as a sole product) the 6β-chloro,7α-hydrin:

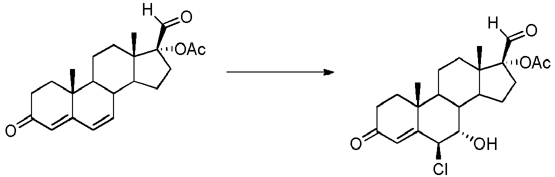

However, when treated with base this compound would form the alpha (down) epoxide.

The present inventors were therefore surprised to find that by reacting a compound of formula (II) with inexpensive and readily available trichloroisocyanuric acid (TCCA) (literature describing use of TCCA: J. Braz. Chem. Soc. 2002, 13 (5), 700-703; Org. Proc. Res. Dev. 2002, 6 (4), 384-393) the beta-epoxide could be accessed in 40% yield *via* forming the 6,7-halohydrin compound (IIIxA) or (IIIyA).

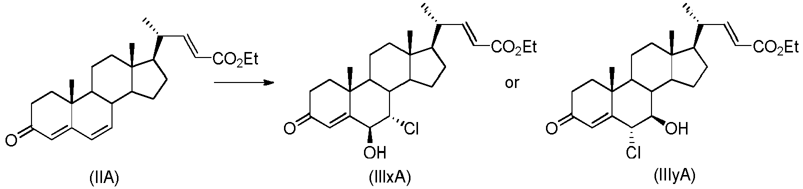

This reaction is described in Example 1.

The halohydrin compound (IIIxA) or (IIIyA) was then cyclized to form the desired beta-epoxide (IA) using a base, 1,8-diazabicycloundec-7-ene (DBU) in 50 % yield. This reaction is described in Example 2.

It should be noted that for the cyclisation reaction to form compound (IA) it is not necessary to identify whether (IIIxA) or (IIIyA) was formed, since both compounds will cyclise under basic conditions to form the same compound (IA).

Thus, in one aspect of the invention is provided a process for preparing a compound of general formula (Ia) comprising the steps of:
(A) reacting a compound of general formula (IIa) with a halogenating agent to give a compound of general formula (IIIxa) and/or a compound of general formula (IIIya): 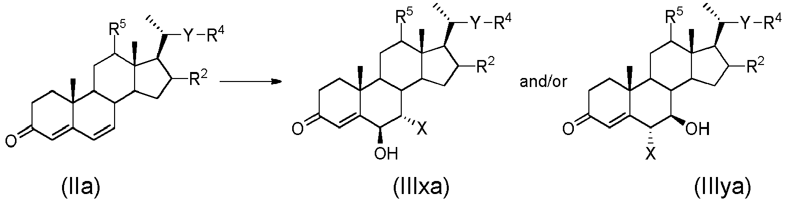
   wherein X is Cl, Br or I;
   and wherein Y, R², R⁴ and R⁵ are as defined for a compound of general formula (Ia); and
(B) reacting a compound of general formula (IIIxa) and/or a compound of general formula (IIIya) with a base to form a compound of general formula (la): 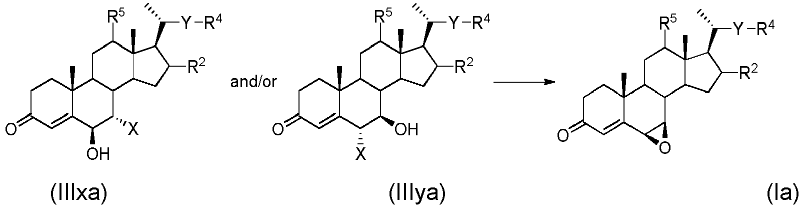
   wherein X is Cl, Br or I;
   and wherein Y, R², R⁴ and R⁵ are as defined for a compound of general formula (Ia).

In another aspect of the invention is provided a process for preparing a compound of general formula (I) comprising the steps of:
(A) reacting a compound of general formula (II) with trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), or 1,3-diiodo-5,5-dimethylhydantoin (DIDMH) to give a compound of general formula (IIIx) and/or a compound of general formula (Illy): 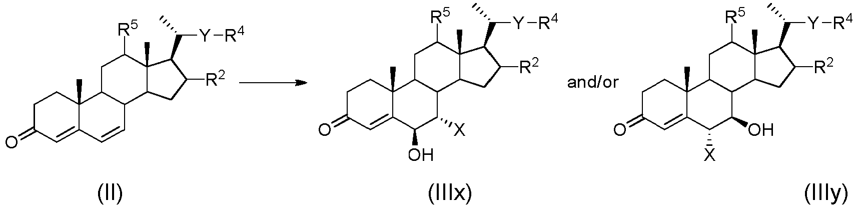
   wherein X is Cl, Br or I;
   and wherein Y, R², R⁴ and R⁵ are as defined for a compound of general formula (I);
   and
(B) reacting a compound of general formula (IIIx) and/or a compound of general formula (Illy) with a base to form a compound of general formula (I): 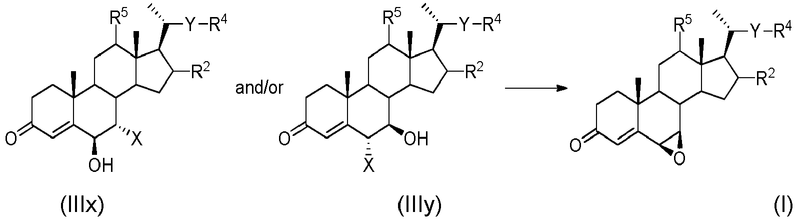
   wherein X is Cl, Br or I;
   and wherein Y, R², R⁴ and R⁵ are as defined for a compound of general formula (I).

Suitably, step (B) is carried out using crude halohydrin intermediates obtained from step (A) without further purification.

Thus, in one aspect of the invention is provided a process for preparing a compound of general formula (Ia) comprising:
reacting a compound of general formula (IIa) with a halogenating agent followed by reaction with base to give a compound of general formula (Ia): 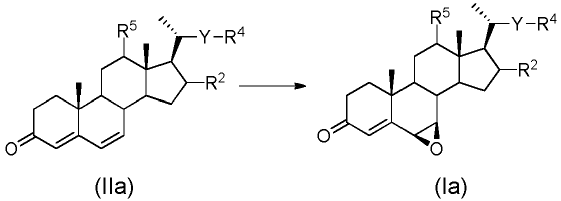 wherein Y, R², R⁴ and R⁵ are as defined for a compound of general formula (Ia) and halogenating agent is as defined above.

In another aspect of the invention is provided a process for preparing a compound of general formula (I) comprising:
reacting a compound of general formula (II) with trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), or 1,3-diiodo-5,5-dimethylhydantoin (DIDMH) followed by reaction with base to give a compound of general formula (I): 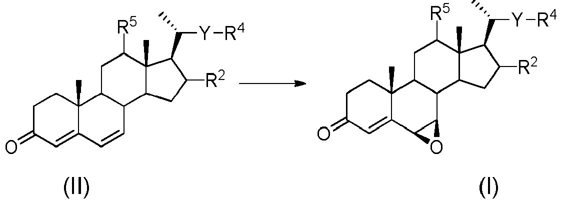 wherein Y, R², R⁴ and R⁵ are as defined for a compound of general formula (I).

References to step (A) and step (B) below apply whether the halohydrin intermediates are isolated and purified, or not.

Suitable halogenating agents are those capable of forming "positive halogen" and include, but are not limited to: Br₂, Cl₂, I₂, *N*-bromosuccinimide (NBS), *N*-chlorosuccinimide (NCS), *N*-iodosuccinimide (NIS), chloramine-T (tosylchloramide), *tert*-butylhypochlorite, trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), or 1,3-diiodo-5,5-dimethylhydantoin (DIDMH).

Suitable halogenating agents also include reagents where Br₂, Cl₂ or I₂ are generated in situ, for example di-*tert*-butyl peroxide with TiCl₄; Ca(OCl)₂ with NaCl in AcOH; or TMSCl with H₂O₂.

Thus, in one embodiment, the halogenating agent is selected from Br₂, Cl₂, I₂, NBS, NCS, NIS, chloramine-T, *tert*-butylhypochlorite, TCCA, TBCA, TICA, DCDMH, DBDMH, DIDMH, di-*tert*-butyl peroxide with TiCl₄, Ca(OCl)₂ with NaCl in AcOH; or TMSCI with H₂O₂. In particular, the halogenating agent is selected from NBS, NCS, NIS, chloramine-T, TCCA, TBCA, TICA, DCDMH, DBDMH and DIDMH, for example, the halogenating agent is selected from TCCA, TBCA, TICA, DCDMH, DBDMH and DIDMH e.g. is selected from TCCA and *tert*-butylhypochlorite, in particular TCCA.

Tribromoisocyanuric acid (TBCA) and triiodoisocyanuric acid (TICA) are equivalents of trichloroisocyanuric acid (TCCA), and have the following structure: 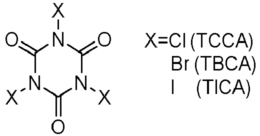 1,3-Dichloro-5,5-dimethylhydantoin (DCDMH), 1,3-dibromo-5,5-dimethylhydantoin (DBDMH), and 1,3-diiodo-5,5-dimethylhydantoin (DIDMH) have the following structure:

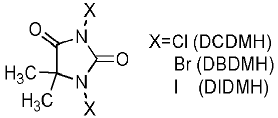

In step (A), suitably the compound of general formula (II) is reacted with TCCA, TBCA or TICA, especially TCCA.

In one embodiment, in step (A) the compound of general formula (II) is reacted with TCCA, TBCA, TICA or *tert*-butylhypochlorite, especially TCCA or *tert*-butylhypochlorite, in particular TCCA.

Suitably, 0.1-2.2 equivalents of halogenating agent are used, for example 0.2-1.5, 0.2-0.9, 0.2-0.6 or about 0.4 equivalents. Thus, in one embodiment, in step (A), 0.1-2.2 equivalents of halogenating agent are used, for example 0.2-1.5, 0.2-0.9, 0.2-0.6 or about 0.4 equivalents

In step (A), typically 0.1-2.2 equivalents of halogenating agent e.g. TCCA, TBCA, TICA, DCDMH, DBDMH or DIDMH, are used, for example 0.1-0.9, 0.2-0.6 or about 0.4 equivalents.

The reaction is suitably carried out in an organic solvent such as acetone, DMF, MeCN or CH₂Cl₂ which may optionally be mixed with a co-solvent such as water and/or an additive such as MeSO₃H or benzoic acid. Other suitable organic solvents include THF, *t*-butyl alcohol, acetic acid, dioxane, DMSO and formic acid. In one embodiment, the reaction is carried out in a solvent selected from acetone, DMF, MeCN or CH₂Cl₂, THF, *t*-butyl alcohol, acetic acid, dioxane, DMSO, formic acid and water, and mixtures thereof. In one embodiment, the reaction solvent is a mixture of acetone and water. In one embodiment, the reaction solvent is neat formic acid. In one embodiment, the reaction solvent is neat acetic acid. In one embodiment, the reaction solvent comprises formic acid or acetic acid.

In one embodiment, the reaction is carried out in the presence of HOC(O)R^{x}, HOR^{y}, or HSO₃R^{z}; wherein R^{x} is H, C₁₋₄ alkyl (e.g. methyl or ethyl), phenyl or benzyl; R^{y} is phenyl, benzyl or C₂₋₄ alkenyl (e.g. allyl); and R^{z} is CF₃ wherein phenyl and benzyl are optionally substituted with one or more substituents selected from C₁₋₄ alkyl, OC₁₋₄ alkyl, halo, nitro, C₁₋₄ haloalkyl and OC₁₋₄ haloalkyl. In one embodiment, HOR^{x}, HOR^{y} or HSO₃R^{z} may be present as an additive in the reaction (suitably in the absence of water and in the presence of an aprotic solvent). In another embodiment, HOR^{x}, HOR^{y} or HSO₃R^{z} is present as the reaction solvent itself.

In embodiments where the reaction is carried out in the presence of HOC(O)R^{x}, HOR^{y}, or HSO₃R^{z} as above defined, in the absence of water or a protic solvent, intermediate compounds of formula (IIIxz) and/or intermediate compounds of formula (IIIyz) (as hereinabove defined) will be formed following treatment of the compound of formula (IIa) or (II) with halogenating agent. In circumstances where group R⁴⁰ is not base labile, the process to form the compound of formula (Ia) or (I) further includes an additional step of removal of the R⁴⁰ group prior to treatment with base. For example, where OR⁴⁰ is Oallyl (formed by reaction of HOR^{y} wherein R^{y} is allyl), the allyl group may be removed by treatment with PdCl₂. Such steps to remove group R⁴⁰ are in effect deprotection steps and are well to those of skill in the art (see Wuts, PGM and Greene, TW (2006) "Greene's Protective Groups in Organic Synthesis", 4th Edition, John Wiley & Sons, Inc., Hoboken, NJ, USA). A further example of such a deprotection step is where OR⁴⁰ is OPMB (PMB = paramethoxybenzyl, formed by reaction of HOR^{y} wherein R^{y} is paramethoxybenzyl), and the PMB group may be removed using DDQ (2,3-dichloro-5,6-dicyano-p-benzoquinone).

Suitably the reaction is carried out at a temperature of between -40 °C and 50 °C, e.g. between 0 °C and room temperature (e.g. 18 °C), or at 0 °C, or at room temperature (e.g. at 18 °C).

In one embodiment, step (A) is the reaction of a compound of general formula (IIa) or a compound of general formula (II) with a halogenating agent e.g. TCCA, TBCA, TICA, DCDMH, DBDMH or DIDMH to give a compound of general formula (IIIxa) or a compound of formula (IIIx), respectively. In one embodiment, step (A) is the reaction of a compound of formula (IIa) or a compound of formula (II) with a halogenating agent e.g. TCCA, TBCA, TICA, DCDMH, DBDMH or DIDMH to give a compound of general formula (IIIyx) or a compound of formula (Illy), respectively. In one embodiment, step (A) is the reaction of a compound of formula (IIa) a compound of formula (II) with a halogenating agent e.g. TCCA, TBCA, TICA, DCDMH, DBDMH or DIDMH to give a mixture of a compound of general formula (IIIxa) and a compound of general formula (IIIya), or a mixture of a compound of general formula (IIIx) and a compound of general formula (Illy), respectively.

In step (B), suitable bases include but are not limited to KOH, NaOH, NaOMe, NaOEt, NaCO₃, K₂CO₃ and non-nucleophilic bases such as *N,N*-diisopropylethylamine (DIPEA), 1,8-diazabicycloundec-7-ene (DBU), and 2,6-di-*tert*-butylpyridine. Suitably, DBU is in CH₂Cl₂, NaOEt is in THF and K₂CO₃ is in EtOH or MeOH. Suitable bases would be well known to the skilled person. In one embodiment, the base is DBU. Suitably 1-2 equivalents such as about 1.5 equivalents of base are used in the reaction.

In one embodiment, step (B) is the reaction of a compound of general formula (IIIxa) or a compound of formula (IIIx) with a base to give a compound of general formula (Ia) or a compound of general formula (I), respectively. In one embodiment, step (B) is the reaction of a compound of formula (IIIya) or a compound of formula (Illy) with a base to give a compound of general formula (Ia) or a compound of formula (I), respectively. In one embodiment, step (B) is the reaction of a mixture of a compound of formula (IIIxa) and a compound of formula (IIIya), or a mixture of a compound of formula (IIIx) and a compound of formula (Illy), with a base to give a compound of general formula (Ia) or a compound of formula (I), respectively.

The reaction is suitably carried out in an organic solvent such as acetone, DMF, MeCN or CH₂Cl₂ which may optionally be mixed with a co-solvent such as water and/or an additive such as MeSO₃H or benzoic acid. Other suitable organic solvents include THF, *t*-butyl alcohol, acetic acid, dioxane, DMSO, formic acid. In one embodiment, the reaction is carried out in a solvent selected from acetone, DMF, MeCN or CH₂Cl₂, THF, *t*-butyl alcohol, acetic acid, dioxane, DMSO, formic acid and water, and mixtures thereof. In one embodiment the reaction solvent is a mixture of acetone and water. In one embodiment, the reaction solvent is neat formic acid. In one embodiment, the reaction solvent is neat acetic acid. Suitably the reaction is carried out at a temperature of between -40 °C and 50 °C, e.g. between 0 °C and room temperature (e.g. 18 °C), or at 0 °C, or at room temperature (e.g. at 18 °C).

In one embodiment, is provided a process for preparing a compound of general formula (I) comprising:
reacting a compound of general formula (II) with trichloroisocyanuric acid (TCCA) or *tert-*butylhypochlorite, or DBDMH (in particular trichloroisocyanuric acid (TCCA) or *tert-*butylhypochlorite) followed by reaction with base to give a compound of general formula (I): 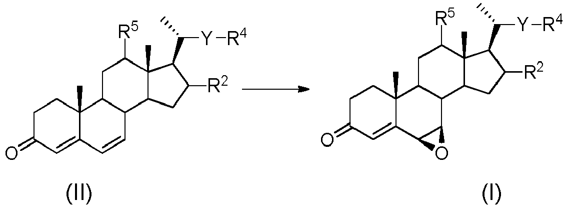
wherein Y, R², R⁴ and R⁵ are as defined for a compound of general formula (I),
wherein the reaction is carried out in a solvent selected from acetone, water, formic acid, acetic acid and mixtures thereof, in particular a mixture of acetone and water, neat formic acid or neat acetic acid.

In this embodiment, prior to reaction with base the halohydrin intermediate(s) is/are isolated but are not purified.

This process for forming the beta-epoxide, in at least some embodiments, is expected to have one or more advantages of:
- good regio- and stereo-selectivity;
- simplified procedure;
- low cost.

### Preparation of compounds of general formula (IIa) and (II)

Compounds of general formula (IIa) or compounds of general formula (II) may be prepared from compounds of general formula (VIIa) or from compounds of general formula (VII), respectively: 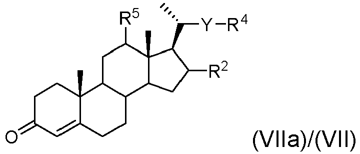
wherein Y, R², R⁴ and R⁵ and are as defined above for general formula (Ia) (for formula (Vlla)) or are as above defined for general formula (I) (for formula (VII));
by reaction with an oxidizing agent such as chloranil.

The reaction may be carried out under acidic conditions, for example in the presence of acetic acid, and in an organic solvent such as toluene.

Some compounds of general formulae (IIa), (II), (VIIa) and (VII) are known. For example Uekawa et al. in Biosci. Biotechnol. Biochem., 2004, 68, 1332-1337 describe the synthesis of (22*E*)-3-oxo-4,22-choladien-24-oic acid ethyl ester (compound (VIIA)) from stigmasterol followed by its conversion to (22*E*)-3-oxo-4,6,22-cholatrien-24-oic acid ethyl ester (referred to herein as compound (IIA)), which has the formula:

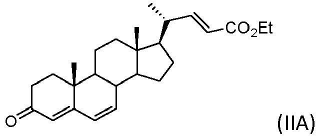

Other compounds of general formulae (IIa), (II), (VIIa), and (VII) may be prepared by analogous methods from phytosterols similar to stigmasterol. Stigmasterol and other phytosterols are plant sterols and are readily available or may be prepared by known routes.

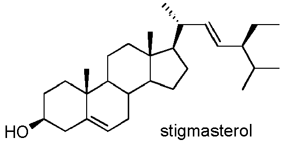

Compounds of general formula (VIIa) or compounds of general formula (VII) may also be prepared from compounds of general formula (VIIIa) or from compounds of general formula (VIII), respectively: 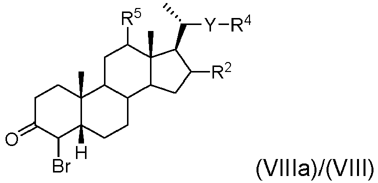
wherein Y, R², R⁴ and R⁵ are as defined in general formula (Ia) (for formula (VIIIa)) or are as defined in general formula (I) (for formula (VIII));
by reaction with lithium bromide and a base such as lithium carbonate. The reaction may be carried out in a solvent such as *N,N*-dimethylformamide (DMF) and at a temperature of about 120 to 180 °C. Such a reaction is described in Example 10 of patent application No. WO2016/079517 .

Compounds of general formula (VIIIa) or compounds of general formula (VIII) may be obtained by bromination of a compound of general formula (IXa), or by bromination of a compound of general formula (IX), respectively: 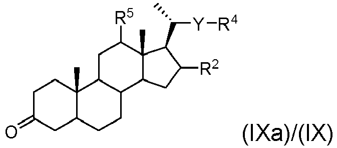
wherein Y, R², R⁴ and R⁵ are as defined in general formula (I) (for formula (IXa)) or are as defined in general formula (Ia) (for formula (IX));
using, for example bromine in acetic acid. Such a reaction is described in Example 9 of patent application No. WO2016/079517.

Compounds of general formula (IXa) or compounds of general formula (IX) may be prepared from compounds of general formula (XIa) or from compounds of general formula (XI), respectively: 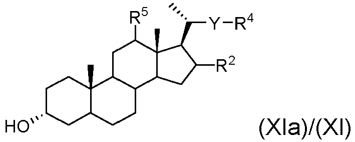
wherein Y, R², R⁴ and R⁵ are as defined in general formula (Ia) (for formula (XIa)) or are as defined in general formula (I) (for formula (XI));
by oxidation, typically with a chromium-based oxidizing agent or with sodium hypochlorite. Such a reaction is described in Example 8 of patent application No. WO2016/079517.

Compounds of general formula (IXa) and compounds of general formula (IX) in which R⁴ is C(O)OR¹⁰, where R¹⁰ is C₁₋₆ alkyl or benzyl, or C₁₋₆ alkyl or phenyl, may be prepared from compounds of general formula (IXa) and from compounds of general formula (IX), respectively, in which R⁴ is C(O)OH by esterification, typically by reaction with an appropriate alcohol under acidic conditions.

Compounds of general formula (XIa) and compounds of general formula (XI) in which R⁴ is C(O)OH and R⁵ is H may be prepared from compounds of general formula (XIIa) and from compounds of general formula (XII), respectively: 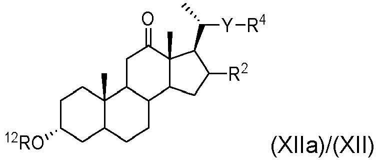
wherein R² and Y are as defined in general formula (Ia) (for formula (XIIa)) and are as defined in general formula (I) (for formula (XII));
R⁴ is C(O)OR¹⁰, where R¹⁰ is C₁₋₆ alkyl or benzyl; and
OR¹² is a protected OH;
by reaction with a reducing agent, typically hydrazine, under basic conditions and in an alcoholic or glycolic solvent, for example diethylene glycol.

Where OR¹² is a protected OH group which is stable under basic conditions, the reaction may be followed by a reaction to remove the protecting group R¹² to leave an OH group.

Protecting groups for OH are discussed above and, for example, R¹² may be a group C(O)R¹⁴, where R¹⁴ is as defined above, in particular, C₁₋₆ alkyl or benzyl; or C₁₋₆ alkyl or phenyl. Silyl ethers are also suitable, and in this case, R¹² may be a group Si(R¹⁶)₃, where each R¹⁶ is independently a group R¹³ as defined above but is especially C₁₋₆ alkyl or phenyl. Other suitable protecting groups for OH are well known to those of skill in the art (see Wuts, PGM and Greene, TW (2006) "Greene's Protective Groups in Organic Synthesis", 4th Edition, John Wiley & Sons, Inc., Hoboken, NJ, USA.

Particularly suitable R¹² groups include groups which are not stable in the presence of a base since this removes the need for the additional step of removing the protecting group. An example of a group R¹² which is not stable in basic conditions is a group C(O)R¹⁴, where R¹⁴ is as defined above, and is particularly C₁₋₆ alkyl or benzyl, or C₁₋₆ alkyl or phenyl.

Alternatively, the reaction may be carried out in 2 steps such that the compound of general formula (XIIa) or a compound of formula (XII) is reacted with a compound of general formula (XXXII):

R²⁰-NH-NH₂ (XXXII)

wherein R²⁰ is a leaving group such as toluene sulfonyl or methane sulfonyl;
to give a compound of general formula (XXXIIIa) or a compound of general formula (XXXIII), respectively: 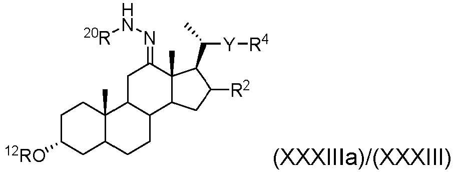
wherein R² and Y are as defined in general formula (Ia);
R⁴ and R¹² are as defined for general formula (Xlla); and
R²⁰ is as defined for general formula (XXXII); (all for formula (XXXIIIa)); or
wherein R² and Y are as defined in general formula (I);
R⁴ and R¹² are as defined for general formula (VII); and
R²⁰ is as defined for general formula (XXXII) (all for formula (XXXIII));
followed by reduction with a suitable reducing agent. Examples of reducing agents which can be used in this reaction include hydrides such as sodium borohydride, sodium cyanoborohydride, lithium aluminum hydride etc. In general formula (XXXIIIa) and in general formula (XXXIII) R²⁰ is as defined above for compounds of general formula (XXXII) and Y, R², R⁴ and R¹² are as defined above for compounds of general formula (XIIa) and for compounds of general formula (XII), respectively.

Compounds of general formula (XIIa) or compounds of general formula (XII) may be prepared from compounds of general formula (XIIIa) or from compounds of general formula (XIII), respectively: 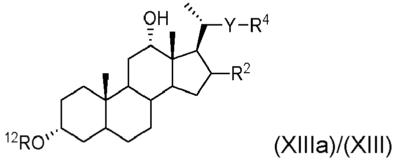
wherein R² and Y are as defined in general formula (Ia) (for formula (XIIIa)) or are as defined in general formula (I) (for formula (XIII));
R⁴ is C(O)OR¹⁰, where R¹⁰ is C₁₋₆ alkyl or benzyl; and
R¹² is as defined above for general formula (XIIa) (for formula (XIIIa)) or is as defined above for general formula (XXII) (for formula (XIII); and is suitably -C(O)C₁₋₆ alkyl;
by reaction with an oxidizing agent, for example sodium hypochlorite. Such a reaction is described in Example 5 of patent application No. WO2016/079517.

The reaction may be carried out under acidic conditions, for example in the presence of acetic acid, and in an organic solvent such as ethyl acetate.

Compounds of general formula (XIIIa) or compounds of general formula (XIII) may be prepared from compounds of general formula (XIVa) or from compounds of general formula (XIV), respectively: 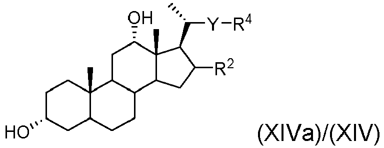
wherein R² and Y are as defined in general formula (Ia) (for formula (XIVa)) or are as defined in general formula (I) (for formula (XIV));
R⁴ is C(O)OR¹⁰, where R¹⁰ is C₁₋₆ alkyl or benzyl;
by reaction with an agent suitable to introduce the protecting group R¹². For example, when R¹² is C(O)R¹⁴, the compound of general formula (XIXa) or the compound of general formula (XIX) may be reacted with a carboxylic acid anhydride or an acid chloride in the presence of a weak base such as pyridine, suitably catalysed by 4-dimethylaminopyridine (DMAP). The reaction may be conducted in a solvent such as ethyl acetate. Such a reaction is described in Example 5 of patent application No. WO2016/079517.

Compounds of general formula (XIVa) or compounds of general formula (XIV) may be prepared by the esterification of compounds of general formula (XVa) or of compounds of general formula (XV), respectively: 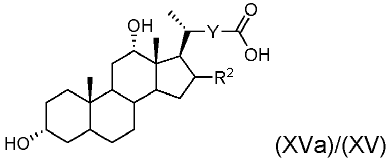 wherein R² and Y are as defined in general formula (Ia) and general formula (I).

The esterification reaction may be carried out by reacting the acid of general formula (XVa) or of general formula (XV) with a suitable alcohol under acidic conditions. Such a reaction is described in Example 5 of patent application No. PCT/GB2015/053516 .

Compounds of general formula (XVa) and of general formula (XV) are known. For example, the compound of general formula (XVa) or of general formula (XV) in which Y is -CH₂CH₂- and R² is H is deoxycholic acid (referred to herein as compound (XVA)), which is readily available from a number of sources.

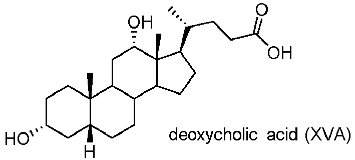

An alternative route to compounds of general formula (VIIa) and to compounds of general formula (VII) in which the group at the R⁴ position is an ester is as shown in Scheme 2 in which 4-androstenedione is converted to a compound of general formula (VIIa) or of general formula (VII) in which R² and R⁵ are H; R⁴ is -C(O)OCH₃ and Y is either -CH₂CH₂-or -CH=CH-.

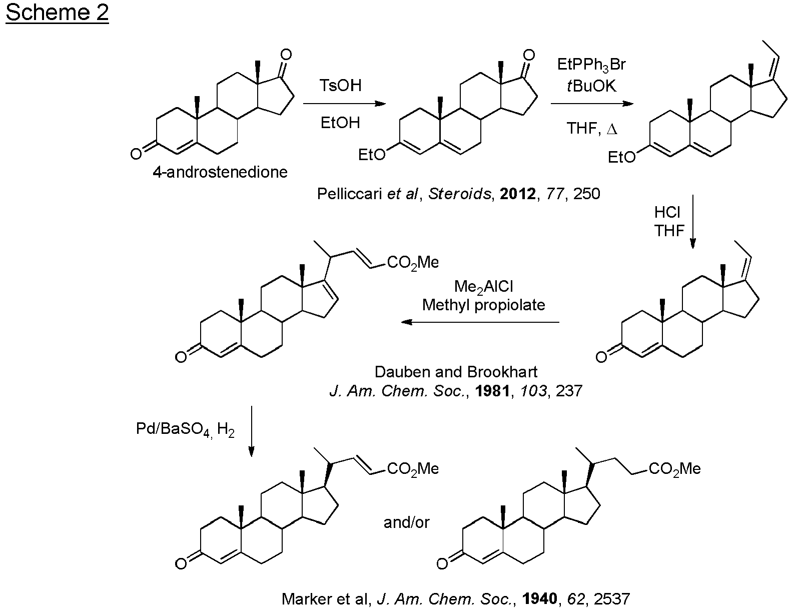

Other compounds with different values for Y and R² can be used as alternative starting materials.

An alternative route to compounds of general formula (IIa) and to compounds of general formula (II) in which Y is an alkenylene group is by use of an olefination reaction, for example a Horner-Wadsworth-Emmons (HWE) olefination of a compound of general formula (XVIa) or of a compound of general formula (XVI), respectively: 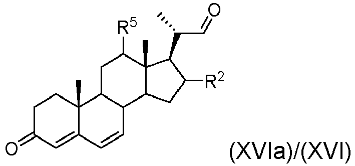 wherein R² and R⁵ are as defined for general formula (Ia) and for general formula (I); using a compound of general formula (XVII): 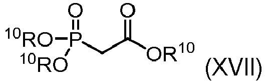 wherein R¹⁰ is as defined for general formula (I).

The reaction may be carried out under standard HWE conditions, for example using a base such as sodium hydride.

Alternatively, an HWE olefination may be used to convert a compound of formula (XI), (IX), (VIII), (VII) and (II).in which R⁴ is C(O)H to a compound in which Y is an alkenylene group.

Compounds of general formula (XVII) are readily available or may be prepared by methods known to those of skill in the art.

Other olefination reactions, such as Tebbe olefination, Wittig type olefination or a Julia-Kocienski olefination, would also give rise to compounds of general formula (IIa) and to compounds of formula (II) in which Y is an alkenylene group. These olefination reactions are familiar to a chemist of skill in the art.

Compounds of general formula (XVIa) or compounds of general formuala (XVI) may be prepared by reaction of a compound of general formula (XVIIIa) or a compound of general formula (XVIII), respectively, with ozone 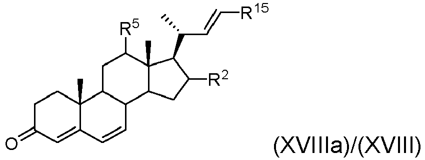 wherein R² and R⁵ are as defined for general formula (Ia) and for general formula (I) and R¹⁵ is C₁₋₆ alkyl.

An example of a reaction of this type is given in patent US2,624,748A (Levin et al. ).

Compounds of general formula (XVIIIa) or compound of general formula (XVIII) may be prepared by reaction of a compound of general formula (XIXa) or a compound of general formula (XIX), respectively: 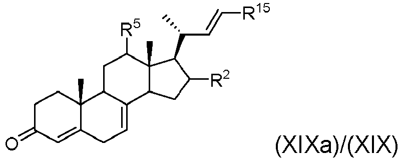
wherein R² and R⁵ are as defined for general formula (Ia) and general formula (I), and R¹⁵ is C₁₋₆ alkyl,
with an acid in a solvent such as methanol.

Compounds of general formula (XIXa) or compounds of general formula (XIX) may be prepared by oxidation of a compound of general formula (XXa) or a compound of general formula (XX), respectively: 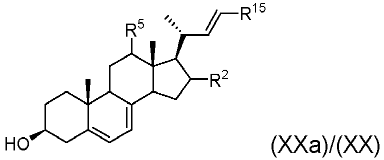 wherein R² and R⁵ are as defined for general formula (Ia) and for general formula (I), and R¹⁵ is C₁₋₆ alkyl, using an Oppenauer oxidation.

Examples of the conversion of compounds of general formula (XXa) to compounds of general formula (XVIIIa) and of the conversion of compounds of general formula (XX) to compounds of general formula (XX) are taught by Shepherd et al, J. Am. Chem. Soc. 1955, 77, 1212-1215 and Goldstein, J. Med. Chem. 1996, 39, 5092-5099.

One example of a compound of general formula (XXa) and of general formula (XX) is ergosterol (referred to herein as (XXA)), which is a fungal sterol and Scheme 3 below shows the conversion of ergosterol to a compound of general formula (II) in which both R² and R⁵ are H, Y is CH=CH₂ and R⁴ is C(O)OR¹⁰, where R¹⁰ is ethyl.

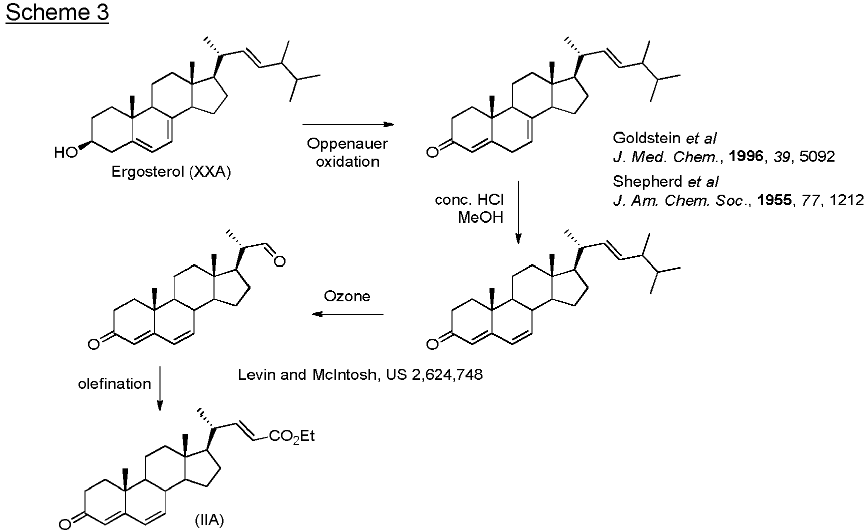

Compounds of general formula (Ia) and (IIa) and compounds of general formula (I) and (II) in which R⁴ is C(O)R¹⁰, C(O)NR¹⁰R¹¹, S(O)R¹⁰, SO₃R¹⁰, or OSO₃R¹⁰ may be prepared from the corresponding compounds in which R⁴ is C(O)OR¹⁰ by reaction with an appropriate reagents using methods well known to those of skill in the art. For example, the methods described in WO2008/002573 and WO2010/014836 or methods similar to those described by Classon et al, J. Org. Chem., 1988, 53, 6126-6130 and Festa et al, J. Med. Chem., 2014, 57, 8477-8495.

### Subsequent reactions of compounds of general formula (IA) and (I)

*Process to form obeticholic acid and obeticholic acid analogues from a compound of general formula (Ia) and general formula (I)*

Compounds of general formula (Ia) and of general formula (I) are of use in the synthesis of compounds of general formula (Xa) and of compounds of general formula (X), respectively: 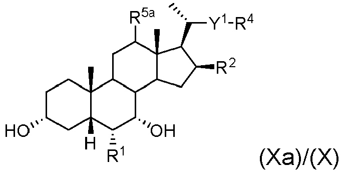 or a salt or an isotopic variant thereof;
wherein,
R¹ is C₁₋₄ alkyl, C₂₋₄ alkenyl or C₂₋₄ alkynyl optionally substituted with one or more substituents selected from halo, OR⁶ and NR⁶R⁷ (for formula (Xa); or
R¹ is C₁₋₄ alkyl optionally substituted with one or more substituents selected from halo, OR⁶ and NR⁶R⁷ (for formula (X));
   wherein each of R⁶ and R⁷ is independently H or C₁₋₄ alkyl;
R² is H, halo or OH; and
Y¹ is a bond, or a C₁₋₂₀ alkylene linker group which is optionally substituted with one or more R³; or
Y¹ and R⁴ together form a =CH₂ group;
R^{5a} is H or OH;
wherein R³ and R⁴ are as described above for a compound of general formula (Ia) (for formula (Xa)) or are as described above for a compound of general formula (I) (for formula (X)).

Compounds of general formula (Xa) and of formula (X) are potent agonists of FXR and TGR5 and include obeticholic acid, which is a compound of formulae (Xa) and (X) in which R¹ is ethyl, R² and R^{5a} are both H, Y¹ is -CH₂CH₂-, and R⁴ is C(O)OH.

The compounds of general formula (Ia) or compounds of general formula (I) may be converted to the compounds of general formula (Xa) or compounds of general formula (X), respectively, in a 4 step process via intermediates of general formulae (IVa), (IV), (Va), (V), (VIa) and (VI) as described below.

In one aspect of the invention is provided a process for preparing a compound of general formula (Xa) from a compound of formula (Ia): 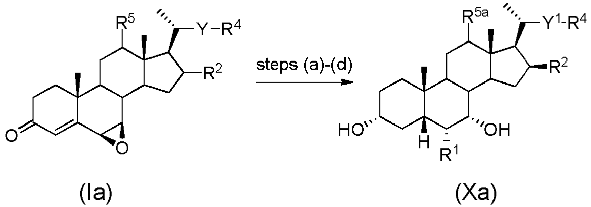 the process comprising the steps of:
(a) selective alkylation of a compound of general formula (Ia) with an organometallic regent to give a compound of general formula (IVa): 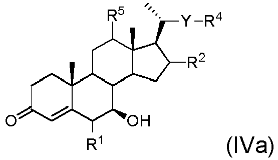
   wherein R¹ is as defined for a compound of formula (Xa);
   and Y, R², R⁴ and R⁵ are as defined for a compound of general formula (Ia);
(b) reducing the compound of general formula (IVa) using a suitable reducing agent to give a compound of general formula (Va): 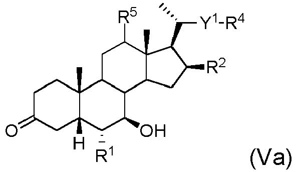
   wherein R¹ and Y¹ are as defined for a compound of formula (Xa);
   and R², R⁴ and R⁵ are as defined for a compound of general formula (Ia);
(c) oxidising the compound of general formula (Va) using a suitable oxidising agent to give a compound of general formula (VIa): 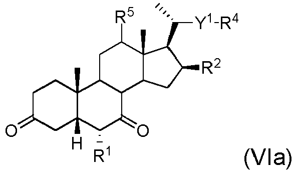
   wherein R¹ and Y¹ are as defined for a compound of formula (Xa);
   and R², R⁴ and R⁵ are as defined for a compound of general formula (Ia);
(d) reduction of the compound of general formula (VIa) using a suitable reducing agent and, where R² and/or R⁵ is a protected OH, removal of the protecting group(s), to give a compound of general formula (Xa) as defined above, wherein removal of the protecting group can take place before or after the reduction.

In another aspect of the invention is provided a process for preparing a compound of general formula (X) from a compound of formula (I): 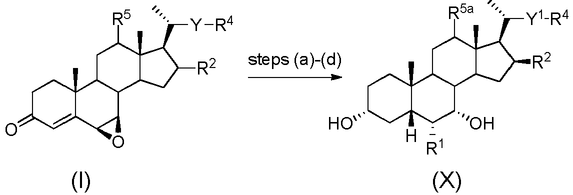 the process comprising the steps of:
(a) selective alkylation of a compound of general formula (I) with an organometallic regent to give a compound of general formula (IV): 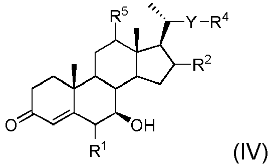
   wherein R¹ is as defined for a compound of formula (X);
   and Y, R², R⁴ and R⁵ are as defined for a compound of general formula (I);
(b) reducing the compound of general formula (IV) using a suitable reducing agent to give a compound of general formula (V): 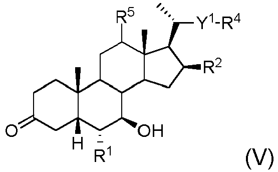
   wherein R¹ and Y¹ are as defined for a compound of formula (X);
   and R², R⁴ and R⁵ are as defined for a compound of general formula (I);
(c) oxidising the compound of general formula (V) using a suitable oxidising agent to give a compound of general formula (VI): 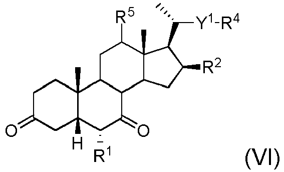
   wherein R¹ and Y¹ are as defined for a compound of formula (X);
   and R², R⁴ and R⁵ are as defined for a compound of general formula (I);
(d) reduction of the compound of general formula (VI) using a suitable reducing agent and, where R² and/or R⁵ is a protected OH, removal of the protecting group(s), to give a compound of general formula (X) as defined above, wherein removal of the protecting group can take place before or after the reduction.

An example of step (a) is shown in Example 6, and example of step (b) in shown in Example 7, and an example of step (c) is shown in Example 8.

In one embodiment is provided a compound of general formula (IVa): 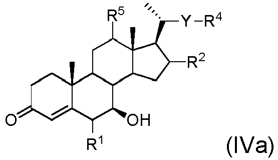
wherein R¹ is as defined for a compound of formula (Xa);
and Y, R², R⁴ and R⁵ are as defined for a compound of general formula (Ia). In the compound of general formula (IVa) suitably R⁴ is C(O)OR¹⁰, CONR¹⁰R¹¹, OSO₂R¹⁰, OSO₃R¹⁰, CN, azide, OR¹⁰, OSi(R¹³)₃, CH[C(O)OR¹⁰]₂, CH(OR¹⁰)(OR¹¹), NR¹⁰CONR¹⁰SO₂R¹¹, NR¹⁰SO₂R¹¹ or tetrazole.

In one embodiment is provided a compound of general formula (Va): 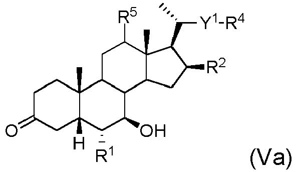
wherein R¹ and Y¹ are as defined for a compound of formula (Xa);
and R², R⁴ and R⁵ are as defined for a compound of general formula (Ia). In the compound of general formula (Va) suitably R⁴ is C(O)OR¹⁰, CONR₁₀R₁₁, OSO₂R¹⁰, OSO₃R¹⁰, CN, azide, OR¹⁰, OSi(R¹³)₃, CH[C(O)OR¹⁰]₂, CH(OR¹⁰)(OR¹¹), NR¹⁰CONR¹⁰SO₂R¹¹, NR¹⁰SO₂R¹¹ or tetrazole.
(5β, 6α, 7β)-6-ethyl-7-hydroxy-3-oxo-cholan-24-oic acid ethyl ester (a compound of formula (V)/(Va) prepared as described above (steps (a) and (b)) may be used in the synthesis of (3α, 5β, 6α, 7β)-6-ethyl-3,7-dihydroxy-cholan-24-oic acid, the 7-beta-hydroxy isomer of obeticholic acid (OCA). This is described in Example 21.

Compounds of general formula (Xa) and of general formula (X) are potent agonists of FXR and TGR5 and include, in particular, compounds in which R¹ is ethyl. Also included are the following.
- Compounds in which R⁴ is C(O)OH, for example:
   ▪ obeticholic acid, which is a compound of formula (Xa)/(X) in which R¹ is ethyl, R² and R^{5a} are both H, Y¹ is -CH₂CH₂-, and R⁴ is C(O)OH; and
   ▪ the compound of formula (Xa)/(X) in which R¹ is ethyl, R² and R^{5a} are both H, Y¹ is -CH₂CH(CH₃)-, and R⁴ is C(O)OH; and
   ▪ the compound of formula (Xa)/(X) in which R¹ is ethyl, R² is H, R^{5a} is OH, Y¹ is -CH₂CH(CH₃)-, and R⁴ is C(O)OH.
- Compounds in which R⁴ is OSO₃H or a salt thereof, for example:
   ▪ the compound of formula (Xa)/(X) in which R¹ is ethyl, R² and R^{5a} are both H, Y¹ is -CH₂CH₂-, and R⁴ is OSO₃H or a salt thereof; and
   ▪ the compound of formula (Xa)/(X) in which R¹ is ethyl, R² is H, R^{5a} is OH, Y¹ is -CH₂CH₂CH₂-, and R⁴ is OSO₃H or a salt thereof; and
   ▪ the compound of formula (Xa)/(X) in which R¹ is ethyl, R² is OH, R^{5a} is H, Y¹ is -CH₂CH₂-, and R⁴ is OSO₃H or a salt thereof.

In the compounds of general formulae (Ia), (Xa), (IVa), (Va) and (VIa) and of general formula (I), (X), (IV), (V) and (VI), more suitable values for R⁴ are as defined for general formula (Ia) and general formula (I), respectively.

In some compounds of general formulae (Xa), (Va) and (VIa) or of general formulae (X), (V) and (VI), Y¹ is a bond.

In other compounds of general formulae (Xa), (Va) and (VIa) or of general formulae (X), (V) and (VI), Y¹ is a C₁₋₁₅ alkylene linker group, more suitably C₁₋₁₂, C₁₋₁₀ or C₁₋₈ alkylene linker group and optionally substituted with one or more R³ as defined above. Typically each R³ is independently halo, OR⁸ or NR⁸R⁹; where each of R⁸ and R⁹ is independently selected from H, methyl or ethyl, especially H or methyl.

In some suitable compounds of general formulae (Xa), (Va) and (VIa) or of general formulae (X), (V) and (VI), Y¹ is an unsubstituted C₁₋₁₅ alkylene or C₂₋₁₅ alkenylene linker, more suitably C₁₋₁₂ alkylene, C₁₋₁₀ alkylnene or C₁₋₈ alkylene, or C₂₋₁₂ alkenylene, C₁₋₁₀ alkenylnene or C₁₋₈ alkenylene.

In suitable compounds of general formulae (Ia), (Xa), (IVa), (Va) and (VIa) or of general formulae (I), (X), (IV), (V) and (VI), R¹ may be C₁₋₄ alkyl optionally substituted with one or more substituents selected from halo, OR⁶ or NR⁶R⁷, where R⁶ and R⁷ are each independently H, methyl or ethyl, especially H or methyl. More suitably, R¹ is unsubstituted C₁₋₄ alkyl.

### Step (a)

Compounds of general formula (IVa) may be prepared from compounds of general formula (Ia): 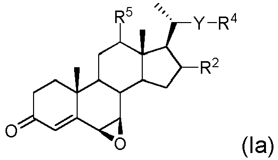 wherein R², R⁴, R⁵ and Y are as defined above;
by selective alkylation with an organometallic reagent, to give a compound of formula: 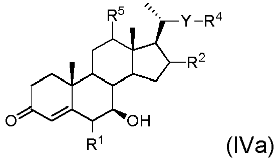 Suitably, the compound of general formula (Ia) is 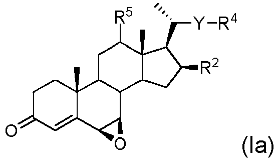 wherein R², R⁴, R⁵ and Y are as defined above.

Compounds of general formula (IV) may be prepared from compounds of general formula (I): 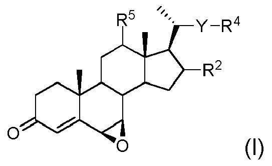 wherein R², R⁴, R⁵ and Y are as defined above;
by selective alkylation with an organometallic reagent, to give a compound of formula:

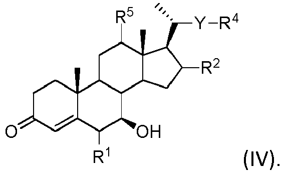

Suitably, the compound of general formula (I) is 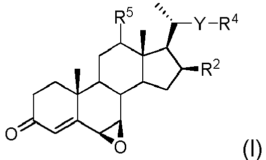 wherein R², R⁴, R⁵ and Y are as defined above.

Suitable organometallic reagents include Gilman reagents formed by reaction of an alkyl lithium compound of formula (XXI):

R¹-Li (XXI)

wherein R¹ is as defined for general formula (Xa) or (X);
and a copper (I) salt, particularly a copper (I) halide such as copper (I) iodide.

The reaction may be conducted in an organic solvent such as tetrahydrofuran, other ethers such as diethylether or a mixture thereof.

Alternatively, the addition can be carried out using Grignard reagents R¹MgX, where R¹ is as defined for general formula (Xa) or (X), and X is a halide, for example ethylmagnesium bromide and the reaction is suitably conducted in the presence of a zinc (II) salt such as zinc chloride and a catalytic amount of a copper (I) or copper(II) salt or complex, for example copper (I) chloride, copper (II) chloride or a copper(I) or copper (II) acetylacetonate (acac) complex.

The reaction may be carried out in an organic solvent, for example an ether such as THF, 2-methyl THF, methyl *tert*-butyl ether (TBME), diethyl ether. Surprisingly, the reaction temperature is not particularly significant and while in some cases the reaction may be carried out at reduced temperature, for example at about -25 to 0 °C, it has also been successfully conducted at higher temperatures of up to about 55 °C.

The method is particularly suitable for the preparation of compounds of general formula (IVa) or compounds of general formula (IV) in which R⁴ is C(O)OR¹⁰ from compounds of general formula (Ia) or from compounds of general formula (I), respectively, where R⁴ is also C(O)OR¹⁰, where R¹⁰ is as defined above but is especially H, C₁₋₆ alkyl or benzyl.

Compounds of general formula (IVa) or of general formula (IV) with other R⁴ groups may be prepared from the above compounds of general formula (IVa) or compounds of general formula (IV), respectively, by methods which are familiar to those of skill in the art, as described below.

A representative method of forming a compound of formula (IVa) or a compound of general formula (IV) is described in Example 6, Example 10 and Example 52.

In one embodiment, the compound of formula (IVa) is: 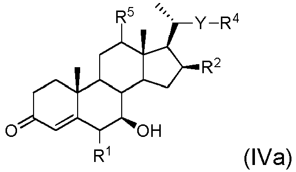
wherein R¹ is as defined above for compounds of general formula (Xa);
and Y, R², R⁴ and R⁵ are as defined above for compounds of general formula (Ia).

In one embodiment, the compound of formula (IVa) is: 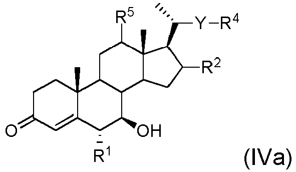
wherein R¹ is as defined above for compounds of general formula (Xa);
and Y, R², R⁴ and R⁵ are as defined above for compounds of general formula (Ia).

In one embodiment, the compound of formula (IVa) is: 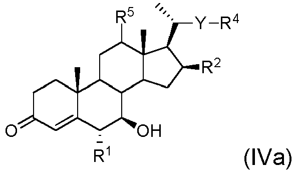
wherein R¹ is as defined above for compounds of general formula (Xa) and Y, R², R⁴ and
R⁵ are as defined above for compounds of general formula (Ia).

In one embodiment, the compound of formula (IV) is: 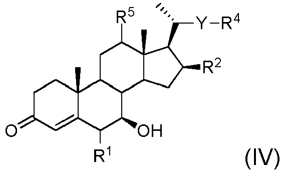
wherein R¹ is as defined above for compounds of general formula (X);
and Y, R², R⁴ and R⁵ are as defined above for compounds of general formula (I).

In one embodiment, the compound of formula (IV) is: 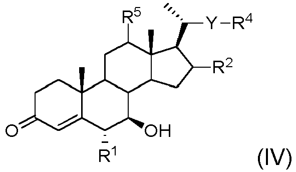
wherein R¹ is as defined above for compounds of general formula (X);
and Y, R², R⁴ and R⁵ are as defined above for compounds of general formula (I).

In one embodiment, the compound of formula (IV) is: 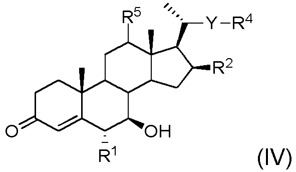
wherein R¹ is as defined above for compounds of general formula (X) and Y, R², R⁴ and
R⁵ are as defined above for compounds of general formula (I).

### Step (b)

The conversion of the compound of general formula (IVa) or the compound of general formula (IV) to the compound of general formula (Va) or to the compound of general formula (V) may be carried out by hydrogenation, usually catalytic hydrogenation. Suitable catalysts for the catalytic hydrogenation include a palladium/carbon, palladium/calcium carbonate, palladium/aluminium oxide, platinum/palladium or Raney nickel catalyst. The reaction may be carried out in an organic solvent, which may be an alcoholic solvent such as methanol, ethanol or isopropanol; ethyl acetate; pyridine; acetic acid; cyclopentyl methyl ether (CPME), acetonitrile (MeCN) or *N,N*-dimethylformamide (DMF). The organic solvent may optionally be mixed with a co-solvent such as acetone or water and/or a base such as triethylamine may also be added.

The choice of catalyst and solvent affects the ratio of the required product of general formula (Va) or general formula (V): 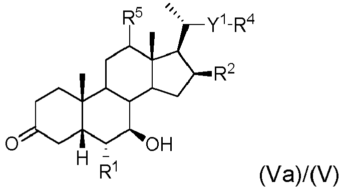 to its isomer of general formula (XXXa) or general formula (XXX):

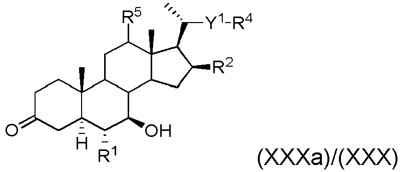

More suitably, a palladium/carbon or palladium/calcium carbonate catalyst is used. Typically, in the catalyst the palladium is present in an amount of 5-10% by weight with respect to the weight of the matrix (where the matrix is the carbon, calcium carbonate etc.).

Particularly suitable solvents and catalysts used for the reaction included a mixture of DMF and MeCN with a palladium/calcium carbonate catalyst and DMF with a palladium/carbon catalyst.

Hydrogenation of a compound of formula (IVa) or a compound of formula (IV) will also reduce any alkene bonds, if present, in the linker Y.

A representative method of forming a compound of general formula (Va) or a compound of general formula (V) is described in Example 7, Example 11 and Example 53.

### Step (c)

The oxidation reaction of a compound of general formula (Va) to a compound of general formula (VIa) or of a compound of general formula (V) to a compound of general formula (VI) may be carried out using any suitable method. One suitable method is a Dess-Martin periodinane (1,1,1-triacetoxy-1,1-dihydro-1,2-benziodoxol-3-(1H)-one) oxidation, which may be carried out in a chlorinated solvent such as chloroform or dichloromethane at a temperature of about 15 to 25 °C, suitably at room temperature.

An alternative oxidation method is oxidation using a hypochlorite, for example sodium hypochlorite, under acidic conditions, for example provided by acetic acid. The reaction may be carried out in an aqueous solvent and at a temperature of 0 to 15 °C, more usually at about 0 to 10 °C.

Other oxidation methods include a Jones reaction using sodium dichromate or, more usually, chromic trioxide in dilute sulfuric acid. This process is known to be reliable for the clean conversion of bile acid hydroxyl groups to the corresponding keto derivatives (Bortolini et al, J. Org. Chem., 2002, 67, 5802).

Alternatively oxidation may be carried out using TEMPO ((2,2,6,6-tetramethyl-piperidin-1-yl)oxy) or a derivative thereof.

A representative example of such a process is described in Example 8, Example 12 and Example 54.

### Step (d)

The reduction of a compound of general formula (VIa) or a compound of general formula (VI) to form a compound of general formula (Xa) or a compound of formula (X), respectively, utilises a reducing agent which is typically a hydride, such as sodium borohydride which may be used in a solvent such as a mixture of tetrahydrofuran and water. Typically, this reaction is carried out under basic conditions, for example in the presence of a strong base such as sodium or potassium hydroxide and at a temperature of about 0 to 110°C, more usually 60 to 100 °C. A compound of general formula (Xa) or a compound of formula (X) in which R⁴ is C(O)OH may be produced by the reduction of a compound in which R⁴ is C(O)OH.

The process further includes one or more optional steps of converting compounds of general formulae (Ia), (IVa), (Va), (VIa) and (Xa) to other compounds of general formulae (Ia), (IVa), (Va), (VIa) and (Xa), or one or more optional steps of converting compounds of general formulae (I), (IV), (V), (VI) and (X) to other compounds of general formulae (I), (IV), (V), (VI) and (X).

The optional steps consist of reacting the side chains of the compounds of general formulae (Ia), (IVa), (Va), (VIa) and (Xa) or of the compounds of general formulae (I), (IV), (V), (VI) and (X) as described below to arrive at compounds with alternative Y and/or R⁴ moieties.

It should be noted that embodiments described above with respect to different R groups apply equally to the process embodiments just described.

### Side chain conversions

The various side chain Y-R⁴ groups of compounds of formulae (Ia), (IIa), (IIIxa), (IIIya), (IIIxz), (IIIyz), and, (IVa)-(XIa) and of compounds of formulae (I), (II), (IIIx), (IIIy) and, (IV)-(XI) may be prepared using conversion steps which are well known to the skilled person e.g. by reactions involving a side chain carboxylic acid, ester, OH or protected OH group. Analogues of the compounds of formulae (Va), (VIa), (V), (VI) and (X) in which a saturated side chain Y¹-R⁴ is converted to an unsaturated side chain Y-R⁴ may also be prepared by these methods as described in more detail below.

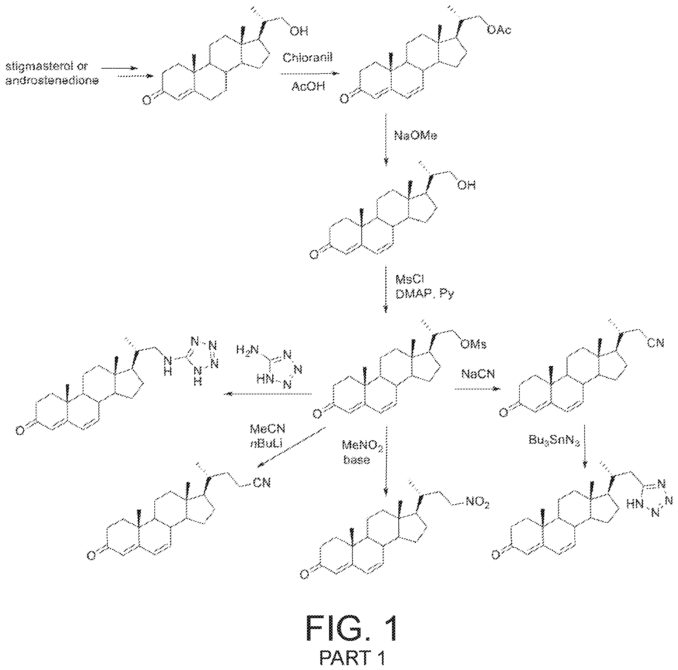

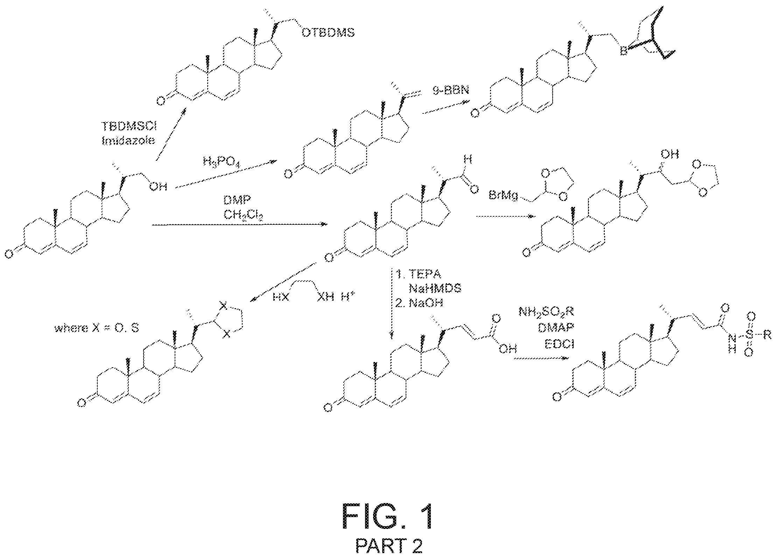

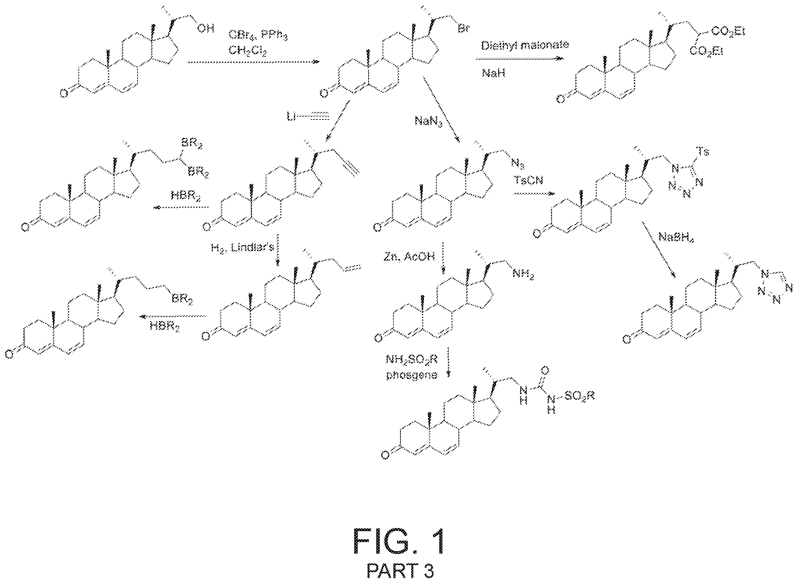

Figure 1 shows the conversion of a compound of general formula (IIa) or of general formula (II) in which the side chain is -CH₂OH to other compounds of general formula (IIa) or of general formula (II), respectively, with different side chains.

Such reactions are equally applicable to compounds of general formulae (Ia), (I), (IIIxa), (IIIx), (IIIya), (Illy), (IIIxz), (IIIyz), (IVa)-(XIa) and (IV)-(XI), wherein appropriate (i.e. where chemically sensible).

As shown in Figure 1, a compound of general formula (IIa) or a compound of general formula (II) wherein Y-R⁴ is CH₂-OH may be prepared from a plant sterol such as stigmasterol.

As shown in Figure 1, a compound of general formula (IIa) or a compound of general formula (II) with the -CH₂OH side chain can be converted to compounds of general formula (IIa) or of general formula (II) with side chains including -CH₂-9-borabicyclo(3.3.1) nonyl, -CH₂CH₂CH[B(alkyl)₂]₂, -CH₂CN, -CH₂CH₂CN, -CH₂Br, -CH₂CH[C(O)OEt]₂, -CH₂-C≡CH, - CH₂-CH=CH₂, =CH₂, -C(O)H, -CH₂NH₂, CH₂OTBDMS, CH₂N₃, CH₂OMs, 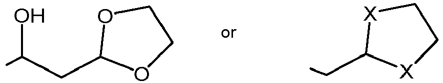
where X is O or S
alkyl may be C₁₋₆ alkyl and Et is ethyl; and also carboxylic acid mimetic groups including -C(O)NHSO₂R¹⁰ and -NHC(O)NH-SO₂R¹⁰.

Compounds of general formulae (Ia), (IIa), (IIIxa), (IIIya), (IIIxz), (IIIyz) and (IVa)-(XIa) and compounds of general formula (I), (II), (IIIx), (IIIy) and (IV)-(XI) with a side chain Y-OH (wherein Y is Y²-CH₂, and Y² is as defined above for Y except it is shorter in length by at least one carbon) can be converted to compounds in which the side chain is -Y²-C(O)H by oxidation, for example using oxalyl chloride suitably in the presence of dimethyl sulfoxide and a base such as triethylamine. Alternatively, the oxidation may be carried out using Dess-Martin periodinane as shown in Example 27.

In compounds of general formulae (Ia), (IIa), (IIIxa), (IIIya), (IIIxz), (IIIyz) and (IVa)-(XIa) and compounds of general formula (I), (II), (IIIx), (IIIy) and (IV)-(XI) in which the side chain is -Y²-C(O)H, the side chain can be extended, for example using an olefination with a compound of general formula (XXII):

Ph₃P=CH-Y³- C(O)OR²⁷ (XXII)

where Y³ is as defined for Y in general formula (Ia) and general formula (IIa) or Y in general formula (I) and general formula (II) except that it may have a shorter carbon chain such that the linker Y of general formula (Ia) and general formula (IIa) or of general formula (I) and general formula (II) can be a moiety -Y²-CH₂CH₂-Y³-, wherein Y² and Y³ are as defined for Y except that they are shorter in length, wherein R²⁷ is suitably C₁₋₆ alkyl or benzyl, to give a compound in which the side chain is Y²-CH=CH-Y³-C(O)OR²⁷. An olefination reaction using (EtO)₂P(O)CH₂Y³C(O)OR²⁷ may also be used.

The olefination may be carried out at about 15 to 25 °C, suitably room temperature, in a solvent such as dichloromethane.

These compounds can, in turn, be converted to compounds in which R⁴ is the carboxylic acid mimetic group C(O)NHSO₂R¹⁰, wherein R¹⁰ is as defined above, by reaction with:

NH₂SO₂R¹⁰

wherein R¹⁰ is as defined above, in the presence of a coupling agent such as 1-ethyl-3(3-dimethylaminopropyl)carbodiimide (EDCI).

Compounds of general formula formulae (Ia), (IIa), (IIIxa), (IIIya), (IIIxz), (IIIyz) and (IVa)-(XIa) and compounds of general formula formulae (I), (II), (IIIx), (IIIy) and (IV)-(XI) in which the group at the R⁴ position is OH can be protected with a silyl protecting group. This may be achieved by reaction with (XXIII) as described below, typically in an organic solvent and in the presence of a base, for example imidazole, or triethylamine. Such a reaction is shown in Example 22.

X¹-Si(R¹⁶)₃ (XXIII)

wherein, R¹⁶ is as defined above and X¹ is a leaving group, for example a halide such as chloride or a sulfonate leaving group such as trifluoromethanesulfonate (triflate), methanesulfonate (mesylate) or toluene sulfate (tosylate).

Compounds of general formulae (Ia), (IIa), (IIIxa), (IIIya), (IIIxz), (IIIyz) and (IVa)-(XIa) and compounds of general formulae (I), (II), (IIIx), (IIIy) and (IV)-(XI) in which R⁴ is OH may also be converted to compounds in which R⁴ is a sulfonate, for example methane sulfonate or toluene sulfonate, by reaction with a sulfonyl halide such as methane sulfonyl chloride, in the presence of a catalyst such as 4-dimethylaminopyridine (DMAP). Such a reaction is shown in Example 13. Alternatively, they may be converted to compounds of general formulae (Ia), (IIa), (IIIxa), (IIIya), (IIIxz), (IIIyz) and (IVa)-(XIa) or to compounds of general formulae (I), (II), (IIIx), (IIIy) and, (IV)-(XI) in which R⁴ is halo, for example bromo, by reaction with a halogenating agent, e.g. a brominating agent such as carbon tetrabromide as illustrated in Example 30 or *N*-bromosuccinimide, as illustrated in Example 35.

Such sulfonate or halide compounds can then be converted to compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is cyano by reaction with a cyanide salt, for example sodium or potassium cyanide (see Example 35). Alternatively, reaction with acetonitrile in the presence of a base such as n-butyllithium leads to a chain lengthening reaction so that, for example, a side chain -CH₂-O-methanesulfonyl or -CH₂-Br is converted to a side chain -CH₂CH₂-CN. Such a reaction is shown in Example 33.

Compounds with a sulfonate side chain can also be converted to compounds in which R⁴ is nitro by reaction with nitromethane in the presence of a base such as sodium or potassium carbonate.

Compounds of formulae (Ia), (IIa), (IIIxa), (IIIya), (IIIxz), (IIIyz) and (IVa)-(XIa) and compounds of general formulae (I), (II), (IIIx), (IIIy) and, (IV)-(XI) in which the side chain is Y²-C(O)OH or an ester thereof may be converted to compounds in which the side chain is Y²-CH=CH₂ by reaction with Phl(OAc)₂ in the presence of copper (II) acetate using a process similar to Hunsdiecker reaction (see J. Org. Chem., 1986, 51, 404-407 and V.C. Edelsztein et al. Tetrahedron, 2009, 65, 3615-3623). Such compounds with side chain -Y²-CH=CH₂ may in turn be oxidised using, for example, osmium tetroxide as described in J. Org. Chem., 1986, 51, 404-407 to give a compound in which the side chain is -Y²-CH(OH)-CH₂-OH. Such compounds may be oxidised to compounds in which the side chain is Y²-CH(OH)-C(O)H, which may then be protected as a 1,3-dioxane or 1,3-dioxolane by reaction with 1,3-propane diol or 1,2-ethandiol in the presence of an acid catalyst such as toluene sulfonic acid. Similar reactions can be used to prepare the equivalent cyclic dithioacetals, and cyclic aminals.

Compounds of general formulae (Ia), (IIa), (IIIxa), (IIIya), (IIIxz), (IIIyz) and (IVa)-(XIa) and compounds of general formulae (I), (II), (IIIx), (IIIy) and (IV)-(XI) with side chain -Y-CH=CH₂ may also be prepared by reduction of a compound with side chain -Y-C≡CH, typically by hydrogenation over a palladium catalyst, suitably Lindlar catalyst, as shown in Figure 1.

Compounds of formulae (Ia), (IIa), (IIIxa), (IIIya), (IIIxz), (IIIyz) and (IVa)-(XIa) and compounds of general formulae (I), (II), (IIIx), (IIIy) and (IV)-(XI) with side chain -Y-C≡CH may be prepared from compounds with side chain Y-X, where X is a halo group, particularly bromo, by reaction with an organometallic reagent, for example:

Li-C≡CH.

Compounds of general formulae (Ia), (IIa), (IIIxa), (IIIya), (IIIxz), (IIIyz) and (IVa)-(XIa) and compounds of general formulae (I), (II), (IIIx), (IIIy) and (IV)-(XI) in which the side chain - Y-R⁴ is -CH₂-OH may also be converted to compounds in which the side chain is =CH₂. This can be achieved by an elimination reaction in which the compound having side chain -Y-R⁴ is -CH₂-OH is reacted with an acid such as phosphoric acid, sulphuric acid or toluene sulphonic acid as shown in Figure 1. A similar reaction can be used to convert a compound with side chain -Y²-CH₂-OH to a compound with side chain -Y²-C=CH₂. Alternatively, compounds in which the side chain is =CH₂ can be prepared by oxidising -Y²-CH₂-OH to Y²-CH(O) and then converting this to an alkene using an olefination reaction.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) with side chain Y-C≡CH, =CH₂ or -Y²-C=CH₂ may be reacted with a borane of formula:

H-BR¹⁰R¹¹

to give compounds in which the side chain is -Y-CH₂-C(BR¹⁰R¹¹)₂, -CH₂-BR¹⁰R¹¹ or -Y²-CH₂-BR¹⁰R¹¹ respectively. An example of this reaction is shown in Figure 1.

Compounds of formulae (Ia), (IIa), (IIIxa), (IIIya), (IIIxz), (IIIyz) and (IVa)-(XIa) and compounds of general formulae (I), (II), (IIIx), (IIIy) and (IV)-(XI) in which the side chain is -CH₂-BR¹⁰R¹¹ or -Y²-CH₂-BR¹⁰R¹¹ may be reacted with, for example phenoxyacetic acid to give a corresponding compound in which the side chain is -CH₂-C(O)OH or -Y²-CH₂-C(O)OH.

Compounds of general formulae (Ia), (IIa), (IIIxa), (IIIya), (IIIxz), (IIIyz) and (IVa)-(XIa) and compounds of general formulae (I), (II), (IIIx), (IIIy) and (IV)-(XI) in which R⁴ is - CH[C(O)OR¹⁰]₂ may be prepared from compounds in which R⁴ is halo, for example bromo, by reaction with a malonate ester in the presence of a base such as sodium hydride, as shown in Figure 1. A reaction of this type is illustrated in Example 32.

Compounds of general formulae (Ia), (IIa), (IIIxa), (IIIya), (IIIxz), (IIIyz) and (IVa)-(XIa) and compounds of general formulae (I), (II), (IIIx), (IIIy) and (IV)-(XI) in which R⁴ is a malonate ester -CH[C(O)OR¹⁰]₂ may be heated under acidic or basic conditions to give compounds in which R⁴ is CH₂C(O)OH.

Compounds of general (Ia), (IIa), (IIIxa), (IIIya), (IIIxz), (IIIyz) and (IVa)-(XIa) and compounds of general formulae (I), (II), (IIIx), (Illy) and (IV)-(XI) in which the side chain is -Y-C(O)OH may also be converted to compounds in which the side chain is -Y-C(O)-CH₂-N₂ by reaction with phosgene to form the acid chloride, followed by reaction with diazomethane.

The diazomethane may be formed *in situ* using conventional methods, e.g. the treatment of *N*-nitroso-*N*-methylurea with aqueous sodium or potassium hydroxide in diethyl ether. Suitably the diazomethane is used in excess, typically in an amount of greater than 2 equivalents compared with the acid chloride. The reaction is typically conducted in an organic solvent such as diethyl ether, toluene or a mixture thereof. The reaction is carried out at a temperature of about -5 to 15 °C, typically 0-10 °C.

The compound with side chain -Y-C(O)-CH₂-N₂ may be treated with an aqueous silver compound, for example silver nitrate, at an elevated temperature and in the presence of an alcohol of formula:

R^{10a}-OH

wherein R^{10a} is as defined for R¹⁰ in general formula (Ia) or in general formula (I) except that it is not H. Most suitably, R^{10a} is C₁₋₆ alkyl or benzyl. Under these conditions, the compound undergoes a Wolff rearrangement to give a compound in which the side chain is -Y-CH₂-C(O)OR^{10a} and thus this sequence can be used to lengthen the side chain.

Compounds of general formulae (Ia), (IIa), (IIIxa), (IIIya), (IIIxz), (IIIyz) and (IVa)-(XIa) or compounds of general formulae (I), (II), (IIIx), (Illy) and (IV)-(XI) in which the side chain is Y-C(O)OH may be converted to compounds in which the side chain is -Y²-CH₂-CN by reaction with sodium nitrite under acidic conditions, for example in the presence of trifluoroacetic acid and trifluroroacetic anhydride (C. D. Schteingart and A. T. Hofmann, Journal of Lipid Research, 1988, 29, 1387-1395; Valentina Sepe et al, Eur. J. Org. Chem. 2012, 5187-5194).

Compounds of general formulae (Ia), (IIa), (IIIxa), (IIIya), (IIIxz), (IIIyz) and (IVa)-(XIa) or compounds of general formulae (I), (II), (IIIx), (Illy) and (IV)-(XI) in which the side chain is Y²-C(O)H (where Y² is as defined above for Y except that it is shorter in length by one carbon) may be converted to compounds in which the side chain is -Y-CH(XR¹⁰)(XR¹¹), where Y is -Y²-CH₂-, for example -Y²-CH(OR¹⁰)(OR¹¹) or -Y² -CH(SR¹⁰)(SR¹¹) where R¹⁰ and R¹¹ together with the atoms to which they are attached join to form a cyclic group. This can be achieved by reacting the compound in which the side chain is Y²-C(O)H with a compound of formula:

HX³-(CH₂)ₚ-X³H

where X³ is O, S or NH and p is 1 to 4 but usually is 2 or 3, or with a protected version of such a compound, for example in which OH or SH groups are protected with trimethylsilyl as shown in Example 28.

Compounds of general formulae (Ia), (IIa), (IIIxa), (IIIya), (IIIxz), (IIIyz) and (IVa)-(XIa) or compounds of general formulae (I), (II), (IIIx), (Illy) and (IV)-(XI) in which the side chain is Y²-C(O)H may also be converted to compounds with side chain -Y²-CH(OH)-CH₂-CH(OR¹⁰)(OR¹¹), -Y²-CH(OH)-CH₂-CH(R¹⁰)(OR¹¹) or -Y²-CH(OH)-CH₂-CH(SR¹⁰)(SR¹¹) by reaction with an appropriate organometallic reagent, typically a Grignard reagent of formula:

XMg-CH₂-R^{4c};

where X is halo, typically bromo, and R^{4c} -CH(OR¹⁰)(OR¹¹), -CH(R¹⁰)(OR¹¹) or CH(SR¹⁰)(SR¹¹).

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which the side chain is -Y²-CH(OH)-CH₂-CH(OR¹⁰)(OR¹¹) can be converted to compounds in which the side chain is -Y²-CH=CH-C(O)H by reaction with an acid. Following this, the aldehyde can be oxidised to give a carboxylic acid and/or the alkylenene bond can be reduced by hydrogenation to give a saturated side chain in which Y is -Y²-CH₂CH₂-.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is -N₃ may be prepared from compounds of (Ia), (IIa), (IIIxa), (IIIya) and (IVa)-(XIa) or compounds of general formulae (I), (II), (IIIx), (IIIy) and (IV)-(XI), respectively, in which R⁴ is a leaving group such as toluene sulfonate, methane sulfonate or compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is halo (for example bromo) or a sulfonyl leaving group such as toluene sulfonate or methane sulfonate, by reaction with sodium azide. This is illustrated in Example 34.

Compounds of general formulae (Ia), (IIa), (IIIxa), (IIIya), (IIIxz), (IIIyz) and (IVa)-(XIa) or compounds of general formulae (I), (II), (IIIx), (IIIy), (IIIxz), (IIIyz) and (IV)-(XI) in which R⁴ is NH₂ may be obtained by reduction of compounds of general formulae (Ia), (IIa), (IIIxa), (IIIya) and (IVa)-(XIa) or compounds of general formulae (I), (II), (IIIx), (IIIy) and (IV)-(XI), respectively, in which R⁴ is azide as illustrated in Example 34.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is - NHC(O)NHSO₂R¹⁰ may be prepared from compounds in which R⁴ is NH₂ using a coupling reaction with a compound of formula:

NH₂SO₂R¹⁰

wherein R¹⁰ is as defined above;

in the presence of a reagent such as *N,N'*-carbonyldiimidazole (CDI).

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is tetrazole-5-yl may be prepared from compounds of general formulae (Ia), (IIa), (IIIxa), (IIIya), (IIIxz), (IIIyz) and (IV)-(XI) or compounds of general formulae (I), (II), (IIIx), (IIIy) and (IV)-(XI), respectively, in which R⁴ is CN by reaction with azidotrimethylsilane/dibutylstannanone or Bu₃SnN₃ as described in US2016/0145295. Alternatively, the compound in which R⁴ is CN may be reacted with sodium azide in the presence of an acid. For example, NaN₃/NH₄Cl in toluene/DMF *(*Organic and Biomolecular Chemistry, 2008, 6, 4108) or NaN₃/ NEt₃.HCl in DMF (Brown et al.; Bioorg Med Chem Lett, 2002, 12, 3171). Alternatively, a compound in which R⁴ is azide may be reacted with a suitable cyanide compound, for example tosyl cyanide, under reducing conditions to give a compound in which R⁴ is tetrazol-1-yl.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is amino tetrazole can be prepared from a compound in which the group at the R⁴ position is mesyl by reaction with 5-amino tetrazole.

Compounds of general (Ia), (IIa), (IIIxa), (IIIya), (IIIxz), (IIIyz) and (IVa)-(XIa) or compounds of general formulae (I), (II), (IIIx), (IIIy) and (IV)-(XI) in which the side chain is -Y²-C(O)H may also be converted to compounds -Y²-CH₂-NR¹⁰R¹¹ by reductive amination, using a reducing agent such as a hydride, borohydride or cyanoborohydride (for example sodium borohydride or sodium cyanoborohydride) and an amine of formula:

H-NR¹⁰R¹¹

where R¹⁰ and R¹¹ are as defined above.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is C(O)OR¹⁰ may be converted to a compound of the same general formula, in which R⁴ is OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, CH(OR¹⁰)(OR¹¹), CH(R¹⁰)(OR¹¹), CH(SR¹⁰)(SR¹¹), NR¹⁰R¹¹, BR¹⁰R¹¹, C(O)CH₂N₂, - CH=CH₂, -C=CH, CH[C(O)OR¹⁰]₂ or CH(BR¹⁰R¹¹)₂, azide or a carboxylic acid mimetic group such as tetrazole.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is SO₃R¹⁰ may be synthesised from compounds in which R⁴ is C(O)OH by the methods taught in WO2008/002573, WO2010/014836 and WO2014/066819.

Thus a compound of general formulae (Ia), (IIa), (IIIxa), (IIIya), (IIIxz), (IIIyz) and (IVa)-(XIa) or a compound of general formulae (I), (II), (IIIx), (IIIy) and (IV)-(XI) in which R⁴ is C(O)OH may first be reacted with a C₁₋₆ alkanoyl or benzoyl chloride or with a C₁₋₆ alkanoic anhydride to protect any OH groups. The protected compound may then be reacted with a reducing agent such as a hydride, suitably lithium aluminium hydride or sodium borohydride in order to reduce the carboxylic acid group to OH. The alcohol group may be replaced by a halogen, for example bromine or iodine, using the triphenyl phosphine/imidazole/halogen method described by Classon et al., J. Org. Chem., 1988, 53, 6126-6130. The halogenated compound may then be reacted with sodium sulphite in an alcoholic solvent to give a compound with a SO₃⁻Na⁺ substituent.

A compound of general formulae (Ia), (IIa), (IIIxa), (IIIya), (IIIxz), (IIIyz) and (IVa)-(XIa) or a compound of general formulae (I), (II), (IIIx), (IIIy) and (IV)-(XI) in which R⁴ is OSO₃R¹⁰ can be obtained by reacting the alcohol obtained from reducing the protected carboxylic acid as described above with chlorosulfonic acid in the presence of a base such as triethylamine to yield the protected triethylamine salt. Protecting groups can be removed using base hydrolysis as described above. Reduction of the carboxylic acid followed by reaction of the resultant alcohol with a sulfonyl chloride yields a compound of general formulae (Ia), (IIa), (IIIxa), (IIIya), (IIIxz), (IIIyz) and (IVa)-(XIa) or a compound of general formulae (I), (II), (IIIx), (IIIy) and (IV)-(XI) in which R⁴ is OSO₂R¹⁰.

Compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is C(O)NR¹⁰R¹¹ may be prepared from the carboxylic acid by reaction with an amine of formula H-NR¹⁰R¹¹ in a suitable solvent with heating. Compounds of general formulae (Ia), (IIa), (IIIxa), (IIIya) and (IVa)-(XIa) or compounds of general formulae (I), (II), (IIIx), (IIIy), (IIIxz), (IIIyz) and (IV)-(XI) in which R⁴ is C(O)NR¹⁰R¹¹ or OSO₃R¹⁰ may also be prepared by methods similar to those described by Festa et al., J. Med. Chem., 2014, 57, 8477-8495.

An example of this is the synthesis of compounds of general formulae (Ia), (IIa), (IIIxa), (IIIya), (IIIxz), (IIIyz) and (IVa)-(XIa) or compounds of general formulae (I), (II), (IIIx), (IIIy) and (IV)-(XI) in which R⁴ is C(O)NH(CH₂)₂SO₃H or C(O)NHCH₂CO₂H or salts thereof from compounds of the same general formula in which R⁴ is C(O)OH by reaction with taurine or glycine respectively in the presence of a coupling reagent such as iso-butylchloroformate and a base such as triethylamine.

An analogue of a compound of general formulae (Ia), (IIa), (IIIxa), (IIIya), (IIIxz), (IIIyz) and (IVa)-(XIa) or a compound of general formulae (I), (II), (IIIx), (IIIy) and (IV)-(XI) in which R⁴ is C(O)R¹⁰ can be obtained by reduction of a compound in which R⁴ is C(O)OR¹⁰ using one equivalent of diisobutyl aluminium hydride (DIBAL-H) to obtain an aldehyde in which R⁴ is C(O)H (see, for example, WO2011/014661).

Alternatively, the aldehyde may be prepared by oxidation of a protected compound in which R⁴ is OH prepared as described above. The oxidation may be Swern oxidation carried out using oxalyl chloride and dimethyl sulfoxide followed by triethylamine (see, for example Xiang-Dong Zhou et al, Tetrahedron, 2002, 58, 10293-10299). Alternatively, the oxidation may be carried out using an oxidising agent such as pyridinium chlorochromate (PCC) as described by Carnell et al (J. Med. Chem., 2007, 50, 2700-2707).

An analogue of a compound of general formulae (Ia), (IIa), (IIIxa), (IIIya), (IIIxz), (IIIyz) and (IVa)-(XIa) or an analogue of compound of general formulae (I), (II), (IIIx), (Illy) and (IV)-(XI) in which R⁴ is C(O)R¹⁰ where R¹⁰ is other than hydrogen can be obtained by known methods, for example by the reaction of the aldehyde in which R⁴ is C(O)H with a suitable Grignard reagent, followed by oxidation. Such methods are well known to those of skill in the art.

### General information

The invention will now be described in greater detail with reference to the examples. In the examples, the following abbreviations were used:
- Ac: acetyl
- AcOH: acetic acid
- nBuOAc: *n*-butyl acetate
- 9-BBN: 9-borabicyclo[3.3.1]nonane
- nBuLi: *n*-butyllithium
- CDCA: chenodeoxycholic acid
- CDI: *N,N'*-carbonyldiimidazole
- CPME: cyclopentyl methyl ether
- DBDMH: 1,3-dibromo-5,5-dimethylhydantoin
- DCDMH: 1,3-dichloro-5,5-dimethylhydantoin
- DCM: dichloromethane
- DDQ: 2,3-dichloro-5,6-dicyano-p-benzoquinone
- DEIPS: diethylisopropylsilyl
- DIBAL-H: diisobutyl aluminium hydride
- DIDMH: 1,3-diiodo-5,5-dimethylhydantoin
- DMAP: 4-dimethylaminopyridine
- DMDO: dimethyldioxirane
- DMF: *N,N*-dimethylformamide
- DMIPS: dimethylisopropylsilyl
- DMP: Dess-Martin periodinane
- DTBMS: di-*tert*-butylmethylsilyl
- EDCI: 1-ethyl-3(3-dimethylaminopropyl)carbodiimide
- EtOAc: ethyl acetate
- EtOH: ethanol
- Et₂O: diethyl ether
- FXR: farnesoid X receptor
- HFIP: 1,1,1,3,3,3-hexafluoro-2-propanol/hexafluoroisopropanol
- HMPO: (20S)-20-hydroxymethyl-pregna-4-en-3-one also known as 20-hydroxymethylpregn-4-en-3-one and 3-keto-bis-norcholenol
- HPLC: high performance liquid chromatography
- HWE: Horner-Wadsworth-Emmons
- IPA: isopropyl alcohol
- IPC: in process control
- mCPBA: *meta*-chloroperoxybenzoic acid
- MeCN: acetonitrile
- MeOH: methanol
- MIBK: methyl isobutyl ketone
- MMPP: magnesium bis(monoperoxyphthalate)
- Ms: methanesulfonyl
- MsCl: methanesulfonyl chloride
- MTO: methyltrioxorhenium (VII)
- NBS: *N*-bromosuccinimide
- NCS: *N*-chlorosuccinimide
- NEt₃: triethylamine
- NIS: *N*-iododuccinimide
- OCA: obeticholic acid
- PCC: pyridinium chlorochromate
- PEG: polyethylene glycol
- PhMe: toluene
- PMB: paramethoxybenzyl
- pTSA.H₂O: *p*-toluenesulfonic acid monohydrate
- TBCA: tribromoisocyanuric acid
- TBDMS: *tert*-butyldimethylsilyl
- TBDPS: *tert*-butyldiphenylsilyl
- TBME: *tert*-butyl methyl ether
- TCCA: trichloroisocyanuric acid
- TDS: thexyldimethylsilyl
- TEMPO: (2,2,6,6-tetramethyl-piperidin-1-yl)oxy
- TEPA: triethyl phosphonoacetate
- TES: triethylsilyl
- TFE: 2,2,2-trifluoroethanol
- THF: tetrahydrofuran
- TIPS: tri-isopropylsilyl
- TICA: triiodoisocyanuric acid
- TLC: thin layer chromatography
- TMS: trimethylsilyl
- TMSOTf: trimethylsilyl trifluoromethanesulfonate
- TPS: triphenylsilyl
- Ts: toluenesulfonyl/tosyl
- UDCA: ursodeoxycholic acid

### EXAMPLES

### GENERAL PROCEDURES

### Examples 1 and 2 - Epoxidation reactions

### Example 1 - Synthesis of (6β, 7β, 22E)-6,7-epoxy-3-oxo-4,22-choladien-24-oic acid ethyl ester (IA) using TCCA in acetone/H₂O

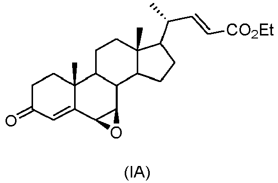

### Synthesis of (6β, 7α, 22E)-7-chloro-6-hydroxy-3-oxo-4,22-choladien-24-oic acid ethyl ester (IIIxA) or (6α, 7β, 22E)-6-chloro-7-hydroxy-3-oxo-4,22-choladien-24-oic acid ethyl ester) (IIIyA)

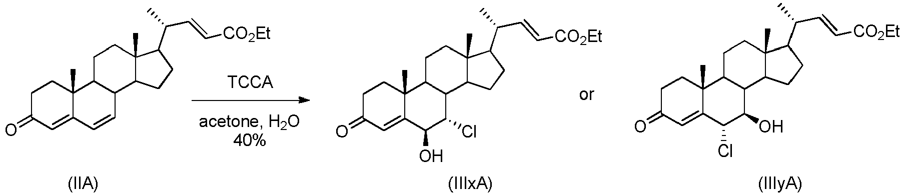

(22E)-3-oxo-4,6,22-cholatrien-24-oic acid ethyl ester (2.0 g, 5.0 mmol, obtained from Stigmasterol as described in Example 1 of patent application No. PCT/GB2015/053516; WO2016/079517A1) was dissolved in acetone:H₂O (12:1, 15 vol). TCCA (0.47 g, 0.4 eq) was added as a single portion and the solution stirred for 1.5 h. The resultant mixture was filtered, diluted with CH₂Cl₂ and washed with 10% NaHSO₃ aq. (25 mL) followed by H₂O (25 mL). The organic layer was dried over Na₂SO₄, filtered and concentrated to give (6β, 7α, 22E)-7-chloro-6-hydroxy-3-oxo-4,22-choladien-24-oic acid ethyl ester (IIIxA) or (6α, 7β, 22*E*)-7-hydroxy-6-chloro-3-oxo-4,22-choladien-24-oic acid ethyl ester (IIIyA) (0.89 g, 2.0 mmol) after column chromatography as an oily solid.
¹H NMR (400 MHz, CDCl₃): δ = 6.8 (1H, dd, J= 15.6, 9.0, C22H), 5.88 (1H, s, C4H), 5.74 (1H, d, *J* = 15.6, C23H), 4.35 (1H, d, *J* = 3.0, C6H), 4.19-4.14 (3H, m, OC*H*₂CH₃ + C7H), 2.54-1.08 (27H, m), 0.79 (3H, s, CH₃).
¹³C NMR (100 MHz, CDCl₃): δ = 199.9, 197.1, 164.7, 154.3, 129.4, 119.2, 77.1, 63.9, 60.2, 54.8, 50.9, 44.5, 42.7, 39.6, 38.9, 37.8, 36.8, 34.2, 34.0, 27.9, 23.1, 20.6, 20.3, 19.2, 14.3, 12.3.
HRMS (ESI-TOF) *m*/*z:* (M+H)⁺ calcd for C₂₆H₃₈Cl₁O₄ 449.2453; found: 449.2425;

### Synthesis of (6β, 7β, 22E)-6,7-epoxy-3-oxo-4,22-choladien-24-oic acid ethyl ester (IA)

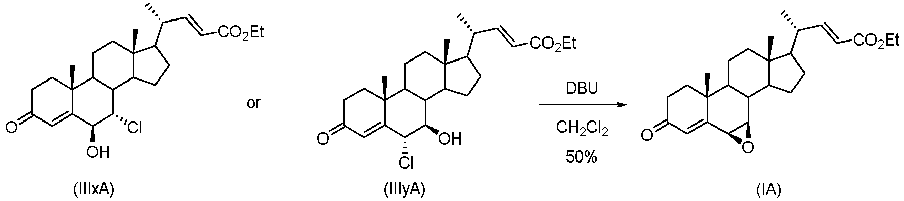

(6β, 7α, 22E)-7-chloro-6-hydroxy-3-oxo-4,22-choladien-24-oic acid ethyl ester (IIIxa) or (6α, 7β, 22*E*)-7-hydroxy-6-chloro-3-oxo-4,22-choladien-24-oic acid ethyl ester (IIIya) (0.89 g, 2.0 mmol) was dissolved in CH₂Cl₂ (10 mL) and DBU (0.45 mL, 1.5 eq) was added. The solution was stirred for 18 h and then additional DBU (0.15 mL, 0.5 eq) added. After stirring for a further 4.5 h DBU (0.15 mL, 0.5 eq) was added and the solution stirred for 19 h. The solution was concentrated to dryness giving the title compound (0.40 g, 1.0 mmol) after column chromatography as an off white solid.
¹H NMR (400 MHz, CDCl₃): δ = 6.8 (1H, dd, *J* = 15.6, 9.0, C22H), 6.13 (1H, s, C4H), 5.74 (1H, d, *J* = 15.6, C23H), 4.17 (2H, q, *J* = 7.1, OC*H*₂CH₃), 3.35-3.33 (2H, m, C6H and C7H), 2.63-2.53 (1H, m), 2.43-2.24 (2H, m), 2.02-1.03 (23H, m), 0.76 (3H, s, CH₃).
¹³C NMR (100 MHz, CDCl₃): δ = 198.2, 166.9, 163.0, 154.0, 129.3, 119.4, 60.2, 59.1, 55.7, 54.7, 52.6, 51.6, 43.7, 39.6, 39.2, 36.6, 36.1, 35.5, 34.1, 28.0, 23.8, 21.3, 19.2, 16.8, 14.3, 12.1.
HRMS (ESI-TOF) *m*/*z:* (M+H)⁺ calcd for C₂₆H₃₇O₄ 413.2686; found: 413.2691.

### Example 2 - Synthesis of (6β, 7β, 22E)-6,7-epoxy-3-oxo-4,22-choladien-24-oic acid ethyl ester using TCCA in acetic acid

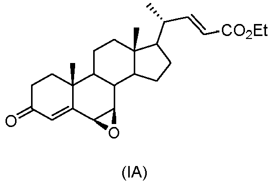

### Synthesis of (6β, 7α, 22E)-6-acetoxy-7-chloro -3-oxo-4,22-choladien-24-oic acid ethyl ester

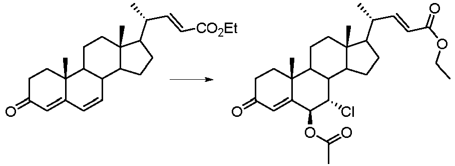

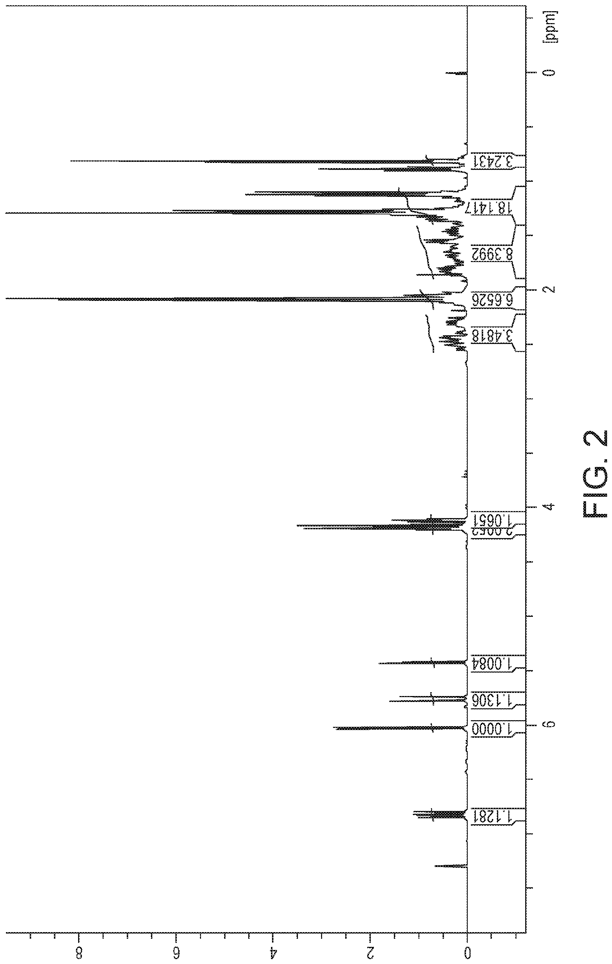

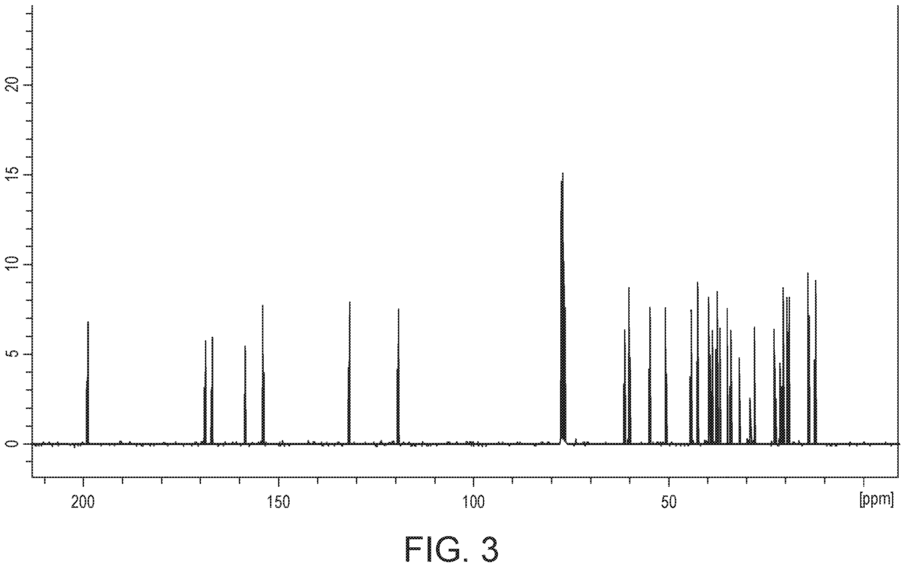

(22*E*)-3-oxo-4,6,22-cholatrien-24-oic acid ethyl ester (1.0 g, 2.5 mmol) was dissolved in AcOH (10 mL). TCCA (235 mg) was added as a single portion and the solution stirred for 18h. The mixture was diluted with EtOAc (100 mL) and washed with 5% aq. NaHSO₃ (100 mL) followed by H₂O (2×50 mL) and 5% aq. NaHCO₃ (50 mL). The organic layer was dried over Na₂SO₄, filtered and concentrated *in vacuo.* (6β, 7α, 22*E*)-6-acetoxy-7-chloro-3-oxo-4,22-choladien-24-oic acid ethyl ester (454 mg, yellow oil) was isolated by column chromatography (SiO₂, eluting with Heptane : EtOAc gradient).
¹H NMR (400 MHz, CDCl₃): δ = 6.81 (1H, dd, *J* 15.6, 9.0, C22H), 6.02 (1H, s, C4H), 5.75 (1H, dd, *J* 0.7, 15.6, C23H), 5.42 (1H, d, *J* 3.0, C6H), 4.15 (2H, dd, *J* 7.1, 14.2, OC*H*₂CH₃), 4.11 (1H, app. t, J2.8, C7H), 2.55-2.25 (3H, m), 2.14-2.00 (3H, m), 2.07 (3H, s, OAc).1.87-1.11 (17H, m), 1.11 (3H, d, *J* 6.8, C21H₃), 0.81 (3H, s, CH₃) (see Figure 2).
¹³C NMR (100 MHz, CDCl₃): δ = 198.9 (C=O), 168.7 (C=O), 166.9 (C=O),158.4 (C5), 154.0 (C22H), 131.9 (C4H), 119.3 (C23H), 76.5 (C6H), 61.2 (C7H), 60.1 (*CH₂*CH₃), 54.7 (CH), 50.8 (CH), 44.3 (CH), 42.6 (C), 39.6 (CH), 38.7 (CH₂), 37.6 (C), 36.8 (CH₂), 35.0 (CH), 34.1 (CH₂), 27.8 (CH₂), 23.1 (CH₂), 21.1 (*CH*₃C(O)), 20.5 (CH₂), 19.5 (*CH*₃CH₂), 19.2 (C21H₂), 14.3 (CH₃), 12.4 (CH₃) (see Figure 3).

### Synthesis of (6β, 7β, 22E)-6,7-epoxy-3-oxo-4,22-choladien-24-oic acid ethyl ester

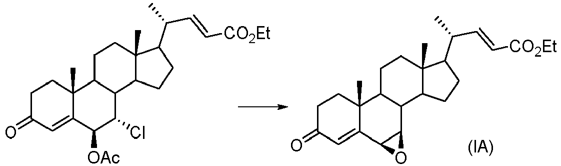

(6β, 7α, 22*E*)-6-acetoxy-7-chloro -3-oxo-4,22-choladien-24-oic acid ethyl ester (48 mg) was dissolved in EtOH (5 mL) and K₂CO₃ (32 mg) was added. The mixture was stirred at reflux for 4 h, then filtered and concentrated *in vacuo,* to give the desired crude β-epoxide in quantitative yield. Identity of the product was confirmed by ¹H NMR and TLC comparison with data obtained for the compound (IA) prepared according to Example 1.

### Example 3 - Synthesis of (6β, 7β, 22E)-6,7-epoxy-3-oxo-4,22-choladien-24-oic acid ethyl ester (IA) using TCCA in formic acid

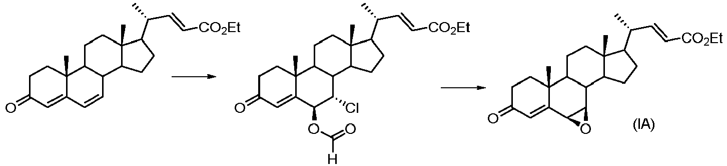

(22*E*)-3-oxo-4,6,22-cholatrien-24-oic acid ethyl ester (1.0 g, 2.5 mmol) was dissolved in formic acid (10 mL). TCCA (235 mg) was added as a single portion and the solution stirred for 15 min. The mixture was diluted with EtOAc (100 mL) and washed with 5% aq. NaHSO₃ (100 mL) followed by H₂O (2×50 mL). The organic layer was dried over Na₂SO₄, filtered and concentrated *in vacuo.* The residue was dissolved in EtOH (10 mL) and K₂CO₃ (698 mg, 5.05 mmol) was added. The mixture was stirred at 65 °C for 3 h and then concentrated *in vacuo.* Purification by column chromatography (SiO₂, eluting with heptane : EtOAc gradient) gave the desired (6β, 7β, 22*E*)-6,7-epoxy-3-oxo-4,22-choladien-24-oic acid ethyl ester (330 mg) as an off white solid. Identity of the product was confirmed by ¹H NM R and TLC comparison with data obtained for the compound (IA) prepared according to Example 1.

### Example 4 - Synthesis of (6β, 7β) and (6α, 7α) mixture of (22E)-6,7-epoxy-3-oxo-4,22-choladien-24-oic acid ethyl ester using DBDMH in acetone/H₂O

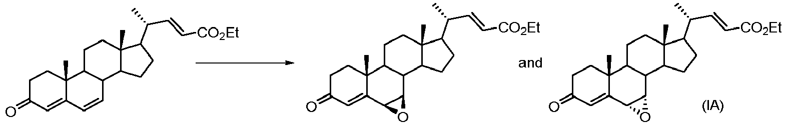

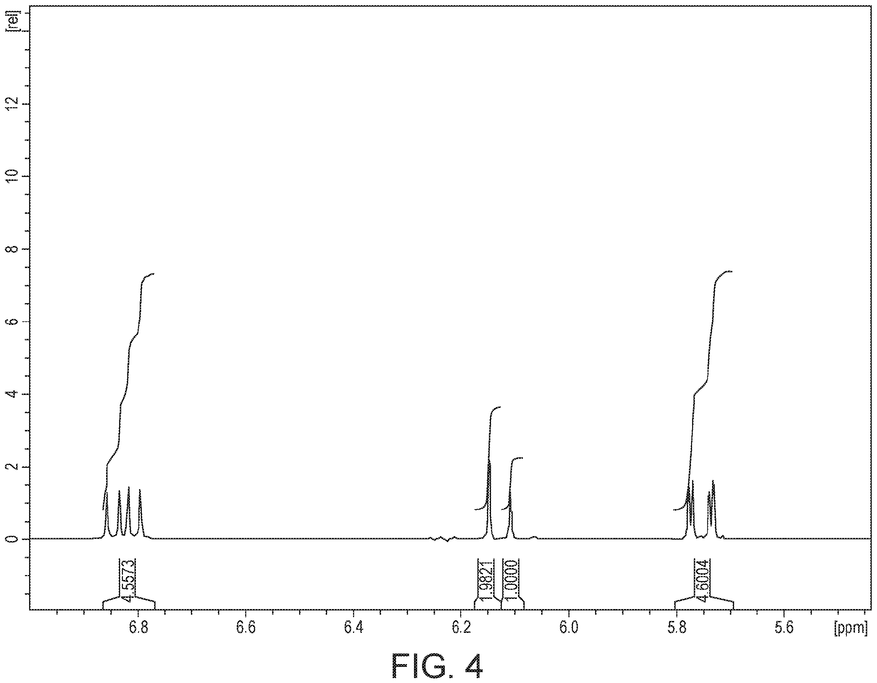

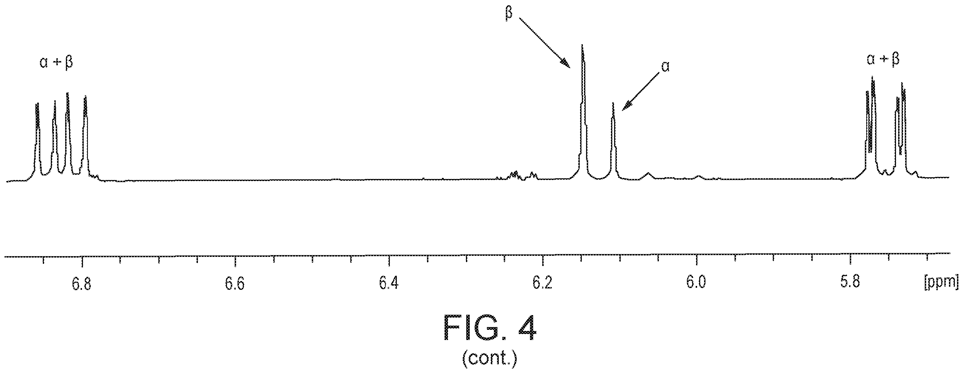

To a solution of (22*E*)-3-oxo-4,6,22-cholatrien-24-oic acid ethyl ester (396 mg, 1.0 mmol) in acetone : H₂O (9:1, 10 mL) at ambient temperature was added benzoic acid (61 mg, 0.5 mmol) followed by 1,3-dibromo-5,5-dimethylhydantoin (286 mg, 1 mmol) and the solution was stirred for 2 h. The mixture was diluted with EtOAc (50 mL) and washed with 5% aq. NaHSO₃ (10 mL) and water (2×25 mL). The organic layer was dried over Na₂SO₄, filtered and concentrated *in vacuo.* The resulting yellow oil was dissolved in CH₂Cl₂ (8 mL) and DBU (0. 5 mL) was added and the solution stirred for 16 h. The mixture was concentrated and the residue (dark brown oil) was purified by column chromatography (SiO₂, eluting with heptane : EtOAc gradient) to give (22*E*)-6,7-epoxy-3-oxo-4,22-choladien-24-oic acid ethyl ester as a 2:1 mixture of (6β,7β) to (6α,7α) epoxides (ratio based on integration of characteristic protons of CH4 in ¹H NMR - see Figure 4) in 33% yield over two steps (137 mg, 0.33 mmol). Identity of the products in the mixture was confirmed by ¹H NMR and compared with data obtained for the compound (IA) prepared according to Example 1 and its 6.7-alpha-epoxy isomer, which was previously synthesized following Uekawa et al. in Biosci. Biotechnol. Biochem. 2004, 68, 1332-1337 and synthesized XXX

### Example 5 - Synthesis of (6β, 7β) and (6α, 7α) mixture of 6,7-epoxy-3-oxo-4,22-choladien-24-oic acid ethyl ester using DBDMH in acetone/H₂O

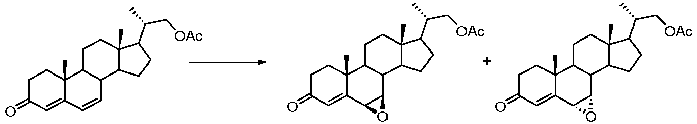

To a solution of (20S)-20-acetoxymethyl-pregna-4,6-dien-3-one (370 mg, 1.00 mmol) in acetone:H₂O (12 : 1, 5 mL) at ambient temperature was added 1,3-dibromo-5,5-dimethylhydantoin (229 mg, 0.80 mmol) in one portion and the solution was stirred for 1 h. A further portion of 1,3-dibromo-5,5-dimethylhydantoin was added (40 mg, 0.14 mmol) and after 10 min the mixture was diluted with EtOAc (50 mL), washed with 5% aq. NaHSO₃ (25 mL) and 5% aq. NaHCO₃ (25 mL). The organic phase was dried over anhydrous Na₂SO₄, filtered and concentrated *in vacuo.* The residue was purified by column chromatography (SiO₂, eluting with heptane : EtOAc gradient), to give 1.2 : 1 mixture of bromohydrins (102 mg, 0.22 mmol) (the ratio was calculated using characteristic peaks in ¹H NMR).
¹H NMR (400 MHz, CDCl₃): δ = 5.95 (1H, s, C4H(a)), 5.86 (1.2H, s, C4H(b)), 4.59 (1H, d, *J* 2.6, C6H(a)), 4.47 (1.2H, d, *J* 2.8, C6H(b)), 4.30 (1.2H, app. t, *J* 2.7, C7H(b)), 4.10-4.04 (2.2H, m, C*H_{A}*H_{B}OAc), 4.04 (1H, app. t, *J* 2.2, C7H(a)), 3.82-3.76 (2.2H, m, CH_{A}*H_{B}*OAc), 3.13 (1.2H, br. s, OH(a)), 2.75 (1H, br. s, OH(b)), 2.60-1.02 (2.2H, m), 2.54-1.08 (57.2H, m), 0.80 (6.6H, s, CH₃).

The isolated mixture of bromohydrins (72 mg, 0.15 mmol) was dissolved in CH₂Cl₂ (3 mL), DBU (50 µL) added and the solution stirred for 18 h. The mixture was concentrated *in vacuo* and the residue purified by column chromatography (SiO₂, eluting with heptane : EtOAc gradient) to give 1.2 : 1 mixture of (6β,7β) to (6α,7α) epoxides in quantitative yield (59 mg, 0.15 mmol).

### Examples 6-9 - Synthesis of (6α, 5β)-3,7-dioxo-6-ethyl-cholan-24-oic acid (VIAA) (precursor to obeticholic acid (OCA))

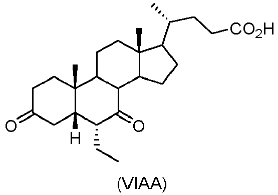

### Example 6 - Synthesis of (6α, 7β, 22E)-6-ethyl-7-hydroxy-3-oxo-4,22-choladien-24-oic acid ethyl ester (IVA)

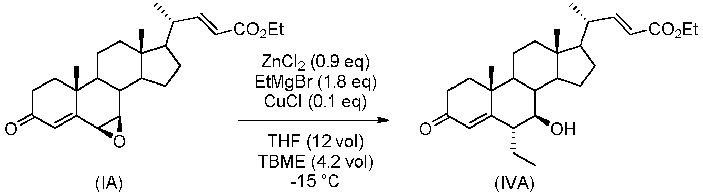

To a solution of ZnCl₂ in THF (0.7 M, 31.2 mL, 21.8 mmol, 0.9 eq) was charged anhydrous THF (40 mL) and the contents then cooled to -10 °C. A solution of EtMgBr in THF (1.0 M, 43.5 mL, 43.5 mmol, 1.8 eq) was added over 25 mins. Solid CuCI (240 mg, 2.6 mmol, 0.1 eq) was added in one portion and a solution of (6β, 7β, 22*E*)-6,7-epoxy-3-oxo-4,22-choladien-24-oic acid ethyl ester (IA) (10 g, 24.2 mmol) in THF (40 mL) was added dropwise over 15 mins. The reaction stirred for 1 h at -10 °C, (TLC 1:1 Heptane:EtOAc, visualised by UV and developed using Ceric Ammonium Molybdate stain) and then quenched by the dropwise addition of sat. aq. NH₄Cl (80 mL). The inorganic salts were filtered off, rinsed with TBME (100 ml) and the filtrate phases were separated. The organic phase was washed with sat. aq. NH₄Cl (80 mL) and 10% aqueous NaCl (80 mL). The organic phase was dried over Na₂SO₄, filtered and concentrated *in vacuo* at 40 °C. Purification by column chromatography (SiO₂, 0-50% Heptane : EtOAc) gave the title compound as a colourless oil solidifying on standing (9.6 g, 90% yield).
¹H NMR (400 MHz, CDCl₃): δ = 6.82 (1H, dd, *J* = 15.6, 9.0, C22H), 5.84 (1H, d, *J* = 1.6, C4H), 5.74 (1H, d, *J* = 15.6, C23H), 4.17 (2H, q, *J* = 7.1, OC*H*₂CH₃), 3.13 (1H, t, *J* = 9.7 C7H), 2.42-2.23 (4H, m), 2.05-0.89 (26H, m), 0.91 (3H, t, *J* = 7.4, CH₃), 0.77 (3H, s, CH₃). ¹³C NMR (100 MHz, CDCl₃): δ = 119.6, 169.1, 167.0, 154.2, 122.8, 119.3, 77.6, 60.2, 55.3, 54.1, 49.8, 48.2, 43.9, 43.1, 39.5, 39.4, 38.6, 35.3, 33.4, 28.5, 26.9, 21.5, 19.4, 18.9, 18.8, 14.3, 12.4, 10.7.
(IR) vₘₐₓ(cm⁻¹): 3473, 2937, 1716, 1650,1606.
HRMS (ESI-TOF) *m*/*z:* (M+H)⁺ calcd for C₂₈H₄₃O₄ 443.3161; found: 443.3155.

### Example 7 - Synthesis of (5β, 6α 7β)-6-ethyl-7-hydroxy-3-oxo-cholan-24-oic acid ethyl ester (VA)

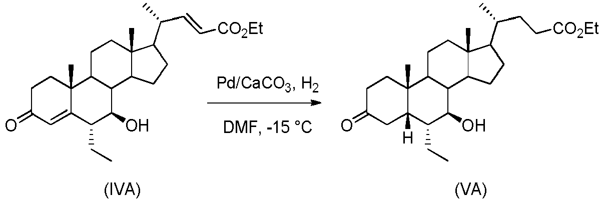

5% Pd/CaCO₃ (1.2 g) was charged to a round bottom flask under an argon atmosphere followed by solution of (6α, 7β, 22*E*)-6-ethyl-7-hydroxy-3-oxo-4,22-choladien-24-oic acid ethyl ester (IVA) (5.6 g) in DMF (25 mL). The mixture was cooled to -10 °C and the flask was evacuated then filled with hydrogen three times with vigorous stirring. The mixture was stirred under an atmosphere of hydrogen for 48 h while maintaining the temperature at -10 °C (IPC by TLC, eluent 1:1 EtOAc: Heptane; visualized with Anisaldehyde stain) then the flask was evacuated, filled with argon and filtered and the catalyst washed with TBME (100 mL). The filtrate was washed with 10% aq. NaCl (3 x 50 mL), dried over Na₂SO₄, filtered and concentrated *in vacuo* at 40 °C. Purification by column chromatography (SiO₂, 2-30% Heptane: EtOAc) gave (5β, 6α, 7β)-6-ethyl-7-hydroxy-3-oxo-cholan-24-oic acid ethyl ester (VA) in 89% yield (4.5 g).
¹H NMR (400 MHz, CDCl₃): δ = 4.12 (2H, q, *J* = 7.1, OC*H*₂CH₃), 3.23 (1H, app. t, *J* = 8.4 C7H), 2.38-0.93 (37H, m), 0.85 (3H, t, *J* = 4.7, CH₃), 0.71 (3H, s).
¹³C NMR (100 MHz, CDCl₃): δ = 212.0, 174.2, 75.1, 60.2, 56.0, 55.0, 45.2, 44.0, 43.7, 43.6, 40.1, 39.6, 37.7, 37.0, 36.6, 35.2, 34.7, 31.3, 31.0, 28.5, 26.8, 22.8, 21.9, 20.7, 18.4, 14.3, 12.2, 11.1.

### Example 8 - Synthesis of (5β, 6α)-3,7-dioxo-6-ethyl-cholan-24-oic acid ethyl ester (VIA)

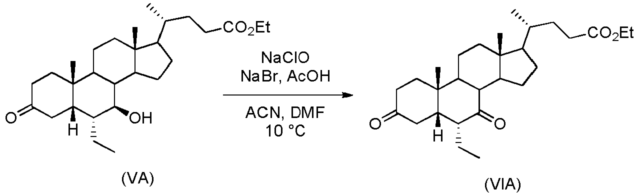

To a solution of (5β, 6α, 7β)-6-ethyl-7-hydroxy-3-oxo-cholan-24-oic acid ethyl ester (VA) (2.4 g) in DMF (7 ml) and CH₃CN (7 mL) was added NaBr (111 mg) followed by AcOH (4.8 mL) and the mixture was cooled to 5 °C. A solution of sodium hypochlorite (-13% w/v, 4.5 mL) was added dropwise over 15 min, then the mixture was stirred for 1 h at 5 °C. Further CH₃CN (20 mL) was added at this point, as the mixture became dense, and the cooling bath removed. The mixture was allowed to stir at ambient temperature for 1 h, then further quantity of AcOH (3 mL) was added followed by a dropwise addition of sodium hypochlorite (-13% w/v, 3 mL). The reaction was stirred for 2 h at ambient temperature and a solution of aq. 5% w/v Na₂SO₃ (360 mL) was charged dropwise with vigorous stirring, followed by EtOAc (150 mL). Phases were separated and the organic phase was washed with 5% aqueous NaHCO₃ (3 × 100 mL), dried with anhydrous Na₂SO₄, filtered and concentrated *in vacuo.* Purification by column chromatography (SiO₂, 0-50% Heptane: EtOAc) gave the title compound as colourless oil in 77 % yield (1.84 g).
¹H NMR (400 MHz, CDCl₃): δ = 4.09 (2H, q, *J* = 7.1, OC*H*₂CH₃), 2.73-2.69 (1H, m), 2.44 (1H, t, *J* = 11.3), 2.35-0.88 (34H, m), 0.78 (3H, t, *J* = 7.4), 0.67 (3H, s).
¹³C NMR (100 MHz, CDCl₃): δ = 212.0, 210.5, 174.2, 60.2, 54.9, 52.4, 52.3, 50.0, 48.9, 43.7, 42.6, 38.9, 38.3, 36.7, 35.9, 35.5, 35.2, 31.3, 31.0, 28.2, 24.6, 22.9, 22.3, 18.6, 18.4, 14.2, 12.1, 11.8.

### Example 9 - Synthesis of (5p, 6α)-3,7-dioxo-6-ethyl-cholan-24-oic acid (VIAA) (precursor to obeticholic acid (OCA))

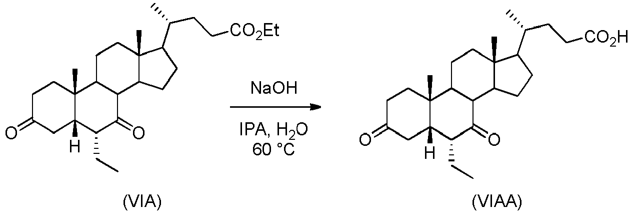

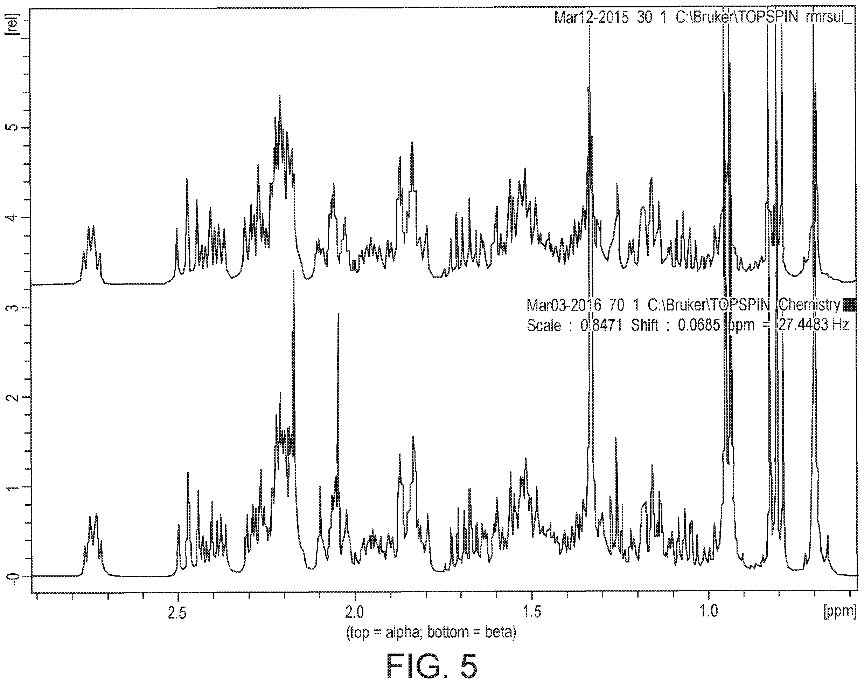

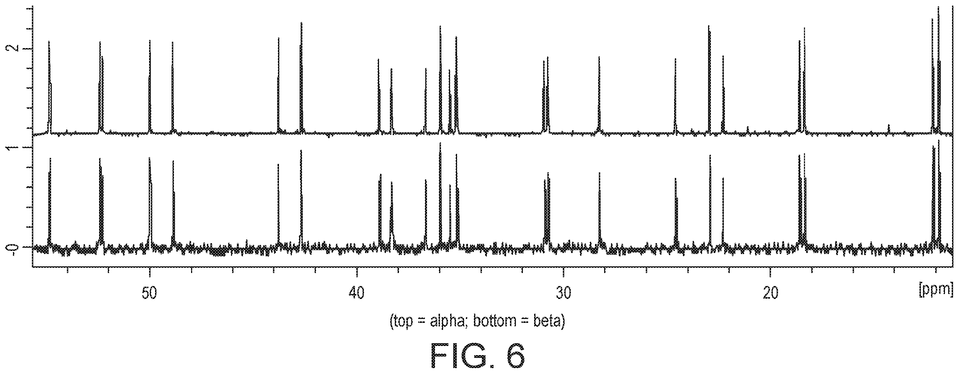

(6α, 5β)-3,7-dioxo-6-ethyl-cholan-24-oic acid ethyl ester (VIA) ((0.5 g) was dissolved in IPA (8 mL) and 0.5M aqueous NaOH (3 mL) was added. The mixture was stirred at 60 °C for 2 h. The IPA was removed under vacuum at 60 °C and then 2M HCI (10 mL) charged followed by EtOAc (50 mL) upon vigorous mixing. Phases were separated and the organic phase washed with brine (2 × 25 mL) and concentrated *in vacuo* to give the title compound (468 mg) in quantitative yield. The obtained material was identical to that synthesized from (6α, 7α, 22*E*)-6,7-epoxy-3-oxo-4,22-choladien-24-oic acid ethyl ester (IVB) by the analogous route (confirmed by ¹H and ¹³C NMR, TLC and HPLC) (see Figures 5 and 6).

### Examples 10-19 - Synthesis of (3α, 5β, 6α, 7α)-6-ethyl-3,7-dihydroxy-cholan-24-oic acid (OCA)

### Example 10 - Synthesis of (6α, 7β, 20S)-20-Acetoxymethyl-6-ethyl-7-hydroxy-pregna-4-en-3-one

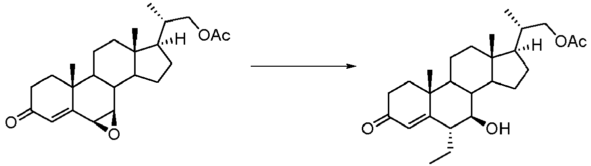

A solution of THF (14 mL) and ZnCl₂ (13 mL of a 0.5 M solution in THF, 6.5 mmol) was cooled to -15 °C. Ethylmagnesium bromide (13 mL, 1 M solution in TBME, 13 mmol) was added dropwise over 25 min. Copper (I) chloride (72 mg, 0.72 mmoL) was added and the mixture stirred at -15 °C for 10 min. (20*S*)-20-Acetoxymethyl-6,7-β-epoxy-pregna-4-en-3-one (2.8 g, 7.24 mmol) was dissolved in THF (11 mL) and added dropwise over 30 min to the organometallic mixture. The mixture was stirred at -15 °C for 1 h. Ethylmagnesium bromide (13 mL, 1 M solution in TBME, 13 mmol) was added dropwise over 30 min, and the reaction mixture stirred for 30 min. Ethylmagnesium bromide (7.5 mL, 1 M solution in TBME, 7.24 mmol) was added dropwise over 30 min, and the reaction mixture stirred for a further 30 min. Copper (I) chloride (78 mg, 0.78 mmol) was added and the mixture stirred at -15 °C for 40 min. Ammonium chloride solution (5 mL of a saturated aq.) was added dropwise and the temperature allowed to warm to 2 °C. The mixture was filtered, and the filter cake washed with TBME (80 mL). The filtrate was washed with ammonium chloride (3 x 50 mL of a saturated aq.) and sodium chloride (50 mL, 5% aq.). The organic phase was concentrated *in vacuo* to afford (6α, 7β, 20S)-20-acetoxymethyl-6-ethyl-7-hydroxy-pregna-4-en-3-one (3.0 g) as a yellow syrup which was used without further purification.

### Example 11 - Synthesis of (5β, 6α, 7β, 20S)-20-Acetoxymethyl-6-ethyl-7-hydroxy-pregna-3-one

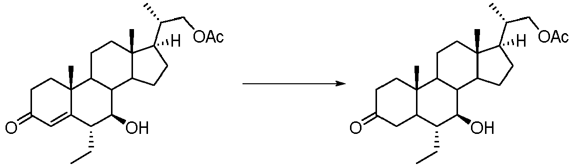

Crude (6α, 7β, 20*S*)-20-acetoxymethyl-6-ethyl-7-hydroxy-pregna-4-en-3-one (0.95 g, 2.4 mmol) was dissolved in DMF and the vessel purged with argon. Pd/CaCO₃ (210 mg) was charged and the mixture cooled to -15 °C. The mixture was degassed and filled with hydrogen three times. The reaction mixture was stirred at -15 °C for 1 h, the allowed to warm to 18 °C. After 4 h at 18 °C the mixture was filtered (0.2 µm PTFE filter) and the filter cake washed with DMF (4 mL). The filtrate was poured into water (50 mL) and extracted with TBME (50 mL). The aq. phase was re-extracted with TBME (3 x 50 mL), and the combined organics washed with sodium chloride (25 mL, 5% aq.) and concentrated *in vacuo.* The residue was purified by flash chromatography on silica gel (heptane-EtOAc) to afford (5β, 6α, 7β, 20S)-20-acetoxymethyl-6-ethyl-7-hydroxy-pregna-3-one (0.29 g, 28% yield over two steps), R_{f}: 0.46 (1:1, heptane : EtOAc); ¹H NMR (700 MHz, CDCl₃): δ = 4.08 (1H, dd, *J* 10.7, 3.4), 3.79 (1 H, dd, *J* 10.7, 7.5), 3.23 (1 H, t, *J* 9.7), 2.30 (1 H, td, *J* 7.4, 5.6), 2.27-2.23 (1H, m), 2.21-2.15 (2H, m), 2.07-2.04 (2H, m), 2.05 (3H, s), 1.91-1.80 (4H, m), 1.75-1.51 (6H, m), 1.47-1.36 (3H, m), 1.32-1.17 (3H, m), 1.07-1.00 (1H, m), 1.04 (3H, s), 1.03 (3H, d, *J* 6.6), 0.85 (3H, t, *J* 7.5), 0.74 (3H, s); ¹³C NMR (176 MHz, CDCl₃): δ = 212.1 (C-3), 171.4 (OC=O), 75.0, 69.5, 55.7, 51.9, 45.1, 44.1, 43.7, 43.6, 39.9, 39.5, 37.6, 37.0, 36.6, 35.7, 34.8, 28.1, 26.9, 22.8, 21.9, 21.0, 20.7, 17.2, 12.3, 11.1.

### Example 12 - Synthesis of (5β, 6α, 20S)-20-Acetoxymethyl-6-ethyl-pregna-3,7-dione

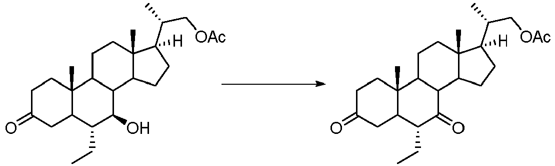

(5β, 6α, 7β, 20*S*)-20-acetoxymethyl-6-ethyl-7-hydroxy-pregna-3-one (197 mg, 0.47 mmol) was dissolved in CH₂Cl₂ (5 mL) and Dess-Martin periodinane (DMP, 240 mg, 0.56 mmol) added. The mixture was stirred at 18°C. After 1 h 40 min a second portion of DMP (124 mg, 0.29 mmol) was added. After 2 h 35 min a third portion of DMP (60 mg, 0.14 mmol) was added. After 2 h 50 min the mixture was quenched with a sodium thiosulfate/sodium bicarbonate solution (15 mL of a 10% thiosulfate / 2% bicarbonate aq.) and stirred until a negative starch/iodide test was obtained. The phases were separated and the aq. phase extracted with CH₂Cl₂ (2 x 5 mL). The combined organic phases were washed with sodium chloride (5 mL of a 5% aq.) and concentrated. The residue was purified by flash chromatography on silica gel (EtOAc-heptane) to afford (5β,6α,20*S*)-20-acetoxymethyl-6-ethyl-pregna-3,7-dione (114 mg, 59%) as a pale yellow syrup which solidified on standing, R_{f}: 0.49 (6:4, heptane:EtOAc); ¹H NMR (700 MHz, CDCl₃): δ = 4.09 (1H, dd, *J* 10.7, 3.4), 3.79 (1H, dd, *J* 10.8, 7.3), 2.76-2.73 (1H, m), 2.47 (1H, t, *J* 11.3), 2.28-2.17 (4H, m), 2.08 (1H, ddd, *J* 14.4, 5.3, 2.7), 2.05 (3H, s), 2.05-2.03 (1H, m), 1.93-1.86 (2H, m), 1.83 (1H, dd, *J* 16.5, 14.9), 1.75-1.50 (6H, m), 1.37-1.32 (1H, m), 1.36 (3H, s), 1.27-1.19 (2H, m), 1.10-1.04 (1H, m), 1.03-1.02 (3H, d, *J* 6.7), 1.00-0.95 (1H, m), 0.81 (3H, t, *J* 7.4), 0.72 (3H, s); ¹³C NMR (176 MHz, CDCl₃): δ = 212.0 (C=O), 210.5 (C=O), 171.4 (OC=O), 69.4, 52.4, 52.2, 51.8, 50.0, 48.7, 43.8, 42.8, 38.7, 38.3, 36.7, 35.9, 35.6, 35.5, 27.8, 24.7, 22.9, 22.3, 21.0, 18.6, 17.3, 12.1, 11.8.

### Example 13 - Synthesis of (5β, 6α, 20S)-6-Ethyl-20-hydroxymethyl-pregna-3,7-dione

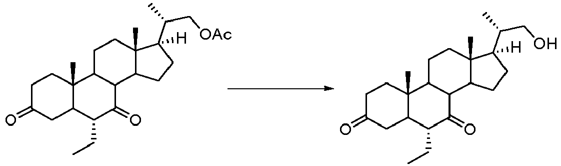

(5β, 6α, 20S)-20-Acetoxymethyl-6-ethyl-pregna-3,7-dione (96 mg, 0.23 mmol) was dissolved in methanol (5 mL) and solid sodium methoxide added to pH 11. The mixture was stirred at 18 °C for 22 h, then neutralised (pH 7-8) with 4:1 ethanol:acetic acid. The mixture was concentrated, then partitioned between EtOAc (20 mL) and water (20 mL). The organic phase was washed with sodium chloride (20 mL of a 5% aq.) and concentrated *in vacuo.* The residue was purified by flash chromatography on silica gel to afford (5β, 6α, 20S)-6-ethyl-20-hydroxymethyl-pregna-3,7-dione (67 mg, 78%) as a colourless syrup.
¹H NMR (700 MHz, CDCl₃): δ = 3.64 (1H, dd, *J* = 10.4, 2.9), 3.37 (1H, dd, *J* = 10.3, 7.1), 2.69 (1H, m), 2.47 (1H, t, *J* = 11.3), 2.30-2.16 (5H, m), 2.10-2.03 (2H, m), 1.94-1.80 (3H, m), 1.72-1.49 (6H, m), 1.43 (1H, br.s), 1.33 (3H, s), 1.32-1.17 (3H, m), 1.06 (3H, d, *J =* 6.7), 0.98 (1H, m), 0.81 (3H, t, *J* = 7.4), 0.71 (3H, s); ¹³C NMR (176 MHz, CDCl₃): δ = 212.1, 210.6, 67.8, 52.4, 52.2, 51.5, 50.0, 48.7, 43.7, 42.7, 38.8, 38.6, 38.3, 36.7, 35.9, 35.5, 27.9, 24.7, 22.9, 22.3, 18.6, 16.8, 12.2, 11.8.

### Example 14 - Synthesis of (3α, 5β, 6α, 20S)-6-ethyl-3-hydroxy-7-oxo-23, 24-dinor-cholane-22-ol [or (3α, 5β, 6α, 20S)-6-ethyl-3-hydroxy-20-hydroxymethyl-pregna-7-one]

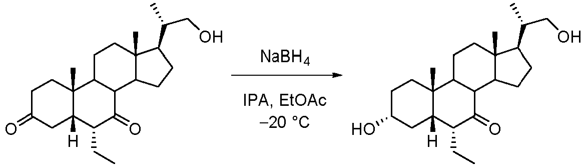

NaBH₄ (136 mg, 3.6 mmol) in IPA (6.5 vol, 9 mL) was cooled to -15 °C, then a solution of (5β, 6α, 20*S*)-6-ethyl-3,7-dioxo-23,24-dinor-cholane-22-ol (1.35 g, 0.3.6 mmol) in EtOAc (6.5 vol, 9 mL) was added dropwise over 10 mins. After 20 mins the reaction was warmed to ambient temperature and quenched by the dropwise addition of 0.7M aq. H₂SO₄ (7 vol, 9.45 mL) over 10 mins. The reaction mixture was diluted with EtOAc (50 mL) and the organic phase washed with H₂O (3 × 50 mL) and 5% aq. NaCl (50 mL). The organic phase was dried over Na₂SO₄, filtered and concentrated *in-vacuo* at 40 °C. Purification by column chromatography and concentration *in-vacuo* at 40 °C gave (3α, 5β, 6α, 20S)-6-ethyl-3-hydroxy-7-oxo-23,24-dinor-cholane-22-ol as a white crystalline solid (0.83 g, 61%). ¹H NMR (700 MHz, CDCl₃): δ = 3.64 (1H, dd, *J* = 10.5, 3.2), 3.53 (1H, m), 3.35 (1H, dd, *J* = 10.4, 7.1), 2.69 (1H, m), 2.35 (1H, t, *J* = 11.2), 2.20 (1H, m), 2.00 (1H, m), 1.92-1.67 (8H, m), 1.57-1.43 (3H, m), 1.34-1.23 (2H, m), 1.23 (3H, s), 1.21-1.10 (4H, m), 1.04 (3H, d, *J* = 6.6), 0.98-0.83 (2H, m), 0.80 (3H, t, *J* = 7.4), 0.67 (3H, s); ¹³C NMR (176 MHz, CDCl₃): δ = 212.9, 71.2, 67.9, 52.0, 51.6, 50.7, 50.0, 48.8, 43.7, 42.8, 38.9, 38.7, 35.7, 34.3, 31.8, 29.6, 27.9, 24.8, 23.5, 21.9, 18.8, 16.8, 12.1, 12.0.

### Example 15 - Synthesis of (3α, 5β, 6α, 7α, 20S)-6-ethyl-3,7-dihydroxy-23,24-dinor-cholane-22-ol (or (3α, 5β, 6α, 7α, 20S)-6-ethyl-3,7-dihydroxy-20-hydroxymethyl-pregnan)

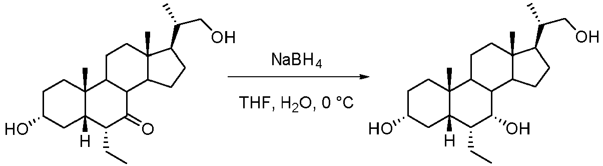

(3α, 5β, 6α, 20S)-6-ethyl-3-hydroxy-7-oxo-23,24-dinor-cholane-22-ol (0.83 g, 2.2 mmol) in THF (30 mL) and water (7.5 mL) was cooled to 0 °C and NaBH₄ (830 mg, 22 mmol) added in 4 portions over 15 mins. After 2 h the reaction was warmed to room temperature and quenched by the addition of 1:1 MeOH : H₂O (15 mL) followed by the dropwise addition of 2M aq. H₂SO₄ (11 mL) over 10 mins. The reaction mixture was diluted with EtOAc (100 mL) and washed with H₂O (100 mL). The aqueous phase was extracted with EtOAc (3 × 100 mL) and the combined organic phases were washed with 5% aq. NaCl (3 × 100 mL). The organic phase was dried over Na₂SO₄, filtered and concentrated *in-vacuo* at 40 °C to give (3α, 5β, 6α, 7α, 20*S*)-6-ethyl-3,7-dihydroxy-23,24-dinor-cholane-22-ol as a white solid (0.53 g, 64%). ¹H NMR (700 MHz, MeOD): δ = 3.64 (1H, s), 3.57 (1H, dd, *J* = 10.6, 3.1), 3.30 (1H, m), 3.23 (1H, dd, *J* = 10.5, 7.4), 2.00 (1H, m), 1.90-1.70 (6H, m), 1.59 (1H, m), 1.57-1.44 (6H, m), 1.42-1.27 (5H, m), 1.21 (2H, m), 1.13 (1H, m), 1.04 (3H, d, *J* = 6.6), 1.00 (1H, m), 0.91 (3H, s), 0.90 (3H, t, *J* = 7.7), 0,71 (3H, s); ¹³C NMR (176 MHz, MeOD): δ = 71.7, 69.7, 66.5, 52.5, 50.0, 45.5, 42.3, 41.7, 40.1, 39.5, 38.8, 35.3, 35.1, 33.1, 32.9, 29.8, 27.5, 23.2, 22.3, 22.0, 20.5, 15.9, 10.9, 10.6.

### Example 16 - Synthesis of (3α, 5β, 6α, 7α)-6-ethyl-3,7-dihydroxy-23,24-dinor-cholane-22-al

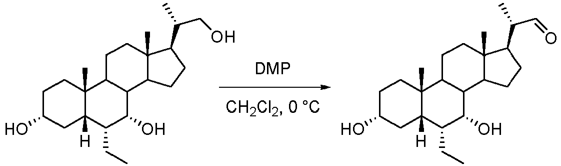

(3α, 5β, 6α, 7α, 20*S*)-6-ethyl-3,7-dihydroxy-23,24-dinor-cholane-22-ol (421 mg, 1.11 mmol) in DMF (50 vol, 20 mL) was cooled to 0°C. Dess Martin periodinane (473 mg, 1.12 mmol) was charged in portions. After 2.5 h (TLC, eluant 7:3 EtOAc: Heptane; visualized with Cerium Ammonium Molybdate stain), the reaction was quenched by the addition of 10% aq. NaHSO₃/2% aq. NaHCO₃ (5 mL) and the mixture stirred for 10 mins. The mixture was diltuted with EtOAc (100 mL) and 5% NaCl (5 mL). The aqueous layer was extracted with EtOAc (50 mL). The combined organic phases were washed with 2M aq. NaOH (50 mL) and 5% aq. NaCl (4 × 50 mL), dried over Na₂SO₄, filtered and concentrated *in-vacuo* at 40°C. Purification by column chromatograph gave (3α, 5β, 6α, 7α)-6-ethyl-3,7-dihydroxy-23,24-dinor-cholane-22-al as a 3:1 mixture with (5β, 6α, 7α)-6-ethyl-7-hydroxy-7-oxo-23,24-dinor-cholane-22-al (white foam, 230 mg). ¹H NMR (700 MHz, CDCl₃): δ = 9.56 (1H, d, J = 3.4), 3.71 (1H, br. s), 3.44 - 3.36 (1H, m), 2.38 - 2.33 (1H, m), 1.94 - 1.86 (2H, m), 1.83 - 1.81 (2H, m), 1.80 - 1.78 (2H, m), 1.74 - 1.36 (10H, m), 1.34 - 1.18 (8H, m), 1.14 (3H, d, J = 6.8), 0.91 (3H, s), 0.88 (3H, t, J = 7.07), 0.71 (3H, s). ¹³C NMR (176 MHz, CDCl₃): δ = 205.1, 72.3, 70.9, 51.0, 49.9, 49.5, 45.1, 43.3, 41.2, 40.0, 39.3, 35.6, 35.5, 34.0, 33.4, 30.6, 27.1, 24.1, 23.1, 22.2, 20.7, 13.5, 12.2, 11.6.

### Example 17 - Synthesis of (3α, 5β, 6α, 7α)-6-ethyl-3,7-dihydroxy-22-cholen-24-oic acid ethyl ester

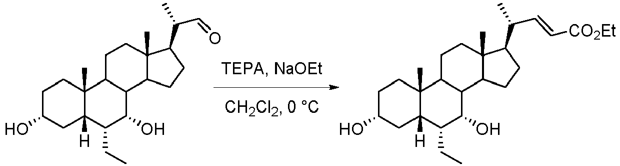

The HWE reagent was prepared by dropwise addition of TEPA (262 µL, 1.32 mmol) to NaOEt (91 mg, 1.3 mmol) in CH₂Cl₂ (2 mL) at 0°C. Thr reaction mixture was added dropwise over 10 minutes to a solution of (3α, 5β, 6α, 7α)-6-ethyl-3,7-dihydroxy-23,24-dinor-cholane-22-al (199 mg, 0.528 mmol) in CH₂Cl₂ (4 mL) at 0 °C. The reaction was warmed to ambient temperature and stirred for 1 hour (TLC, eluant 1:1 EtOAc: Heptane; visualized with Cerium Ammonium Molybdate stain). The mixture was diluted with H₂O (20 mL) and CH₂Cl₂ (15 mL). The aqueous layer was separated and extracted with CH₂Cl₂ (3 × 20 mL). The combined organic phases were dried over Na₂SO₄, filtered and concentrated. Purification by column chromatography gave (3α, 5β, 6α, 7α)-6-ethyl-3,7-dihydroxy-22-cholen-24-oic acid ethyl ester as a white foam (158 mg). The isolated product is a 4:1 mixture of the desired (3α, 5β, 6α, 7α)-6-ethyl-3,7-dihydroxy-22-cholen-24-oic acid ethyl ester and (5β, 6α, 7α)-6-ethyl-7-dihydroxy-3-oxo-22-cholen-24-oic acid ethyl ester. ¹H NMR (700 MHz, CDCl₃): δ= 6.83 (1H, dd, J = 9.0, 15.6), 5.73 (1H, d, J = 15.3), 4.17 (2H, q, J = 7.1), 3.69 (1H, m), 3.40 (1H, m), 2.30 - 2.25 (1H, m), 1.92 (1H, m), 1.85 - 1.76 (2H, m), 1.76 - 1.62 (5H, m), 1.59 (1H, m), 1.54 - 1.34 (7H, m), 1.29 (3H, t, J = 7.1), 1.33 - 1.23 (6H, m), 1.09 (3H, d, J = 6.6), 0.90 (3H, s), 0.90 (3H, t, J = 7.4), 0.68 (3H, s). ¹³C NMR (176 MHz, CDCl₃): δ = 167.1, 154.7, 119.0, 72.3, 70.8, 60.1, 54.9, 50.4, 45.2, 43.0, 41.0, 40.1, 39.8, 39.5, 35.6, 35.5, 34.0, 33.3, 30.6, 28.2, 23.7, 23.1, 22.2, 20.7, 19.3, 14.3, 12.1, 11.7.

### Example 18 - Synthesis of (3α, 5β, 6α, 7α)-6-ethyl-3,7-dihydroxy-cholan-24-oic acid ethyl ester

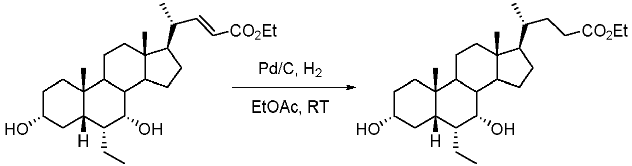

10% Palladium on Carbon (79 mg) was charged to a flask under argon. A solution of (3α, 5β, 6α, 7α)-6-ethyl-3,7-dihydroxy-22-cholen-24-oic acid ethyl ester (135 mg, 0.312 mmol) in EtOAc (51 vol, 7.0 mL) was charged and purged with H₂. After 70 h (TLC, eluant 1:1 EtOAc: Heptane; visualized with Anisaldehyde stain) the reaction mixture was filtered through a 0.45 µm PTFE filter and the filter washed with EtOAc (10 mL). Concentration *in-vacuo* at 40 °C gave (3α, 5β, 6α, 7α)-6-ethyl-3,7-dihydroxy-cholan-24-oic acid ethyl ester (134 mg) as a 4:1 mixture with (5β, 6α, 7α)-6-ethyl-7-hydroxy-3-oxo-cholan-24-oic acid ethyl ester. ¹H NMR (500 MHz, CDCl₃): δ= 4.13 (2H, q, J = 7.2), 3.46 - 3.37 (1H, m), 2.41 - 2.32 (1H, m), 2.28 - 2.19 (1H, m), 1.89 - 1.76 (6H, m), 1.76 - 1.57 (5H, m), 1.54 - 1.34 (12H, m), 1.27 (3H, t, J = 7.1), 1.25 - 1.12 (4H, m), 0.98 - 0.88 (9H, m), 0.68 (3H, s). ¹³C NMR (126 MHz, CDCl₃): δ = 167.1, 154.7, 119.0, 72.3, 70.8, 60.1, 54.9, 50.4, 45.2, 43.0, 41.0, 40.1, 39.8, 39.5, 35.6, 35.5, 34.0, 33.3, 30.6, 28.2, 23.7, 23.1, 22.2, 20.7, 19.3, 14.3, 12.1, 11.7.

### Example 19 - Synthesis of (3α, 5β, 6α, 7α)-6-ethyl-3,7-dihydroxy-cholan-24-oic acid (obeticholic acid)

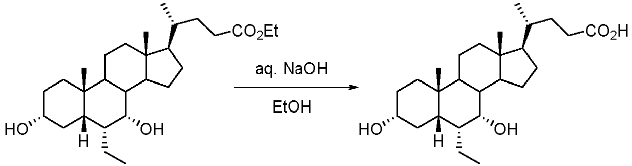

To (3α, 5β, 6α, 7α)-6-ethyl-3,7-dihydroxy-cholan-24-oic acid ethyl ester (118 mg, 0.272 mmol) in EtOH (34 vol, 4 mL) at 50°C, was added 0.5M aq. NaOH (1.2 mL, 0.61 mmol) dropwise. The reaction mixture was stirred at 50°C for 2.5 h (TLC, eluent 1:1 EtOAc: Heptane; visualized with Cerium Ammonium Molybdate stain) and then 0.5M aq. NaOH (1 mL, 0.5 mmol) was added. After 1 h, the reaction was quenched with 3M aq. HCI (2 mL). The aqueous phase was separated and extracted with EtOAc (3 × 15 mL). The combined organic phases were dried over Na₂SO₄, filtered and concentrated *in-vacuo* at 40°C. Purification by column chromatography gave (3α, 5β, 6α, 7α)-6-ethyl-3,7-dihydroxy-cholan-24-oic acid (108 mg, white foam) as a 4:1 mixture with (5β, 6α, 7α)-6-ethyl-3,7-dihydroxy-cholan-24-oic acid. NMR data was consistent with an authentic sample of OCA.

### Examples 20 and 21 - Synthesis of (3a, 5β, 6α, 7β)-6-ethyl-3,7-dihydroxy-cholan-24-oic acid (analogue of obeticholic acid (OCA))

### Example 20 - Synthesis of (5β, 6α, 7β)-6-ethyl-7-hydroxy-3-oxo--cholan-24-oic acid

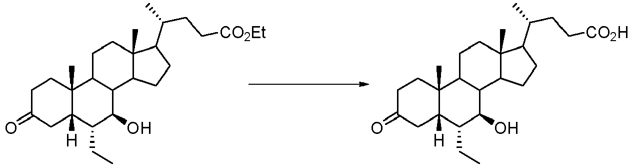

To a solution of (6α, 7β)-6-ethyl-7-hydroxy-3-oxo-cholanic acid ethyl ester (510 mg, 1.14 mmol) in IPA (20 mL) was added 0.5 M aq. NaOH (10 mL) and the mixture was heated at 60 °C for 2.5 h. The volatiles were removed under reduced pressure and the residue was partitioned between EtOAc (10 mL) and 10% aq. citric acid (10 mL). The layers were separated and the aqueous was extracted with EtOAc (2 x 25 mL). The combined organics were washed with 10% aq. NaCl (40 mL), dried over sodium sulfate and were concentrated under reduced pressure. The material was used directly in the next step without purification.

### Example 21 - Synthesis of (3α, 5β, 6α, 7β)-6-ethyl-3,7-dihydroxy-cholan-24-oic acid (7-beta-hydroxy isomer of obeticholic acid (OCA))

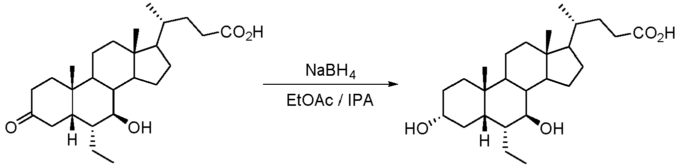

To a solution of (6α, 5β, 7β)-6-ethyl-7-hydroxy-3-oxo-cholan-24-oic acid ethyl ester (473 mg, 1.13 mmol) in EtOAc (20 mL) was added IPA (4 mL) and the mixture was cooled in an ice bath. NaBH₄ (214 mg, 5.65 mmol) was added and the reaction was allowed to warm to room temperature and was stirred for 17 h. EtOAc (10 mL) and 10% aq. Citric acid (15 mL) were added and the layers were separated. The aqueous layer was extracted with EtOAc (2 x 15 mL). The combined organic phases were washed with 10% aq. NaCl (15 mL), dried over sodium sulfate and concentrated under reduced pressure. The residue was purified by column chromatography on silica gel (10-50% acetone in toluene) to give the desired product (300 mg, 63%) as a colourless solid. ¹H NMR (400 MHz, MeOD); δ = 3.45 (1H, m), 3.08 (1H, dd, *J* 10.4, 9.4), 2.33 (1H, ddd, *J* 15.3, 9.8, 5.3), 2.20 (1H (ddd, *J* 16.2, 9.4, 6.9), 2.04 (1H, br. dt, *J* 12.2, 2.9), 1.98-1.76 (5H, m), 1.75-1.34 (10H, m), 1.33-1.00 (11H, m), 0.97 (3H, d, *J* 6.6, C21-C*H*₃), 0.95 (3H, s, C19-C*H*₃), 0.86 (3H, t, *J* 7.4, ethyl C*H*₃), 0.72 (3H, s, C18-C*H*₃); ¹³C NMR (100 MHz, MeOD) δ = 178.2, 76.3, 72.4, 57.8, 56.6, 45.2, 45.0, 44.7, 44.6, 41.7, 41.1, 36.7, 36.5, 35.6, 32.4, 32.0, 31.2, 30.8, 29.6, 27.9, 24.0, 22.6, 22.0, 18.9, 12.7, 11.5.

### Examples 22-40 - Synthesis of further epoxidation precursors

### Example 22 - Synthesis of (20S)-20-hydroxymethyl-pregna-4-en-3-one

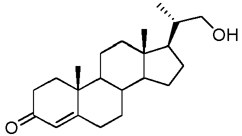

(20*S*)-20-Hydroxymethyl-pregna-4-en-3-one (HMPO) can be prepared by chemoselective reduction of dinorcholenaldehyde ((20S)-20-formyl-pregn-4-en-3-one) with NaBH₄ in primary alcohol (Barry M. Trost, Alvin C. Lavoie J. Am. Chem. Soc., 1983, 105 (15), 5075-5090).

### Example 23 - Synthesis of (20S)-20-acetoxymethyl-pregna-4,6-dien-3-one

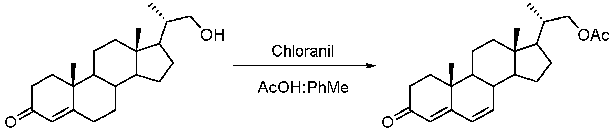

HMPO (300 g, 0.913 mol) was charged to a reaction vessel, followed by AcOH (0.9 L) and toluene (0.3 L) with stirring. *p*-Chloranil (245 g, 1.00 mol) was then charged and the reaction mixture heated to 110 °C and maintained at this temperature for 6 h. The mixture was then cooled to 5 °C and held at that temperature for 2 h. The resulting solid was filtered and the filter-cake washed with cold, premixed 3:1 AcOH : Toluene (4 x 150 mL) and the filtrate was concentrated *in-vacuo.* The residue was dissolved in acetone (900 mL), then 3.5% w/w aqueous NaOH (3.0 L) was charged dropwise with stirring, maintaining the temperature below 30 °C. The resulting solids were collected by filtration and the filter cake was washed with premixed 1:1 acetone : water (1.5 L). The filter cake was then slurried in 1:1 acetone : water (600 mL) at 20 °C, filtered and washed with premixed 1:1 acetone : water (1.0 L). The solid was dried under vacuum at 65-70 °C to give the desired product (224 g, 67%) as a tan solid. δH (400 MHz, CDCl₃); 6.17-6.12 (1H, m, C6-C*H*), 6.10 (1H, dd, *J* 9.9, 2.0, C7-C*H*), 5.68 (1H, s, C4-C*H*), 4.10 (1H, dd, *J* 10.7, 3.5, C22-C*H*ₐH_{b}), 3.79 (1H, dd, *J* 10.7, 7.4, C22-CHa*H*_{b}), 2.58 (1H, ddd, *J* 17.9, 14.4, 5.4, C2-C*H*a*H*_{b}), 2.49-2.39 (1H, m, C2-C*H*a*H*_{b}), 2.20 (1H, brt, *J* 10.2, C8-C*H*), 2.10-1.97 (1H, m), 2.06 (3H, s, OC(O)C*H*₃), 1.96-1.66 (4H, m), 1.62-1.53 (1H, m), 1.52-1.16 (8H, m), 1.12 (3H, s, C19-C*H*₃), 1.04 (3H, d, J 6.6, C21-C*H*₃), 0.79 (3H, s, C18-C*H*₃); δC (100 MHz, CDCl₃); 199.6, 171.3, 163.8, 141.2, 127.9, 123.6, 69.4, 53.2, 52.6, 50.7, 43.6, 39.4, 37.7, 36.1, 35.8, 33.9, 33.9, 27.6, 23.8, 21.0, 20.7, 17.1, 16.3, 11.9.

### Example 24 - Synthesis of (20S)-20-hydroxymethyl-pregna-4,6-dien-3-one

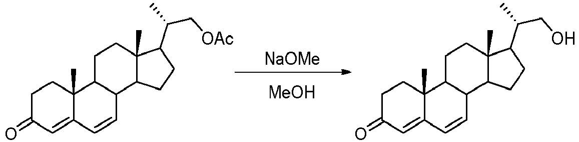

(20S)-20-Acetoxymethyl-pregna-4,6-dien-3-one (25 g, 67.5 mmol) was suspended in MeOH (250 mL) and sodium methoxide (25% w/v solution in MeOH) was added until pH 12 was achieved. The resulting mixture was stirred at room temperature for 4 h. The pH was adjusted to pH 4 by addition of Finex CS08GH⁺ resin. The mixture was filtered and the filtrate was concentrated under reduced pressure, co-evaporating with PhMe (2 × 250 mL). The residue was dried in a vacuum oven at 30 °C for 48 h to give the desired product (22.15 g, 99%) as a light brown solid. δH (400 MHz, CDCl₃); 6.16-6.11 (1H, m, C7-C*H*), 6.09 (1H, dd, *J* 9.9, 2.3, C6-C*H*), 5.67 (1H, s, C4-C*H*), 3.65 (1H, dd, *J* 10.5, 3.3, C22-C*H*ₐH_{b}), 3.59 (1H, dd, *J* 10.5, 6.7, C22-CHₐ*H*_{b}), 2.57 (1H, ddd, *J* 18.0, 14.4, 5.5, C2-C*H*ₐH_{b}), 2.45-2.38 (1H, m, C2-C*H*ₐ*H*_{b}), 2.19 (1H, brt, *J* 10.4, C8-C*H*), 2.11-1.76 (5H, m), 1.71 (1H, td, *J* 13.9, 5.3, C1-C*H*ₐH_{b}), 1.65-1.16 (9H, m), 1.11 (3H, s, C19-C*H*₃), 1.06 (3H, d, *J* 6.6, C21-C*H*₃), 0.78 (3H, s, C18-C*H*₃); δC (100 MHz, CDCl₃); 199.7, 164.0, 141.4, 127.9, 123.5, 67.8, 53.2, 52.3, 50.7, 43.5, 39.4, 38.7, 37.8, 36.1, 33.9, 33.9, 27.6, 23.8, 20.7, 16.7, 16.3, 12.0.

### Example 25 - Synthesis of (20S)-20-tertbutyldimethylsilyloxymethyl-pregna-4,6-dien-3-one

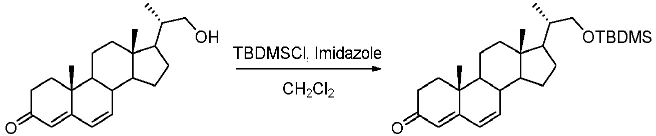

(20S)-20-Hydroxymethyl-pregna-4,6-dien-3-one (1.00 g, 3.04 mmol) was dissolved in anhydrous CH₂Cl₂ (10 mL) and the solution was cooled to 0 °C. Imidazole (414 mg, 6.09 mmol) and TBDMSCI (551 mg, 3.65 mmol) were added and the reaction was stirred at 0 °C for 4 h. The reaction was warmed to room temperature and CH₂Cl₂ (10 mL) and water (20 mL) were added. The layers were separated and the organic phase was washed with water (20 mL), saturated aqueous sodium chloride (20 mL), dried over sodium sulfate and was concentrated under reduced pressure. The residue was purified by column chromatography on silica gel (0-25% EtOAc in heptane) to give the desired product (890 mg, 66%) as a light yellow solid. δH (400 MHz, CDCl₃); 6.14 (1H, dd, *J* 9.9, 1.3, C7-C*H*), 6.09 (1H, dd, *J* 9.8, 2.4, C6-C*H*), 5.66 (1H, s, C4-C*H*), 3.58 (1H, dd, *J* 9.7, 3.4, C22-CHₐ*H*_{b}), 3.28 (1H, dd, *J* 9.7, 7.2, C22-CHₐ*H*_{b}), 2.57 (1H, ddd, *J* 17.9, 14.4, 5.4, C2-C*H*ₐ*H*_{b}), 2.47-2.37 (1H, m, C2-C*H*ₐ*H*_{b}), 2.19 (1H, brt, *J* 10.3, C8-C*H*), 2.07 (1H, dt, *J* 12.9, 3.3), 2.00 (1H, dd, *J* 8.5, 2.1), 1.94-1.63 (3H, m), 1.60-1.15 (9H, m), 1.11 (3H, s, C19-C*H*₃), 1.00 (3H, d, *J* 6.7, C21-C*H*₃), 0.89 (9H, s, SiC(C*H*₃)₃), 0.77 (3H, s, C18-C*H*₃), 0.03(6H, s, Si(C*H*₃)₂); δC (100 MHz, CDCl₃); 199.6, 163.9, 141.5, 127.8, 123.5, 67.7, 53.2, 52.5, 50.7, 43.5, 39.4, 39.0, 37.8, 36.1, 34.0, 33.9, 27.6, 25.9, 25.9, 25.9, 23.9, 20.7, 18.4, 16.9, 16.3, 12.0, -5.3, -5.4; (IR) vₘₐₓ(cm⁻¹): 3027, 2956, 2930, 2891, 2857, 1677, 1077, 753; HRMS (ESI-TOF) *m*/*z:* (M+H)⁺ calculated for C₂₈H₄₆O₂Si 442.3267, found 443.3338.

### Example 26 - Synthesis of (20S)-tert-butyldiphenylsilyloxymethyl-pregna-4,6-dien-3-one

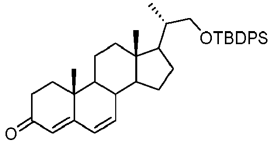

To a solution of (20S)-hydroxymethyl-pregna-4,6-dien-3-one (500 mg, 1.5 mmol) in DMF (5mL, 10vol) under argon was added TBDPSCI (510mg, 1.2 eq) and imidazole (217mg, 2 eq) and the reaction stirred at 20 °C. After 16 h the reaction mixture was poured onto H₂O (50 mL) and extracted with TBME (2 x 20 mL). The combined organic phases were washed with aqueous 5% w/v NaCl (2 x 20 mL), dried over Na₂SO₄, filtered and concentrated *in vacuo.* Purification by column chromatography on silica gel afforded (20*S*)-*tert-*butyldiphenylsilyloxymethyl-pregna-4,6-dien-3-one (742 mg, 86%), R_{f} 0.69 (1:1, EtOAc:Heptane); ¹H NMR (700 MHz, CDCl₃): 7.67 -7.65 (4H, m), 7.43 - 7.40 (2H, m), 7.39-7.36 (4H, m), 6.13-6.07 (2H, m), 5.66 (1H, s), 3.61 (1H, dd, J 9.3, 3.3), 3.37 (1H, dd, J 9.8, 6.9), 2.57 (1H, ddd, J 18.0, 14.6, 5.5), 2.45-2.40 (1H, m), 2.17 (1H, t, J 8.3), 2.06 (1H, dt, J 12.9, 3.4), 2.00 (1H, ddd, J 13.2, 5.4, 2.0), 1.75-1.67 (3H, m), 1.58-1.53 (1H, m), 1.43 (1H, qd, J 12.9, 3.9), 1.32-1.16 (7H, m), 1.11 (3H, s), 1.10 (3H, d, J 2.7), 1.05 (9H, s), 0.73 (3H, s); ¹³C NMR (175 MHz, CDCl₃): 199.7, 164.0, 141.5, 135.7, 135.6, 134.1, 129.5, 127.8, 127.6, 127.5, 123.5, 68.5, 53.2, 50.6, 43.5, 39.4, 39.0, 37.8, 36.1, 34.0, 33.9, 27.5, 26.9, 23.8, 20.7, 19.4, 17.2, 16.3, 14.1, 11.9.

### Example 27 - Synthesis of (20S)-20-formyl-pregna-4,6-dien-3-one

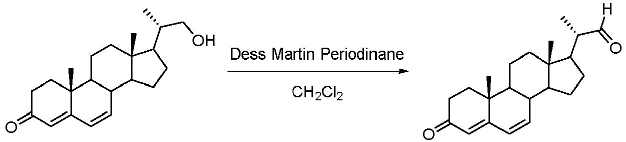

(20S)-20-Hydroxymethyl-pregna-4,6-dien-3-one (3.01 g, 9.16 mmol) was dissolved in anhydrous CH₂Cl₂ (60 ml) and the solution was cooled to 0 °C. Dess-Martin periodinane (5.83 g, 13.7 mmol) was added portion-wise over 10 minutes and the reaction was allowed to slowly warm to room temperature and was stirred for 22 h. The mixture was cooled to 0 °C and a 1 : 1 mixture of 10% aq. Na₂S₂O₃ and 2% aq. NaHCO₃ (75 ml) was added portionwise. CH₂Cl₂ (50 mL) was added and the layers were separated. The aqueous phase was extracted with CH₂Cl₂ (2 × 50 mL) and the combined organics were dried over sodium sulfate and concentrated under reduced pressure. The residue was purified by column chromatography on silica gel (0-25% EtOAc in heptane) to give the desired product (1.23 g, 41%) as a pale yellow solid. δH (400 MHz, CDCl₃); 9.59 (1H, d, *J* 3.2, C*H*O), 6.12 (2H, s, C6-C*H* and C7-C*H*), 5.68 (1H, s, C4-C*H*), 2.58 (1H, ddd, *J* 17.9, 14.4, 5.4), 2.49-2.36 (2H, m), 2.22 (1H, t, *J* 10.6, C8-C*H*), 2.08-1.81 (4H, m), 1.73 (1H, td, *J* 13.8, 5.1, C1-C*H*ₐH_{b}), 1.65-1.20 (8H, m), 1.15 (3H, d, *J* 6.9, C21-C*H*₃), 1.13 (3H, s, C19-C*H*₃), 0.82 (3H, d, C18-C*H*₃); δC (100 MHz, CDCl₃); 204.6, 199.5, 163.6, 140.8, 128.1, 123.7, 52.8, 50.8, 50.7, 49.4, 44.0, 39.2, 37.6, 36.0, 33.9, 33.9, 27.0, 24.1, 20.6, 16.3, 13.5, 12.3; (IR) Vₘₐₓ(cm⁻¹): 3030, 2934, 2706, 1717, 1655, 1615, 15811; HRMS (ESI-TOF) *m*/*z:* (M+H)⁺ calculated for C₂₂H₃₀O₂ 326.2246; found 327.2318.

### Example 28 - Synthesis of (20S)-20-(ethylenedioxymethyl)-pregna-4,6-dien-3-one

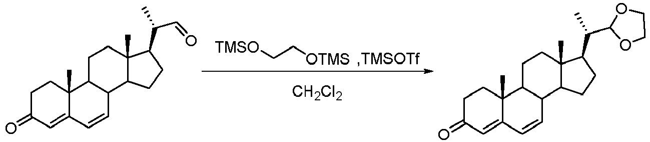

To a solution of (20S)-20-formyl-pregna-4,6-dien-3-one (3.89 g, 12 mmol) in CH₂Cl₂ (5 vol, 20 mL) under an argon atmosphere was added 1,2-bis (trimethylsilyloxy) ethane (2.94 mL, 12 mmol). The reaction mixture was cooled to -78 °C and TMSOTf (108 µL, 0.6 mmol) was added. After 2 h the reaction mixture was diluted with CH₂Cl₂ (100 mL) and washed with water (2 × 100 mL) and 5% aq. NaCl (100 mL). The organic phase was dried over Na₂SO₄ and was concentrated under reduced pressure. Purification by column chromatography on silica gel gave the desired product (2.42 g, 55%) as a colourless crystalline solid. δH (700 MHz, CDCl₃); 6.12 (2H, m), 5.67 (1H, m), 4.86 (1H, d, *J* 2.0), 3.94 (2H, m), 3.86 (2H, m,), 2.56 (1H, m), 2.43 (1H, m), 2.19 (1H, t, *J* 10.6), 2.05-1.95 (3H, m), 1.85 to 1.20 (12H, m), 1.11 (3H, s), 0.95 (3H, d, *J* 6.7), 0.77 (3H, s). δC (176 MHz, CDCl₃); 199.7, 163.9, 141.4, 127.9, 123.6, 105.6, 65.3, 65.1, 52.9, 52.2, 50.6, 43.7, 39.3, 39.3, 37.8, 36.1, 34.0, 33.9, 27.3, 23.9, 20.67, 16.3, 11.7, 11.6.

### Example 29 - Synthesis of (20S)-20-(1-mesyloxymethyl)-pregna-4,6-dien-3-one

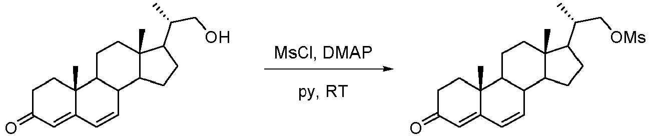

To a solution of (20S)-20-hydroxymethyl-pregna-4,6-dien-3-one (1.00 g, 3.05 mmol) in pyridine (10 mL) was added DMAP (19 mg, 0.15 mmol). MsCI (1.18 mL, 15.2 mmol) was added dropwise and the reaction was stirred at room temperature for 18 h. The reaction was cooled in an ice bath and water (10 mL) was added dropwise. EtOAc (20 mL) was added and the layers were separated. The aqueous layer was extracted with EtOAc (3 x 20 mL). The combined organic phases were washed with 2 M aq. HCI (20 mL), dried over sodium sulfate and were concentrated under reduced pressure. The residue was purified by column chromatography on silica gel (0-50% EtOAc in heptane) to give the desired product (1.01 g, 82%) as an orange solid. δH (400 MHz, CDCl₃); 6.12 (2H, brs, C6-C*H* and C7-C*H*), 5.68 (1H, s, C4-C*H*), 4.21 (1H, dd, *J* 9.4, 3.2, C22-C*H*ₐH_{b}), 4.01 (1H, dd, *J* 9.4, 6.6, C22-CHₐ*H*_{b}), 3.01 (3H, s, OS(O₂)C*H*₃), 2.58 (1H, ddd, *J* 18.0, 14.4, 5.5, C2-C*H*ₐH_{b}), 2.49-2.39 (1H, m, C2-CHₐ*H*_{b}), 2.21 (1H, brt, *J* 10.5, C8-C*H*), 2.09-1.80 (5H, m), 1.73 (1H, td, *J* 13.8, 5.2, C1-C*H*ₐH_{b}), 1.63-1.53 (1H, m), 1.52-1.18 (7H, m), 1.13 (3H, s, C19-C*H*₃), 1.12 (3H, d, *J* 6.1, C21-C*H*₃), 0.80 (3H, s, C18-C*H*₃); δC (100 MHz, CDCl₃); 199.5, 163.6, 140.9, 128.0, 123.7, 74.8, 53.1, 51.8, 50.6, 43.6, 39.3, 37.7, 37.2, 36.3, 36.0, 33.9, 33.9, 27.5, 23.8, 20.6, 16.9, 16.3, 12.0.

### Example 30 - Synthesis of (20S)-20-(bromomethyl)-pregna-4,6-dien-3-one

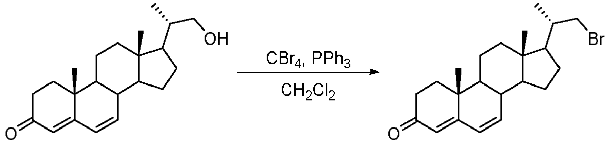

To a solution of (20S)-20-hydroxymethyl-pregna-4,6-dien-3-one (1.00 g, 3.05 mmol) in anhydrous CH₂Cl₂ (10 mL) was added carbon tetrabromide (1.52 g, 4.57 mmol). Triphenylphosphine (1.20 g, 4.57 mmol) was added and the mixture was heated at reflux for 2 h. The reaction was allowed to cool to room temperature and water (20 mL) was added. The layers were separated and the organic layer was washed with 5% aq. NaHCO₃ (20 mL), 10% aq NaCl (20 mL) and was concentrated under reduced pressure. The residue was purified by column chromatography on silica gel (0-25% acetone in heptane) to give the desired product (980 mg, 82%) as a light yellow crystalline solid. δH (400 MHz, CDCl₃); 6.09-6.00 (2H, m, C6*H* and C7*H*), 5.59 (1H, s, C4*H*), 3.43 (1H, dd, *J* 9.8, 2.7, C22*H*ₐH_{b}), 3.29 (1H, dd, *J* 9.8, 5.8, C22Hₐ*H*_{b}), 2.50 (1H, ddd, *J* 17.9, 14.4, 5.4, C2*H*ₐH_{b}), 2.40-2.30 (1H, m, C2*H*ₐH_{b}), 2.13 (1H, brt, *J* 9.8, C8*H*), 2.01-1.57 (5H, m), 1.55-1.45 (1H, m), 1.44-1.10 (8H, m), 1.05 (3H, s, C19*H*₃), 1.03 (3H, d, *J* 6.5, C21*H*₃), 0.72 (3H, s, C18*H*₃); δC (100 MHz, CDCl₃); 199.2, 163.6, 141.0, 127.9, 123.6, 53.5, 53.1, 50.6, 43.4, 43.3, 39.2, 37.7, 37.6, 36.0, 33.9, 33.9, 27.4, 23.6, 20.6, 18.6, 16.3, 12.3;

### Example 31 - Synthesis of (20S)-(N-phthalimidomethyl)-pregna-4,6-dien-3-one

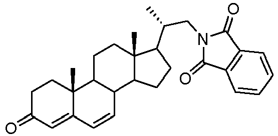

(20S)-Bromomethyl-pregna-4,6-dien-3-one (1.25 g, 3.2 mmol) was dissolved in DMF (25 mL, 20 vol) and potassium phthalimide (0.65 g, 1.1 eq) was added. The mixture was stirred at 50 °C under argon for 65 h and cooled to 25 °C. TBME (80 mL, 64 vol) was added and the reaction mixture was washed with water (80 mL, 64 vol). The aqueous phase was separated, extracted with TBME (80 mL) and the organic phases were combined, washed with 0.2M NaOH (80 mL), aqueous 5% w/v NaCl (80mL) and concentrated to give (20*S*)-(*N*-phthalimidomethyl)-pregna-4,6-dien-3-one (0.97 g, 66%). R_{f}: 0.30 (3:7, EtOAc:Heptane); ¹H NMR (700 MHz, CDCl₃): 7.84 (2H, m), 7.72 (2H, m), 6.15 (1H, dd, J 9.7, 1.4), 6.11 (1H, dd, J 9.8, 2.7), 5.67 (1H, s), 3.65 (1H, dd, J 13.3, 3.8), 3.44 (1H, dd, J 13.6, 10.5), 2.57 (1H, ddd, J 17.8, 14.4, 5.4), 2.43 (1H, m), 2.21 (1H, t, J 10.6), 2.11-2.03 (2H, m), 2.02-1.96 (2H, m), 1.87 (1H, m), 1.72 (1H, td, J 13.9, 5.1), 1.66, (1H, m), 1.55 (1H, m), 1.43 (1H, qd, J 13.1, 4.0), 1.36 (1H, m), 1.29-1.20 (4H, m) 1.11 (3H, s), 0.91 (3H, d, J 6.6), 0.80, (3H, s); ¹³C NMR (175 MHz, CDCl₃): 199.7, 168.8, 163.9, 141.3, 133.9, 132.1, 127.9, 123.6, 123.2, 54.5, 53.2, 50.6, 43.8, 43.7, 39.4, 37.7, 36.2, 36.1, 34.0, 33.9, 27.8, 23.9, 20.6, 17.0, 16.3, 12.0.

### Example 32 - Synthesis of 23-ethoxyformyl-3-oxo-4,6-choladien-24-oic acid ethyl ester

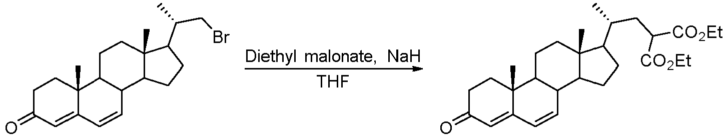

Sodium hydride (60% dispersion in mineral oil, 226 mg, 5.64 mmol) was suspended in anhydrous THF (10 mL) and the mixture was cooled to 0 °C. Diethyl malonate (1.17 mL, 7.68 mmol) was added drop-wise and the mixture was stirred at 0 °C for 15 minutes. A solution of (20*S*)-20-(bromomethyl)-pregna-4,6-dien-3-one (1.00 g, 2.56 mmol) in anhydrous THF (10 mL) was added drop-wise and the reaction was heated at reflux for 18 h. The reaction was allowed to cool to room temperature and water (10 mL) was added. EtOAc (25 mL) was added and the layers were separated. The aqueous layer was extracted with EtOAc (3 x 50 mL) and the combined organics were washed with 10% aq. NaCl (50 mL), dried over sodium sulfate and were concentrated under reduced pressure. The residue was purified by column chromatography on silica gel (0-25% acetone in heptane) to give the desired product (1.00 g, 83%) as a clear oil. δH (400 MHz, CDCl₃); 6.17-6.07 (2H, m, C6*H* and C7*H*), 5.67 (1H, s, C4*H*), 4.29-4.14 (4H, m, 2x C(O)OC*H*₂), 3.44 (1H, dd, *J* 10.9, 3.7, EtO₂CC*H*), 2.57 (1H, ddd, *J* 17.9, 14.4, 5.4, C2*H*ₐH_{b}), 2.43 (1H, dddd, *J* 17.8, 5.1, 2.0, 0.8, C2Hₐ*H*_{b}), 2.24-2.12 (2H, m), 2.10-1.93 (3H, m), 1.87-1.77 (1H, m), 1.71 (1H, td, *J* 16.2, 5.2, C1*H*ₐH_{b}), 1.59-1.35 (4H, m), 1.34-1.14 (12H, m), 1.11 (3H, s, C18*H*₃), 0.96 (3H, d, *J* 6.2, C21*H*₃), 0.75 (3H, s, C19*H*₃); δC (100 MHz, CDCl₃); 199.5, 170.0, 169.6, 163.8, 141.3, 127.9, 123.6, 61.4, 61.2, 56.2, 53.4, 50.6, 49.8, 43.5, 39.5, 37.7, 36.1, 35.0, 34.3, 34.0, 33.9, 28.0, 23.7, 20.7, 18.2, 16.3, 14.2, 14.1, 11.9.

### Example 33 - Synthesis of 23-cyano-24-nor-chol-4,6-dien-3-one

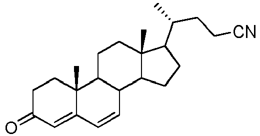

### Synthesis of (20S)-20-bromomethyl-3,3-ethylenedioxy-4-pregnene and (20S)-20-bromomethyl-3,3-ethylenedioxy-5-pregnene

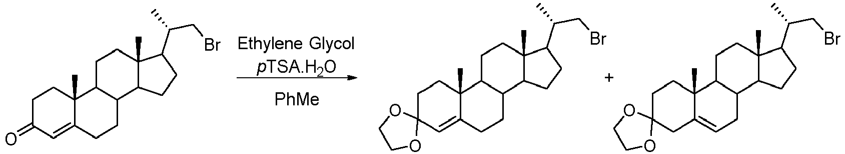

To a solution of (20*S*)-20-bromomethyl-4-pregnen-3-one (1.00 g, 2.59 mmol) and ethylene glycol (2.0 mL, 36.25 mmol) in toluene (30 mL) was added *p*TSA.H₂O (9.86 mg, 0.05 mmol) and the mixture was heated to reflux using a Dean Stark apparatus for 5 h. The reaction mixture was allowed to cool to room temperature before being poured onto 5% aq. NaHCO₃ (30 mL). The layers were separated and the aqueous layer was extracted with CH₂Cl₂ (2 x 30 mL). The combined organics were dried over sodium sulfate and were concentrated under reduced pressure. The residue was used in the next step without purification. A sample was purified by column chromatography (heptane/EtOAc) to give a mixture of (20S)-20-bromomethyl-3,3-ethylenedioxy-4-pregnene and (20S)-20-bromomethyl-3,3-ethylenedioxy-5-pregnene in 68 % yield (the ratio of Δ⁵:Δ⁴ was approximately 3.6:1). δH (700 MHz, CDCl₃); 5.35 (0.8H, dt, *J* = 4.4, 2.2), 5.23 (0.2H, s), 4.02-3.96 (4H, m, C*H*₂O), 3.51 (0.8H, dd, *J* 9.7, 2.7), 3.51-3.49 (0.2H, m), 3.34 (0.8H, dd, *J* 9.7, 6.0), 3.33 (0.2H, dd, *J* 9.7, 6.1), 2.56 (0.8H, dq, *J* 14.1, 2.9), 2.20 (0.2H, td, *J* 13.9, 4.9, 1.8), 2.12 (0.8H, dd, *J* 14.2, 2.9), 2.05 (0.2H, ddd, *J* 14.0, 4.2, 2.4), 1.99-1.93 (2H, m), 1.91-1.83 (1H, m), 1.81-1.75 (2H, m), 1.74-1.62 (4H, m), 1.60 (0.8H, s), 1.561.51 (1H, m), 1.50-1.41 (2H, m), 1.37-1.25 (3H, m), 1.21 (1H, td, *J* 6.5, 4.2), 1.17-1.04 (3H, m), 1.09 (3H, d, *J* 6.4), 1.03 (3H, s), 1.01-0.84 (0.8H,m), 0.71 (2.4H, s), 0.70 (0.6H, s); δC (176 MHz, CDCl₃); 151.6, 140.2, 122.1, 119.65, 109.5, 106.2, 64.6, 64.5, 64.2, 64.2, 56.4, 55.7, 53.8, 53.7, 53.7, 49.6, 43.6, 43.5, 42.5, 42.4, 41.8, 39.5, 39.5, 37.9, 37.8, 37.4, 36.6, 36.3, 35.8, 34.9, 32.4, 32.1, 31.9, 31.9, 31.7, 31.1, 30.0, 27.6, 27.6, 24.2, 24.1, 21.0, 18.9, 18.7, 18.6, 17.6, 12.3, 12.2.

### Synthesis of 3,3-ethylenedioxy-4-choleno-24-nitrile and 3,3-Ethylenedioxy-5-choleno-24-nitrile

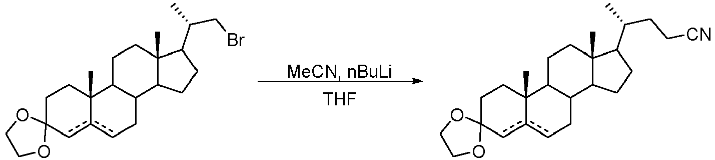

### Procedure A

A solution containing MeCN (26.0 mg, 0.63 mmol) in THF (1.85 mL) was cooled to -78 °C under argon and nBuLi (0.32 mL, 2 M in cyclohexane, 0.63 mmol) was charged dropwise over 2 min. To this mixture, a solution containing (20S)-20-bromomethyl-3,3-ethylenedioxy-4-pregnene and (20S)-20-bromomethyl-3,3-ethylenedioxy-5-pregnene (185 mg, 0.423 mmol) in THF (2.15 mL) was charged dropwise over 30 min. The reaction mixture was allowed to warm to 0 °C over 4 h, cooled to -78 °C and quenched with 10% aq. NH₄Cl (3 mL). The reaction mixture was diluted with EtOAc (20 mL) and 10% aq. NH₄Cl (20 mL) and the organic phase was separated. The aqueous phase was extracted with EtOAc (20 mL), and the combined organic phases were washed with 5% aq. NaCl (20 mL), dried over sodium sulfate and concentrated under reduced pressure. The residue was purified by column chromatography on silica gel using heptane: EtOAc (5:1) as the eluent. A fraction containing 3,3-ethylenedioxy-4-choleno-24-nitrile and 3,3-ethylenedioxy-5-choleno-24-nitrile was obtained in 49% yield (the ratio of Δ⁵:Δ⁴ was approximately 7:1). δH (700 MHz, CDCl₃); 5.35 (0.9H, dt, *J* 4.5, 2.2), 5.2 (0.1H, br s), 4.02-3.86 (4H, m), 2.56 (0.9H, dq, *J* 14.2, 2.9), 2.39-2.34 (0.1H, m), 2.34 (0.9H, ddd, *J* 16.9, 8.6, 5.1), 2.27 (0.9H, dt, *J* 16.8, 8.4), 2.27 (0.1H, dt, *J* 16.8, 8.4), 2.20 (0.1H, td, *J* 13.9, 5.0, 1.8), 2.12 (0.9H, dd, *J* 14.2, 3.0), 2.05 (0.1H, ddd, *J* 13.8, 4.4, 2.2), 2.01-1.95 (2H, m), 1.87-1.75 (4H, m), 1.73-1.70 (0.3H, m), 1.69-1.59 (3.4H, m), 1.58-1.52 (2H, m), 1.50-1.43 (2H, m), 1.39-1.25 (4.6H, m), 1.18 (1H, td, *J* 6.5, 4.2), 1.14-0.99 (4H, m), 1.03 (3H, s), 0.96 (2.7H, d, *J* 6.6), 0.94 (0.3H, d, *J* 6.7), 0.88 (0.9H, t, *J* 14.3), 0.70 (2.7H, s), 0.70 (0.3H, s); δC (176 MHz, CDCl₃); 151.6, 140.1, 122.1, 120.2, 119.6, 109.5, 106.2, 64.6, 64.4, 64.2, 56.7, 56.0, 55.5, 55.5, 53.8, 49.6, 42.6, 42.5, 41.8, 39.8, 39.7, 37.4, 36.6, 36.3, 35.7, 35.2, 35.2, 34.9, 32.4, 32.1, 31.9, 31.9, 31.7, 31.6, 31.5, 31.1, 30.0, 29.7, 28.1, 28.1, 24.2, 24.1, 22.7, 21.0, 18.9, 17.9, 17.9, 17.6, 14.3, 14.2, 14.1, 12.0, 11.9.

### Procedure B

A solution of MeCN (2.06 mL, 39.43 mmol) in THF (34 mL) was charged dropwise over 1.2 h to a solution of nBuLi (19.72 mL, 2 M in cyclohexane, 39.43 mmol) in THF (69 mL) at -60 °C under argon. To the resulting white suspension, a solution containing (20S)-20-bromomethyl-3,3-ethylenedioxy-4-pregnene and (20*S*)-20-bromomethyl-3,3-ethylenedioxy-5-pregnene (6.9 g, 15.77 mmol) in THF (69 mL) was charged dropwise over 1.2 h. The thick suspension that formed was warmed to 0 °C over 15 min and water (69 mL) was charged dropwise. The layers were separated and the aqueous phase was extracted with EtOAc (2 x 100 mL). The combined organic phases were washed with 5% aq. NaCl (2 x 100 mL) and concentrated under reduced pressure. The residue was purified by column chromatography on silica gel using a gradient of EtOAc in heptane as the eluent. A fraction containing 3,3-ethylenedioxy-4-choleno-24-nitrile and 3,3-ethylenedioxy-5-choleno-24-nitrile was obtained which also contained the product from double-alkylation of MeCN (mass 3.88 g).

### Synthesis of 3-oxo-4-choleno-24-nitrile

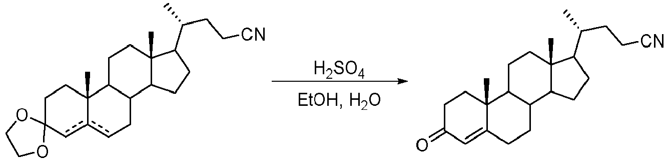

To a solution of 3,3-ethylenedioxy-4-choleno-24-nitrile and 3,3-ethylenedioxy-5-choleno-24-nitrile (3.75 g, 9.43 mmol) in EtOH (75 mL) was added a solution of H₂SO₄ (1 mL, conc, 18.86 mmol) in water (7.5 mL). The reaction mixture was heated at reflux for 30 min and cooled to room temperature. A white solid was removed by filtration and the filter-cake was washed with EtOH (2 x 20 mL). Pyridine (3 mL) was added to the combined wash and filtrate and the mixture was concentrated under reduced pressure. The residue was dissolved in EtOAc (100 mL), washed with 1M aq. H₂SO₄ (100 mL), 5% aq. NaHCO₃ (100 mL), 5% aq. NaCl (2 x 100 mL), dried over sodium sulfate and was concentrated under reduced pressure to give the desired product (2.36 g). ¹H NMR (700 MHz, CDCl₃): δ = 5.72 (1H, s, C4-C*H*), 2.45-2.25 (6H, m), 2.04-2.00 (2H, m), 1.89-1.82 (3H, m), 1.69 (1H, td, J 7.0, 4.6), 1.67-1.62 (1H, m), 1.59-1.51 (3H, m), 1.44 (1H, qd, J 13.1, 4.0), 1.39-1.25 (3H, m), 1.20-1.10 (3H, m), 1.18 (3H, s), 1.05-0.99 (2H, m), 0.96 (3H, d, J 6.6), 0.95-0.91 (1H, m), 0.73 (3H, s); ¹³C NMR (176 MHz, CDCl₃): δ = 199.6 (C=O), 171.4 (C=CH), 123.8 (C=CH), 120.2 (CN), 55.8, 55.5, 53.7, 42.6, 39.6, 38.6, 35.7, 35.6, 35.1, 34.0, 32.9, 32.0, 31.5, 28.1, 24.1, 21.0, 17.9, 17.4, 14.3, 12.0.

### Synthesis of 3-oxo-4,6-choladieno-24-nitrile

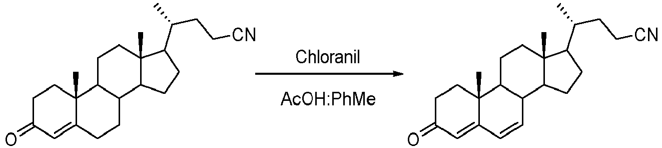

To a solution of 3-oxo-4-choleno-24-nitrile (2.25 g, 0.64 mmol) in toluene (2.25 mL) and AcOH (6.75 mL) was added chloranil (1.72 g, 0.70 mmol). The mixture was heated at 100 °C for 45 min and was then allow to cool to room temperature. The mixture was filtered, washing with AcOH : toluene (3 : 1, 20 mL) and the combined filtrates were concentrated under reduced pressure. The residue was concentrated from toluene (3 x 40 mL) and acetone (3 x 40 mL) and was then dissolved in acetone (6.75 mL). The solution was charged to an aqueous solution of NaOH (22.5 mL, 3% w/v) and the sticky solid that formed was collected by filtration and washed with water: acetone (2 x 20 mL, 2 : 1). The solid was purified by chromatography on silica gel using a gradient of EtOAc in heptane as the eluent to give the desired product as a yellow solid (1.33 g, 59% yield). ¹H NMR (700 MHz, CDCl₃): δ = 6.13 (1H, d, J 11.0), 6.10 (1H, dd, J 9.8, 2.3), 5.67 (1H, s), 2.57 (1H, ddd, J 17.9, 14.5, 5.4), 2.45-2.41 (1H, m), 2.39 (1H, ddd, J 17.0, 8.3, 5.1), 2.29 (1H, dt, J 16.8, 8.4), 2.20 (1H, t, J 10.6), 2.05 (1H, dt, J 12.9, 3.4), 2.00 (1H, ddd, J 13.2, 5.3, 2.0), 1.95-1.89 (1H, m), 1.88-1.80 (2H, m), 1.71 (1H, td, J 9.7, 1.3), 1.62-1.54 (2H, m), 1.44 (1H, qd, J 9.7, 1.3), 1.41-1.34 (2H, m), 1.30 (1H, ddd, J 24.0, 11.7, 5.8), 1.25-1.19 (3H, m), 1.17 (1H, q, J 9.5), 1.11 (3H, s), 0.97 (3H, d, J 6.7), 0.78 (3H, s); ¹³C NMR (176 MHz, CDCl₃): δ = 199.6, 163.8, 141.1, 127.9, 123.6, 120.1, 55.4, 53.4, 50.6, 43.6, 39.5, 37.7, 36.0, 35.2, 34.0, 33.9, 31.4, 28.1, 23.7, 20.6, 17.9, 16.3, 14.4, 11.9.

### Example 34 - Synthesis of (20S)-20-(1-aminomethyl)-pregna-4,6-dien-3-one

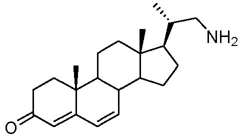

### Synthesis of (20S)-tosyloxymethyl-pregna-4,6-dien-3-one

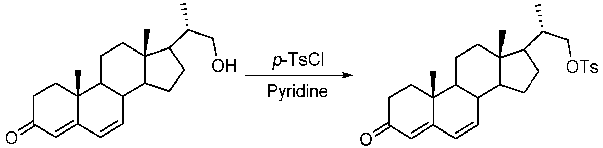

To a solution of (20S)-hydroxymethyl-pregna-4,6-dien-3-one (1.50 g, 4.58 mmol) in pyridine (50 mL) at 0 °C was added p-toluenesulfonyl chloride (1.79 g, 9.39 mmol). The reaction was stirred at 0 °C for 1 h and ambient for 17 h. The reaction was quenched with 1 M aq. HCI (75 mL) and was diluted with ethyl acetate (150 mL). The organic phase was separated and washed with water (50 mL), 5% aq. sodium bicarbonate (75 mL), 5% aq. NaCl (50 mL) and was concentrated *in vacuo.* The residue was purified by column chromatography on silica gel (heptane-EtOAc) to give the desired product (1.59 g, 72%) as a yellow powder. R_{f}: 0.36 (3:2, heptane:ethyl acetate); ¹H NMR (700 MHz, CDCl₃): δ = 7.78 (2H, d, *J* 8.2, Ar-H), 7.35 (2H, d, *J* 8.2, Ar-H), 6.10 (2H, br. s, C6H and C7H), 5.67 (1H, s, C4H), 3.97 (1H, dd, *J* 9.3, 3.2, C22H), 3.80 (1H, dd, *J* 9.3, 6.4, C22H), 2.56 (1H, ddd, *J* 17.6, 14.6, 5.6, C2H), 2.45-2.41 (4H, m, C2H and Ts-C*H*₃), 2.17 (1H, t, *J* 10.5), 2.01-1.96 (2H, m), 1.80-1.67 (4H, m), 1.54 (1H, dq, *J* 13.5, 3.1), 1.41 (1H, qd, *J* 13.1, 3.9), 1.30-1.23 (3H, m), 1.23-1.17 (3H, m),1.10 (3H, s, C19H), 1.00 (3H, d, *J* 6.7, C21H), 0.73 (3H, s, C18H). ¹³C NMR (176 MHz, CDCl₃): δ = 197.9, 162.0, 142.9, 139.2, 131.3, 128.0, 126.2, 126.1, 121.9, 73.6, 51.3, 49.9, 48.8, 41.7, 37.4, 35.9, 34.4, 34.3, 32.2, 32.1, 25.6, 21.9, 20.0, 18.8, 15.1, 14.5, 10.1.

### Synthesis of (20S)-azidomethyl-pregna-4,6-dien-3-one

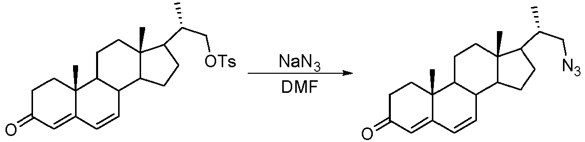

To a suspension of (20S)-tosyloxymethyl-pregna-4,6-dien-3-one (1.58 g, 3.27 mmol) in DMF (24 mL) and water (59 µL) was added sodium azide (273 mg, 4.20 mmol). The reaction was heated to 70 °C and stirred for 1 h. The reaction was quenched with 2% aq.sodium bicarbonate solution (50 mL) at 40 °C, and was diluted with ethyl acetate (100 mL). The layers were separated and the organic layer was washed with 2% aq. sodium bicarbonate (50 mL), 5% aq. NaCl (50 mL) and was concentrated *in vacuo.* The residue was purified by column chromatography on silica gel (heptane-EtOAc) to give the desired product (1.01 g, 91% yield) as a colourless crystalline solid. R_{f}: 0.54 (3:2, heptane:ethyl acetate); ¹H NMR (700 MHz, CDCl₃): δ = 6.12 (1H, d, *J* 9.9, C6H), 6.10 (1H, dd, *J* 9.9, 2.1, C7H), 5.67 (1H, s, C4H), 3.38 (1H, dd, *J* 11.9, 3.3, C22H), 3.07 (1H, dd, *J* 11.9, 7.3, C22H), 2.57 (1H, ddd, *J* 17.8, 14.7, 5.4, C2H), 2.46-2.41 (1H, m, C2H), 2.17 (1H, t, *J* 10.6), 2.04 (1H, dt, *J* 12.8, 3.3), 2.00 (1H, ddd, *J* 13.2, 5.4, 2.1), 1.93-1.86 (1H, m), 1.86 -1.81 (1H, m), 1.75-1.65 (2H, m), 1.56 (1H, dq, *J* 13.4, 3.7), 1.44 (1H, qd, *J* 13.0, 4.0), 1.40-1.28 (6H, m), 1.11 (3H, s, C19H), 1.06 (3H, d, *J* 6.7, C21H), 0.77 (3H, s, C18H). ¹³C NMR (176 MHz, CDCl₃): δ = 199.9, 163.8, 141.1, 128.0, 123.6, 57.9, 53.2, 53.0, 50.6, 43.6, 39.3, 37.7, 36.9, 36.0, 34.0, 33.9, 27.8, 23.8, 20.6, 17.8, 16.3, 12.0.

### Synthesis of (20S)-aminomethyl-pregna-4,6-dien-3-one

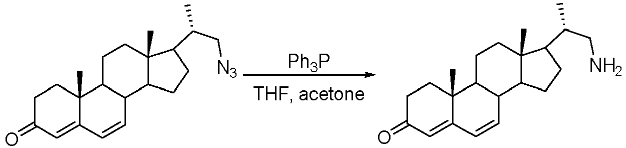

To a solution of (20S)-azidomethyl-pregna-4,6-dien-3-one (99 mg, 0.292 mmol) and triphenylphosphine (106 mg, 0.404 mmol) in THF (1.1 mL) under argon atmosphere was added acetone (300 µL). The reaction was stirred at room temperature for 64 h. The reaction was diluted with ethyl acetate (10 mL) and 2 M aq. hydrochloric acid solution (10 mL). The layers were separated and the aqueous phase was basified with 2 M aq. sodium hydroxide (6.5 mL) to pH 11, and was then extracted with ethyl acetate (10 mL). The organic phase was separated and concentrated *in vacuo.* The residue was purified by column chromatography on silica gel (DCM-MeOH) to give the desired product (28 mg, 30% yield) as an off-white powder. R_{f} 0.23 (4:1, CH₂Cl₂:MeOH); ¹H NMR (700 MHz, CDCl₃): δ = 6.12-6.07 (2H, m, C6H and C7H), 5.67 (1H, s, C4H), 3.05 (1H, dd, *J* 12.7, 3.1, C22*H*ₐH_{b}), 2.74 (1H, dd, *J* 12.7, 8.3, C22Hₐ*H*_{b}), 2.58 (1H, ddd, *J* 17.9, 14.5, 5.4, C2*H*ₐH_{b}), 2.46-2.41 (1H, m, C2*H*ₐH_{b}), 2.18 (1H, t, *J* 10.5), 2.05-1.94 (3H, m), 1.90-1.81 (2H, m), 1.68 (1H, td, *J* 13.9, 5.6), 1.55 (1H, dq, *J* 13.4, 3.4), 1.45-1.17 (9H, m), 1.20 (3H, obscured d, *J* 6.7, C21H), 1.11 (3H, s, C18H), 0.78 (3H, s, C19H). ¹³C NMR (140 MHz, CDCl₃): δ = 199.5, 163.6, 140.8, 128.0, 123.7, 53.2, 52.8, 50.6, 45.3, 43.6, 39.3, 37.6, 36.0, 36.0, 35.1, 34.0, 33.9, 27.8, 23.7, 20.7, 17.3, 16.3.

### Example 35 - Synthesis of (20R)-20-(1-cyanomethyl)-pregna-4,6-dien-3-one

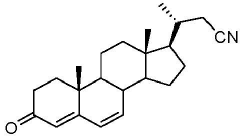

### Synthesis of (20S)-20-bromomethyl-4-pregnen-3-one

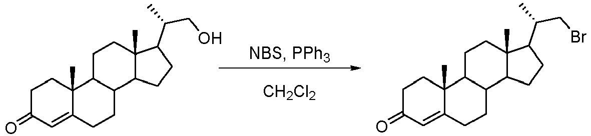

To a solution of (20*S*)-hydroxymethyl-4-pregnen-3-one (50 g, 0.15 mol) in CH₂Cl₂ (350 mL) at 0 °C was added triphenylphosphine (43.6 g, 0.17 mol). *N*-bromosuccinimide (29.6 g, 0.17 mol) was added portionwise and the reaction mixture was stirred at 18 °C. After 18 h, the reaction mixture was cooled to 0 °C and triphenylphosphine (19.8 g, 0.08 mol) was added, followed by *N*-bromosuccinimide (13.5 g, 0.08 mol) portionwise. The mixture was warmed to 18 °C. After 2 h the reaction mixture was washed with water (350 mL) and the aqueous phase extracted with CH₂Cl₂ (350 mL). The combined organic phases were washed with 5% aq. sodium bicarbonate (350 mL), and the aqueous phase extracted with CH₂Cl₂ (100 mL). The combined organic phases were washed with 5% aq. sodium chloride (150 mL), dried over sodium sulfate and were concentrated *in vacuo.* The residue was purified by column chromatography on silica gel (heptane-EtOAc) to give the desired product (47.1 g, 79%) as a yellow solid. ¹H NMR (700 MHz, CDCl₃): δ = 5.72 (1H, s), 3.50 (1H, dd, J = 9.8, 2.7, C22- C*H*ₐH_{b}), 3.35 (1H, dd, J = 9.8, 5.9, C22- CHₐ*H_{b}*), 2.45-2.32 (3H, m), 2.27 (1H, ddd, J = 14.6, 4.1, 2.5), 2.04-1.98 (2H, m), 1.91-1.82 (2H, m), 1.72-1.64 (3H, m), 1.56-1.50 (2H, m), 1.43 (1H, qd, J = 13.1, 4.1), 1.33-1.27 (2H, m), 1.22 (1H, dd, J = 13.0, 4.2), 1.20-1.13 (1H, m), 1.18 (3H, s), 1.09 (3H, d, J = 6.4), 1.09-1.00 (2H, m), 0.94 (1H, ddd, J = 12.3, 10.9, 4.1), 0.74 (3H, s); ¹³C NMR (176 MHz, CDCl₃): δ = 197.5, 169.3, 121.8, 53.5, 51.6, 51.6, 41.4, 40.4, 37.3, 36.5, 35.7, 33.6, 33.6, 31.9, 30.8, 29.9, 25.5, 22.0, 18.9, 16.6, 15.3, 10.3.

### Synthesis of (20R)-cyanomethyl-4-pregnen-3-one

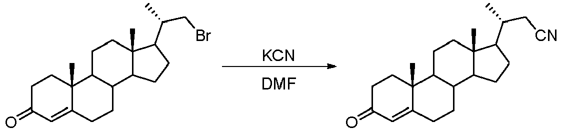

To a suspension of (20S)-20-bromomethyl-4-pregnen-3-one (15 g, 38.1 mmol) in DMF (225 mL) was added potassium cyanide (7.5 g, 114 mmol). The suspension was stirred at 80 °C for 41 h before cooling to room temperature. EtOAc (250 mL) and water (500 mL) were added and the layers were separated. The aqueous layer was extracted with EtOAc (2 x 250 mL) and the combined organic phases were washed with 5% aq. NaCl (250 mL) and were concentrated under reduced pressure. The residue was purified by column chromatography on silica gel (heptane/EtOAc) to afford the desired product (9.7 g, 75%) as a white solid. δH (700 MHz, CDCl₃); 5.73 (1H, s, C4-C*H*), 2.45-2.32 (4H, m), 2.27 (1H, ddd, J = 14.6, 4.2, 2.7), 2.24 (1H, dd, J = 16.8, 7.1), 2.04-1.99 (2H, m), 1.89-1.78 (3H, m), 1.72-1.65 (2H, m), 1.57-1.51 (2H, m), 1.43 (1H, qd, J = 13.2, 4.0), 1.31-1.16 (4H, m), 1.18 (3H, s), 1.17 (3H, d, J = 6.7), 1.11-1.01 (2H, m), 0.94 (1H, ddd, J = 12.3, 10.7, 4.1), 0.74 (3H, s); δC (176 MHz, CDCl₃); 199.5, 171.2, 123.9, 118.9, 55.7, 54.7, 53.6, 42.5, 39.2, 38.5, 35.7, 35.6, 34.0, 33.6, 32.8, 31.9, 28.0, 24.8, 24.1, 20.9, 19.3, 17.4, 12.1.

### Synthesis of (20R)-cyanomethyl-4,6-pregnadien-3-one

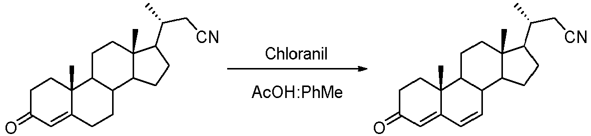

To a suspension of (20*R*)-cyanomethyl-4-pregnen-3-one (9.1 g, 26.8 mmol) in toluene (36 mL) and acetic acid (0.15 mL) was added *p*-chloranil (7.2 g, 29.5 mmol). The mixture was heated at reflux for 90 minutes before allowing to cool to room temperature. The suspension was filtered, washing with toluene (25 mL). The filtrate was concentrated under reduced pressure and the residue was purified by column chromatography on silica gel (heptane/EtOAc). The material was then dissolved in acetone (35 mL) and methanol (23 mL) and 0.5 M aq. NaOH (200 mL) was added dropwise. Water (100 mL) was added and the resulting solid was filtered, washing with water (2 x 50 mL) and 2 : 1 acetone : water (2 x 20 mL). The solid was dried *in vacuo* to afford the desired product (5.4 g, 60%) as a pale brown solid. δH (700 MHz, CDCl₃); 6.11 (2H, s), 5.67 (1H, s), 2.57 (1H, ddd, *J* = 18.0, 14.4, 5.4), 2.45-2.42 (1H, m), 2.37 (1H, dd, *J* = 16.7, 3.7), 2.25 (1H, dd, *J* = 16.7, 7.2), 2.01 (1H, t, J = 10.4), 2.03 (1H, dt, *J* = 12.8, 3.3), 2.00 (1H, ddd, *J* = 13.2, 5.4, 2.1), 1.96-1.91 (1H, m), 1.88-1.81 (1H, m), 1.74-1.70 (1H, m), 1.58 (1H, dq, *J* = 13.4, 3.6), 1.44 (1H, qd, *J* = 4.4, 3.9), 1.36-1.20 (7H, m), 1.18 (3H, d, *J* = 6.7), 1.11 (3H, s), 0.79 (3H, s); δC (176 MHz, CDCl₃); 199.6, 163.67, 140.8, 128.1, 123.7, 118.8, 54.6, 53.2, 50.5, 43.5, 39.1, 37.6, 36.0, 33.9, 33.9, 33.5, 28.0, 24.8, 23.6, 20.6, 19.3, 16.3, 12.0.

### Example 36 - Synthesis of (20S)-(N-benzyl)aminomethyl-pregna-4,6-dien-3-one

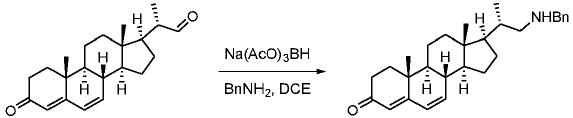

(20S)-Formyl-pregna-4,6-dien-3-one (98 mg, 0.30 mmol) and benzylamine (21 µL, 0.30 mmol) were dissolved in 1,2-dichloroethane (1.0 mL) under an argon atmosphere. Sodium triacetoxyborohydride (96 mg, 0.45 mmol) was added. The reaction mixture was stirred at 20 °C for 2 h, then quenched with aq. sodium bicarbonate solution (5%, 2 mL). The mixture was diluted with EtOAc (10 mL) and water (5 mL). The aq. phase was separated and extracted with EtOAc (2 × 5 mL). The organic phases were combined and concentrated *in vacuo.* The residue was purified by silica column chromatography (heptane-EtOAc) to yield (20*S*)-(*N*-benzyl)aminomethyl-pregna-4,6-dien-3-one as a beige powder (51 mg, 41% yield). R_{f} 0.15 (EtOAc); ¹H NMR (500 MHz, CDCl₃): δ = 7.34 (4H, d, *J* 4.5, Bn-CH), 7.29-7.23 (1H, m, Bn-CH), 6.15 (1H, d, *J* 10.2, C6), 6.11 (1H, dd, *J* 9.6, 2.0, C7H), 5.68 (1H, s, C4H), 3.84 (1H, d, *J* 13.1, Bn-C*H*ₐH_{b}), 3.75 (1H, d, *J* 13.1, Bn-CHₐ*H_{b}*), 2.69 (1H, dd, *J* 11.6, 3.0, C22*H*ₐH_{b}), 2.58 (1H, ddd, *J* 17.2, 14.5, 5.3, C2*H*ₐH_{b}), 2.44 (1H, dd, *J* 17.4, 4.4, C2*H*ₐH_{b}), 2.35 (1H, dd, *J* 11.5, 8.3, C22*H*ₐH_{b}), 2.20 (1H, t, *J* 10.7, H8), 2.07 (1H, dt, *J* 12.6, 3.0), 2.04-1.97 (1H, m, C1*Hₐ*H_{b}), 1.92-1.68 (3H, m), 1.68-1.60 (1H, m, C20H), 1.60-1.52 (1H, m), 1.44 (1H, qd, *J* 12.8, 3.9), 1.40-1.18 (7H, m), 1.13 (3H, s, C18H), 1.04 (3H, d, *J* 6.6, C21H), 0.78 (3H, s, C19H). ¹³C NMR (126 MHz, CDCl₃): δ = 199.7, 164.0, 141.4, 140.5, 128.4, 128.1, 127.8, 126.9, 123.5, 54.9, 54.2, 54.0, 53.3, 50.7, 43.5, 39.5, 37.7, 36.5, 36.0, 34.0, 33.9, 27.9, 23.8, 20.7, 17.8, 16.3, 12.0.

### Example 37 - Synthesis of N-((22E)-3,24-dioxo-4,6,22-cholatrien-24-yl)cyclopropylsulfonamide

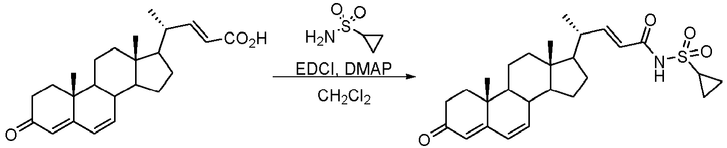

To a solution of (22*E*)-3-oxo-4,6,22-cholatrien-24-oic acid (2.00 g, 5.43 mmol) in CH₂Cl₂ (40 mL) was added EDCI (1.69 g, 10.9 mmol) and DMAP (1.33 g, 10.9 mmol). Cyclopropane sulfonamide (1.97 g, 16.3 mmol) was added and the reaction was stirred at room temperature for 22 h. Water (25 mL) was added and the layers were separated. The aqueous layer was extracted with CH₂Cl₂ (2 x 25 mL) and the combined organics were washed with 2 M aq HCI (20 mL), 10% aq. NaCl (10 mL), dried over sodium sulfate and concentrated under reduced pressure. The residue was purified by column chromatography on silica gel (0-10% acetone in toluene) to give the desired product (1.68 g, 66%) as an off-white solid. δH (400 MHz, CDCl₃); 8.90 (1H, s, N*H*), 6.95 (1H, dd, *J* 15.5, 9.0, C23-C*H*), 6.11 (2H, brs, C6-C*H* and C7-C*H*), 5.86 (1H, dd, *J* 15.5, 0.5, C22-C*H*), 5.68 (1H, s, C4-C*H*), 3.00 (1H, dddd, *J* 12.8, 9.5, 8.1, 4.8, SO₂C*H*), 2.64 (1H, ddd, *J* 18.1, 14.4, 5.4, C2-C*H*ₐ*H*_{b}), 2.51-2.41 (1H, m, C2-CHₐ*H*_{b}), 2.40-2.28 (1H, m), 2.25-2.15 (1H, m), 2.09-1.96 (2H, m), 1.85-1.64 (3H, m), 1.63-1.52 (1H, m), 1.51-1.17 (9H, m), 1.17-1.07 (5H, m), 1.12 (3H, s, C19-C*H*₃), 0.80 (3H, s, C18-C*H*₃); δC (100 MHz, CDCl₃); 200.0, 164.2, 164.1, 155.5, 141.3, 127.9, 123.6, 119.4, 54.7, 53.2, 50.6, 43.8, 39.8, 39.3, 37.8, 36.1, 33.9, 33.9, 31.5, 28.1, 23.7, 20.6, 19.1, 16.3, 12.2, 6.3, 6.3.

### Example 38 - Synthesis of N-((22E)-3,24-dioxo-4,6,22-cholatrien-24-yl)-4-(trifluoromethoxy)benzenesulfonamide

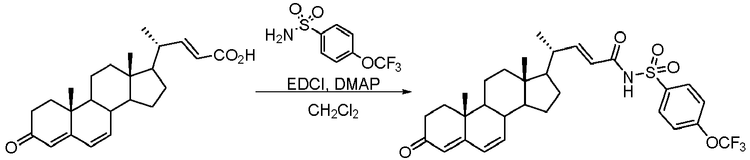

To a solution of (22*E*)-3-oxo-4,6,22-cholatrien-24-oic acid (2.00 g, 5.43 mmol) in CH₂Cl₂ (40 mL) was added EDCI (1.69 g, 10.9 mmol) and DMAP (1.33 g, 10.9 mmol). 4-(Trifluoromethoxy)benzene sulfonamide (3.93 g, 16.3 mmol) was added and the reaction was stirred at room temperature for 22 h. Water (25 mL) was added and the layers were separated. The aqueous layer was extracted with CH₂Cl₂ (2 x 25 mL) and the combined organics were washed with 2 M aq HCI (20 mL), 10% aq. NaCl (10 mL), dried over sodium sulfate and concentrated under reduced pressure. The residue was used in the next step without purification. A portion was purified by column chromatography on silica gel (0-50% EtOAc in heptane) to give the desired product as an off-white solid. δH (400 MHz, MeOD); 8.16-8.11 (2H, m, ArH), 7.52-7.46 (2H, m, ArH), 6.82 (1H, dd, *J* 15.4, 9.0, C23-C*H*), 6.20 (1H, brdd, *J* 9.8, 1.4, C6-C*H*), 6.15 (1H, dd, *J* 9.9, 1.4, C7-C*H*), 5.82 (1H, dd, *J* 15.4, 0.7, C22-C*H*), 5.64 (1H, s, C4-C*H*), 2.62 (1H, ddd, *J* 18.2, 14.5, 5.4, C2-C*H*ₐ*H*_{b}), 2.42-2.20 (3H, m), 2.12-1.98 (2H, m), 1.88-1.63 (3H, m), 1.63-1.55 (1H, m), 1.49 (1H, dd, *J* 12.6, 3.8), 1.40-1.18 (7H, m), 1.14 (3H, s, C19-C*H*₃), 1.08 (3H, d, *J* 6.6, C21-C*H*₃), 0.81 (3H, s, C18-C*H*₃); δC (100 MHz, MeOD); 202.3, 167.2, 165.9, 156.7, 154.0, 143.3, 139.7, 131.8, 128.8, 123.9, 123.0 (q, *J* 254), 121.9, 120.6, 56.0, 54.6, 52.2, 44.9, 40.9, 40.6, 39.1, 37.4, 35.0, 34.7, 30.2, 29.0, 24.7, 21.7, 19.5, 16.6, 12.5.

### Example 39 - Synthesis of (20S)-20-(5-Tosyltetrazol-1-yl)methyl-pregna-4,6-dien-3-one

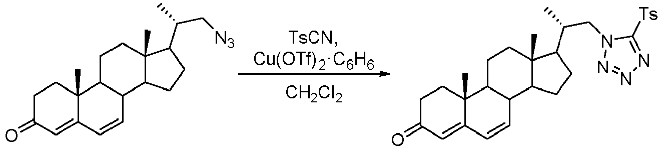

To a solution of (20S)-azidomethyl-pregna-4,6-dien-3-one (500 mg, 1.41 mmol) in CH₂Cl₂ (5 mL) was added *p*-toluenesulfonyl cyanide (282 mg, 1.55 mmol). Copper(I) trifluoromethanesulfonate benzene complex (71 mg, 0.141 mmol) was added and the mixture was stirred at room temperature for 18 h. Toluene (5 mL), added *p*-toluenesulfonyl cyanide (128 mg, 0.708 mmol) and copper(I) trifluoromethanesulfonate benzene complex (71 mg, 0.141 mmol) were added and the mixture was heated to 60 °C for 24 h. Water (10 mL) and CH₂Cl₂ (30 mL) were added and the layers were separated. The organic layer was washed with 10% aq. Na₂S₂O₃/2% aq. NaHCO₃ (2 x 20 mL), 10% aq. NaCl (20 mL), was dried over sodium sulfate and was concentrated under reduced pressure. The residue was purified by column chromatography on silica gel (0-50% EtOAc in heptane) to give the desired product (381 mg, 50%) as a light yellow solid. δH (400 MHz, CDCl₃); 8.03-7.97 (2H, m, ArH), 7.46 (2H, m, ArH), 6.14 (2H, brs, C6-C*H* and C7-C*H*), 5.69 (1H, s, C4-C*H*), 4.80 (1H, dd, *J* 13.4, 3.9, C22-C*H*ₐH_{b}), 4.45 (1H, dd, *J* 13.4, 10.5, C22-CHₐ*H*_{b}), 2.26-2.53 (1H, m), 2.51 (3H, s, ArC*H*₃), 2.49-2.28 (2H, m), 2.24 (1H, appt, *J*, 10.5), 2.13-1.97 (2H, m), 1.96-1.87 (1H, m), 1.79-1.63 (2H, m), 1.53-1.18 (8H, m), 1.13 (3H, s, C19-C*H*₃), 0.89 (3H, d, *J* 6.6, C21-C*H*₃), 0.86 (3H, s, C18-C*H*₃); δC (100 MHz, CDCl₃); 199.5, 163.6, 147.5, 140.8, 134.3, 130.4, 129.3, 128.1, 123.7, 55.1, 53.9, 53.2, 50.7, 44.0, 39.4, 37.8,37.6, 36.0, 33.9, 33.9, 31.9, 27.5, 23.8, 22.7, 21.9, 20.6, 16.5, 16.3, 12.0.

### Example 40 - Synthesis of 23-carboxy-3-oxo-4,6-choladien-24-oic acid dimethyl ester

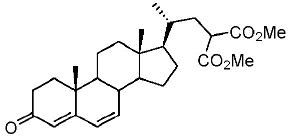

### Synthesis of 23-carboxy-3-oxo-4-cholen-24-oic acid dimethyl ester

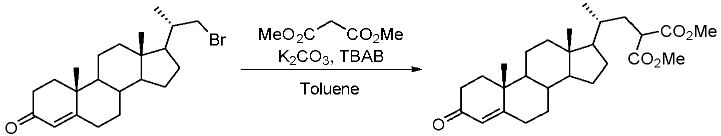

To a suspension of (20S)-20-bromomethyl-4-pregnen-3-one (15 g, 38.1 mmol), tetrabutylammonium bromide (1.2 g, 3.8 mmol) and potassium carbonate (26.3 g, 191 mmol) in toluene (150 mL) was added dimethylmalonate (13.1 mL, 114 mmol) and the reaction mixture was stirred at 80 °C for 91 h. The reaction mixture was then cooled to room temperature and was poured onto water (150 mL). The layers were separated and the aqueous phase was extracted with EtOAc (2 × 100 mL). The combined organic phases were washed with 5% aq. sodium chloride (100 mL) and were concentrated under reduced pressure. The residue was purified by column chromatography on silica gel (heptane-EtOAc) to give the desired product (14.8 g, 87%) as a yellow solid. ¹H NMR (700 MHz, CDCl₃): δ = 5.72 (1H, s), 3.75 (3H, s), 3.72 (3H, s), 3.48 (1H, dd, J = 11.0, 4.0), 2.44-2.36 (2H, m), 2.33 (1H, dt, J = 17.0, 3.6), 2.27 (1H, ddd, J = 14.6, 4.1, 2.4), 2.18 (1H, ddd, J = 13.7, 11.1, 2.5), 2.03-2.00 (2H, m), 1.95-1.89 (1H, m), 1.85-1.82 (1H, m), 1.71-1.67 (1H, m), 1.64-1.60 (1H, m), 1.54-1.39 (4H, m), 1.37-1.30 (2H, m), 1.19-1.09 (3H, m), 1.18 (3H, s), 1.05-0.99 (2H, m), 0.94-0.90 (1H, m), 0.93 (3H, d, J = 6.5), 0.70 (3H, s); ¹³C NMR (176 MHz, CDCl₃): δ = 199.6, 171.5, 170.4, 170.0, 123.8, 56.3, 55.8, 53.7, 52.6, 52.4, 49.4, 42.5, 39.6, 38.6, 35.7, 35.6, 35.1, 34.3, 34.0, 32.9, 32.0, 28.0, 24.1, 21.0, 18.1, 17.4, 11.9.

### Synthesis of 23-carboxy-3-oxo-4,6-choladien-24-oic acid dimethyl ester

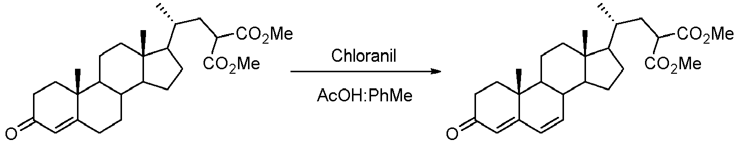

23-Carboxy-3-oxo-4-cholen-24-oic acid dimethyl ester (14.5 g, 32.7 mmol) was suspended in toluene (60 mL) and acetic acid (0.19 mL, 3.3 mmol). p-Chloranil (8.8 g, 35.9 mmol) was added and the mixture stirred at reflux for 65 min. The reaction mixture was cooled to room temperature and filtered. The filter cake was washed with toluene (45 mL) and the filtrate concentrated under reduced pressure. The residue (21.6 g) was used without further purification. A small portion was purified by column chromatography on silica gel (heptane-EtOAc) to give the product. ¹H NMR (700 MHz, CDCl₃): δ = 6.12 (1H, d, J = 10.8), 6.08 (1H, dd, J = 9.8, 2.2), 5.65 (1H, s), 3.74 (3H, s), 3.71 (3H, s), 3.47 (1H, dd, J = 11.0, 3.9), 2.58 (1H, dd, J = 14.3, 5.3), 2.53 (1H, dd, J = 14.3, 5.3), 2.44-2.38 (1H, m), 2.21-2.15 (2H, m), 2.05-1.92 (3H, m), 1.83-1.77 (1H, m), 1.69 (1H, td, J = 13.9, 5.2), 1.55-1.34 (5H, m), 1.31-1.11 (5H, m), 1.10 (3H, s), 0.93 (3H, d, J = 6.3), 0.73 (3H, s); ¹³C NMR (176 MHz, CDCl₃): δ = 199.6, 170.4, 170.0, 163.9, 141.4, 127.8, 123.5, 56.1, 53.4, 52.6, 52.4, 50.6, 49.4, 43.5, 39.5, 37.7, 36.0, 35.1, 34.3, 33.9, 33.9, 28.0, 23.7, 20.6, 18.1, 16.3, 11.9.

### Example 41-50 - Further epoxidation reactions

### Example 41 - Epoxidation of (20S)-20-hydroxymethyl-pregna-4,6-dien-3-one to form (6β, 7β, 20S)-6,7-epoxy-20-hydroxymethyl-pregna-4-en-3-one

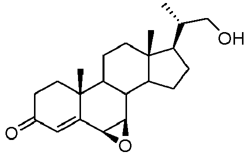

(20S)-(20)-Hydroxymethyl-pregna-4,6-dien-3-one (188 mg, 0.57 mmol) was dissolved in formic acid (3.4 mL) and the solution was protected from light. *t*-Butylhypochlorite (57 µL, 0.50 mmol) was added. The reaction was stirred at 18 °C for 15 min, and then quenched by the addition of aq. sodium bisulfite solution (10%, 0.60 mL). The mixture was concentrated *in vacuo,* and then diluted with CH₂Cl₂ (5 mL) and water (5 mL). The aq. phase was separated and extracted with CH₂Cl₂ (2 × 5 mL). The combined organic phases were washed with aq. sodium bicarbonate solution (5%, 10 mL). The sodium bicarbonate phase was diluted with aq. sodium chloride solution (5%, 10 mL) and re-extracted with CH₂Cl₂ (2 × 10 mL). All organic phases were combined and concentrated *in vacuo* to give the intermediate as a yellow viscous oil (207 mg, R_{f}: 0.44 (3:2 heptane:EtOAc), which was used in the next step without further purification. The residue was dissolved in ethanol (5.0 mL) and a solution of potassium carbonate (132 mg, 0.955 mmol) in water (1.4 mL) was added. The reaction was heated to reflux for 15 min then cooled to 40 °C and acetic acid (80 µL) was added. The reaction mixture was diluted with water (5 mL) and CH₂Cl₂ (10 mL), the aq. phase was separated and extracted with CH₂Cl₂ (2 × 10 mL). The combined organic phases were then concentrated *in vacuo.* The crude product (220 mg) was purified by flash chromatography on silica gel (heptane-EtOAc) to yield (6β, 7β, 20S)-6,7-epoxy-20-hydroxymethyl-pregna-4-en-3-one as a white powder (69 mg, 35% yield). R_{f}: 0.18 (3:2, heptane:EtOAc); ¹H NMR (500 MHz, CDCl₃): δ = 6.14 (1H, s, C4H), 3.63 (1H, dd, *J* 10.5, 3.1, C22H), 3.38 (1H, dd, *J* 10.5, 6.8, C22H), 3.35 (2H, br. s, C6H and C7H), 2.54 (1H, ddd, *J* 17.5, 15.1, 5.0, C2H), 2.40 (1H, br d, *J* 17.5, C2H), 2.02 (1H, dt, *J* 12.8, 3.3), 1.99-1.83 (4H, m), 1.76 (1H, br. s, O*H*), 1.64 (1H, td, *J* 13.9, 4.3), 1.65-1.54 (1H, m), 1.51 (1H, dq, *J*13.5, 3.1), 1.47-1.23 (5H, m), 1.23-1.14 (1H, obscured m), 1.20 (3H, obscured s, C19H), 1.10-1.02 (1H, obscured m), 1.05 (3H, obscured d, *J* 6.7, C21H), 0.75 (3H, s, C18H). ¹³C NMR (100 MHz, CDCl₃): δ = 198.5, 163.3, 129.3, 67.6, 59.3, 55.7, 52.5, 52.2, 51.6, 43.4, 39.2, 38.6, 36.6, 36.1, 35.5, 34.1, 27.6, 23.9, 21.2, 16.8, 16.7, 11.9.

### Example 42 - Epoxidation of (20S)-20-(bromomethyl)-pregna-4,6-dien-3-one to form (6β, 7β, 20S)-6,7-epoxy-20-bromomethyl-pregna-4-en-3-one

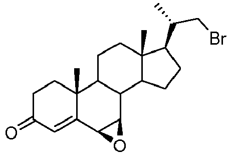

(20S)-20-(Bromomethyl)-pregna-4,6-dien-3-one (202 mg, 0.517 mmol) was dissolved in formic acid (3.6 mL) and the solution was protected from light. *t*-Butylhypochlorite (62 µL, 0.548 mmol) was added. The reaction was stirred at 18 °C for 15 min and then quenched by the addition of aq. sodium bisulfite solution (10%, 0.66 mL). The reaction mixture was diluted with CH₂Cl₂ (5 mL) and water (5 mL). The organic phase was separated and washed aq. sodium bicarbonate solution (5%, 10 mL). The sodium bicarbonate phase was re-extracted with CH₂Cl₂ (2 × 5 mL). The combined organic phases were concentrated *in vacuo* to afford the intermediate as a yellow viscous oil (287 mg, R_{f}: 0.49 (3:2, heptane:EtOAc), which was used in the next step without further purification. The residue was dissolved in ethanol (6.0 mL) and a solution of potassium carbonate (146 mg, 1.06 mmol) in water (1.4 mL) was added. The reaction was heated to reflux for 15 min, then cooled to 40 °C, and acetic acid (112 µL) was added. The reaction mixture was diluted with water (5 mL) and CH₂Cl₂ (10 mL), the aq. phase was separated and extracted with CH₂Cl₂ (2 × 5 mL). The combined organic phases were concentrated *in vacuo.* The crude product (244 mg) was purified by flash chromatography on silica gel (heptane-EtOAc) to yield (6β, 7β, 20S)-6,7-epoxy-20-bromomethyl-pregna-4-en-3-one as a colourless oil, which solidified to a white solid (152 mg, 72% yield). R_{f}: 0.50 (3:2, heptane:EtOAc); ¹H NMR (500 MHz, CDCl₃): δ = 6.14 (1H, s, C4H), 3.49 (1H, dd, *J* 9.8, 2.6, C22H), 3.37 (1H, obscured m, C22H), 3.35 (2H, obscured m, C6H and C7H), 2.58 (1H, ddd, *J* 17.5, 15.0, 5.0, C2H), 2.40 (1H, br d, *J* 17.5, C2H), 2.02-1.86 (5H, m), 1.73 (1H, m), 1.64 (1H, td, *J* 14.4, 4.4), 1.52 (1H, dq, *J* 13.7, 3.6), 1.47-1.21 (6H, m), 1.20 (3H, s, C19H), 1.11-1.05 (1H, obscured m), 1.09 (3H, obscured d, *J* 6.5, C21H), 0.76 (3H, s, C18H). ¹³C NMR (100 MHz, CDCl₃): δ = 198.2, 162.9, 129.4, 59.1, 55.7, 53.4, 52.5, 51.5, 43.4, 43.3, 39.0, 37.5, 36.6, 36.1, 35.6, 34.1, 27.4, 23.8, 21.2, 18.6, 16.8, 12.2.

### Example 43 - Epoxidation of (20S)-20-(1-mesyloxymethyl)-pregna-4,6-dien-3-one to form (20S)-methanesulfonyloxymethyl-6,7-β-epoxy-4-pregnen-3-one

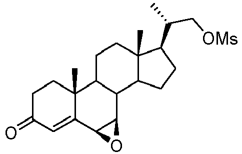

To a solution of (20S)-20-(1-mesyloxymethyl)-pregna-4,6-dien-3-one (200 mg, 0.5 mmol)) in formic acid (3.6 mL), under argon, was added *t*BuOCl (59 µL, 0.52 mmol) and the reaction mixture stirred at 18°C temperature. After 30 min the reaction was quenched with aq. 10% NaHSO₃ (0.66 mL) and stirred for 10 min. The reaction mixture was diluted with CH₂Cl₂ (100 mL), washed with aq. 5% NaHCO₃ (2 x 25 mL) and aq. 5% NaCl (25 mL). The organic phase was dried over Na₂SO₄ and concentrated *in vacuo* to give 186 mg of crude material. The crude material was taken up in EtOH (3.7 mL) at 18°C temperature and K₂CO₃ (105mg, 0.76 mmol) and H₂O were added. After 30 min the reaction mixture was heated to 80 °C for 2 h and then cooled to 18°C temperature and quenched with a mixture of 1:4 AcOH: EtOH (0.1 mL). The organic solvents were removed *in vacuo* and the residue taken up in CH₂Cl₂ (15 mL). The organic phase was washed with aq 5% NaCl (15 mL) and the aq. phase extracted with CH₂Cl₂ (2 x 10 mL). The combined organic phases were dried over Na₂SO₄ and concentrated *in vacuo.* Purification by column chromatography on silica gel gave (20*S*)-methanesulfonyloxymethyl-6,7-β-epoxy-4-pregnen-3-one (yield 41%). R_{f}: 0.36 (1:1, heptane:EtOAc); ¹H NMR (700 MHz, CDCl₃): 6.15 (1H, s, C4H), 4.19 (1H, dd, *J* 9.4, 3.1 Hz, C22H), 4.02 (1H, dd, *J* 9.4, 6.4 Hz, C22H) 3.37 (2H, m, C6H, C7H), 3.01 (3H, s), 2.59 (1H, ddd, *J* 20.0, 15.1, 5.0 Hz), 2.42 (1H, m), 2.05 - 1.89 (5H, m), 1.87 - 1.83 (1H, m), 1.66 (1H, td, *J* 14.5, 4.5 Hz), 1.53 (1H, ddd, *J* 13.6, 7.1, 3.6 Hz), 1.43 - 1.23 (6H, m), 1.21 (3H, m), 1.10 (3H, d, *J* 6.6 Hz, C23H), 1.08 (1H, td, J 12.2, 2.9 Hz) 0.77 (3H, s); ¹³C NMR (175 MHz, CDCl₃): 198.3, 163.0, 129.4, 74.8, 59.1, 55.7, 52.4, 51.6, 51.5, 43.5, 39.0, 37.2, 36.6, 36.3, 36.1, 35.5, 34.1, 27.5, 23.9, 21.2, 16.9, 16.8, 11.9.

### Example 44 - Epoxidation of (20R)-20-(1-cyanomethyl)-pregna-4,6-dien-3-one to form (20R)-cyanomethyl-6,7-β-epoxy-4-pregnen-3-one

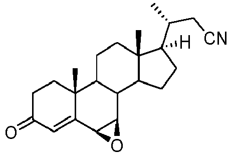

(20R)-20-(1-cyanomethyl)-pregna-4,6-dien-3-one (1.1 g, 3.2 mmol) was dissolved in formic acid (19.4 mL) and *tert*-butyl hypochlorite (383 µL, 3.39 mmol) added. The reaction mixture was stirred at 18 °C. After 20 min the mixture was quenched with sodium bisulfite (3 mL, 10% aq.) and diluted with EtOAc (100 mL), water (100 mL), and sodium chloride solution (50 mL, 5% aq.). The aq. phase was extracted with EtOAc (50 mL), and the combined organic phases washed with sodium bicarbonate (3 × 100 mL of a 5% aq.) and concentrated *in vacuo* to afford the intermediate which was used without further purification. The residue was suspended in ethanol (28 mL) and warmed to 40°C to dissolve. Potassium carbonate (885 mg, 6.4 mmol) was dissolved in water (7 mL) and added to the ethanolic solution. After 15 min at 18 °C TLC indicated complete consumption of the intermediate and the mixture was heated to reflux. After 40 min the reaction mixture was cooled to 18 °C temperature and neutralised with 4:1 ethanol:acetic acid. The mixture was concentrated to ∼7 mL to remove ethanol, then diluted with dichloromethane (80 mL) and sodium chloride solution (80 mL, 5% aq.). The aq. phase was re-extracted with CH₂Cl₂ (3 × 30 mL) and then the combined organic phases were washed with sodium chloride (30 mL, 5% aq.). The aq. phase was re-extracted with CH₂Cl₂ (30 mL) and the combined organic phases concentrated *in vacuo.* The residue was purified by flash chromatography on silica gel (heptane-EtOAc) to afford (20*R*)-cyanomethyl-6,7-β-epoxy-4-pregnen-3-one (0.55 g, 48%) as a colourless syrup, R_{f}: 0.29 (3:2, heptane:EtOAc); ¹H NMR (500 MHz, CDCl₃): δ = 6.17 (1H, d, *J* 0.7), 3.38 (1H, d, *J* 3.7), 3.37 (1H, d, *J* 3.7), 2.61 (1H, ddd, *J* 17.5, 14.9, 5.0), 2.46-2.41 (1H, m), 2.39 (1H, dd, *J* 16.7, 3.8), 2.30 (1H, dd, *J* 16.7, 6.9), 2.04-1.91 (4H, m), 1.88-1.83 (1H, m), 1.71-1.64 (2H, m), 1.56 (1H, dq, *J* 13.2, 3.5), 1.50-1.26 (6H, m), 1.23 (3H, s), 1.20 (3H, d, *J* 6.7), 1.10 (1H, td, *J* 6.0, 3.7), 0.78 (3H, s); ¹³C NMR (126 MHz, CDCl₃): δ = 198.2 (C=O), 162.8 (C=CH), 129.4 (C=CH), 118.7 (CN), 59.0, 55.7, 54.4, 52.6, 51.4, 43.5, 39.0, 36.6, 36.1, 35.5, 34.1, 33.4, 27.9, 24.8, 23.8, 21.2, 19.3, 16.8, 11.9.

### Example 45 - Epoxidation of (20S)-20-acetoxymethyl-pregna-4,6-dien-3-one to form (20S)-20-acetoxymethyl-6,7-β-epoxy-pregna-4-en-3-one

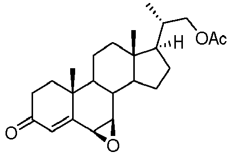

(20S)-20-acetoxymethyl-pregna-4,6-dien-3-one (5 g, 13.5 mmol) was dissolved in formic acid (90 mL) and *tert*-butyl hypochlorite (1.62 mL, 14.3 mmol) added. The reaction mixture was stirred at 18 °C. After 20 min the mixture was quenched with sodium bisulfite (15 mL, 10% aq.), diluted with EtOAc (250 mL), water (250 mL), and sodium chloride solution (50 mL, 5 % aq.). The aq. phase was re-extracted with EtOAc (50 mL) and the combined organic phases washed with sodium bicarbonate (4 × 250 mL, 5% aq.) and concentrated *in vacuo* to afford the intermediate which was used without further purification. The residue was dissolved in ethanol (116 mL) and a solution of potassium carbonate (3.7 g, 27 mmol) in water (29 mL) was added. After 15 min at 18 °C TLC indicated complete consumption of intermediate and the mixture was heated to reflux. After 50 min the mixture was allowed to cool to 18 °C and was neutralised with 4:1 EtOH : AcOH (to pH 7). The mixture was concentrated to -30 mL to remove ethanol, then diluted with CH₂Cl₂ (80 mL) and sodium chloride (80 mL of a 5% aq.). The aq. phase was re-extracted with CH₂Cl₂ (3 × 50 mL). The combined organic phases were concentrated *in vacuo* and purified by flash chromatography on silica gel (heptane-EtOAc) to afford (20*S*)-20-acetoxymethyl-6,7-β-epoxy-pregna-4-en-3-one (2.87 g, 54%) as a pale yellow syrup which solidified on standing, R_{f}: 0.41 (3:2, heptane:EtOAc); ¹H NMR (700 MHz, CDCl₃): δ = 6.15 (1H, d, *J* 0.6), 4.09 (1H, dd, *J* 10.7, 3.5), 3.80 (1H, dd, *J* 10.7, 7.4), 3.37 (2H, s), 2.59 (1H, ddd, *J* 17.5, 15.0, 5.0), 2.43-2.40 (1H, m), 2.06 (3H, s), 2.03 (1H, dt, *J* 12.9, 3.4), 1.98 (1H, t, *J* 11.6), 1.93-1.90 (3H, m), 1.80-1.74 (1H, m), 1.65 (1H, dd, *J* 7.0, 4.3), 1.52 (1H, dq, *J* 13.5, 3.6), 1.48-1.37 (3H, m), 1.34 (1H, qd, *J* 6.6, 3.8), 1.30-1.25 (1H, m), 1.23-1.19 (1H, m), 1.21 (3H, s), 1.08 (1H, td, *J* 6.0, 3.6), 1.03 (3H, d, *J* 6.7), 0.77 (3H, s); ¹³C NMR (176 MHz, CDCl₃): δ = 198.4, 171.3, 163.1, 129.3, 69.3, 59.2, 55.7, 52.5, 51.6, 43.5, 39.2, 36.6, 36.1, 35.7, 35.5, 34.1, 27.6, 23.9, 21.3, 21.0, 17.1, 16.8, 11.9.

### Example 46 - Epoxidation of (20S)-tert-butyldiphenylsilyloxymethyl-pregna-4,6-dien-3-one to form (6β, 7β, 20S)-6,7-epoxy-20-tert-butyldiphenylsiloxymethyl-pregna-4-en-3-one

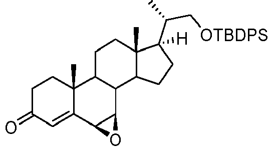

To a solution of (20*S*)-*tert*-butyldiphenylsilyloxymethyl-pregna-4,6-dien-3-one (230 mg, 0.41 mmol) in acetone : H₂O (12:1, 15 vol) was added TCCA (38mg, 0.4 eq) and the reaction stirred at 20°C. After 3 h the reaction mixture was filtered, diluted with DCM (25 mL) and washed with 10% w/v aq. NaHSO₃ (25mL) followed by H₂O (25mL). The organic phase was dried over Na₂SO₄ and concentrated *in vacuo.* The residue was taken up in EtOH (3.88 mL, 20 vol) at ambient temperature and K₂CO₃ (85mg, 2 eq) and H₂O (0.97 mL, 5 vol) added. The reaction mixture was heated to 80°C. After 2 h the reaction was cooled to ambient temperature and quenched with a mixture of 1:4 AcOH: EtOH (0.2 mL). The organic solvents were removed *in vacuo* and the residue taken up in DCM (25 mL). The organic phase was washed with 5% w/v aq. NaCl (25 mL) and the resulting aqueous phase extracted with DCM (2 x 10 mL). The combined organic phases were dried over Na₂SO₄ and concentrated *in vacuo.* Purification by column chromatography on silica gel gave (6β, 7β, 20*S*)-20-*tert*-butyldiphenylsilyloxymethyl-6,7-epoxy-pregna-4,6-dien-3-one (68 mg, yield 29%), R_{f} 0.67 (1:1, EtOAc: Heptane); ¹H NMR (500 MHz, CDCl₃): 7.72-7.68 (4H, m), 7.47-7.39 (6H, m), 6.17 (1H, s), 3.65 (1H, dd, J 9.8, 3.1), 3.45-3.40 (1H, m), 3.38 (2H, m), 2.72-2.70 (1H, m), 2.62 (1H, ddd, J 17.6, 15.0, 5.0), 2.48-2.42 (1H, m), 2.10-2.05 (1H, m), 2.01-1.92 (2H, m), 1.90-1.51 (6H, m), 1.42-1.19 (5H, m), 1.23 (3H, s), 1.13 (3H, d, J 6.6), 1.09 (9H, s), 0.75 (3H, s); ¹³C NMR (125 MHz, CDCl₃): 198.4, 163.3, 135.7, 135.6, 134.1, 134.0, 129.5, 129.3, 129.6, 129.5, 68.5, 59.3, 55.8, 52.6, 52.2, 51.7, 43.4, 39.3, 38.9, 36.7, 36.1, 35.6, 34.1, 27.5, 24.0, 21.3, 19.4, 17.2, 16.9, 11.9.

### Example 47 - Epoxidation of (20S)-azidomethyl-pregna-4,6-dien-3-one to form (6β, 7β, 20S)-6,7-epoxy-20-azidomethyl-pregna-4-en-3-one

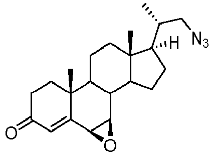

To a solution of (20S)-azidomethyl-pregna-4,6-dien-3-one (256 mg, 0.754 mmol) in formic acid (4.6 mL), *t*-butylhypochlorite (90 µL, 0.799 mmol) was added. The reaction mixture was stirred at 18 °C for 15 min, then quenched with aq. sodium bisulfite solution (10%, 0.84 mL). The mixture was diluted with CH₂Cl₂ (20 mL) and the organic phase washed with water (10 mL) and aq. sodium bicarbonate solution (5%, 10 mL). The organic phase was concentrated *in vacuo* to the intermediate as an orange viscous oil (392 mg, R_{f}: 0.48 (3:2, heptane:EtOAc), which was used without further purification. The residue was suspended in ethanol (8.6 mL) and a solution of potassium carbonate (219 mg, 1.58 mmol) in water (2.0 mL) was added. Reaction was stirred at 18 °C for 15 min, and then heated to reflux for 20 min. After cooling to 40 °C acetic acid (157 µL) was added. The mixture was concentrated *in vacuo,* and diluted with EtOAc (20 mL) and water (20 mL). The organic phase was separated and concentrated *in vacuo* to yellow solid (583 mg). The residue was purified by silica column chromatography on silica gel (heptane-EtOAc) to afford (6β, 7β, 20S)-6,7-epoxy-20-azidomethyl-pregna-4-en-3-one as a pale yellow oil (98 mg, 37% yield), R_{f}: 0.23 (4:1, heptane:EtOAc); ¹H NMR (700 MHz, CDCl₃): δ = 6.15 (1H, s, C4H), 3.40-3.35 (3H, m, C6H, C7H, C22*H*ₐH_{b}), 3.10 (1H, dd, *J* 11.9, 7.1, C22Hₐ*H*_{b}), 2.59 (1H, ddd, *J* 20.0, 15.0, 5.1, C2*H*ₐH_{b}), 2.44-2.39 (1H, m, C2*H*ₐH_{b}), 2.01 (1H, dt, *J* 13.0, 3.4), 1.94-1.89 (3H, m), 1.72-1.62 (3H, m), 1.52 (1H, dq, *J* 14.0, 3.8), 1.43-1.24 (5H, m), 1.23-1.18 (1H, obscured m), 1.21 (3H, obscured s, C18H), 1.10-1.15 (1H, obscured m), 1.06 (3H, d, *J* 6.7, C21H), 0.76 (3H, s, C19H). ¹³C NMR (176 MHz, CDCl₃): δ = 198.4, 163.0, 129.4, 59.2, 57.9, 55.7, 52.9, 52.6, 51.6, 43.5, 39.1, 36.8, 36.6, 36.1, 35.5, 34.1, 27.8, 23.9, 21.2, 17.7, 16.8, 11.9.

### Example 48 - Epoxidation of (20S)-(N-phthalimidomethyl)-pregna-4,6-dien-3-one to form (6β, 7β, 20S)-6,7-epoxy-20-(N-phthalimidomethyl)-pregna-4,6-dien-3-one

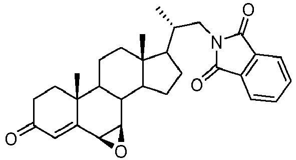

(20*S*)-(*N*-phthalimidomethyl)-pregna-4,6-dien-3-one (100 mg, 0.22 mmol) was dissolved in formic acid (1.8 mL) and *tert*-butyl hypochlorite (27 µL, 0.24 mmol) added. The reaction mixture was stirred at 23 °C. After 15 mins the mixture was quenched with 10% w/v aq. sodium bisulfite (0.33 mL) and diluted with DCM (10 mL). The organic phase was washed with water (2 x 10 mL), 5% w/v aq. sodium bicarbonate (10 mL) and 5% w/v aq. sodium chloride (10 mL) then concentrated *in vacuo* to afford the intermediate which was used without further purification. The residue was suspended in ethanol (2 mL), warmed to 50°C and DMF (2 mL) added. Potassium carbonate (51 mg, 0.37 mmol) dissolved in water (0.5 mL) was added and the mixture was heated to 90°C. After 2 h the reaction mixture was cooled to 23°C, diluted with water (10 mL) and extracted with TBME (2 x 20 mL). The combined organic phases were washed with 5% w/v aq. sodium chloride (10 mL) and concentrated. The residue was purified by chromatography on silica gel (ethyl acetate-heptane) to afford (6β, 7β, 20*S*)-6,7-epoxy-20-(*N*-phthalimidomethyl)-pregna-4,6-dien-3-one (19 mg, 21%) as a white solid, R_{f}: 0.57 (1:1, heptane:ethyl acetate); ¹H NMR (500 MHz, CDCl₃): δ = 7.85 (2H, m), 7.72 (2H, m), 6.16 (1H, s), 3.66 (1H, dd, J 13.3, 3.7), 3.44 (1H, dd, J 13.4, 10.2), 3.38 (2H, m), 2.59 (1H, m), 2.42 (1H, m), 2.85-1.87 (6H, m), 1.73-1.62 (2H, m), 1.54-1.62 (9H, m), 1.09 (1H, m), 0.91 (3H, d, J 6.7), 0.78 (3H, s); ¹³C NMR (126 MHz, CDCl₃): δ = 198.4, 168.8, 163.4, 113.9, 132.0, 129.4, 123.3, 59.3, 55.7, 54.4, 52.5, 51.6, 43.8, 43.6, 39.2, 36.6, 36.1, 36.1, 35.5, 34.1, 27.8, 24.1, 21.2, 17.0, 16.8, 11.9.

### Example 49 - Epoxidation of (20S)-20-(ethylenedioxymethyl)-pregna-4,6-dien-3-one to form (6β, 7β, 20S)-6,7-epoxy-20-formyl-pregna-4-en-3-one

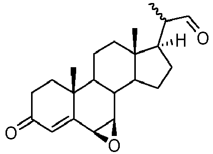

To a solution of (20S)-20-(ethylenedioxymethyl)-pregna-4,6-dien-3-one (168 mg) in formic acid (3 mL, 18 vol) under argon was added *t*BuOCl (54 uL, 1.06 eq) and the reaction mixture stirred at ambient temperature. After 30 min the reaction was quenched with aq. 10% NaHSO₃ (0.55 mL, 3.3 vol) and stirred for 10 min (-ve peroxide test). The reaction mixture was diluted with CH₂Cl₂ (100 mL), washed with aq. 5% NaHCO₃ (2 x 100 mL) and aq. 5% NaCl (100 mL). The organic phase was dried over Na₂SO₄, and concentrated *in vacuo* to give 1.76 g of wet residue. The crude material was taken up in EtOH (35 mL) at 18 °C and K₂CO₃ (912mg, 6.6 mmol) and DI H₂O (8.8 mL) added. After 30 min the reaction mixture was heated to 80 °C. After 2 hrs the reaction was cooled to 18 °C and quenched with a mixture of 1:4 AcOH: EtOH (1 mL). The organic solvents were removed in vacuo and the residue taken up in CH₂Cl₂ (50 mL). The organic phase was washed with aq 5% NaCl (50 mL) and the resulting aq. phase washed with CH₂Cl₂ (2 x 20 mL). The combined organic phases were dried over Na₂SO₄, and concentrated in vacuo. Purification by column chromatography on silica gel gave (6β, 7β, 20*S*)-6,7-epoxy-20-formyl-pregna-4-en-3-one (76 mg, 49%) as a 1:1 mixture of C21 epimers, R_{f} 0.52 (1:1, EtOAc: Heptane); ¹H NMR (700 MHz): 9.60 (0.5H, d, *J* 3.1), 9.54 (0.5Hz, d, *J* 5.1), 6.15 (1H, s), 3.37 (2H, m), 2.65-2.55 (1H, m), 2.45-2.35 (2H, m), 2.02-1.88 (4H, m), 1.70-1.25 (9H, m), 1.22 (1.5H, s), 1.20 (1.5H, s), 1.14 (1.5H, d, *J* 6.9), 1.13 - 1.08 (1H, m), 1.07 (1.5H, d, *J* 6.9), 0.79 (1.5H, s), 0.75 (1.5H, s); ¹³C NMR (175 MHz, CDCl₃): 205.4, 204.5, 198.4, 198.3, 162.9, 162.8, 129.4, 59.1, 59.0, 55.7, 55.6, 52.3, 52.1, 51.7, 51.6, 51.5, 50.7, 49.4, 48.7, 43.9, 43.1, 39.0, 38.0, 36.6, 36.1, 36.0, 35.5, 35.4, 34.1, 34.0, 31.9, 27.0, 26.3, 24.2, 23.5, 21.2, 21.0, 16.9, 16.8, 13.6, 13.4, 12.8, 12.2.

### Example 50 - Epoxidation of (20S)-20-(ethylenedioxymethyl)-pregna-4,6-dien-3-one to form (6β, 7β, 20S)-6,7-epoxy-20-(ethylenedioxymethyl)-pregna-4-en-3-one

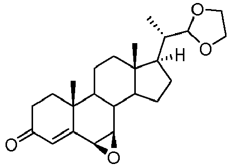

To a solution of (20S)-20-(ethylenedioxymethyl)-pregna-4,6-dien-3-one (168 mg) in formic acid (3 mL, 18 vol) under argon was added *t*BuOCl (54 uL, 1.06 eq) and the reaction mixture stirred at ambient temperature. After 30 min the reaction was quenched with aq. 10% NaHSO₃ (0.55 mL, 3.3 vol) and stirred for 10 min (-ve peroxide test). The reaction mixture was diluted with CH₂Cl₂ (100 mL), washed with aq. 5% NaHCO₃ (2 x 100 mL) and aq. 5% NaCl (100 mL). The organic phase was dried over Na₂SO₄ and concentrated *in vacuo.* 99 mg of the resulting crude material was dissolved in dry CH₂Cl₂ (1 mL, 10 vol) and 1,2-bis(trimethylsiloxy)ethane added (59 µL, 1 eqv). The reaction mixture was cooled to -78 °C and TMSOTf added (3 µL, 0.05 eqv). After 1 hour the reaction mixture was warmed to 18 °C and quenched with pyridine (50 µL, 1 vol). The mixture was taken up in CH₂Cl₂ and washed with water (2 x 20 mL) and aq. 5% NaCl (2 x 20 mL). The organic phase was dried over Na₂SO₄ and concentrated *in vacuo.* The residue was taken up in EtOH (1 mL) at ambient temperature and K₂CO₃ (17mg, 2 eqv) and DI H₂O (0.25 mL) added. After 30 min the reaction mixture was heated to 80°C. After 2 hrs the reaction was cooled to ambient temperature and quenched with a mixture of 1:4 AcOH: EtOH (0.1 mL). The organic solvents were removed *in vacuo* and the residue taken up in DCM (25 mL) and washed with aq 5% NaCl (20 mL). The aq. phase was extracted with DCM (2 x 10 mL), and the combined organic phases dried over Na₂SO₄ and concentrated *in vacuo.* Purification by column chromatography gave (6β, 7β, 20*S*)-6,7-epoxy-20-(ethylenedioxymethyl)-pregna-4-en-3-one (26 mg, 15%). R_{f} 0.47 (1:1, EtOAc:Heptane); ¹H NMR (700 MHz, CDCl₃): 6.15 (1H, s, C4H), 4.86 (1H, d, J = 2.0), 3.98-3.93 (2H, m), 3.88-3.83 (2H, m), 3.37 (2H, s, C6H, C7H), 2.59 (1H, ddd, J = 17.6, 15.0, 5.0), 2.41 (1H, m), 2.04-1.96 (2H, m), 1.93-1.88 (2H, m), 1.86-1.82 (1H, m), 1.66 (1H, td, J = 13.5, 4.6), 1.48-1.44 (3H, m), 1.40-1.36 (2H, m), 1.35-1.31 (1H, m), 1.28-1.22 (2H, m), 1.21 (3H, s), 1.10-1.05 (1H, td, J = 12.0, 3.8), 0.94 (3H, d, J = 6.7, C21H), 0.75 (3H, s); ¹³C NMR (175 MHz, CDCl₃): 198.4, 163.1, 129.3, 105.9, 65.3, 65.1, 59.3, 55.8, 52.2, 52.1, 51.6, 43.7, 39.2, 39.1, 36.6, 36.1, 35.6, 34.1, 27.2, 24.1, 21.3, 16.8, 11.7, 11.5.

### Example 51 - Epoxidation of 23-carboxy-3-oxo-4,6-choladien-24-oic acid dimethyl ester of (6β, 7β)-6,7-epoxy-3-oxo-4-cholen-23-carboxy-24-oic acid dimethyl ester

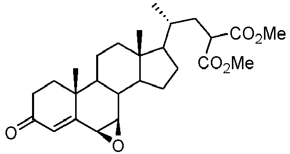

To a solution of 23-carboxy-3-oxo-4,6-choladien-24-oic acid dimethyl ester (1.28g) in formic acid (23 mL, 18 vol) under argon was added tBuOCI (348 uL, 1.06 eq) and the reaction mixture stirred at 18 °C. After 30 min the reaction mixture was quenched with aq. 10% NaHSO₃ (4.2 mL, 3.3 vol) and stirred for 10 min (-ve peroxide test). The reaction mixture was diluted with CH₂Cl₂ (100 mL), washed with aq. 5% NaHCO₃ (2 x 100 mL) and aq. 5% NaCl (100 mL). The organic phase was dried over Na₂SO₄ and concentrated *in vacuo* to give 1.76g of wet residue. The crude material was taken up in EtOH (35 mL, 20 vol) at ambient temperature and K₂CO₃ (912mg, 2 eq) and DI H₂O (8.8 mL, 5 vol) added. After 30 min the reaction mixture was heated to 80 °C. After 2 hrs the reaction was cooled to 18 °C and quenched with a mixture of 1:4 AcOH: EtOH (1 mL). The organic solvents were removed *in vacuo* and the residue taken up in CH₂Cl₂ (50 mL). The organic phase was washed with aq 5% NaCl (50 mL) and re-extracted with CH₂Cl₂ (2 x 20 mL). The combined organic phases were dried over Na₂SO₄, and concentrated *in vacuo.* Purification by column chromatography on silica gel gave (6α, 7β)-6-ethyl-7-hydroxy-3-oxo-4-cholen-23-carboxy-24-oic acid dimethyl ester (yield 49%), R_{f} 0.52 (1:1, EtOAc: Heptane); ¹H NMR (700 MHz): 4.22 (1H, t, *J* 7.1 Hz), 3.76 (3H, s, COOMe), 3.73 (3H, s, COOMe), 3.48 (1H, dd, *J* 10.9, 3.9 Hz), 2.59 (1H, ddd, 17.5, 15.0, 7.0 Hz), 2.41 (1H, m), 2.19 (1H, m), 2.03 - 1.95 (5H, m), 1.70 (1H, m), 1.64 (1H, td, *J* 14.4, 4.4 Hz), 1.53 - 1.25 (8H, m), 1.21 - 1.15 (5H, m), 1.06 (1H, td, *J* 12.0, 3.6 Hz), 0.94 (3H, d, *J* 6.4 Hz), 0.73 (3H, s); ¹³C NMR (175 MHz, CDCl₃):198.4, 170.6, 170.0, 163.2, 129.3, 59.2, 56.1, 55.7, 52.7, 52.6, 52.5, 51.6, 49.4, 43.5, 39.3, 36.6, 36.1, 35.5, 35.1, 34.3, 34.1, 28.0, 23.8, 21.3, 18.1, 16.8, 11.8.

### Examples 52-54 - Synthesis of (5β, 6α)-6-ethyl-3,7-dioxo-cholan-23-carboxy-24-oic acid dimethyl ester (precursor of OCA)

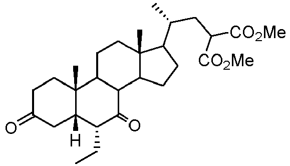

### Example 52 - Synthesis of (6α, 7β)-6-ethyl-7-hydroxy-3-oxo-4-cholen-23-carboxy-24-oic acid dimethyl ester

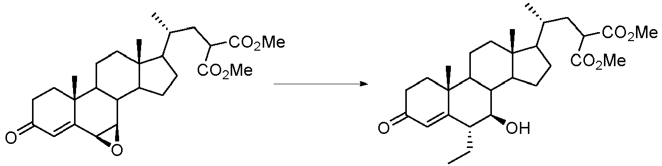

To a solution of ZnCl₂ (0.5M in THF, 2.1 mL, 0.6 eq) in THF (2.7 mL, 4 vol) under argon at -15 °C was added EtMgBr (1M in TBME, 2.1 mL, 1.8 eq) dropwise over 20 min. CuCI (12 mg, 0.05 eq) was added in one portion and the suspension stirred for 10 min. (6β, 7β)-6,7-Epoxy-3-oxo-4-cholen-23-carboxy-24-oic acid dimethyl ester (535 mg) dissolved in THF (2.2mL, 4 vol) was added dropwise over 30 min and the mixture stirred for 90 min. Saturated NH₄Cl (aq, 1.5 mL, 2.5 vol) was added dropwise and the mixture warmed to room temperature. The reaction mixture was diluted with EtOAc (25 mL) and washed with saturated NH₄Cl (aq, 2 x 20 mL) and water (2 x 20 mL). The organic phase was dried (Na₂SO₄), and concentrated *in vacuo.* Purification by column chromatography on silica gel afforded (6α, 7β)-6-ethyl-7-hydroxy-3-oxo-4-cholanen-23-carboxy-24-oic acid dimethyl ester as a white crystalline solid (49%), R_{f} 0.43 (1:1, EtOAc: Heptane); ¹H NMR (700 MHz): 5.83 (1H, s), 4.20 (1H, m), 3.75 (3H, s, COOMe), 3.72 (3H, s, COOMe), 3.48 (1H, dd, *J* 11.0, 3.9 Hz), 3.13 (1H, td, *J* 9.8, 4.4 Hz), 2.40-2.28 (3H, m), 2.21-2.15 (1H, m), 2.05-1.90 (5H, m), 1.83 (1H, m), 1.73 (1H, td, *J* 13.2, 4.8 Hz), 1.65-1.53 (4H, m), 1.50-1.33 (4H, m), 1.19 (3H, s), 1.16 (1H, m), 1.08 (1H, m), 1.01 (1H, td, *J* 12.2, 3.8 Hz), 0.94 (3H, d, *J* 6.5 Hz), 0.91 (3H, t, *J* 7.4Hz), 0.72 (3H, s); ¹³C NMR (175 MHz, CDCl₃): 199.8, 170.4, 170.0, 169.5, 122.6, 61.5, 61.3, 60.4, 55.5, 55.3, 52.6, 52.4, 49.7, 49.4, 48.2, 43.7, 43.0, 39.5, 38.5, 35.3, 35.1, 34.2, 33.4, 28.5, 26.8, 21.5, 18.8, 18.3, 12.1.

### Example 53 - Synthesis of (5β, 6α, 7β)-6-ethyl-7-hydroxy-3-oxo-cholan-23-carboxy-24-oic acid dimethyl ester

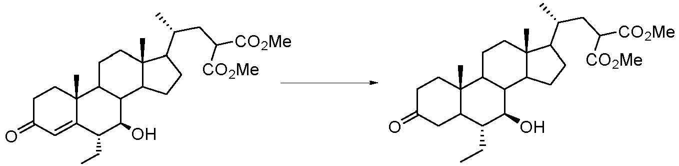

A solution of (6α, 7β)-6-ethyl-7-hydroxy-3-oxo-4-cholen-23-carboxy-24-oic acid dimethyl ester (250mg) in DMF (0.75 mL, 3 vol) and MeCN (1.5 mL, 6 vol) was evacuated and purged with argon 3 times then cooled to 0 °C. 10% Pd on C was added in one portion and the flask evacuated and filled with argon 3 times. The flask was evacuated and filled with hydrogen 3 times and stirred for 18 hrs under an atmosphere of hydrogen. The flask was evacuated and purged with argon 3 times and the suspension filtered through a PTFE HPLC filter cartridge and the cake washed with TBME (2 x 20 mL). The filtrate was washed with water (2 x 20 mL) and 5% NaCl (aq, 20 mL), dried over Na₂SO₄, and concentrated *in vacuo.* Purification by column chromatography on silica gel gave (5β, 6β, 7a)-6-ethyl-7-hydroxy-3-oxo-cholan-23-carboxy-24-oic acid dimethyl ester (70 mg, 28%), R_{f} 0.53 (1:1, EtOAc:Heptane), ¹H NMR (700 MHz): 4.20 (1H, m), 3.75 (3H, s, COOMe), 3.72 (3H, s, COOMe), 3.48 (1H, dd, *J* 11.0, 4.0 Hz), 3.23 (1H, td, *J* 9.9, 4.8 Hz). 2.33 - 2.23 (2H, m), 2.21 - 2.17 (3H, m), 2.12 - 2.03 (2H, m), 1.98 - 1.77 (4H, m), 1.65 (1H, m), 1.6 - 1.34 (9H, m), 1.24 (1H, m), 1.20 (1H, td, *J* 13.2, 4.2 Hz), 1.10 (1H, q, J 9.7 Hz), 1.04 (3H, s), 1.03 - 0.99 (1H, m), 0.94 (3H, d, J 6.5 Hz), 0.88 (3H, t, *J* 7.0 Hz), 0.70 (3H, s); ¹³C NMR (175 MHz, CDCl₃): 212.1, 170.4, 170.0, 75.0, 56.0, 55.5, 52.6, 52.4, 49.4, 45.1, 44.0, 43.7, 43.5, 40.1, 39.5, 37.7, 37.0, 36.6, 35.2, 34.8, 34.3, 28.5, 26.8, 22.6, 21.9, 20.7, 18.3, 12.2, 11.1.

### Example 54 - Synthesis of (5β, 6a)-6-ethyl-3,7-dioxo-cholan-23-carboxy-24-oic acid dimethyl ester (precursor of OCA)

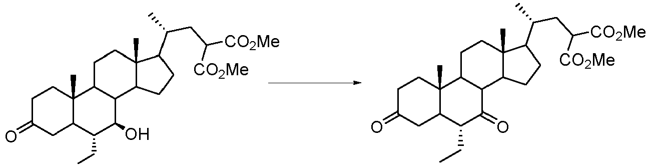

To a solution of (5β, 6α, 7β)-6-ethyl-7-hydroxy-3-oxo-cholan-23-carboxy-24-oic acid dimethyl ester (95 mg) in CH₂Cl₂ (2.4 mL, 25 vol) under argon was added Dess-Martin Periodinane (DMP, 98 mg, 1.2 eq). After 60 min a further portion of DMP (50mg, 0.6 eq) was added. After another 60 min a further portion of DMP (50mg, 0.6 eq) was added. After 30 min the mixture was partitioned between EtOAc (10 mL) and 10% Na₂S₂O₃/2% NaHCO₃ (95 mL) and stirred for 30 min. The aq. phase was extracted with EtOAc (10 mL) and the combined organic phases washed with 1M NaOH (aq, 2 x 10 mL). The organic phase was dried over Na₂SO₄ and concentrated *in vacuo.* Purification by column chromatography on silica gel gave (5β, 6a)-6-ethyl-3,7-dioxo-cholan-23-carboxy-24-oic acid dimethyl ester (67%) as a white crystalline solid, R_{f} 0.36 (1:1, EtOAc: Heptane);
¹H NMR (700 MHz, CDCl₃): δ= 3.75 (3H, s), 3.73 (3H, s), 3.47 (1H, dd, J = 11.0, 4.0), 2.74 (1H, dd, J = 11.0, 6.6), 2.47 (1H, t, J = 11.3), 2.29- 2.16 (5H,m), 2.09 -1.96 (3H, m), 1.89 - 1.80 (2H, m), 1.72 - 1.46 (6H, m), 1.39 - 1.34 (1H, m), 1.33 (3H, s), 1.32 -1.23 (2H, m), 1.21 - 1.13 (2H, m), 1.10 - 1.07 (1H, m), 0.99 - 0.95 (1H, m), 0.94 (3H, d, J = 6.5), 0.81 (3H, t, J = 7.4), 0.68 (3H, s); ¹³C NMR (176 MHz, CDCl₃): δ= 212.1, 210.5, 170.3, 170.0, 55.3, 52.6, 52.4, 52.3, 52.2, 49.9, 49.34, 48.8, 43.7, 42.7, 38.8, 38.3, 36.6, 35.9, 35.4, 35.1, 34.2, 28.2, 24.5, 22.9, 22.2, 18.6, 18.2, 12.1, 11.8.

### Examples 55 - 58 - Synthesis of OCA from (5β, 6α)-6-ethyl-3,7-dioxo-cholan-23-carboxy-24-oic acid dimethyl ester

### Example 55 - Synthesis of (3α, 5β, 6α)-6-ethyl-3-hydroxy-7-oxo-cholan-23-carboxy-24-oic acid dimethyl ester.

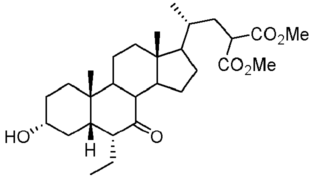

To a suspension of NaBH₄ (27 mg, 1 eq) in IPA (2.3 mL) at -20 °C was added a solution of (5β, 6a)-6-ethyl-3,7-dioxo-cholan-23-carboxy-24-oic acid dimethyl ester (350 mg) in EtOAc (2.3 mL, 6.5 vol) over 10 mins. After 30 mins 0.7M H₂SO₄ (2.5 mL) was added dropwise over 10 mins and the solution allowed to warm to 18 °C. The solution was diluted with EtOAc (50 mL) and the organic phase washed with water (3 x 50 mL) and 5% aq. NaCl (50 mL). The organic phase was dried over Na₂SO₄, filtrered and concentrated *in vacuo.* Purification by column chromatography gave (3α, 5β, 6a)-6-ethyl-3-hydroxy-7-oxo-cholan-23-carboxy-24-oic acid dimethyl ester (298 mg, 85%)
¹H NMR (700 MHz, CDCl₃): δ= 3.74 (3H, s), 3.72 (3H, s), 3.52 (1H, m), 3.47 (1H, dd, J = 11.0, 4.0), 2.69 (1H, dd, J = 12.8, 5.9), 2.34 (1H, t, J = 11.3), 2.21 - 2.16 (2H, m), 1.99 - 1.94 (2H, m), 1.85 - 1.68 (7H, m), 1.50 - 1.43 (4H, m), 1.37 - 1.23 (5H, m), 1.21 (3H, s), 1.20 - 1.10 (4H, m), 0.92 (3H, d, J = 6.5), 0.80 (3H, t, J = 7.4), 0.64 (3H, s); ¹³C NMR (176 MHz, CDCl₃): δ= 212.8, 170.4, 170.0, 71.1, 55.3, 52.6, 52.4, 52.0, 50.7, 49.9, 49.4, 49.0, 43.7, 42.7, 39.0, 35.7, 35.1, 34.3, 34.2, 31.8, 29.8, 28.3, 24.6, 23.5, 21.8, 18.8, 18.2, 12.0, 12.0.

### Example 56 - Synthesis of (3α, 5β, 6α, 7a)-6-ethyl-3,7-dihydroxy-cholan-23-carboxy-24-oic acid dimethyl ester.

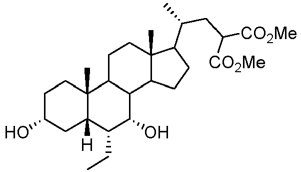

To a solution of (3α, 5β, 6a)-6-ethyl-3-hydroxy-7-oxo-cholan-23-carboxy-24-oic acid dimethyl ester (200 mg) in THF (20 mL, 100 vol) and water (5 mL, 25 vol) at 0 °C was added NaBH₄ (154 mg, 10 eq) in 3 portions. The solution was stirred for one h, allowing to warm to 18 °C. MeOH/water (10 mL, 1:1) was added dropwise and the organic solvent removed *in vacuo.* To the aqueous solution was added 2M aq. HCI (20 mL) dropwise. The aqueous solution was extracted with EtOAc (2 x 30 mL) and the combined organic phases washed with 5% aq.NaHCO₃ (30 mL) and water (30mL). The organic phase was dried over Na₂SO₄, filtered and concentrated *in vacuo.* Purification by column chromatography gave (3α, 5β, 6α, 7α)-6-ethyl-3,7-dihydroxy-cholan-23-carboxy-24-oic acid dimethyl ester (90 mg, 45%).
¹H NMR (700 MHz, CDCl₃): δ= 3.75 (3H, s), 3.72 (3H, s), 3.48 (1H, dd, J = 11.0, 4.0), 3.69 (1H, bs), 3.40 (1H, m), 2.18 (1H, m), 1.97 - 1.93 (2H, m), 1.85 - 1.75 (4H, m), 1.73 - 1.57 (4H, m), 1.51 - 1.11 (18H, m), 1.00 (1H, td, J = 14.3, 3.4), 0.93 (3H, d, J = 6.5), 0.90 (3H, t, J = 7.3), 0.64 (3H, s); ¹³C NMR (176 MHz, CDCl₃): δ= 170.5, 170.1, 72.3, 70.9, 56.3, 52.6, 52.4, 50.5, 49.4, 45.2, 42.8, 41.2, 40.0, 39.6, 35.6, 35.5, 35.2, 34.4, 34.0, 33.2, 30.6, 28.2, 23.7, 23.2, 22.2, 20.7, 18.2, 11.8, 11.7.

### Example 57-Synthesis of (3α, 5β, 6α, 7α)-6-ethyl-3,7-dihydroxy-cholan-23-carboxy-24-oic acid.

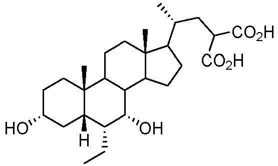

To a solution of (3α, 5β, 6α, 7a)-6-ethyl-3,7-dihydroxy-cholan-23-carboxy-24-oic acid dimethyl ester (70 mg) in IPA (2 mL, 28 vol) was added 0.5 M aqueous NaOH (2 mL, 28 vol) and the mixture stirred at 60 °C for 2 h. The organic solvent was removed *in vacuo* and the aqueous solution adjusted to pH1 with 2M aq. H₂SO₄. EtOAc (20 mL) was added and the mixture stirred for 5 mins. The aqueous phase was re-extracted with EtOAc (10 mL). The combined organic phases were washed with 5% aq.NaCl (2 x 10 mL), dried over Na₂SO₄, filtered and concentrated *in vacuo* to give (3α, 5β, 6α, 7a)-6-ethyl-3,7-dihydroxy-cholan-23-carboxy-24-oic acid as a white solid (54 mg, 81%).
¹H NMR (700 MHz, d-6 Acetone): δ= 3.58 (1H, bs), 3.32 (1H, dd, J = 11.1, 3.6), 3.18 (1H, m), 2.03 (1H, m), 1.90 - 1.62 (6H, m), 1.57 (1H, m), 1.48 - 1.31 (8H, m), 1.28 - 1.13(6H, m), 1.11 - 1.05 (3H, m), 0.98 (3H, m), 0.87 (3H, d, J = 6.1), 0.85 (1H, m) 0.79 (3H, s), 0.75 (3H, t, J = 7.3), 0.74 (3H, s); ¹³C NMR (176 MHz, d-6 Acetone): δ= 171.7, 171.3, 72.5, 70.4, 57.5, 51.4, 46.7, 43.4, 42.6, 41.3, 40.7, 36.7, 36.3, 36.2, 35.3, 34.6, 34.0, 31.5, 30.6, 29.0, 24.3, 23.7, 23.2, 21.6, 18.7, 12.3, 12.1.

### Example 58 - Synthesis of (3α, 5β, 6α, 7α)-6-ethyl-3,7-dihydroxy-cholan-24-oic acid (OCA).

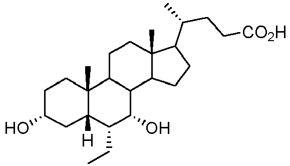

(3α, 5β, 6α, 7α)-6-ethyl-3,7-dihydroxy-cholan-23-carboxy-24-oic acid (25 mg) was taken up in xylene (1.25 mL, 50 vol) and pyridine (250 µL, 10 vol) and the solution heated to reflux for 90 mins. The cooled solution was diluted with EtOAc (20 mL) and washed with 1M aq. HCI (3 x 10 mL). The organic phase was washed with water (3 x 10 mL), 5% aq. NaCl (10 mL), dried over Na₂SO₄, filtered and concentrated. Purification by column chromatography gave (3α, 5β, 6α, 7a)-6-ethyl-3,7-dihydroxy-cholan-24-oic acid as a white solid (19 mg, 82%).
¹H and ¹³C NMR were consistent with an authentic sample of (3α, 5β, 6α, 7α)-6-ethyl-3,7-dihydroxy-cholan-24-oic acid.

## Claims

1. A compound of general formula (Ia): 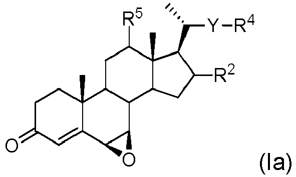 wherein:
R² is H, halo, OH or a protected OH group;
Y is a bond, or a C₁₋₂₀ alkylene, C₂₋₂₀ alkenylene or C₂₋₂₀ alkynylene linker group any of which is optionally substituted with one or more R³;
wherein each R³ is independently H, halo, OH, a protected OH group or NR⁸R⁹;
wherein each of R⁸ and R⁹ is independently H, C₁₋₆ alkyl, C(O)Ph, benzyl, phthalimide, *tert*-butyloxycarbonyl or carboxybenzyl;
R⁴ is C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azide, NO₂, NR¹⁰C(O)NR¹⁰SO₂R¹¹, NR¹⁰C(O)NR¹⁰SO₂N R¹⁰R¹¹, NR¹⁰SO₂R¹¹, C(O)NR¹⁰SO₂R¹¹, CH(XR¹⁰)(XR¹¹), CH(R¹⁰)(XR¹¹), phthalimide or a carboxylic acid mimetic group such as tetrazole;
wherein each X is independently O, S or NR⁸;
wherein each R¹⁰ and R¹¹ is independently:
a. hydrogen;
or
b. C₁₋₂₀ alkyl, C₃₋₇ cycloalkyl, C₂₋₂₀ alkenyl or C₂₋₂₀ alkynyl, any of which is optionally substituted with one or more substituents selected from:
C₁₋₄ alkyl, C₁₋₄ haloalkyl, halo, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ and a 6-to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
or
c. a 6- to 14- membered aryl, 5- to 14-membered heteroaryl group or 3- to 10-membered heterocyclic ring, any of which is optionally substituted with one or more substituents selected from:
C₁₋₆ alkyl, C₃₋₇ cycloalkyl, C₁₋₆ haloalkyl, halo, NO₂, CN, OR¹⁹, C(O)C₁₋₄alkyl, SR¹⁹, C(O)OR¹⁹, C(O)N(R¹⁹)₂, SO₂R¹⁹, SO₃R¹⁹, N(R¹⁹)₂, phenyl, 5- to 14-membered heteroaryl, 3- to 10-membered heterocyclic ring, methylenedioxy and ethylenedioxy;
or
d. a polyethylene glycol residue;
or
e. when R⁴ is C(O)NR¹⁰R¹¹, CH(XR¹⁰)(XR¹¹), NR¹⁰R¹¹, BR¹⁰R¹¹, CH[C(O)OR¹⁰]₂ or CH(BR¹⁰R¹¹)₂ an R¹⁰ and an R¹¹ group, together with the atom or atoms to which they are attached, may combine to form a 3- to 10-membered heterocyclic ring;
wherein each R¹⁹ is independently:
H, C₁₋₆ alkyl, C₁₋₆ haloalkyl, or a 6- to 14- membered aryl or 5- to 14-membered heteroaryl group either of which is optionally substituted with one or more substituents selected from halo, C₁₋₆ alkyl and C₁₋₆ haloalkyl;
and wherein each R¹³ is independently:
a. C₁₋₂₀ alkyl, C₂₋₂₀ alkenyl or C₂₋₂₀ alkynyl, any of which is optionally substituted with one or more substituents selected from:
halo, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SO₂R¹⁹, SO₃R¹⁹ and N(R¹⁹)₂;
or
b. a 6- to 14- membered aryl or 5- to 14-membered heteroaryl group either of which is optionally substituted with one or more substituents selected from:
C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
wherein each R¹⁹ is independently:
H, C₁₋₆ alkyl or C₁₋₆ haloalkyl; or
Y and R⁴ together form a =CH₂ group; and
R⁵ is H, OH or a protected OH group;
or a salt or isotopic variant thereof.

2. A compound according to claim 1, of general formula (I): 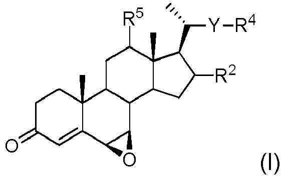 wherein:
R² is H, halo, OH or a protected OH group;
Y is a bond, or a C₁₋₂₀ alkylene, C₂₋₂₀ alkenylene or C₂₋₂₀ alkynylene linker group any of which is optionally substituted with one or more R³;
wherein each R³ is independently halo, OR⁸ or NR⁸R⁹;
wherein each of R⁸ and R⁹ is independently H or C₁₋₄ alkyl;
R⁴ is C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halo, CN, CH(OR¹⁰)(OR¹¹), CH(R¹⁰)(OR¹¹), CH(SR¹⁰)(SR¹¹), NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azide or a carboxylic acid mimetic group such as tetrazole;
wherein each R¹⁰ and R¹¹ is independently:
a. hydrogen;
or
b. C₁₋₂₀ alkyl, C₂₋₂₀ alkenyl or C₂₋₂₀ alkynyl, any of which is optionally substituted with one or more substituents selected from:
halo, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
or
c. a 6- to 14- membered aryl or 5- to 14-membered heteroaryl group either of which is optionally substituted with one or more substituents selected from: C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
or
d. a polyethylene glycol residue;
or
e. when R⁴ is C(O)NR¹⁰R¹¹, CH(OR¹⁰)(OR¹¹), CH(SR¹⁰)(SR¹¹), NR¹⁰R¹¹, BR¹⁰R¹¹, CH[C(O)OR¹⁰]₂ or CH(BR¹⁰R¹¹)₂ an R¹⁰ and an R¹¹ group, together with the atom or atoms to which they are attached, may combine to form a 3- to 10-membered heterocyclic ring;
wherein each R¹⁹ is independently:
H, C₁₋₆ alkyl, C₁₋₆ haloalkyl, or a 6- to 14- membered aryl or 5- to 14-membered heteroaryl group either of which is optionally substituted with one or more substituents selected from halo, C₁₋₆ alkyl and C₁₋₆ haloalkyl;
and wherein each R¹³ is independently:
a. C₁₋₂₀ alkyl, C₂₋₂₀ alkenyl or C₂₋₂₀ alkynyl, any of which is optionally substituted with one or more substituents selected from:
halo, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SO₂R¹⁹, SO₃R¹⁹ and N(R¹⁹)₂;
or
b. a 6- to 14- membered aryl or 5- to 14-membered heteroaryl group either of which is optionally substituted with one or more substituents selected from:
C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂;
wherein each R¹⁹ is independently:
H, C₁₋₆ alkyl or C₁₋₆ haloalkyl; or
Y and R⁴ together form a =CH₂ group; and
R⁵ is H, OH or a protected OH group;
or a salt or isotopic variant thereof.

3. A compound according to claim 1 or claim 2, wherein R² is H.

4. A compound according to any one of claims 1 to 3, wherein Y is a bond or a C₁₋₃ alkylene or C₂₋₃ alkenylene linker group, either of which is optionally substituted with one or two R³ groups, wherein R³ is as defined in claim 1 or claim 2; and is preferably -CH₂CH₂- or -CH=CH-.

5. A compound according to any one of claims 1 to 4, wherein R⁴ is C(O)OR¹⁰, OR¹⁰, C(O)NR¹⁰R¹¹, SO₃R¹⁰, or OSO₃R¹⁰; or
R⁴ is halo, CN, CH(XR¹⁰)(XR¹¹), CH=CH₂, -C≡CH, CH[C(O)OR¹⁰]₂, BR¹⁰R¹¹ or Y and R⁴ together form a =CH₂ group.

6. A compound according to any one of claims 1 to 4, wherein R⁴ is C(O)OR¹⁰, OC(O)R¹⁰, OR¹⁰, OSi(R¹³)₃, OSO₂R¹⁰, halo, CN, NR¹⁰R¹¹, CH[C(O)OR¹⁰]₂, azide, C(O)NR¹⁰SO₂R¹¹ CH(XR¹⁰)(XR¹¹); phthalimide, tetrazole or substituted tetrazole.

7. A compound according to any one of claims 1 to 6, wherein each R¹⁰ and R¹¹ is independently:
a. hydrogen; or
b. C₁₋₁₀ alkyl, C₂₋₁₀ alkenyl or C₂₋₁₀ alkynyl, any of which is optionally substituted with one or more substituents selected from:
C₁₋₄ alkyl, C₁₋₄ haloalkyl, halo, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ and a 6- to 14- membered aryl or 5- to 14-membered heteroaryl group, either of which is optionally substituted with one or more substituents selected from C₁₋₆ alkyl, C₁₋₆ haloalkyl, halo, NO₂, CN, OR¹⁹, SR¹⁹, C(O)OR¹⁹, C(O)N(R¹⁹)₂, SO₂R¹⁹, SO₃R¹⁹ and N(R¹⁹)₂; or
c. a 6- to 10- membered aryl or 5 to 10-membered heteroaryl group either of which is optionally substituted with one or more substituents selected from:
C₁₋₆ alkyl, C₃₋₇ cycloalkyl, C₁₋₆ haloalkyl, halo, NO₂, CN, OR¹⁹, C(O)C₁₋₄alkyl, SR¹⁹, C(O)OR¹⁹, C(O)N(R¹⁹)₂, SO₂R¹⁹, SO₃R¹⁹, N(R¹⁹)₂, phenyl, 5- to 14-membered heteroaryl, 3- to 10-membered heterocyclic ring, methylenedioxy and ethylenedioxy; or
d. a polyethylene glycol residue; or
e. when R⁴ is C(O)NR¹⁰R¹¹, CH(XR¹⁰)(XR¹¹), NR¹⁰R¹¹ or BR¹⁰R¹¹, an R¹⁰ and an R¹¹ group, together with the atom or atoms to which they are attached, may combine to form a 3- to 10-membered heterocylic ring.

8. A compound according to any one of claims 1 to 7, wherein R¹⁰ is hydrogen; or C₁₋₆ such as C₁₋₃ alkyl or C₂₋₆ such as C₂₋₃ alkenyl, either of which is optionally substituted with 6- to 14-membered aryl such as phenyl, especially C₁₋₆ alkyl or benzyl.

9. A compound according to any one of claims 1 to 8, wherein R⁵ is H.

10. A process for preparing a compound of general formula (Ia) according to any one of claims 1 to 9, comprising:
reacting a compound of general formula (IIa) with a halogenating agent followed by reaction with base: 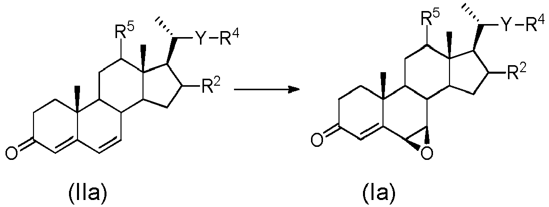 wherein Y, R², R⁴ and R⁵ are as defined in any one of claims 1 to 9.

11. A process according to claim 10, wherein the halogenating agent is Br₂, Cl₂, I₂, *N-*bromosuccinimide (NBS), *N*-chlorosuccinimide (NCS), *N*-iodosuccinimide (NIS), chloramine-T (tosylchloramide), *tert*-butylhypochlorite, trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), 1,3-diiodo-5,5-dimethylhydantoin (DIDMH), di-*tert*-butyl peroxide with TiCl₄; Ca(OCl)₂ with NaCl in AcOH; or TMSCI with H₂O₂.

12. A process according to claim 11, wherein the halogenating agent is trichloroisocyanuric acid (TCCA), tribromoisocyanuric acid (TBCA), triiodoisocyanuric acid (TICA), 1,3-dichloro-5,5-dimethylhydantoin (DCDMH), 1,3-dibromo-5,5-dimethylhydantoin (DBDMH), or 1,3-diiodo-5,5-dimethylhydantoin (DIDMH); or
wherein the halogenating agent is trichloroisocyanuric acid (TCCA) or *tert-*butylhypochlorite.

13. A process according to any one of claims 10 to 12, wherein the reaction is carried out in a solvent selected from acetone, DMF, MeCN or CH₂Cl₂, THF, *t*-butyl alcohol, acetic acid, dioxane, DMSO, formic acid and water, and mixtures thereof; and is preferably carried out in a solvent selected from acetone, water, formic acid, acetic acid and mixtures thereof.

14. A process for preparing a compound of general formula (Xa): 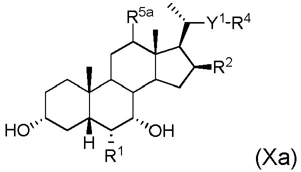 or a salt or an isotopic variant thereof,
wherein,
R¹ is C₁₋₄ alkyl, C₂₋₄ alkenyl or C₂₋₄ alkynyl optionally substituted with one or more substituents selected from halo, OR⁶ and NR⁶R⁷;
wherein each of R⁶ and R⁷ is independently H or C₁₋₄ alkyl;
R² is H, halo or OH;
R^{5a} is H or OH; and
Y¹ is a bond, or a C₁₋₂₀ alkylene linker group which is optionally substituted with one or more R³; or
Y¹ and R⁴ together form a =CH₂ group;
wherein R³ and R⁴ are as defined in any one of claims 1 to 9;
the process comprising the steps of:
(a) selective alkylation of a compound of general formula (Ia) with an organometallic regent to give a compound of general formula (IVa): 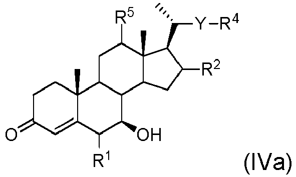
wherein R¹ is as defined for a compound of general formula (Xa); and
Y, R², R⁴ and R⁵ are as defined in claim 1;
(b) reducing the compound of general formula (IVa) using a suitable reducing agent to give a compound of general formula (Va): 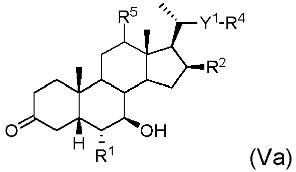
wherein Y¹ and R¹ are as defined for a compound of general formula (Xa); and
R², R⁴ and R⁵ are as defined in claim 1;
(c) oxidising the compound of general formula (Va) using a suitable oxidising agent to give a compound of general formula (VIa): 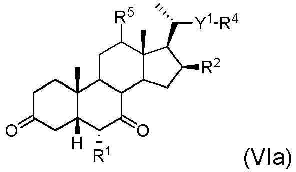
wherein Y¹ and R¹ are as defined for a compound of general formula (Xa);and
R², R⁴ and R⁵ are as defined in claim 1;
(d) reduction of the compound of general formula (VIa) using a suitable reducing agent and, where R² and/or R⁵ is a protected OH, removal of the protecting group(s), to give a compound of general formula (Xa) as defined above, wherein removal of the protecting group can take place before or after the reduction.

15. A compound of general formula (Ia) according to claim 1 selected from:
(6β, 7β, 22E)-6,7-epoxy-3-oxo-4,22-choladien-24-oic acid ethyl ester (Example 1);
(6β, 7β, 20S)-6,7-epoxy-3-oxo-4,22-choladien-24-oic acid ethyl ester (Example 5);
(6β, 7β, 20S)-6,7-epoxy-20-hydroxymethyl-pregna-4-en-3-one (Example 41);
(6β, 7β, 20S)-6,7-epoxy-20-bromomethyl-pregna-4-en-3-one (Example 42);
(20S)-methanesulfonyloxymethyl-6,7-β-epoxy-4-pregnen-3-one (Example 43);
(20R)-cyanomethyl-6,7-β-epoxy-4-pregnen-3-one (Example 44);
(20S)-20-acetoxymethyl-6,7-β-epoxy-pregna-4-en-3-one (Example 45);
(6β, 7β, 20S)-6,7-epoxy-20-*tert*-butyldiphenylsiloxymethyl-pregna-4-en-3-one (Example 46);
(6β, 7β, 20S)-6,7-epoxy-20-azidomethyl-pregna-4-en-3-one (Example 47);
(6β, 7β, 20S)-6,7-epoxy-20-(*N*-phthalimidomethyl)-pregna-4,6-dien-3-one (Example 48);
(6β, 7β, 20S)-6,7-epoxy-20-formyl-pregna-4-en-3-one (Example 49);
(6β, 7β, 20S)-6,7-epoxy-20-(ethylenedioxymethyl)-pregna-4-en-3-one (Example 50); and
(6β, 7β)-6,7-epoxy-3-oxo-4-cholen-23-carboxy-24-oic acid dimethyl ester (Example 51);
or a salt or isotopic variant thereof.

## Patentansprüche

1. Verbindung der allgemeinen Formel (Ia): 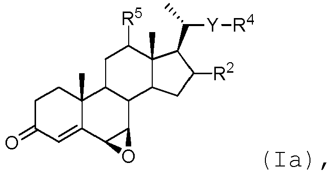 wobei:
R² für H, Halogen, OH oder eine geschützte OH-Gruppe steht,
Y für eine Bindung oder eine C₁₋₂₀-Alkylen-, C₂₋₂₀-Alkenylen- oder C₂₋₂₀-Alkinylen-Linkergruppe, jeweils gegebenenfalls substituiert durch ein oder mehrere R³, steht,
wobei R³ jeweils unabhängig für H, Halogen, OH, eine geschützte OH-Gruppe oder NR⁸R⁹ steht,
wobei R⁸ und R⁹ jeweils unabhängig für H, C₁₋₆-Alkyl, C(O)Ph, Benzyl, Phthalimid, tert.-Butyloxycarbonyl oder Carboxybenzyl stehen,
R⁴ für C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, Halogen, CN, NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, Azid, NO₂, NR¹⁰C(O)NR¹⁰SO₂R¹¹-, NR¹⁰C(O)NR¹⁰SO₂N R¹⁰R¹¹, NR¹⁰SO₂R¹¹, C(O)NR¹⁰SO₂R¹¹, CH(XR¹⁰) (XR¹¹), CH(R¹⁰)(XR¹¹), Phthalimid oder eine carbonsäuremimetische Gruppe wie Tetrazol steht,
wobei X jeweils unabhängig für O, S oder NR⁸ steht,
wobei R¹⁰ und R¹¹ jeweils unabhängig für:
a. Wasserstoff
oder
b. C₁₋₂₀-Alkyl, C₃₋₇-Cycloalkyl, C₂₋₂₀-Alkenyl oder C₂₋₂₀-Alkinyl, jeweils gegebenenfalls substituiert durch einen oder mehrere Substituenten ausgewählt aus:
C₁₋₄-Alkyl, C₁₋₄-Halogenalkyl, Halogen, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ und einer 6- bis 14-gliedrigen Aryl- oder 5- bis 14-gliedrigen Heteroarylgruppe, jeweils gegebenenfalls substituiert durch einen oder mehrere Substituenten ausgewählt aus C₁₋₆-Alkyl, C₁₋₆-Halogenalkyl, Halogen, NO₂, CN, OR¹⁹, SR¹⁹, C(O)OR¹⁹, C(O)N(R¹⁹)₂, SO₂R¹⁹, SO₃R¹⁹ und N(R¹⁹)₂,
oder
c. eine 6- bis 14-gliedrige Aryl- oder 5- bis 14-gliedrige Heteroarylgruppe oder einen 3- bis 10-gliedrigen heterocyclischen Ring, jeweils gegebenenfalls substituiert durch einen oder mehrere Substituenten ausgewählt aus:
C₁₋₆-Alkyl, C₃₋₇-Cycloalkyl, C₁₋₆-Halogenalkyl, Halogen, NO₂, CN, OR¹⁹, C(O)C₁₋₄-Alkyl, SR¹⁹, C(O)OR¹⁹, C(O)N(R¹⁹)₂, SO₂R¹⁹, SO₃R¹⁹, N(R¹⁹)₂, Phenyl, 5- bis 14-gliedriges Heteroaryl, einen 3- bis 10-gliedrigen heterocyclischen Ring, Methylendioxy und Ethylendioxy,
oder
d. einen Polyethylenglykolrest
stehen oder
e. wenn R⁴ für C(O)NR¹⁰R¹¹, CH(XR¹⁰) (XR¹¹), NR¹⁰R¹¹, BR¹⁰R¹¹, CH[C(O)OR¹⁰]₂ oder CH(BR¹⁰R¹¹)₂ steht, eine R¹⁰- und eine R¹¹-Gruppe zusammen mit dem Atom oder den Atomen, an das/die sie gebunden sind, einen 3- bis 10-gliedrigen heterocyclischen Ring bilden können,
wobei R¹⁹ jeweils unabhängig für:
H, C₁₋₆-Alkyl, C₁₋₆-Halogenalkyl oder eine 6- bis 14-gliedrige Aryl- oder 5- bis 14-gliedrige Heteroarylgruppe, jeweils gegebenenfalls substituiert durch einen oder mehrere Substituenten ausgewählt aus Halogen, C₁₋₆-Alkyl und C₁₋₆-Halogenalkyl, steht,
und wobei R¹³ jeweils unabhängig für:
a. C₁₋₂₀-Alkyl, C₂₋₂₀-Alkenyl oder C₂₋₂₀-Alkinyl, jeweils gegebenenfalls substituiert durch einen oder mehrere Substituenten ausgewählt aus:
Halogen, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ und einer 6- bis 14-gliedrigen Aryl- oder 5- bis 14-gliedrigen Heteroarylgruppe, jeweils gegebenenfalls substituiert durch einen oder mehrere Substituenten ausgewählt aus C₁₋₆-Alkyl, C₁₋₆-Halogenalkyl, Halogen, NO₂, CN, OR¹⁹, SO₂R¹⁹, SO₃R¹⁹ und N(R¹⁹)₂,
oder
b. eine 6- bis 14-gliedrige Aryl- oder 5- bis 14-gliedrige Heteroarylgruppe, jeweils gegebenenfalls substituiert durch einen oder mehrere Substituenten ausgewählt aus:
C₁₋₆-Alkyl, C₁₋₆-Halogenalkyl, Halogen, NO₂, CN, OR¹⁹, SR¹⁹, C(O)OR¹⁹, C(O)N(R¹⁹)₂, SO₂R¹⁹, SO₃R¹⁹ und N(R¹⁹)₂, steht,
wobei R¹⁹ jeweils unabhängig für:
H, C₁₋₆-Alkyl oder C₁₋₆-Halogenalkyl steht, oder
Y und R⁴ zusammen eine =CH₂-Gruppe bilden und
R⁵ für H, OH oder eine geschützte OH-Gruppe steht,
oder ein Salz oder eine isotopische Variante davon.

2. Verbindung nach Anspruch 1 mit der allgemeinen Formel (I): 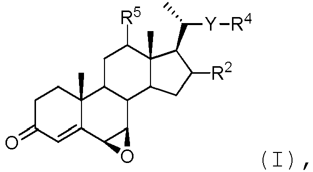 wobei:
R² für H, Halogen, OH oder eine geschützte OH-Gruppe steht,
Y für eine Bindung oder eine C₁₋₂₀-Alkylen-, C₂₋₂₀-Alkenylen- oder C₂₋₂₀-Alkinylen-Linkergruppe, jeweils gegebenenfalls substituiert durch ein oder mehrere R³, steht,
wobei R³ jeweils unabhängig für Halogen, OR⁸ oder NR⁸R⁹ steht,
wobei R⁸ und R⁹ jeweils unabhängig für H oder C₁₋₄-Alkyl stehen,
R⁴ für C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, Halogen, CN, CH(OR¹⁰)(OR¹¹), CH(R¹⁰)(OR¹¹), CH(SR¹⁰)(SR¹¹), NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, Azid oder eine carbonsäuremimetische Gruppe wie Tetrazol steht,
wobei R¹⁰ und R¹¹ jeweils unabhängig für:
a. Wasserstoff
oder
b. C₁₋₂₀-Alkyl, C₂₋₂₀-Alkenyl oder C₂₋₂₀-Alkinyl, jeweils gegebenenfalls substituiert durch einen oder mehrere Substituenten ausgewählt aus:
Halogen, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ und einer 6- bis 14-gliedrigen Aryl- oder 5- bis 14-gliedrigen Heteroarylgruppe, jeweils gegebenenfalls substituiert durch einen oder mehrere Substituenten ausgewählt aus C₁₋₆-Alkyl, C₁₋₆-Halogenalkyl, Halogen, NO₂, CN, OR¹⁹, SR¹⁹, C(O)OR¹⁹, C(O)N(R¹⁹)₂, SO₂R¹⁹, SO₃R¹⁹ und N(R¹⁹)₂,
oder
c. eine 6- bis 14-gliedrige Aryl- oder 5- bis 14-gliedrige Heteroarylgruppe, jeweils gegebenenfalls substituiert durch einen oder mehrere Substituenten ausgewählt aus:
C₁₋₆-Alkyl, C₁₋₆-Halogenalkyl, Halogen, NO₂, CN, OR¹⁹, SR¹⁹, C(O)OR¹⁹, C(O)N(R¹⁹)₂, SO₂R¹⁹, SO₃R¹⁹ und N(R¹⁹)₂,
oder
d. einen Polyethylenglykolrest
stehen oder
e. wenn R⁴ für C(O)NR¹⁰R¹¹, CH(OR¹⁰)(OR¹¹), CH(SR¹⁰)(SR¹¹), NR¹⁰R¹¹, BR¹⁰R¹¹, CH[C(O)OR¹⁰]₂ oder CH(BR¹⁰R¹¹)₂ steht, eine R¹⁰- und eine R¹¹-Gruppe zusammen mit dem Atom oder den Atomen, an das/die sie gebunden sind, einen 3- bis 10-gliedrigen heterocyclischen Ring bilden können,
wobei R¹⁹ jeweils unabhängig für:
H, C₁₋₆-Alkyl, C₁₋₆-Halogenalkyl oder eine 6- bis 14-gliedrige Aryl- oder 5- bis 14-gliedrige Heteroarylgruppe, jeweils gegebenenfalls substituiert durch einen oder mehrere Substituenten ausgewählt aus Halogen, C₁₋₆-Alkyl und C₁₋₆-Halogenalkyl, steht und wobei R¹³ jeweils unabhängig für:
a. C₁₋₂₀-Alkyl, C₂₋₂₀-Alkenyl oder C₂₋₂₀-Alkinyl, jeweils gegebenenfalls substituiert durch einen oder mehrere Substituenten ausgewählt aus:
Halogen, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ und einer 6- bis 14-gliedrigen Aryl- oder 5- bis 14-gliedrigen Heteroarylgruppe, jeweils gegebenenfalls substituiert durch einen oder mehrere Substituenten ausgewählt aus C₁₋₆-Alkyl, C₁₋₆-Halogenalkyl, Halogen, NO₂, CN, OR¹⁹, SO₂R¹⁹, SO₃R¹⁹ und N(R¹⁹)₂,
oder
b. eine 6- bis 14-gliedrige Aryl- oder 5- bis 14-gliedrige Heteroarylgruppe, jeweils gegebenenfalls substituiert durch einen oder mehrere Substituenten ausgewählt aus:
C₁₋₆-Alkyl, C₁₋₆-Halogenalkyl, Halogen, NO₂, CN, OR¹⁹, SR¹⁹, C(O)OR¹⁹, C(O)N(R¹⁹)₂, SO₂R¹⁹, SO₃R¹⁹ und N(R¹⁹)₂, steht, wobei R¹⁹ jeweils unabhängig für:
H, C₁₋₆-Alkyl oder C₁₋₆-Halogenalkyl steht, oder
Y und R⁴ zusammen eine =CH₂-Gruppe bilden und
R⁵ für H, OH oder eine geschützte OH-Gruppe steht,
oder ein Salz oder eine isotopische Variante davon.

3. Verbindung nach Anspruch 1 oder Anspruch 2, wobei R² für H steht.

4. Verbindung nach einem der Ansprüche 1 bis 3, wobei Y für eine Bindung oder eine C₁₋₃-Alkylen- oder C₂₋₃-Alkenylen-Linkergruppe steht, jeweils gegebenenfalls substituiert durch eine oder zwei R³-Gruppen, wobei R³ wie in Anspruch 1 oder Anspruch 2 definiert ist und vorzugsweise für -CH₂CH₂- oder -CH=CH- steht.

5. Verbindung nach einem der Ansprüche 1 bis 4, wobei R⁴ für C(O)OR¹⁰, OR¹⁰, C(O)NR¹⁰R¹¹, SO₃R¹⁰ oder OSO₃R¹⁰ steht oder
R⁴ für Halogen, CN, CH(XR¹⁰) (XR¹¹), CH=CH₂, -C≡CH, CH[C(O)OR¹⁰]₂, BR¹⁰R¹¹ steht oder Y und R⁴ zusammen eine =CH₂-Gruppe bilden.

6. Verbindung nach einem der Ansprüche 1 bis 4, wobei R⁴ für C(O)OR¹⁰, OC(O)R¹⁰, OR¹⁰, OSi(R¹³)₃, OSO₂R¹⁰, Halogen, CN, NR¹⁰R¹¹, CH[C(O)OR¹⁰]₂, Azid, C(O)NR¹⁰SO₂R¹¹, CH(XR¹⁰) (XR¹¹), Phthalimid, Tetrazol oder substituiertes Tetrazol steht.

7. Verbindung nach einem der Ansprüche 1 bis 6, wobei R¹⁰ und R¹¹ jeweils unabhängig für:
a. Wasserstoff oder
b. C₁₋₁₀-Alkyl, C₂₋₁₀-Alkenyl oder C₂₋₁₀-Alkinyl, jeweils gegebenenfalls substituiert durch einen oder mehrere Substituenten ausgewählt aus:
C₁₋₄-Alkyl, C₁₋₄-Halogenalkyl, Halogen, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ und einer 6- bis 14-gliedrigen Aryl- oder 5- bis 14-gliedrigen Heteroarylgruppe, jeweils gegebenenfalls substituiert durch einen oder mehrere Substituenten ausgewählt aus C₁₋₆-Alkyl, C₁₋₆-Halogenalkyl, Halogen, NO₂, CN, OR¹⁹, SR¹⁹, C(O)OR¹⁹, C(O)N(R¹⁹)₂, SO₂R¹⁹, SO₃R¹⁹ und N(R¹⁹)₂, oder
c. eine 6- bis 10-gliedrige Aryl- oder 5- bis 10-gliedrige Heteroarylgruppe, jeweils gegebenenfalls substituiert durch einen oder mehrere Substituenten ausgewählt aus:
C₁₋₆-Alkyl, C₃₋₇-Cycloalkyl, C₁₋₆-Halogenalkyl, Halogen, NO₂, CN, OR¹⁹, C(O)C₁₋₄-Alkyl, SR¹⁹, C(O)OR¹⁹, C(O)N(R¹⁹)₂, SO₂R¹⁹, SO₃R¹⁹, N(R¹⁹)₂, Phenyl, 5- bis 14-gliedriges Heteroaryl, einen 3- bis 10-gliedrigen heterocyclischen Ring, Methylendioxy und Ethylendioxy, oder
d. einen Polyethylenglylolrest stehen oder
e. wenn R⁴ für C(O)NR¹⁰R¹¹, CH(XR¹⁰) (XR¹¹), NR¹⁰R¹¹ oder BR¹⁰R¹¹ steht, eine R¹⁰- und eine R¹¹-Gruppe zusammen mit dem Atom oder den Atomen, an das/die sie gebunden sind, einen 3- bis 10-gliedrigen heterocyclischen Ring bilden können.

8. Verbindung nach einem der Ansprüche 1 bis 7, wobei R¹⁰ für Wasserstoff oder C₁₋₆ wie C₁₋₃-Alkyl oder C₂₋₆ wie C₂₋₃-Alkenyl, jeweils gegebenenfalls substituiert durch 6- bis 14-gliedriges Aryl wie Phenyl, insbesondere C₁₋₆-Alkyl oder Benzyl steht.

9. Verbindung nach einem der Ansprüche 1 bis 8, wobei R⁵ für H steht.

10. Verfahren zur Herstellung einer Verbindung der allgemeinen Formel (Ia) nach einem der Ansprüche 1 bis 9, bei dem man:
eine Verbindung der allgemeinen Formel (IIa) mit einem Halogenierungsmittel umsetzt, gefolgt von einer Umsetzung mit Base: 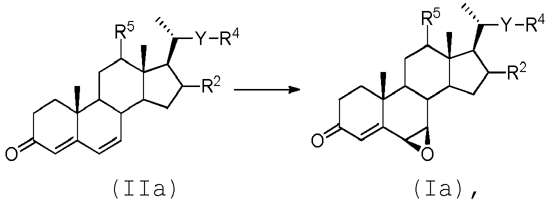 wobei Y, R², R⁴ und R⁵ wie in einem der Ansprüche 1 bis 9 definiert sind.

11. Verfahren nach Anspruch 10, wobei es sich bei dem Halogenierungsmittel um Br₂, Cl₂, I₂, N-Bromsuccinimid (NBS), N-Chlorsuccinimid (NCS), N-Iodsuccinimid (NIS), Chloramin-T (Tosylchloramid), tert.-Butylhypochlorit, Trichlorisocyanursäure (TCCA), Tribromisocyanursäure (TBCA), Triiodisocyanursäure (TICA), 1,3-Dichlor-5,5-dimethylhydantoin (DCDMH), 1,3-Dibrom-5,5-dimethylhydantoin (DBDMH), 1,3-Diiod-5,5-dimethylhydantoin (DIDMH), Di-tert.-butylperoxid mit TiCl₄; Ca(OCl)₂ mit NaCl in AcOH oder TMSCl mit H₂O₂ handelt.

12. Verfahren nach Anspruch 11, wobei es sich bei dem Halogenierungsmittel um Trichlorisocyanursäure (TCCA), Tribromisocyanursäure (TBCA), Triiodisocyanursäure (TICA), 1,3-Dichlor-5,5-dimethylhydantoin (DCDMH), 1,3-Dibrom-5,5-dimethylhydantoin (DBDMH) oder 1,3-Diiod-5,5-dimethylhydantoin (DIDMH) handelt oder
wobei es sich bei dem Halogenierungsmittel um Trichlorisocyanursäure (TCCA) oder tert.-Butylhypochlorit handelt.

13. Verfahren nach einem der Ansprüche 10 bis 12, wobei die Umsetzung in einem aus Aceton, DMF, MeCN oder CH₂Cl₂, THF, t-Butylalkohol, Essigsäure, Dioxan, DMSO, Ameisensäure und Wasser sowie Mischungen davon ausgewählten Lösungsmittel durchgeführt wird und vorzugsweise in einem aus Aceton, Wasser, Ameisensäure, Essigsäure und Mischungen davon ausgewählten Lösungsmittel durchgeführt wird.

14. Verfahren zur Herstellung einer Verbindung der allgemeinen Formel(Xa): 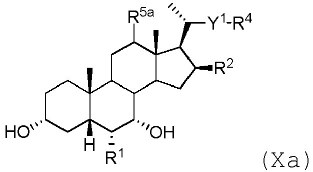 oder eines Salzes oder einer isotopischen Variante davon, wobei
R¹ für C₁₋₄-Alkyl, C₂₋₄-Alkenyl oder C₂₋₄-Alkinyl, gegebenenfalls substituiert durch einen oder mehrere Substituenten ausgewählt aus Halogen, OR⁶ und NR⁶R⁷, steht,
wobei R⁶ und R⁷ jeweils unabhängig für H oder C₁₋₄-Alkyl stehen,
R² für H, Halogen oder OH steht,
R^{5a} für H oder OH steht und
Y¹ für eine Bindung oder eine C₁₋₂₀-Alkylen-Linkergruppe, gegebenenfalls substituiert durch ein oder mehrere R³, steht oder
Y¹ und R⁴ zusammen eine =CH₂-Gruppe bilden,
wobei R³ und R⁴ wie in einem der Ansprüche 1 bis 9 definiert sind,
wobei das Verfahren die folgenden Schritte umfasst:
(a) die selektive Alkylierung einer Verbindung der allgemeinen Formel (Ia) mit einem metallorganischen Reagenz unter Erhalt einer Verbindung der allgemeinen Formel (IVa): 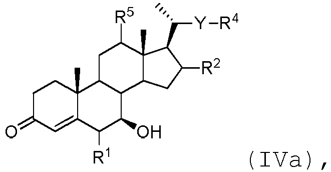
wobei R¹ wie für eine Verbindung der allgemeinen Formel (Xa) definiert ist und
Y, R², R⁴ und R⁵ wie in Anspruch 1 definiert sind,
(b) die Reduktion der Verbindung der allgemeinen Formel (IVa) mit einem geeigneten Reduktionsmittel unter Erhalt einer Verbindung der allgemeinen Formel (Va): 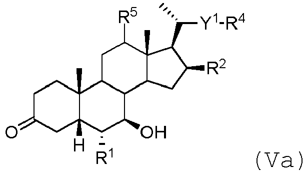
wobei Y¹ und R¹ wie für eine Verbindung der allgemeinen Formel (Xa) definiert sind und
R², R⁴ und R⁵ wie in Anspruch 1 definiert sind,
(c) die Oxidation der Verbindung der allgemeinen Formel (Va) mit einem geeigneten Oxidierungsmittel unter Erhalt einer Verbindung der allgemeinen Formel (VIa): 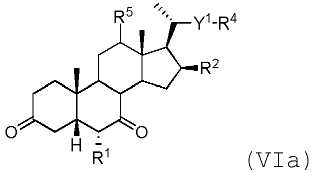
wobei Y¹ und R¹ wie für eine Verbindung der allgemeinen Formel (Xa) definiert sind und
R², R⁴ und R⁵ wie in Anspruch 1 definiert sind,
(d) die Reduktion der Verbindung der allgemeinen Formel (VIa) mit einem geeigneten Reduktionsmittel und, wenn R² und/oder R⁵ für geschütztes OH stehen/steht, das Entfernen der Schutzgruppe(n) unter Erhalt einer wie oben definierten Verbindung der allgemeinen Formel (Xa), wobei das Entfernen der Schutzgruppe vor oder nach der Reduktion stattfinden kann.

15. Verbindung der allgemeinen Formel (Ia) nach Anspruch 1, ausgewählt aus:
(6β, 7β, 22E)-6,7-Epoxy-3-oxo-4,22-choladien-24-säureethylester (Beispiel 1),
(6β, 7β, 20S)-6,7-Epoxy-3-oxo-4,22-choladien-24-säureethylester (Beispiel 5),
(6β, 7β, 20S)-6,7-Epoxy-20-hydroxymethylpregna-4-en-3-on (Beispiel 41),
(6β, 7β, 20S)-6,7-Epoxy-20-brommethylpregna-4-en-3-on (Beispiel 42),
(20S)-Methansulfonyloxymethyl-6,7-β-epoxy-4-pregnen-3-on (Beispiel 43),
(20R)-Cyanomethyl-6,7-β-epoxy-4-pregnen-3-on (Beispiel 44),
(20S)-20-Acetoxymethyl-6,7-β-epoxypregna-4-en-3-on (Beispiel 45),
(6β, 7β, 20S)-6,7-Epoxy-20-*tert*.-butyldiphenylsiloxymethylpregna-4-en-3-on (Beispiel 46), (6β, 7β, 20S)-6,7-Epoxy-20-azidomethylpregna-4-en-3-on (Beispiel 47),
(6β, 7β, 20S)-6,7-Epoxy-20-(N-phthalimidomethyl)pregna-4,6-dien-3-on (Beispiel 48),
(6β, 7β, 20S)-6,7-Epoxy-20-formylpregna-4-en-3-on (Beispiel 49),
(6β, 7β, 20S)-6,7-Epoxy-20-(ethylendioxymethyl)pregna-4-en-3-on (Beispiel 50) und
(6β, 7β)-6,7-Epoxy-3-oxo-4-cholen-23-carboxy-24-säuredimethylester (Beispiel 51)
oder ein Salz oder eine isotopische Variante davon.

## Revendications

1. Composé de formule générale (Ia) : 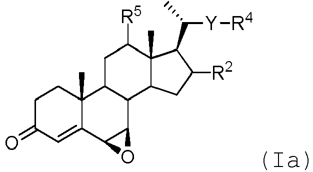
R² étant H, halogéno, OH ou un groupe OH protégé ;
Y étant une liaison, ou un groupe lieur C₁₋₂₀ alkylène, C₂₋₂₀ alcénylène ou C₂₋₂₀ alcynylène, l'un quelconque parmi lesquels étant éventuellement substitué par un ou plusieurs R³ ;
chaque R³ étant indépendamment H, halogéno, OH, un groupe OH protégé ou NR⁸R⁹ ;
chacun parmi R⁸ et R⁹ étant indépendamment H, C₁₋₆ alkyle, C(O)Ph, benzyle, phtalimide, *tert-*butyloxycarbonyle ou carboxybenzyle ;
R⁴ étant C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halogéno, CN, NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azoture, NO₂, NR¹⁰C(O)NR¹⁰SO₂R¹¹, NR¹⁰C(O)NR¹⁰SO₂NR¹⁰R¹¹, NR¹⁰SO₂R¹¹, C(O)NR¹⁰SO₂R¹¹, CH(XR¹⁰)(XR¹¹), CH(R¹⁰)(XR¹¹), phtalimide ou un groupe mimétique d'acide carboxylique tel que tétrazole ; chaque X étant indépendamment O, S ou NR⁸ ;
chaque R¹⁰ et R¹¹ étant indépendamment :
a. hydrogène ;
ou
b. C₁₋₂₀ alkyle, C₃₋₇ cycloalkyle, C₂₋₂₀ alcényle ou C₂₋₂₀ alcynyle, l'un quelconque parmi lesquels étant éventuellement substitué par un ou plusieurs substituants choisis parmi :
C₁₋₄ alkyle, C₁₋₄ halogénoalkyle, halogéno, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ et un groupe aryle à 6 à 14 chaînons ou hétéroaryle à 5 à 14 chaînons, l'un ou l'autre parmi lesquels étant éventuellement substitué par un ou plusieurs substituants choisis parmi C₁₋₆ alkyle, C₁₋₆ halogénoalkyle, halogéno, NO₂, CN, OR¹⁹, SR¹⁹, C(O)OR¹⁹, C(O)N(R¹⁹)₂, SO₂R¹⁹, SO₃R¹⁹ et N(R¹⁹)₂ ;
ou
c. un groupe aryle à 6 à 14 chaînons, hétéroaryle à 5 à 14 chaînons ou un cycle hétérocyclique à 3 à 10 chaînons, l'un quelconque parmi lesquels étant éventuellement substitué par un ou plusieurs substituants choisis parmi :
C₁₋₆ alkyle, C₃₋₇ cycloalkyle, C₁₋₆ halogénoalkyle, halogéno, NO₂, CN, OR¹⁹, C(O)C₁₋₄alkyle, SR¹⁹, C(O)OR¹⁹, C(O)N(R¹⁹)₂, SO₂R¹⁹, SO₃R¹⁹, N(R¹⁹)₂, phényle, hétéroaryle à 5 à 14 chaînons, cycle hétérocyclique à 3 à 10 chaînons, méthylènedioxy et éthylènedioxy ;
ou
d. un radical polyéthylèneglycol ;
ou
e. lorsque R⁴ est C(O)NR¹⁰R¹¹, CH(XR¹⁰)(XR¹¹), NR¹⁰R¹¹, BR¹⁰R¹¹, CH[C(O)OR¹⁰]₂ ou CH(BR¹⁰R¹¹)₂, un groupe R¹⁰ et un groupe R¹¹, conjointement avec l'atome ou les atomes au(x)quel(s) ils sont fixés, peuvent se combiner pour former un cycle hétérocyclique à 3 à 10 chaînons ;
chaque R¹⁹ étant indépendamment :
H, C₁₋₆ alkyle, C₁₋₆ halogénoalkyle, ou un groupe aryle à 6 à 14 chaînons ou hétéroaryle à 5 à 14 chaînons, l'un ou l'autre parmi lesquels étant éventuellement substitué par un ou plusieurs substituants choisis parmi halogéno, C₁₋₆ alkyle et C₁₋₆ halogénoalkyle ;
et chaque R¹³ étant indépendamment :
a. C₁₋₂₀ alkyle, C₂₋₂₀ alcényle ou C₂₋₂₀ alcynyle, l'un quelconque parmi lesquels étant éventuellement substitué par un ou plusieurs substituants choisis parmi :
halogéno, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ et un groupe aryle à 6 à 14 chaînons ou hétéroaryle à 5 à 14 chaînons, l'un ou l'autre parmi lesquels étant éventuellement substitué par un ou plusieurs substituants choisis parmi C₁₋₆ alkyle, C₁₋₆ halogénoalkyle, halogéno, NO₂, CN, OR¹⁹, SO₂R¹⁹, SO₃R¹⁹ et N(R¹⁹)₂ ;
ou
b. un groupe aryle à 6 à 14 chaînons ou hétéroaryle à 5 à 14 chaînons, l'un ou l'autre parmi lesquels étant éventuellement substitué par un ou plusieurs substituants choisis parmi :
C₁₋₆ alkyle, C₁₋₆ halogénoalkyle, halogéno, NO₂, CN, OR¹⁹, SR¹⁹, C(O)OR¹⁹, C(O)N(R¹⁹)₂, SO₂R¹⁹, SO₃R¹⁹ et N(R¹⁹)₂ ;
chaque R¹⁹ étant indépendamment :
H, C₁₋₆ alkyle ou C₁₋₆ halogénoalkyle ; ou
Y et R⁴ formant ensemble un groupe =CH₂ ; et
R⁵ étant H, OH ou un groupe OH protégé ;
ou sel ou variante isotopique correspondant(e).

2. Composé selon la revendication 1, de formule générale (I) : 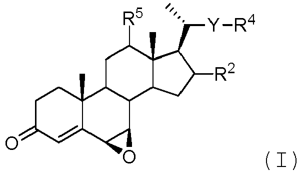
R² étant H, halogéno, OH ou un groupe OH protégé ;
Y étant une liaison, ou un groupe lieur C₁₋₂₀ alkylène, C₂₋₂₀ alcénylène ou C₂₋₂₀ alcynylène, l'un quelconque parmi lesquels étant éventuellement substitué par un ou plusieurs R³ ;
chaque R³ étant indépendamment halogéno, OR⁸ ou NR⁸R⁹ ;
chaque R⁸ et R⁹ étant indépendamment H ou C₁₋₄ alkyle ;
R⁴ étant C(O)OR¹⁰, OC(O)R¹⁰, C(O)NR¹⁰R¹¹, OR¹⁰, OSi(R¹³)₃, S(O)R¹⁰, SO₂R¹⁰, OSO₂R¹⁰, SO₃R¹⁰, OSO₃R¹⁰, halogéno, CN, CH(OR¹⁰)(OR¹¹), CH(R¹⁰)(OR¹¹), CH(SR¹⁰)(SR¹¹), NR¹⁰R¹¹, BR¹⁰R¹¹, C(O)CH₂N₂, -CH=CH₂, -C≡CH, CH[C(O)OR¹⁰]₂, CH(BR¹⁰R¹¹)₂, azoture ou un groupe mimétique d'acide carboxylique tel que tétrazole ;
chaque R¹⁰ et R¹¹ étant indépendamment :
a. hydrogène ;
ou
b. C₁₋₂₀ alkyle, C₂₋₂₀ alcényle ou C₂₋₂₀ alcynyle, l'un quelconque parmi lesquels étant éventuellement substitué par un ou plusieurs substituants choisis parmi :
halogéno, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ et un groupe aryle à 6 à 14 chaînons ou hétéroaryle à 5 à 14 chaînons, l'un ou l'autre parmi lesquels étant éventuellement substitué par un ou plusieurs substituants choisis parmi C₁₋₆ alkyle, C₁₋₆ halogénoalkyle, halogéno, NO₂, CN, OR¹⁹, SR¹⁹, C(O)OR¹⁹, C(O)N(R¹⁹)₂, SO₂R¹⁹, SO₃R¹⁹ et N(R¹⁹)₂ ;
ou
c. un groupe aryle à 6 à 14 chaînons ou hétéroaryle à 5 à 14 chaînons, l'un ou l'autre parmi lesquels étant éventuellement substitué par un ou plusieurs substituants choisis parmi :
C₁₋₆ alkyle, C₁₋₆ halogénoalkyle, halogéno, NO₂, CN, OR¹⁹, SR¹⁹, C(O)OR¹⁹, C(O)N(R¹⁹)₂, SO₂R¹⁹, SO₃R¹⁹ et N(R¹⁹)₂ ;
ou
d. un radical polyéthylèneglycol ;
ou
e. lorsque R⁴ est C(O)NR¹⁰R¹¹, CH(OR¹⁰)(OR¹¹), CH(SR¹⁰)(SR¹¹), NR¹⁰R¹¹, BR¹⁰R¹¹, CH[C(O)OR¹⁰]₂ ou CH(BR¹⁰R¹¹)₂, un groupe R¹⁰ et un groupe R¹¹, conjointement avec l'atome ou les atomes au(x)quel(s) ils sont fixés, peuvent se combiner pour former un cycle hétérocyclique à 3 à 10 chaînons ;
chaque R¹⁹ étant indépendamment :
H, C₁₋₆ alkyle, C₁₋₆ halogénoalkyle, ou un groupe aryle à 6 à 14 chaînons ou hétéroaryle à 5 à 14 chaînons, l'un ou l'autre parmi lesquels étant éventuellement substitué par un ou plusieurs substituants choisis parmi halogéno, C₁₋₆ alkyle et C₁₋₆ halogénoalkyle ;
et chaque R¹³ étant indépendamment :
a. C₁₋₂₀ alkyle, C₂₋₂₀ alcényle ou C₂₋₂₀ alcynyle, l'un quelconque parmi lesquels étant éventuellement substitué par un ou plusieurs substituants choisis parmi :
halogéno, NO₂, CN, OR¹⁹, SR¹⁹, C(O)OR¹⁹, C(O)N(R¹⁹)₂, SO₂R¹⁹, SO₃R¹⁹, OSO₃R¹⁹, N(R¹⁹)₂ et un groupe aryle à 6 à 14 chaînons ou hétéroaryle à 5 à 14 chaînons, l'un ou l'autre parmi lesquels étant éventuellement substitué par un ou plusieurs substituants choisis parmi C₁₋₆ alkyle, C₁₋₆ halogénoalkyle, halogéno, NO₂, CN, OR¹⁹, SO₂R¹⁹, SO₃R¹⁹ et N(R¹⁹)₂ ;
ou
b. un groupe aryle à 6 à 14 chaînons ou hétéroaryle à 5 à 14 chaînons, l'un ou l'autre parmi lesquels étant éventuellement substitué par un ou plusieurs substituants choisis parmi :
C₁₋₆ alkyle, C₁₋₆ halogénoalkyle, halogéno, NO₂, CN, OR¹⁹, SR¹⁹, C(O)OR¹⁹, C(O)N(R¹⁹)₂, SO₂R¹⁹, SO₃R¹⁹ et N(R¹⁹)₂ ;
chaque R¹⁹ étant indépendamment :
H, C₁₋₆ alkyle ou C₁₋₆ halogénoalkyle ; ou
Y et R⁴ formant ensemble un groupe =CH₂ ; et
R⁵ étant H, OH ou un groupe OH protégé ;
ou sel ou variante isotopique correspondant(e).

3. Composé selon la revendication 1 ou la revendication 2, R² étant H.

4. Composé selon l'une quelconque des revendications 1 à 3, Y étant une liaison ou un groupe lieur C₁₋₃ alkylène ou C₂₋₃ alcénylène, l'un ou l'autre parmi lesquels étant éventuellement substitué par un ou deux groupes R³, R³ étant tel que défini dans la revendication 1 ou la revendication 2 ; et étant préférablement -CH₂CH₂- ou -CH=CH-.

5. Composé selon l'une quelconque des revendications 1 à 4, R⁴ étant C(O)OR¹⁰, OR¹⁰, C(O)NR¹⁰R¹¹, SO₃R¹⁰, ou OSO₃R¹⁰ ; ou
R⁴ étant halogéno, CN, CH(XR¹⁰)(XR¹¹), CH=CH₂, -C≡CH, CH[C(O)OR¹⁰]₂, BR¹¹R¹¹ ou Y et R⁴ formant ensemble un groupe =CH₂.

6. Composé selon l'une quelconque des revendications 1 à 4, R⁴ étant C(O)OR¹⁰, OC(O)R¹⁰), OR¹⁰, OSi(R¹³)₃, OSO₂R¹⁰, halogéno, CN, NR¹⁰R¹¹, CH[C(O)OR¹⁰]₂, azoture, C(O)NR¹⁰SO₂R¹¹CH(XR¹⁰)(XR¹¹); phtalimide, tétrazole ou tétrazole substitué.

7. Composé selon l'une quelconque des revendications 1 à 6, chaque R¹⁰ et R¹¹ étant indépendamment :
a. hydrogène ; ou
b. C₁₋₁₀ alkyle, C₂₋₁₀ alcényle ou C₂₋₁₀ alcynyle, l'un quelconque parmi lesquels étant éventuellement substitué par un ou plusieurs substituants choisis parmi :
C₁₋₄ alkyle, C₁₋₄ halogénoalkyle, halogéno, NO₂, CN, OR¹⁹, SR¹⁹, C(O)OR¹⁹, C(O)N(R¹⁹)₂, SO₂R¹⁹, OSO₂R¹⁹, SO₃R¹⁹, OSO₃R¹⁹, N(R¹⁹)₂ et un groupe aryle à 6 à 14 chaînons ou hétéroaryle à 5 à 14 chaînons, l'un ou l'autre parmi lesquels étant éventuellement substitué par un ou plusieurs substituants choisis parmi C₁₋₆ alkyle, C₁₋₆ halogénoalkyle, halogéno, NO₂, CN, OR¹⁹, SR¹⁹, C(O)OR¹⁹, C(O)N(R¹⁹)₂, SO₂R¹⁹, SO₃R¹⁹ et N(R¹⁹)₂ ; ou
c. un groupe aryle à 6 à 10 chaînons ou hétéroaryle à 5 à 10 chaînons, l'un ou l'autre parmi lesquels étant éventuellement substitué par un ou plusieurs substituants choisis parmi :
C₁₋₆ alkyle, C₃₋₇ cycloalkyle, C₁₋₆ halogénoalkyle, halogéno, NO₂, CN, OR¹⁹, C(O)C₁₋₄alkyle, SR¹⁹, C(O)OR¹⁹, C(O)N(R¹⁹)₂, SO₂R¹⁹, SO₃R¹⁹, N(R¹⁹)₂, phényle, hétéroaryle à 5 à 14 chaînons, cycle hétérocyclique à 3 à 10 chaînons, méthylènedioxy et éthylènedioxy ; ou
d. un radical polyéthylèneglycol ; ou
e. lorsque R⁴ est C(O)NR¹⁰R¹¹, CH(XR¹⁰)(XR¹¹), NR¹⁰R¹¹ ou BR¹⁰R¹¹, un groupe R¹⁰ et un groupe R¹¹, conjointement avec l'atome ou les atomes au(x)quel(s) ils sont fixés, peuvent se combiner pour former un cycle hétérocyclique à 3 à 10 chaînons.

8. Composé selon l'une quelconque des revendications 1 à 7, R¹⁰ étant hydrogène ; ou C₁₋₆ alkyle tel que C₁₋₃ alkyle ou C₂₋₆ alcényle tel que C₂₋₃ alcényle, l'un ou l'autre parmi lesquels étant éventuellement substitué par aryle à 6 à 14 chaînons tel que phényle, notamment C₁₋₆ alkyle ou benzyle.

9. Composé selon l'une quelconque des revendications 1 à 8, R⁵ étant H.

10. Procédé pour la préparation d'un composé de formule générale (Ia) selon l'une quelconque des revendications 1 à 9, comprenant :
la mise en réaction d'un composé de formule générale (IIa) avec un agent d'halogénation suivie par une réaction avec une base : 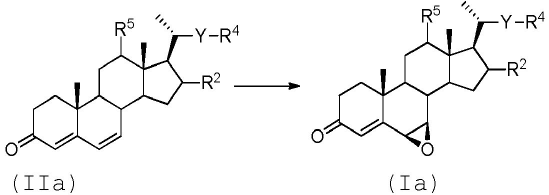 Y, R², R⁴ et R⁵ étant tels que définis dans l'une quelconque des revendications 1 à 9.

11. Procédé selon la revendication 10, l'agent d'halogénation étant Br₂, Cl₂, I₂, le N-bromosuccinimide (NBS), le N-chlorosuccinimide (NCS), le N-iodosuccinimide (NIS), la chloramine-T (tosylchloramide), l'hypochlorite de tert-butyle, l'acide trichloroisocyanurique (TCCA), l'acide tribromoisocyanurique (TBCA), l'acide triiodoisocyanurique (TICA), la 1,3-dichloro-5,5-diméthylhydantoïne (DCDMH), la 1,3-dibromo-5,5-diméthylhydantoïne (DBDMH), la 1,3-diiodo-5,5-diméthylhydantoïne (DIDMH), le peroxyde de di-tert-butyle avec TiCl₄ ; Ca(OCl)₂ avec NaCl dans AcOH ; ou TMSCl avec H₂O₂.

12. Procédé selon la revendication 11, l'agent d'halogénation étant l'acide trichloroisocyanurique (TCCA), l'acide tribromoisocyanurique (TBCA), l'acide triiodoisocyanurique (TICA), la 1,3-dichloro-5,5-diméthylhydantoïne (DCDMH), la 1,3-dibromo-5,5-diméthylhydantoïne (DBDMH), ou la 1,3-diiodo-5,5-diméthylhydantoïne (DIDMH) ; ou
l'agent d'halogénation étant l'acide trichloroisocyanurique (TCCA) ou l'hypochlorite de tert-butyle.

13. Procédé selon l'une quelconque des revendications 10 à 12, la réaction étant mise en œuvre dans un solvant choisi parmi l'acétone, le DMF, le MeCN ou le CH₂Cl₂, le THF, l'alcool t-butylique, l'acide acétique, le dioxanne, le DMSO, l'acide formique et l'eau, et des mélanges correspondants ; et étant préférablement mise en œuvre dans un solvant choisi parmi l'acétone, l'eau, l'acide formique, l'acide acétique et des mélanges correspondants.

14. Procédé pour la préparation d'un composé de formule générale (Xa) : 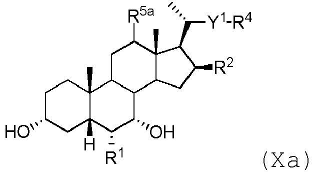 ou d'un sel ou d'une variante isotopique correspondant(e),
R¹ étant C₁₋₄ alkyle, C₂₋₄ alcényle ou C₂₋₄ alcynyle éventuellement substitués par un ou plusieurs substituants choisis parmi halogéno, OR⁶ et NR⁶R⁷ ;
chacun parmi R⁶ et R⁷ étant indépendamment H ou C₁₋₄ alkyle ;
R² étant H, halogéno ou OH ;
R^{5a} étant H ou OH ; et
Y¹ étant une liaison, ou un groupe lieur C₁₋₂₀ alkylène qui est éventuellement substitué par un ou plusieurs R³ ; ou
Y¹ et R⁴ formant ensemble un groupe =CH₂ ;
R³ et R⁴ étant tels que définis dans l'une quelconque des revendications 1 à 9 ;
le procédé comprenant les étapes de :
(a) alkylation sélective d'un composé de formule générale (Ia) avec un réactif organométallique pour donner un composé de formule générale (IVa) : 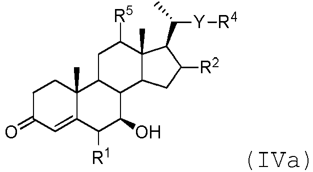
R¹ étant tel que défini pour un composé de formule générale (Xa) ; et
Y, R², R⁴ et R⁵ étant tels que définis dans la revendication 1 ;
(b) réduction du composé de formule générale (IVa) à l'aide d'un agent de réduction approprié pour donner un composé de formule générale (Va) : 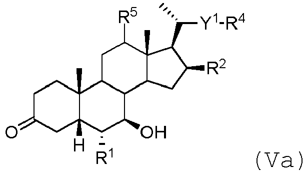
Y¹ et R¹ étant tels que définis pour un composé de formule générale (Xa) ; et
R², R⁴ et R⁵ étant tels que définis dans la revendication 1 ;
(c) oxydation du composé de formule générale (Va) à l'aide d'un agent d'oxydation approprié pour donner un composé de formule générale (VIa) : 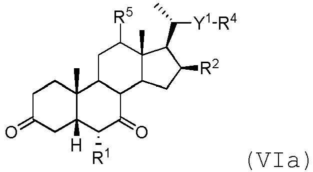
Y¹ et R¹ étant tels que définis pour un composé de formule générale (Xa) ; et
R², R⁴ et R⁵ étant tels que définis dans la revendication 1 ;
(d) réduction du composé de formule générale (VIa) à l'aide d'un agent de réduction approprié et, lorsque R² et/ou R⁵ est un OH protégé, élimination du ou des groupe(s) protecteur(s), pour donner un composé de formule générale (Xa) tel que défini ci-dessus, l'élimination du groupe protecteur pouvant avoir lieu avant ou après la réduction.

15. Composé de formule générale (Ia) selon la revendication 1 choisi parmi :
ester éthylique de l'acide (6β, 7β, 22E)-6,7-époxy-3-oxo-4,22-choladién-24-oïque (Exemple 1) ;
ester éthylique de l'acide (6β, 7β, 20S)-6,7-époxy-3-oxo-4,22-choladien-24-oïque (Exemple 5) ;
(6β, 7β, 20S)-6,7-époxy-20-hydroxyméthyl-pregna-4-én-3-one (Exemple 41) ;
(6β, 7β, 20S)-6,7-époxy-20-bromométhyl-pregna-4-én-3-one (Exemple 42) ;
(20S)-méthanesulfonyloxyméthyl-6,7-β-époxy-4-pregnén-3-one (Exemple 43) ;
(20R)-cyanométhyl-6,7-β-époxy-4-pregnén-3-one (Exemple 44) ;
(20S)-20-acétoxyméthyl-6,7-β-époxy-pregna-4-én-3-one (Exemple 45) ;
(6β, 7β, 20S)-6,7-époxy-20-tert-butyldiphénylsiloxyméthyl-pregna-4-én-3-one (Exemple 46) ;
(6β, 7β, 20S)-6,7-époxy-20-azidométhyl-pregna-4-én-3-one (Exemple 47) ;
(6β, 7β, 20S)-6,7-époxy-20-(N-phtalimidométhyl)-pregna-4,6-dién-3-one (Exemple 48) ;
(6β, 7β, 20S)-6,7-époxy-20-formyl-pregna-4-én-3-one (Exemple 49) ;
(6β, 7β, 20S)-6,7-époxy-20-(éthylènedioxyméthyl)-pregna-4-én-3-one (Exemple 50) ; et
ester diméthylique de l'acide (6β, 7β)-6,7-époxy-3-oxo-4-cholén-23-carboxy-24-oïque (Exemple 51) ;
ou sel ou variante isotopique correspondant(e).
